(19) Europäisches Patentamt
European Patent Office
Office européen des brevets

(11) EP 2 258 871 B1

# (12) EUROPEAN PATENT SPECIFICATION

(45) Date of publication and mention of the grant of the patent:
**14.05.2014 Bulletin 2014/20**

(51) Int Cl.:
***C12Q 1/68*** *(2006.01)*

(21) Application number: **10156846.7**

(22) Date of filing: **18.01.2008**

## (54) Methods and nucleic acids for analyses of cell proliferative disorders

Verfahren und Nukleinsäuren zur Analyse von zellproliferativen Krankheiten

Procédés et acides nucléiques pour les analyses de troubles prolifératifs

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **19.01.2007 EP 07100829**
**11.06.2007 EP 07110019**
**30.07.2007 EP 07113449**

(43) Date of publication of application:
**08.12.2010 Bulletin 2010/49**

(62) Document number(s) of the earlier application(s) in accordance with Art. 76 EPC:
**08701160.7 / 2 115 165**

(73) Proprietor: **Epigenomics AG**
**10178 Berlin (DE)**

(72) Inventors:
- **Liebenberg, Volker**
**14050 Berlin (DE)**
- **Distler, Jürgen**
**12163 Berlin (DE)**
- **Lewin, Jörn**
**10115 Berlin (DE)**
- **Model, Fabian**
**12683 Berlin (DE)**
- **Tetzner, Reimo**
**10439 Berlin (DE)**
- **Cortese, Rene**
**Toronto, ON M5R 2N9 (CA)**

(74) Representative: **Zwicker, Jörk et al**
**ZSP Patentanwälte**
**Partnerschaftsgesellschaft**
**Radlkoferstrasse 2**
**81373 München (DE)**

(56) References cited:
**WO-A-2006/008128 WO-A1-98/14568**
**WO-A2-2006/060653**

- **DATABASE DBsnp [Online] NCBI; 1 June 2004 (2004-06-01), XP002605423 retrieved from http: //www.ncbi.nlm.nih.gov/SNP/snp_ref.cg i? searchType=adhoc_search&type=rs&rs=13074 768 Database accession no. rs13074768**
- **DATABASE DBsnp [Online] NCBI; 8 August 2002 (2002-08-08), XP002605424 retrieved from http: //www.ncbi.nlm.nih.gov/SNP/snp_ref.cg i? searchType=adhoc_search&type=rs&rs=32231 19 Database accession no. rs3223119**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 258 871 B1

Sequence listing

<110> Epigenomics AG

<120> METHODS AND NUCLEIC ACIDS FOR ANALYSES OF CELL PROLIFERATIVE DISORDERS.

<160> 118

<210> 1
<211> 3905
<212> DNA
<213> Homo Sapiens

<400> 1

ttcaaaaaag cagatccgaa gatgtttaaa gtaataatgg tgctgttaga atggtgctcc 60
ctctccctcc caggggatga ctttgaagga cactacaata acagatgtgt aaattctgaa 120
atatatggct aaaatgaggt ttattatttt ctagtcacca cttagacatc ttttagtcat 180
ttgtttatga cagtttataa atgagaaaga agtccattta aaatatctcc atgaggacct 240
caaggtaaat aataggaatc gatcattcat caatctggcc tggccccggc ccactgaaga 300
aagttccttc ttgagtgcat tcaaagctct catggtccag ccctgttcaa cctgagcagc 360
atcctatctg ctcctccccc agacgttccg atgccctgca ccagattcaa gagagagcca 420
agttccccaa caggctatgc acgtggctat taacacccag cacacgctat ccctggccag 480
ctctctcctt cttccctaag aattaaacat tctttggcct gagttttcct cctttatgtc 540
accataaagt tgagttattc attattaggg tctatacatg agtagccaaa atatacgggt 600
ctctatattt aggtcaaggg gccttcagca ggagaacatg aataattagg agtgaggcca 660
tgagacagag gaagaaaagc tcataggaat gggaagacag cctctcagat gagaggtgtg 720
cggagtgatt gctgtgagga attgctcaat aggaaagtta gccaacctga ggacgtttct 780
aggggaggaa aagggaaact catatggttc tctgctaatt cttaaaaata aggtagattc 840
ttactcatct agggtgcttg ctcgttatga acacaggaag attaaggaat gagtatggac 900
tgtttctact ctatgttttc ggacaagaag aataataaaa aagcactggg tagctggaac 960
aactgcagag gccccaacaa tgatggagac tttctggtgc accgtggcac aggggtctct 1020
tccaaaggga ctcaatcaac cccaacaggc atcatcttct gtgactcaag atgtcctttt 1080
taggagtgct tgtacttttt aatgctcaga gaagttcgta ttactgattc gggaacactg 1140
agtttttcag ctcctgtaaa actattttca ggtttatttt caagtacatt ctttacctcg 1200
gggggtcaaa tcttttggtt tccagtgaat atcgtggtta tacctgtggt tacccactga 1260
cttctcatct gcctagctta tctgataaca ggcaaatggg gaaagaggct ttaatacccc 1320
tcaaaagggg aagattgata attagttatt caccgcatag aatcaggcca ggtcaactct 1380
ctccaagaag aaaagcaatc tggatattgc actttggggt ccctttttcat tttggcatta 1440
caggcctcat gaggatgtag tctttctgta tttctttctt tctttttaaa ttgttattta 1500
tttatttatt ttaaccaggt caaagcatta catactgcag aagagaaagc aacttcagct 1560
tctcttaaac ggtgtcattt tcatgacagc tcgtaaattt tggcctccta tgggtccagg 1620
atctgcgcgc atccgcccgg ggacttgttg gtggatccat ccctcatggc ggagcagcaa 1680
ggggatcctt tagaaaaagc aatgggcgaa gtaactgaaa gagcgacgca gaaagcaaca 1740
gccagaaacg gcggggacgc gagcggccca gacaggaagg gaggcggtgg cgcagctctg 1800
gtgcgcagcg cgccgcagcg acggaacttc tgcaaaagct gcctgcccgc gcgttatcag 1860
cggcgcgcag gcctgtggtt ttctcgctct cgcaaccctg ctttaactgc cggtttattt 1920
ttcgacaaac aggatgcctc catctgaggc tgtcagcatc ccaggctctt tgaaagaaaa 1980
gaagtagagc ggccccaccc tagaggcaag gacggggtct gtgtcaagag gcttcccaga 2040
gaagtgaaaa ctctgcaggt gcagccgctg ggagagcatc aagaagggca gggtggaggg 2100
gcagggggcg aagggagggg gtgaagcccg caccctaccc ccacatgaaa ctgattccac 2160
taccccatct ctgcaagcgt ccagaggcag agaggccaac atttcgggga cagcttggag 2220
gcgggagatt taggcagggc tccttaaact tttatgtgca tgaaaatcag gccaatcacg 2280
gggctcttga gcaaatgggg acgatgattc agcaggtctg ggctgaggcc tcagattctg 2340
cacttctaac aagttcccag gtggtagtga tgctgccagt ccaaagacca cactgtgaat 2400
agcaaggcgt cgtcgggaca gtctgcttta gagtaaaacc taatagatgt gcaaatattt 2460
aaaccaagct gtatttattc ctgaggccct ggtttaactg gagccacctt gctaatggaa 2520
aatggaaaat gaaattcctt taggttccaa agggaaggaa agtttgaaat ggttaaggta 2580
tttaaaaaat cctagttggg agtggccttg gggattcggg agagttgaag ttaggttctg 2640
agccaagaga tgggggagga agggtgggat gtggcaaata aatataaaca cacacacaca 2700
aaccccagcc caaccacagg ttctttccta tcaatgaaac atttgcatct tgcaggtaga 2760
actcacacgt gtccacattt ttcagaagtt gaatggtttt gtgtaatggt acagaatttg 2820

```
gagcccatat ttgaatattc tagatccaat gttatctcag actggcttta ggatcttgtg     2880
gcctacattt aaaaatgcat gtttctgagt catgtttaat tagtagtagt atctgagaac     2940
tgagagacta gactaagaaa tccatggtat atgaataaaa tctccagaca gcagctagaa     3000
catagaggtc cctccagagg cagtgccagt tgtcagctgt aaaaataggt tctgctcagt     3060
tttgcttagg cctgaagttt cctgaagatt tcatttagac ttcatcaaaa ccagtcaaag     3120
acttcctgtt acagaaaggt tcttaaagta tggctgggac tacagggcta ttaagatgca     3180
gattcttggg cctctctgcc tcactcatca tggtttctca gggtgggtgc cgggaacctg     3240
cattttaaca aacgctttga ggattcttat gtactgccaa gtttaggagt ccctggtctg     3300
caggtttaag ttcaaccttc tcactgcagc attcaaagac ctggcttcaa ttttgctgtt     3360
cacaggcgag cctcagtgac ttcctcactg ccctctgagc actcctggag ccacctggct     3420
gcctcattca tttcttctcc tctgtctttc cccagccctg gaataaaatg acttccatct     3480
tcctctccga actctcttca gatcatggct tcccactcag caagcagtta ctttcctggt     3540
ctgccactgc tgtggtatta atgataggcc actcgattct cattttgttg actgaatatt     3600
tctcatgctt aggctttgcc tccctgacca gacagtaagt tctctgaagc catttcatgc     3660
tatataatct ttatgttcaa gactactttc taacgcagtg tcatgtccac atggatcatt     3720
cagtaaattt accgatggct tatctatcac caacatgttt tacagagtgt tattaagcag     3780
ggacactctc tatgggtcta ggatcagaag aagctgggaa gagaaatggg gtagtggtga     3840
gagtgtcatg agaatctggt tatacagtca tcaaagacac agaggaagtg ggaaaaaggg     3900
agtgt                                                                 3905
```

<210> 2
<211> 5338
<212> DNA
<213> Homo Sapiens

<400> 2

```
ggcaacctct tttgccatca ctttttgcat aaaatatgca agatgcttcc attccatcag      60
cactgaaacc aaagaccaaa agagagaaag gtgttggacc gaaaaggagg cggcgcaacg     120
gccacccctt ccagcacctc ggctttgtcc ttcccgggga aggcggccac atccctaccc     180
gccttgctcc tgaacgtagt aaacaatctc acaaacaacc accgctgccc acgctctcca     240
tccgtcctcc cggccttatc agacctccgt tctcccgcac tcttcgggca gggtccctaa     300
taagctcagg ctgaaagaac gtttgccacc tcccccaccc tcgttgaaag aaaaggaaga     360
aaaacagcag cagcgagaaa cctccggggc gactcctccc ccgcccccaa gcaccagcgc     420
acagcatccc cctctgtctt tgttgtggtt ctccgttgct tcgggccacg ccgttcagcc     480
aagcaacccg gacctgagag tgcacagcca ggactagctt aggggggcgag gggttggtct     540
ttgggaaacc aagcgctcag gacagaggtg gaaagtgggt cccgggagcc agaaaagaga     600
gagaagggca gacggctggg tggcaaatac aaaaatagaa ataatttagg gggatgcccg     660
ccaggctttt gcgcctgctc cttctccccc aattcggagc aggttccctt cggcctcccg     720
cgcccccgggg cgccccctgg cggcagcggc agcagcgggc aacgcgcgga gggctcaggg     780
ggcgcacagg ggactcccgg gcacactcag agaggcgggc gcggccccct ggcggtggcg     840
acgtagttat ctggtgagcg gagcctcgtc cctctggtcc ggcgggctca cggccgtctt     900
actaagcacc gcggccgagt agggcaggaa gccgctctcg gaacgcggcg ccgccgcgct     960
gcagccgaag tccgagcctc ccccggcccc ggcgccccccg ccgccgccgc caccaccacc    1020
accgccgccg ccaccgccac cccgggaacc cagggctgcg gcagctgctg ccgcggctgc    1080
cgccgccgac tgactgctgt ggcaactgag gcacgagcag ggtgcagagc cgccgctggg    1140
gggcgcgccg gccgccgccg ccgaggaggc cgcagccgct gcggctgccg cggctgccgc    1200
ggcagaggcc gcgctgttga gccccgcggc ggccgcggga gcctggtaga gaccagggtg    1260
gcggaagcta cacagcagct ccggccgaga gtaggggtgc gagagggcgc ggaaggtgtc    1320
cagtggccgg atggaagtag cgaagggcga cgaagccgcg gccgccgcgc ctgaggctgc    1380
agccgcggcc gccgccgccg tgacgcccac gtgcgggtag tagtgcagcg gcacgtgcga    1440
gtggaagggg tagggcaggc ttccggtggc ggccgcgtgc gtcatcatgt aggtgtagaa    1500
gctggggtcg gctgggtgcg gccaggacat ggccaggcgc tgccgcttgt ccttcatgcg    1560
ccggttctgg aaccacacct gcggggagag acgcgccgca gcctgggtta gggagcgccc    1620
cgtgttccca gctcctgtcc caggacctct gccccttccg gacctctgaa tggcttggtc    1680
tacttctctc cgaccaagcc caaccccgag taccctgtgg tctcccagct gggaaagtgt    1740
ggacggcagt gtgtggaccg ccgtgggcac accgtcctca acgaagaggg tcctctcccc    1800
cgcgtccggc tgctgctgct cctcaggctt ttattccctt acttcttgct gcactttttt    1860
gtcccaatcc aacctttcct ctccctcccg ccacccacca gtgccggtct cgctgagcac    1920
ccgtctctca atcccaggat ttgtacgggg attctgggca gcctttgaag agaggctgcc    1980
gctcagcttt tctgagaggt cgccgcgcga agtcttgagc cctccgaact gcaaggacct    2040
gcccctaggg gcacgggtcc gattttgata ttgaagggat gattttgttg gaatcgtttg    2100
ccttaaatga gtgggtagag caatgtctcc ataaacgggg aaggggacgt ttccacccct    2160
```

```
cccaacacta tctaataaag acatcattcg tcacaactct aaattaaaga aagcccgatc     2220
aaaccagagg gagacttcca cactcctccc ctaccccgtg gagatttttt cttttttctg     2280
cagtgtcgaa cgctccatga agggctcacc aaatcgccta acctcccggc tatctctccc     2340
agacgtataa taaaattaat aacctaaagt tatatataaa taaggacaat ttcgttgcat     2400
ttttccccgc aggaggttgc ccttttttcg ttgcccaaga ggaaaatgtt caggaaacta     2460
ctgtctcaaa ccaatcgatt ttaaagatac agtatccttt tctccgtgta acgatttatg     2520
cggaaaataa atctccaagc tcaagagcaa atgaaaagtt tcacctctgg ttcctgcttg     2580
aggaacaaag accaactggg cttgccgcct aggggaaagt ggggccgtgg gtatgggcga     2640
gggggcatct ggccaggcgt tgggcacaat ggagcagggg cgagtgcttt cagcattgga     2700
gtcaccattc ggggggccttc ttagatccgt caggccggac aaccgttcgg attcggtggc     2760
cgggaaataa ataagccaat tcctttggtg actacccccc gcggatttcc agacccttag     2820
ctaaatctag ccacccagaa aggggaaagg ggaaaaagaa acaatcaacc cagatgcccc     2880
cggggaggcc agagcaggca tgcactggaa ttgatacctt gatggtggtt tcgggcaggt     2940
tgagtgccgc ggccagctcg caccggcggg gccgcgacac atagttctcc cggtagaact     3000
ccttctccag gcgcgcgatc tgctcgcggg tgaacgccgt acggtagcgc cgcacttgat     3060
ccgcgccaga gccggagcca cccagcgccg cgctcccgcc gctgcctccg ctgcctccat     3120
gcaggcttcc gaggcctgag cccgacgccg acgtcgtggt gccggcagcc gagccgctct     3180
ctgcgtaccc tggcaaacaa acgaccaaca gcgcatgagt ggctgtagga ccaacagccc     3240
ggcgctggcg ctgcgcgcgg atcggggaag ccccgtcagg aaggagagtc gctgccggaa     3300
ttgatggggt ctgtcatgct tacaaattgc tgccgttaat ggaatcaata aagtttgggg     3360
agcccttcat aagcaaataa tagaaacgga attaggagat ttcttttttta ataattagaa     3420
attttcaacc aaggaggaaa agtggccgag ggaaaatgcc tacctctggg cgcagtttgg     3480
gaagctctgg gtttcccatg gtctggagac cgcagggcag ctttttatac ggcctctaac     3540
ctttatctga gtcttcgggg tttcaaatat tttaagagtt ccaacacagc agtagctcaa     3600
acccaagcca ataggggggtg aaatatactt ttcaactctt tctcccgctg acctaaacag     3660
agacgccggc gaggcttcct ccgacttacc cagaagaatg ccccctccag ccctgagggc     3720
acaggggcag gggagttcag agtagttgcc cagggtacct ttcccaaaag caactcggct     3780
gctgactagg ggtctaccgg ctcgctcggg ctcttagaca cgagcgcaga aacatttttct     3840
cggactcagg agcgagggcg ggtgggcctg gtgttccagg ctccgcaggc ctgagcctgg     3900
cgggaaagct caaggagcag aactggaagg cacggtccca gacatgcgtc ccgccccccgc     3960
ccgtgctagc tcggtgtagc ttgcctgtgg agggtctgag aggggaaaag gcaccgggaa     4020
aggctggcgg gggccgcgga ggagcaaaga ggatgggact ggagagcgcg gtgcggccgg     4080
cgcggttacc tttgccattg ttttccttaa gctgagcggc gccgaggccc ccgggggagc     4140
gaagcgcgga gcagcccacc tccacgtcgc tgctcatgtc ggcctcagcg gccgcctctg     4200
aataatggcc cggcttcttg cggctctcgg cggcggagga gatttcggag gagacggtgc     4260
tttcgctgcc cgtgtgctgc aggttgaaca aagtgtctat ttcgaatttg cccttggcgg     4320
ggagttctcc cagagcgctg tgcagggggg cagacggcag gcgcgggctt aggcgagccg     4380
ggtgctgcga atttccagg gcctcgagca cagcattgcc agccgagttg gacaaattgg     4440
agaatctctt gcccgccgta gggctgtgca gccctctctc catcagaatc atctcttttc     4500
ttattctttc catcatctca gctttcttaa aaatgtcaca gtggccctgc tgtcccgtcc     4560
taatgatagg ctgcgcctag ggctaattct cattcagccc cagcgaacgc ctctaaatat     4620
tattatcgct ttcggaggga ggcggaaaaa ttgtgggatg ccagagcgag ggaatgactg     4680
gtcaaaatga cccatacatc cttccgatcc cgagatgtca ttcatcaaaa agtagcgccc     4740
gctcgtcatt aaggtacgaa tgacgctgtt cgaataatca tttattgtaa caggtttata     4800
agcaaataaa tacaccctcc tgtcagtggg taatgaaggc agcttcagag cagacaaata     4860
gatccaggta ggaggcgaga agagacaagt gaggaggaag gctccggtcc tttgcccgct     4920
ccgagccagt tctgctcgcc cgggggtttg gcctcggggg tgaaattgac agtctgatta     4980
atagagcgcg ccgcggcaca tttccccctt catttagggg catcattacg ctctcagttt     5040
gctaggttgc cccttaggtc ctaatcatgc agcgccttct tcatttttcc ttggacacag     5100
atacgtatta aataatagat aatggtttga ctttccagag taggtcctcg ggaaggtttg     5160
gttttttttt tttttttttt tttttttttt accatttaca agctgggggc ataagtgggg     5220
ggagataagg gggaggatgc aggaagagtt gcgtaaggag aagaccttgc gcctctccta     5280
tctggattac ttatctttcg gatttcggaa ccaaatatgt attttattta acaaatag       5338
```

<210> 3
<211> 5332
<212> DNA
<213> Homo Sapiens

<400> 3

```
ctagtttcaa accctgcacc cgcactctcc agacaggaag tccctctccc tcgaccaggg      60
attgagggag aagagattca gaatgcatct ccttgcccta ggcctttcgg gggggacatg     120
```

gccttagaca gtcaccccga aattgggctc aggtcctcac agtaagtcga gtgtcccccc 180
acatccccaa ccccggccct ttcactaaag tttaaaagat tcttttgttc ctcaggcttc 240
tcccgcttgg ttgcttccat tattccccaa ctgcggaaac accagccacc atatgtccgc 300
cggagaataa gcgtgagtgt cgcggtggac agttgccgcc aaaggtgtcc agacggatgg 360
cattctcaaa gtagggatct tagcatcctc tccatacatc atcacccaag aatggtagag 420
gaaagagtag gagtggggtt acagatagga gagaaaggag gttgaggcca caaaattggc 480
atttctctaa ttatcaaccc cctcctccac catcctgcca ccatttgtta tggcaaagtt 540
ctctttctgt gccccttccc taataaagaa atcctcagtc agcctccacc tactcctttt 600
gatttgcagg aagccaaact gccagcagat tgagctttct aagggatacc tttgggatat 660
gttggaattc tgatttgttc aagatgatga cggtcatcag tacaaatcag aaacaatgcc 720
actcttcagg gtgtcatttg taagtgcaat aacacggatt catttgcaaa acccagctaa 780
actgcgtctg gataagtgac tagatccaaa cgacaaattt cttccatcac tcagctcctc 840
ccaagctctg cgggggcagc acagaagact gctttagggt gaaactaagg ctcagctcac 900
catttgtgcc agcttgaggg accctggaaa tgttgactct gagaaaaaga caaaacaaaa 960
caaaacaaaa caaaacccaa tcacaaaaca tatcctactg tctacaaagc aaacagaaac 1020
acaaagttgt ttccaaaatc accttttcag caaataagta tgtgatattc tgagcaggag 1080
caggagggag agaaagactg ccacttaaaa aaacatgaaa gcattaagta ataatttaaa 1140
acacggctgt ttagaaaaat atttatattt attgcagctg ctcaattggc ctcgttaaag 1200
tcggcaagca tttaaattgt gttacaatct catttaaatc ccgttccgtt ccagcaggct 1260
gaagagcttg aatagaccaa tcacttcata atgatgatga atgagaaatt aattcagata 1320
caagcaagac aatttaggcc ttcatctgtt taaatagcct tccaatatta ttcgccatcg 1380
cgggtcacag attgaatcaa ttgtccaatc ttgtgaaagt catatccttg cgtcttcatc 1440
gagattattt atagcgcagt gagcgctgct gaaaggtata cgctgttaac agggacaatt 1500
acttaaaggg aagcgtttgt aaaatggaaa acaaatgccg gggttggtaa caaatgggat 1560
caaaagcagc cattccaatc tggctccccg agggaaagga gcgggcgtgg ccctgtagga 1620
ttaggggcgc ttctgacctc cccaagaccc aggtgaggcc gagggtcccc ggcgcgccag 1680
cctgcgccgt ggctgtggct gcggtggcga ctcgggccgg gcctcgctct cgccggcttc 1740
aggttcccgc ctccctcgcg caggcagcgg gcgcgtgtgg cccgggctgg gcaagccgag 1800
gaacagcgag cccccggacg ctgactgcag gacgtcccag tttgtgcccg ggtctccgtc 1860
cctccccgta cggggctcgt acccccgggc ctgggtctga cccacagggc gctgaggcct 1920
ttgtagctga agtcggaagg cctcgttgcg agcgcggcac aggttgctgg tagcttctgg 1980
actctggagg cttggccttc cttctaagcc gatggcgggg aaagaacctc gtttccacag 2040
cttccccgac ccccgccgct tgccatttgg ggacgggaag cgcgcccggg tcgcttcacg 2100
tccctctggg ccggagccct ttccatggct ggctcctctg gggcccttg ggcctgtgag 2160
cagcgtctac ttccctcaga gaagaatcct ttccttcccc catcgaagtg tccctttctg 2220
tatcctgaaa taacccctcc tgggtgaggc cagttcccct ctgtcgccct cctcccgcag 2280
gcgtccggga gcctcgtgag gaccccgtgc agttgagtcc aggcgacagg tgcctcccca 2340
ggtgtctact tgccctccct caacctgttt agggaaagac cagaacgatg tgcgcgggga 2400
ggtggttttg tttctcccga aactggcgct cttgggtaaa accgcgtgcc gcgtctacgc 2460
gggctctgtt agtgtgcgtg tcccagaaga gtgggccttc taggcggcac tctttggaga 2520
agtaagtgag gtgaactcag aacagagaag cgggaacgat cgtttgatgt gccgagccgg 2580
aaaaagagag gagagggaga ggctcccagc gagcgcggag acagaggagc ccacgcggca 2640
cagcacgagc cctactcttg cccgcgtaga ggtgtgtgtg tgtgtgtgtg tgtgtgtgtg 2700
tgtgtgtgtg tgtgtgtgtt ttcctctgta tttctgtgtt tgaaacaaaa gcccaactaa 2760
cggattcctg gtgctctgag gactcactcc cgggagcgac tttgatgtat tgctgtccaa 2820
ttagtgcctt taattggtgt cctcggagac aggcaggccc ggagctggga cagagcctcc 2880
tctcgcctct cctgcacact ggaaaggtaa aatttataac acactgtggc caccccttgg 2940
gagagcgaaa gagagtggga ggcagggaag aaagggagag atgcagctgt ttgcagagga 3000
cggagcctcc agccccgggg gtcgtgggga agggcccaga ccaccgggtt cgactcagtt 3060
ttctgtgtaa gagggttcgg gccggctgag tcgctgagga ttatgaattt gctcgcagaa 3120
aggcctaggg agctctcatt gtctgtacaa acccacccca tgtggtctac catctcgtta 3180
ataatggctg tttgttaaca aggatgcgga ggttaaataa tccgggtgga agattaaaca 3240
cgtctcatca aggcgcttgg aagccgcgcg gtgcagagac ctgcgctgga gccgcctggc 3300
tcactggagc ctggagggag atgggagcac agtctgcaag gagccccggg gtagaagagg 3360
aggcaaggtc tagtctccct cttcagggtg tcctgctatg ccaggacagg tctaactctt 3420
ccacatgtca aacagaaaat ccacatagaa gtcagcccca atgaggtgtg gaggtcaaac 3480
cctcggagta gttggctgga gggcagacat ggagcatctg gggtctgaac caaaggcccc 3540
cttttgagga ggggtggcca tcccctgcct cccagtcctt acatgtcatg catctggcaa 3600
tttgtgccta catgggatta gcattggatt agaagacaga agatgtggtt tctagtctgg 3660
gttctgcccc taaatcactg tagaattttt attacattat tctcccagct tcatttttct 3720
actgtgtaag agaaaggaga tggggcttga tgtagatgat gtacagcatt tcttcttttg 3780
tccagcttcc caccagagtt agggaactga acaccttata agatgctctg tctcccctaa 3840
accttgcagg atgcagtcaa aaagaatttt atttagcaaa tatctgaggg cctactctgc 3900

accagtcatg gtgcagtatg tattttttaa ctgagagcag aatatcagtt catattcccc 3960
tgttattctc aaatataatg ttttataaca aaagatgaaa gagaaagaac ttcaaagaga 4020
atagaatagg atgggtatgt atgtacaggg tagagaaact atggaggtaa aggtaaaggc 4080
attgacaaaa gctgtgaaga acttgttgaa ctggcaagga agtcagtgga taaatactgc 4140
agaagtagct ccttaacaga gatactatgt aggagagtag agagaaggtc ccctcctgaa 4200
tcctggtgat ttggcagaga acaaaagggt aagggttgca gaatttgact aggaggccca 4260
agttgtatgc atctaatatt gccaattttt ggtttatata ttctttcagt agttggaatt 4320
tctctctctc tctctctctc tctttttctt tttgagatgg ggtcttgctc tgttgctcag 4380
gctggagtgc agtggcccca tcaaggctca ctgcagcctt aaactcccag gctcaagtga 4440
tcctctcatc tcagtctccc gagtagcaag ggccacaggc atatgccacc atgcctggat 4500
aatttttttt tttggttgag gggtacagat ggggtcttgc tgtgttaccc aggctggtct 4560
caagcttcta ggctcaatga accctcccac cctggtgtcc caaagtgttg agattacagg 4620
aatgagccac tgtgcccggc ctaggaagtt tactctttag ctaattttga aacattactt 4680
tctgaatcca ccaatcagga aactcggctg ctaggctggg actcttcaag cttctgggtg 4740
atgggcattt agcctcaagg aaggcaatga tgcttaaatc actaaaccat gtctcagttt 4800
ctgtgagtca ggaatctagt gagcttagct gtgtgtccct ggctccaggc tattccactg 4860
gggttgtggt cttatctaaa gactcagctg ggtgaggata tgttccgggc tcactcatgt 4920
ggttgtttgg caggattcag ttccttgctg gtgggctatt tggctgaagg cctcagttcc 4980
ttcttggtgg ttggccagag gtttctctgt tccttaccat atgagcctct ctatagggta 5040
gcttataaag tggcagctgt ctttcctcag actgagtaag caagagtgag agtggagggg 5100
ggagaaagca cccaagacag aagctacact cttttggtca tctaatcttg gaagtgactt 5160
tccatcacta tgccatatta tatttgttag aagtgagtca ctagatccag cccacactaa 5220
aggaaagggg attacagaag gcatcaaatc caggaggcag ggatcactgg ggactgtctt 5280
agaggctgct gcccagaccc cttttatata acagctctga ggcttatatg gt 5332

<210> 4
<211> 4215
<212> DNA
<213> Homo Sapiens

<400> 4

```
ggtgcccgac caagcgcagc ccgcagtctg ggcactgcca actgactcca actcctttta     60
tggtgagagg atggattctt cgttatttcc ccgcccaatc tggtacccac ccacccaccc    120
acctaccacg tccgctgggc gcacccaagt ctaaccccgg ggcgcacgcg ctagcgcaga    180
caccgtattt cttctccttt ctcggccaac cctaggtaga atcctaaaac aactgccctc    240
tcttccacga tctagatgtt gcggcccgcg gacaggaggt tcaagaaata gtacactccg    300
agcggcaggc agcgaggcgg aaacggtcgc cggtttcagt ggtggcccca ctggaagccg    360
agttcaggag cggctaagcg gtcgccgggg aaagcaccgg ggcttcccag ggtcccctcc    420
gagttcccac tccgcacctc cgagggcgtg aaaaccacgg gagccgcccc accccgcgcg    480
cccagccccg ccccagccca gacaccgccc cccgccagtc ttccctgcgg cgcccaggga    540
ggacgcgctc cgcccccttc caatccggcc aatgggcgcc cgggcagcgc gcggtttgcc    600
tccgcctccg ccagggaaac ttggaggagg agaaaagttt gtacagaggg tggaaaggcg    660
agagcggagc tccaagcccg gcagcccgag aggaagatga acagccccag gccagagcct    720
ctggcagagt ggaccccgag ccgcccccag gtagccagga gcggcctcag cggcagccgc    780
aaactccagt agccgcccgt gctgcccgtg gctggggcgg agggcagcca gagctgggga    840
ccaaggctcc gcgccacctg cgcgcacagc ctcacacctg aacgctgtcc tcccgcagac    900
gagaccggcg ggcactgcaa agctgggact cgtctttgaa ggaaaaaaaa tagcgagtaa    960
gaaatccagc accattcttc actgacccat cccgctgcac ctcttgtttc ccaagttttt   1020
gaaagctggc aactctgacc tcggtgtcca aaaatcgaca gccactgaga ccggctttga   1080
gaagccgaag atttggcagt ttccagactg agcaggacaa ggtgaaagca ggttggaggc   1140
gggtccagga catctgaggg ctgaccctgg gggctcgtga ggctgccacc gctgctgccg   1200
ctacaggtga gatggcgttg ggctgacgtt ggggtcaacg ggtagagaac gcagggatgc   1260
ggccctcgcc gaagagagcc aagaagggaa gagcgcgctc tccaaattgc ttttgtaact   1320
tgttttcagt gagcatttta ttgattcaga atctatcgag aatagcacta gcgagctact   1380
tttcccttga gatgggtctt attcatcttg gcaatggagt gagttggatt gtggggagga   1440
agaggaatgg gaaaatcagt ttataaatat taatgtcagc aagagtgtgc tgttggcagg   1500
acgtatcgcg agcctggaga ttttggtggc cgcagttggt aagtggctac aatccagaaa   1560
gtaggatcga gttgctcccc ttgtcttatc agtgtatcgt ttctcgggcg cgggtctaac   1620
accttacaag tggtaatttc cgctcacggc agctttgtct ctcttctacc atccccagac   1680
ccagccttgc actccaaggc tgcgcaccgc cagccactat catgtccact cccggggtca   1740
attcgtccgc ctccttgagc cccgaccggc tgaacagccc agtgaccatc ccggcggtga   1800
tgttcatctt cggggtggtg ggcaacctgg tggccatcgt ggtgctgtgc aagtcgcgca   1860
```

aggagcagaa ggagacgacc ttctacacgc tggtatgtgg gctggctgtc accgacctgt 1920
tgggcacttt gttggtgagc ccggtgacca tcgccacgta catgaagggc caatggcccg 1980
ggggccagcc gctgtgcgag tacagcacct tcattctgct cttcttcagc ctgtccggcc 2040
tcagcatcat ctgcgccatg agtgtcgagc gctacctggc catcaaccat gcctatttct 2100
acagccacta cgtggacaag cgattggcgg gcctcacgct ctttgcagtc tatgcgtcca 2160
acgtgctctt ttgcgcgctg cccaacatgg gtctcggtag ctcgcggctg cagtacccag 2220
acacctggtg cttcatcgac tggaccacca acgtgacggc gcacgccgcc tactcctaca 2280
tgtacgcggg cttcagctcc ttcctcattc tcgccaccgt cctctgcaac gtgcttgtgt 2340
gcggcgcgct gctccgcatg caccgccagt tcatgcgccg cacctcgctg ggcaccgagc 2400
agcaccacgc ggccgcggcc gcctcggttg cctcccgggg ccaccccgct gcctccccag 2460
ccttgccgcg cctcagcgac tttcggcgcc gccggagctt ccgccgcatc gcgggcgccg 2520
agatccagat ggtcatctta ctcattgcca cctccctggt ggtgctcatc tgctccatcc 2580
cgctcgtggt gagtgaccgg ggctggggcc ctactcggcc tttttctcgc atccacctcc 2640
cgcgtccatt ccccgctccc tgctttccct ctgagtcctt ggcagtgaac gtgtcgcctt 2700
taggtcgggg ctgggattcc cacactgttt ctcagagcag gcccaaccct ctttgaagtc 2760
ccaaccctaa cgagatttag caggtgcttt gcccctacat cccccagttt atgttcccgg 2820
aagcctgggt ttctttctcc accgagacag ccccttacccc ttgctgcctg acactggccg 2880
agtcttccaa gaaacccccc cgccccctct gttagacgtg gaggggagcc tgctgtagtg 2940
tgacttagcc cattcctccg tactgtgaac tgtgaactgc aaaacctgaa atgtcaggga 3000
tggtgatctg ttcgttgtaa accattttta aggaaagtgg ggtttttggt gttttgatgt 3060
atgcaaggtt tcttgaattt cttccccccgt ggattgcgta gaattttaaa gctagaagag 3120
atatcaagag gtgttttagt ctaactccct cacttcactg agggagtgaa tgatacgctt 3180
aaaataatcc agccgaacct acacagtcaa gctaggctcc ctgctcttta ccatggtgtg 3240
ccctgggagg gcagcttaaa gcacacaaat tctaattcat atacagggca gggtccagac 3300
tcagtttctt tcattctgtc tagaccagga aagcaaagtt tctcagggtg actaattatg 3360
gacaggctga tatacatagg gtatgggatt tgaattctcg acaactgtgc gaggtacagt 3420
accattttag agaaaccgag gtttagagat ggctgtcttt gtttcttcaa gccaaagctc 3480
tacagcttgt aatggacctt aggccgtctg aggccagacc cagctcatgt gttattacat 3540
cacgtctccc tcgtagaaca tttctcattt tggcgtttgt aaagcgtggg ttggagtttt 3600
ggtacacttc atttttagtt catcttttaa gaattgcctt tggattaact tttaaaatat 3660
gataaacaga aaggctcaaa ccctacctag ttttctactt tgcctgtggc taaaatggaa 3720
tgcaccagaa gcagatgggc agaataatgc aatactttat ctagagtttc tcttgaaagc 3780
aactagttat gagttcttcc atcttccaaa acactgcatt cctggaaatt ctaaactacc 3840
aactcaaaaa gagataactg ctttttctta tgcttgttgg gaacaagaga atataaaatg 3900
taatgggcta ttattccttc cttttcctta ttgatactgt ccagggatct ttggtgtaaa 3960
aatatatctt tgataaaagt gactgtaaat ggaaggaact tagaggggggc tcagagccag 4020
aatacagtag gttcttacca atttgtataa acagaagtaa atataatagg ccataagagc 4080
ctttaagcca ttccaggacc caatataatt tacagagtta agtcacactt gagccacata 4140
aatatatttt actgtctgta atggcaacct agaataaagt gagattttgg aagaaacaac 4200
actgctcctg atggg 4215

<210> 5
<211> 5473
<212> DNA
<213> Homo Sapiens

<400> 5

```
gtaatgttaa ttagcttcat cttaaagaaa aaccaaacac cagagcaaat aatgactcct      60
ttgcggagaa ggagttgtaa gggttgggga gaggtaatta gtgtcataaa agcctcacta     120
caaagaagtt tagaatttct ctgttctgct gtgcagcaga gtgaaacaca gtagcagaat     180
aattgtgcac cattatcatg ataatctaat ttgcttcttt agaaaaagcc actgtttgca     240
gttaggatag taggaaaaca tctagaaaat aactaaaatg taaataaaag tatgaaacaa     300
caataaagtt cacttcttaa gagaaaaaaa gaagagcact cagcgaaagg agaatgaagt     360
atatataaag caagtaactc ttaggtactt tgttctcctg gatgttacct gcttaaactt     420
gacaactttt acaggcagta tgacagattt tttaaaattc aaataaagct tgcttttgta     480
ttccactttc aataataaag catattgatt gactcacaaa ttaaagtttt taaaacccca     540
ttaaagtaaa aatactgctt cagagctttc attggtatgt ttttgaaagc aagtgtcata     600
gtcaatgtaa atcttcaaaa atttctctgc tatttagtac aacagagggt ggattttgtg     660
ccagggtggc atcctttgtg taattcacat ggcctgaggc ttcttgcctt tttttcccct     720
agaagagttg gaattaagat actaataatc ataactgtaa taattatcat catgttttta     780
aggggttgct gagcctctgg agatctcagt gcacagagcg aagcaggaga ggcttaaaaa     840
ggaaggctct gctgctgatg ctgtgatgac caaggcagcc tattattact tttcctcttc     900
```

```
aacgtgactg ctctgttttt tttttcctg atcctgttac aagatgtggg gaagcccctt     960
aagaattgtg catagttcgc tacagatggt ggtctctgtg cccagagacc gaaagagtat    1020
ctgttacagc gatactttgt aacagctgca gatgtgcggg cctaaaactg ctccatgtgt    1080
tcaaaccaat aataactatc attttattct attttattat ctgagcggaa atacaatctg    1140
aaacatttaa atttgttccc cctaaagcta gaaggatatg tgtatgtgaa tgtacacaat    1200
ttgctccttt aaatgccccc aaagcctatt tttgctatta catttgcctc cacaaccaat    1260
aaagaaaatc tgtttcagtc ctaacttcat tgggtatggc acgtgctgga gggcaggctt    1320
tggaaatata aatactaata tatgtggttt gttttaaaat ctaatgtatt tagggtcagt    1380
ttcgttgcct tggttaaatg aatgaacaaa gagtgaccaa aatgcgttta tttctccgct    1440
atttcacaca cagtttgaga tcgaccgcta acagcagact gtaaatttct gcacttaaca    1500
gttactaatt ttctttatag tctgcacatc tataaaccta attgggatat ccgcttgaca    1560
aacctagacc ctgcagagca gactttcaag gaaatccaac tccagagaac aaaatactct    1620
ttggaacttt taaggcatcc agagataaaa cgttttctgt gggatttttt tttaagcctt    1680
ataaaagtag gcccattagg attaaaccac attaaagcca gtgcgaggca agggggggtgt   1740
ggtttataga aactctggga gaaagcccag cacagctttc ttcttctggg aacatatgtt    1800
aggatggaaa aaaaaaaaag gaggggagtg ctataggact aagatctact ttggaaaata    1860
agacctctag taaagatatc tctttccttc tcatcttaca ccagacacta gaagcaccac    1920
ctcccgagtg tgtcccccaa gcctctttcc aaccccaggc tctctctgct ccctgggccc    1980
tcgacctcct ggcagctggg tacctgcact cgggcctccg acaggcccag tcgctggctc    2040
agttcctctc gcatgaaggc gtcgggatag tgggtctcgt caaaaagcct ctccagctca    2100
ttgagttgtt ccagggtgaa attggtccga cttcgcctct gcttgatttt ggtctggcct    2160
tcgtcctcca tccctttcgc atcctctttg cgatctttca gctccgggga cactggaggg    2220
ggcaccccag cggggccaca cgtgcatcca cacgaacaca cacacacacg cacacacaca    2280
cggacgaaaa cagcacagca aatcctgtta ccagatttgt cgcaaaaaag aatagagaat    2340
ccttccccct cgtggaatcc tggtccggct tctcaaccca cccggaggaa gaatatcccg    2400
gagaagcgca aaggtcaagt ctgagcggcc gcctggggca aggtgggcag tcatccacca    2460
cctccttcac aaacgtacct cgactcaccg cctcactatt caccccccag tcaccggcct    2520
tccccagcaa acaccaaatg gtttccactc actttcgcac tcacttgctc cgcagcactc    2580
acagaaatca aaggcgcagc gccaggcctc tcacagagca aagtctggac ataagggcgc    2640
ggcaaggcca cccactggcc tctcacaccg gccattccca gaaggctggc tcgttctcag    2700
gccagctctc accaccgctg gctctctgcc taccgcaaac ttgctggtct aatttaggaa    2760
caattgggcc gaaaggtatc agcgagagca acagaccccg gtgttgtgcc gcacagggag    2820
ccgcatccgc agacgcccct cgctgcccct gggctcgggc caaaccctgc ataaggtccc    2880
ctggacagcc aggtaatctc cgtcccgcct gcccgaccgg ggtcgcacga gcacaggcgc    2940
ccacgccatg ttggctgccc aaagggctcg ccgcccaagc cgggccagaa ggcaggaggc    3000
ggaaaaccag cctccggtgg cgggcgaaag caaccgctct ttctgttctc tcttcgccct    3060
ccctcgtgga aacgcagact cgaccctaaa cgcttaaccc acagagatca acaggttcaa    3120
gcggaatatt cgcgatcctc ggtttctatt ggttgctcaa agcctttca tgcaaccagc     3180
agctcggatg tttaataaaa tatgaatttt tctgctctgg gtttgcacac atccgttgcc    3240
actttcctgt ccttctcctc cctttaaaaa caggagctct tctagtgtga gcatatacag    3300
ccaccatata tatatccccc tggaaccttt atcctctgcg cccgaatcag agactccggg    3360
ttttaagaag tcaaagctga gtttagatag tcgaggctgt ttgcagttct tcccagcgca    3420
ggatccaggc cagcatgggt gttaaaaatc atttccggaa gagccggtct gcagagaaat    3480
taatacttgc cctgagcttg gaaatcaaca atcccaggaa ctgcgcccgc cggggggttca   3540
gacctgaacc tcgccgtcag gcgcgctggc gaacctgtag ttcgggcctg tagttgtgga    3600
ctcatggccg ggaggctgca ctgggcacct tgacggtaac tccaccactt tacaaattta    3660
ggaagacctg gtgctggttt ggaggaagaa gagtcgctag atctcggccc tgccgggcat    3720
tctagctacg gtgatcttaa atattgccgt attttctagt tctcagagtt agttttgtgg    3780
acatttgtcc tttctttagg aaggaacatt atagtaaaca ttatgggcca atttaatagc    3840
aaagtatttt tactgatatt gtattgaaat ttaaatatga aaacacaccc ttcaaattct    3900
gccaatgagg tgggaaggct ttgtctatta catgtcatct acacagtgga tgccactcaa    3960
agttttgggg cttcgaggag atgtccaaat ttcaagttga tccatagact taataaaatc    4020
tctatttttg tccccaaatt tccaattact gatctaaaca ggaacagacc gtgtcataaa    4080
caacttaatt gcttgcttaa atatgtgatt ccacgcatgc cacatatttt cttaattgga    4140
aaaatatggc agtacaccag ttactgcagg caccaagtgc caaatcaatt ttattgttga    4200
actcctttga attggtatag aacgctctgc cattgagata aagacgtttc ctaacactga    4260
gctagcacca aaggattttt gctctaagag aaccgagtac tgggtgattg aaaacactaa    4320
aggaattgtg taaatataga tccccacctc ccccgccgcc cccccccccc gcccccaaca    4380
caccaagaaa ccgaaacgcc tcgcccggga gaagcagcgt agcctggacc tcagctttcg    4440
ttggcttcct tctaccttga aagagaatgg gaagttttgg cccctaagat ccaaagattc    4500
aggggctctg cggagttcga ggggtcttgg cggccccaaa cacctaggcg accggagggt    4560
taagctttcc acctctgccc cttctccctc ccggcaaact ctgcgctaga ggctagcttt    4620
agtcccgccg tctggttcag caccccctcc tgcagcccgg ccccgcgaac ttccacgtcc    4680
```

```
gcctgcgggc catccgggct gcgggcccat cctcccgctg gtgaaaaatg catttcggtg    4740
gaatgggaac atctgggaag ggcgcgcaca aaccccacac gcttcccccc acgccacccc    4800
cacaacacac taggacccca cccatgaccg agcataccac cggacccccca cccttccact    4860
atcactctag ctgacaaagc tggggtccag ggccctctcc gtgtccctct cccctccccg    4920
ctgggcctcg gagtcctctc ccgcccgaga ggagtccgga aggcgggaag gggaaccgct    4980
gccgggggtc agtcaggtcg ttaccctccg tcagtcgcgg gctgcccggc tccctgcttc    5040
tctcggcggc gcccatgtcc agctcccgga cgggagagcg ccctcctcca gctcctccgc    5100
ctgctcctcc tcctcctaca cctcctccgc ctcctccgcc gccgcctccg cctcctccgc    5160
cgccgcctcc gccggccgcc cggactgccg ggctgctgcg gtcgtcgcgg cccgcctcgg    5220
tgcagccggt cggctccttg gccccgcgca gcggcccgct ctccagcacc tcccggtacg    5280
tgatcgcctc cttcttctcc ttcactttct ggtcaaaaga cttggagacg aacgccgtaa    5340
gttcttccat cgccgccgca cgtcagcccg gcgctcaacc tctgccagca gagccccgct    5400
ctttttttcc ttcttctttt tttactgctc cagccccccc aataataaca catcaatggg    5460
acaggaggtg ggg                                                       5473
```

<210> 6
<211> 5328
<212> DNA
<213> Homo Sapiens

<400> 6

```
ttgacaagag aaaaggtgtt gagggcaggt cgcgctgtct tatttacagc ccactgcttg     60
ctgtagagtc tttgttagga ctggaaagct aatttagtgc tgatcaaaag caccacagag    120
ttaaaggact ggtaatgact gctaatttca tttcagcctc atgcatcccc aactgctgta    180
attaaagaca cgctatttaa atacaggtga agggtttttt ttttttaagt tgggttgggg    240
gggggtgggt gggtggatta caattttatt tagatcttct actctacttg ggtgttatgt    300
agtcccaaat tagattttat tattattatt atttagagcc gctccttgtc atttatacct    360
ggctttgcag aatcggaagt gtgaaatgtg tcactcacat aaaagcccct gtttaaagaa    420
agggtgggac gggggagtgc agaggagtaa ctatttaggg gtgatttaaa tatttaggtc    480
tccttttttt ctccttcaaa ttaatagaaa ggccttcgat atagttaaaa gtatttgaaa    540
ctgggggcaa ggttaacagg ccaaagggat cagtgtcagc tttttaagta cttctaaaaa    600
gatcatgtga acagatggaa aaatatattt ttaattaaat aatttccccc acactatagc    660
tgcagtgttg gctcagaaac cgtgtaattt ctcatccgat aggagtttct ttatgagaac    720
atcaaaggat aaaaagctct gcattttcgt agttggttaa aattgataac cacccccccca    780
tgcgcgcgcg cgcacacaca catacacaca tatacacaca cacacaccaa cacacacaca    840
cacacacaca cacacacaca cacacacaca tatccccaaa aaagaattta gcctttaatc    900
tctttttatc cacgaagact aaagctctgt actcgacccc gcgagcggct ctcgccttcc    960
ggggctggaa cccgactcag atgtgaagcg gcactgagcc gctgcgcagg ttcgtgccca   1020
gcgcccccctg ttggacacag taggagctgc gcagcgccag tcccagaaat cgctggaatc   1080
ctgctctggg gttagagaaa actcttttat tgccctccaa aaaagaaacg ggatgcgaaa   1140
acaaattttt atatccttga aaaataatat aatattaact caataaaaaa ctaaagaggc   1200
gatactacaa ttttaattca ataaaaaata aagactgtcc tcctcacatc ccagccatcc   1260
cccacccggt ttggttttgt aactcttttg aaaggttctg cgatggtaaa acaggtaaag   1320
aaaagtgacg aaaaacaaag catttttctg tagccgtgga gaatcaagca aagtatactt   1380
cctccgtttt gtccacattt caaaaaatta aacggaagat gctgctgctt gctgttttga   1440
cgttgtcagc agcgtcttga atgccctcag aggtcctgca agtatcgtaa ctgagccttc   1500
gagtaaacga aataacttgc ttttatgtgt ttatttccag agtataatta aaagaccagt   1560
ctcttctttg agtgtttccc atcccctaga ccaatcctgg tcaaagtttt tccttcccag   1620
atactggcta agaaacacat actggctaag aaacaggtgc ctggtatctt ttttctttca   1680
tcttagccgt aggatttcgg ggaaggtgtg ccaatggtag ggaaggtttt ctttgaaatt   1740
cagcatttcg ttgaccagga atccaaatta tcctagggaa gggggagtat ttttacactt   1800
ccaaaaaact acctgtctac ataataaaga gctttgcctt ttttattatt attatttctt   1860
cttcttcttt ttggtcagca agtgagggca aaagtagcct ttgaattatt tcagaaagag   1920
aaactgtaaa aatggcacat cctctgaatt taaaacaata gggaaatgtg ggtttgctta   1980
attgtttaaa atcaaataga aataattact gtgctaaatg aacaagatgt tttaagattt   2040
acatcttcag ggttatggtt attgatttta actttaccct taagaagttt agagatcgcg   2100
tttttaaagt ttgcttattg acggaaatcg gcttaatttt tcataaaaga atggcttttt   2160
aaaatattga atactatggc tatgcaaggt ttatgatacc tttatgttta ttcttcccac   2220
ctcccaaaat tagcgaattc gagataacat ttcaagggga attgctattt agtgcgatga   2280
gagatgactt ctccatctgg ttccagaatt agatctcggc ttaggttttt tagtaagtag   2340
gttcagatgc aatggaagga gagaagaatc aagccttttg ataaaatctc taccccgaat   2400
tctatggaaa agacaggcaa gcgggattcc agccacagca cccccacctt ttgctctacc   2460
```

```
aaaaggcttg tccccattac agaaataata tagtcggtgc tagttttgtg aacccgcgcc 2520
aaccgataag gtaagaaacg gtgcgctgac agccgaggtc cgaaggctgc tcagtggttc 2580
ccgcagcccg tggggtaggg aggccaaagc cagcaaaatg ttggaggcga cgccagaaag 2640
agagtatatg cctgctattc ggaagtgatg tcgaacgtgc aatagcagag tcctggagat 2700
ttccaccccg ttccgcaccc agggatccca gcctgtgggg tagcaggttg ttgggtgaag 2760
gcaggaggga gaagccgcac ctggctgagg gcgggtgggc gtgccgggag cgagtgccca 2820
actttctagg gcccatggag aaagcataac ccctagttga ggaaagagag ctaaacaagt 2880
gggtggccca ggcctgggaa tgccacccag ctctcccgac gcagtgttat ttctgtggtc 2940
ccaggggggc ccagcacctg cccgaatccc actccccaag gccgccagca ggaaggcagc 3000
ccctccatct ttgagggctg aggagggggc agcgagtgac ttgattcctt agtagcaggg 3060
aaaaccgcag cggctggcaa cgggccgcgt ttctccgcgg cggcaaagcg ccctttaggg 3120
gcggggcggg catttttggt ctcctgtccc agcgtccagg cctgagcccg cgcgggttgg 3180
ctgggcgccc tgctttgtaa gtaaccaatc ttgctggatt ctgaggacta tccaggcgga 3240
tgggcccact tagcttaggc ccactgagac cctgccaact cctcagaccc ttttcccctt 3300
catccctcac acctttcatg atgaacccag gaaagaaccc gaactctgaa aaacagtaca 3360
tgtggtttga agtttgatag ttatttgggt gtgagggaca tgactctatt aataaagact 3420
aagattcaag gctgagctct catttattaa atttgctctt tcactctgtt ttcaaactct 3480
ggagatcttt cagtttcgtt gctccaaccc tgacacagac aaaggcgcca gggagaaagc 3540
ccaggcgggc ttgaagcagt tgaggtatag actgatactc atctctccat cactcagacg 3600
ggcagataag cactgccaga ctgggggtcc taatccaggg cccagcggct acgtgggttg 3660
ctgcggggtg ttgaaaccac aagtacaact tgacaccatt gagcccacgg tgcttgtcaa 3720
taatctgtga attaggtgca gagattgtca ctgtagatca taacagtttc tgaaggactc 3780
attacagcct atcgtgtcaa tgatcttcaa attaaggagg ggaggtgggg cgagacgaaa 3840
aaggagtggg gagagccagc aaaaagggaa gaagggtggg agggaagata aaagtcagag 3900
ccccagctcc gaaggagcct ttaaagatta tcaggccagc gatctgagaa ttccattggg 3960
atgggtttgg aaacccaact ccatctgagc caggactgga agagggggcgc tgggggcaag 4020
cagaagtggg cccctgggaa ggccgacttc tcaggcattt cttttgaggc cggaatagct 4080
tttgggagag ggttctagat gtcaatggga ggccttgagg atgccagccc gcccaggatg 4140
ggtgtacact ttgatttaga gcactttcca gggcaagccg cagctgcaga tctttgaaaa 4200
aagtggtaat agtttcgttt agggatgatg gtgattgtag gatgactttt tttttccaat 4260
ggtttgcttt aacttctttt catgccacag gtgaaagctt tcttcctttt cctctgatag 4320
tattcctgtc tgtccctcac atgttacgta gatctaattt ctttccccaa cttgtttttt 4380
tgggtgccaa acacatcagc cctctgagat tttatcactt ttgtggtttg tattgagtcc 4440
aactgtaatt ctggaaaggg aacttgtgga taacacagta gcagtatgtt ccataagatt 4500
ccagaagaaa gaagactaaa ttccatttat gcggtgtggc aggggccagg ctaagggaaa 4560
gaggttttcc ctgcttggta aattgtcctt gcagtgaaag ggtagcccag ccttcactgg 4620
agtgtcattt aatactggct agagaaaggt taaggaaagt gtaggtttgt ttttcccaca 4680
aaagttgtgg tcacaactaa acgaagtaat cacagttgga gaatttaaaa caactgtttt 4740
ttctctaaaa tgagggtaat aaccctagcc actggaaaat tgggattcta aggaaaacct 4800
tttcctttgg tataattaat tgcataagat agtgtggtca atcagagaca tatgtattta 4860
gactcaagca atgatttaca ggtgtaaaag gtccagtggc tttttgggct acctggagtt 4920
cctactgatc attttgcctt ttaaaaaatt ttcttctaat tttaaaaata aaagtcatag 4980
atttaatgca actgaaaacc actgatggca gctcttcagg ttttcagttt atgtttttaa 5040
agggaattta tatttaaaat aattaaatca tttaatggtc acactacatt ttctcctgga 5100
caacagctgt agtgcaggga tagaatctta acttacatta aaattgcttg ataattcaga 5160
aatggaaatg aaattgtgaa ttaatttctc cagaaaatat ttgagctaga atataagaag 5220
gatgttcata tataatactg taagtaacat gatgattatt atatgcagct tgaaagacct 5280
acacaaatga ttatatacta cttctgttgc tataatttaa agtaaggg 5328
```

<210> 7

<211> 4324

<212> DNA

<213> Homo Sapiens

<400> 7

```
gtcccccagt atcaccatcc taaaggcact tccacttcct ctatcatcag ggaaggagtg      60
tgcagttctg atttagctcc tcagtggagt aaagggaatt tagaggaagg gggatttctg     120
cagaaattgt cagttacaca agtaagttcc tcttggttcc ccgtttcatc aaaagtgaaa     180
aactgttcac aggagtgccc atgtcccctg tttccctatg catcactaga gccaagaggg     240
gactccgcca gcaaggcacc ctctgctctg tcccgcagat cacagtcccc gactttcacc     300
ccattgtttg tgcaatgtgc agaactggag cccaaagcag cgggctcagg atcactctcc     360
aggcccggcg ggcccataga ctccttcctt ggctgggaag aggggctttc gtgaaagcag     420
```

agcttgacaa gccccagtac tgtcatggtc aagatcacca acactactac tgctgtgctc 480
acaaatatga agaccacggc agaggaggag tcgaaagcct gaggagtggc agaggaagtc 540
gtagaattaa acttggaaat cacgctccct gatggggtga tagtggcctt tgactcggct 600
tgaagggaca tttgaagggt agacatcgtg ctctgtgatc cccatcgagg aatctcagga 660
atagatgtta ctgaattgtc ttgttcaggg acaagtggtg tctctcccag cttctcgtcg 720
accctgattg gccatgttct ctgcggcacg gggctggttg cagtggccgg cgggcgcctg 780
gtgggcaccc cggtcccccc aagggtcggc tgtccttccc cactggtcac acaagagcgg 840
ccgtccttcc ccagctcgaa gcccgtagca cattcgcagg caaagcctcc caagtcgtct 900
aggcagttag ggagctctgc gcatttgcca gcacggaggt acctcccggg gcagggacac 960
aacacatcgc ccgagagttt gtcccagcga gcgccgattt cgtccgcgat gcaagtaact 1020
gagatcggga gctgtccccg gcagagcgca ctcacctcgg tcccaggtgg actgaagtcc 1080
agagcggcgc tgtgcagctg gaagggcgcg cgatagctca agttagaggc ggccccgggg 1140
cgcggcgcag gacacaagac ctcaaactgg tacttgcaca ggtagccgtt ggcgcgcagg 1200
tggcatcgca tctccttcca gcctgcgggc tcgaccccac cggtggcctg gagtaccgcg 1260
catctccgcg cggtgcagga gcgttggggc tcctccaccc actgcagcgt gtcgctttcg 1320
agaccgccgg ggtcggagga cagccaggag aaaccccgca aaggctcgtt ctccagggtg 1380
cagtgggaac gcctgcgctc cagtgcgacc cagaacagca ggtctttgga gccccctccg 1440
ggccctgggc ctgcccgcag gagcgcgagc acagcgcgca gctcggcgcc cgcacgcacg 1500
gtgctgagcg ccccacctcg caggatgcag gcctcctcgg ccgcctgccg cttcatggta 1560
gcgtggtgca ggctgtagca ggcccccgag gccgagcagc cagcacggtc ggcagtgggg 1620
tgttcgccgc cgcccggccc gggccagagc gcctgccaga ggaggcacag ggcgaacgcc 1680
ggcctcattc tctgaggccc cgcacgcaga gctgctccca acttggatct gtcccgctcg 1740
aggacgcaga gctgtgtctg gagcgcggtc accgccctct acagctggct tccccaaccc 1800
ggttccccct cccttcccct ccgccctcgc tcccgacgga cccccaact gccaagaatc 1860
cccccgcggg cccagcggcc aacagtttcc cttaggccgc gacaggaaat gtgtgcggag 1920
cgctgccagg cccggtgcag ccctgagtta ttctggggtc aggaacacag ctggcgccgg 1980
gtacagggga cgcagtcgac tagaaggtca aacaccctgc ttagagcaag aggagaaatt . 2040
cgccactggg atgaagtgtg tggtttttct ccgttcaggg agtttcatca ggaccgcaga 2100
acccataaca cgtaagcgaa cataggcagg aaggacttga gaaaggaact ggcttatgat 2160
tataagacca cgcccgtcta ttgtgtttct gtccggagct gtggagttag cgtcagaata 2220
attcgtagac ggctccagtg tttgggggc ctgtgcctgt gggggaaaaa tatgttgtga 2280
aacaacttag caactgcttt tgccaccaga ggcagcacta tttaaccgca tatctctcta 2340
tgggtctttg cttctctatt atgtcaagag atttcaggag tcctaactac taaagaattt 2400
ggagttgttt gggtatttta aatgggaaat tctgcttctt ccatcagcac ttgctttaga 2460
aatttatttt tcacttccac gtgtttctct ttgagttaga ccatattgat tcattacaga 2520
aaggaaatgt atttctagga ctaaatgaaa aataatgacc atagatctta acagttaggt 2580
tgttagcttt ccttgccact gtattggttc aaaaattctt caagtttata ggttatacat 2640
aaatgagttc gtggccttat aaatattcga gaacaactgc cctatgtgtg attttagcct 2700
tgaactattt tagtatcatt ttctcctatg attgagtggc agcatttata atagtaagtt 2760
gtaaacatct cctttaagga aaattagagt tccttaagaa attaaaatgt ttattccatt 2820
ataatgaata ttgtatactg tcaaaaaata tcttgtaatt tgaaagatgg gtgagccaat 2880
ttctttagaa ttccagtggg tgttttgttt taatataaac atttcaactc agtgggaagt 2940
ggcaaggaat taaagatgaa taaagaagta aaggttaaaa actgtactgt tatgaaagga 3000
tttcttgtta gcaacttaga caattcagta tcagtttaga aaacgtacat tctatcttgg 3060
ttcataatgg ccaaatacgt tgccatcaga aatacataga gagtttcagc taagccctct 3120
tagagatgtc tgatgctttt aaatatcttt aatcagaatg aaagttgcca ttccatggat 3180
aatgcatata agctcacttt gttttactgc attcaggttg atgaggggtg ggagggaagg 3240
tttcaggatc acatctgtat gcttccatct tatttttacc ataactctca ttcttctttc 3300
ctcctggaaa ttccatgtat cttaacaaac aacagtaagc ctatgagtca tcatcatact 3360
taccttgctt ttgctgagta tggagctttg cactaggatt tctctcctgc cctatttccc 3420
aatttaaaga acattcctta gtccttcctt cagatgtctg agaatcacct taaacgtggc 3480
tttgcgttta aattaggtaa cttagtgtct tataagaaag aaacatgaaa atccagaaat 3540
caaattgaat ggtttttaaa aaatacttag tcacttgggc cagctctcta tagaccattg 3600
caatcttatg actgtgtttg taaatgtgtc catgtagtgc aatattttta ataaaatctt 3660
tttcaaactt aagtagtcaa tggaaatttc ttagatagct ttaaaaatcg tttgggatga 3720
gatgaggtgt ctacagtaag aaagaatgaa tagctggaaa gcttaagtaa atacatgatc 3780
tcactccctg acattctgtc taggggagtg ggattatagc agagaacaca ttggccataa 3840
cgactatatg agagtactga ccatgaccat gaccatatca tggaatgaca caagagttag 3900
aaaaaattag ctcaaagtag gacgtaaata gttttgtaat tagtttgaaa tagacagttt 3960
atctgaccaa attagaaatg gaggaaagtt tctatttttt aataatattt attaagataa 4020
taatatagaa ccattttacc cccactggat tccaaatgcc taaaaatctt ataataccaa 4080
atgtgaatct attggctctt ttacatgtta cagattagag caaccacttt ggaaaaattt 4140
gacactttttt ttcactaaaa ttgaatattt gcaaactcta gcacttccat gataaggtat 4200

aaaactaaga gaaatttttg cacatgtaca acagaagata tgtaaaaaat gtttatagtg 4260
acactattca tattaacaaa acgttgaatc aatcaaaata tccaaaaaca gtataaaaat 4320
aaac 4324

<210> 8
<211> 5683
<212> DNA
<213> Homo Sapiens

<400> 8

ataatgctaa atgtaaattc tttgttttgt tcaatgttct tattttatgg ttggcaaaca 60
aaaacaacaa aacagcattg acaacaacaa cagaaacaac caactgaagc tgcagctttt 120
aatctttcta aagacaatag gctaaagtga ggtcctcata ataagatgct agtcaccagg 180
aaggaagcca gggaaaatct cctcccaggg ggtaattgat ccctattggc ctaacactgc 240
atgtagcact gagttaggag gctccatgga gaagtgtctg cttcttttcc tctgctgtca 300
gaacaaagtt cacaaacaac tttgttttcc ccacaccttt ggagttagct agttagggcc 360
cctatttatg tccaggggac cagacctgga catttgtaaa agaactgaat atagatggat 420
agctaagaaa aaaacaaatg ttaagattat cattatttat agcccaatga tttagtaatc 480
taaaaaaatc caagaacaac aaaataatcc ctaagaagga caagaatttt aaaacatgca 540
gtttagaaaa ccaaatttct caggcctaga gctcaagtaa aagaaaatct gagataatga 600
agtctggaac actgggcctg cattctgctc ctgactctgt gcccttgggt aagacattga 660
acttccccac cccagattca tcctacaccc taaggagggt gtaggatttt gtagccttcc 720
tagtgatggc atactattaa tcagaacctt aaccagctct attctcacta tcccagtaaa 780
atgatgttaa aaattagacc tcgtgacatg agagaagagg ggaaattaat tacagattac 840
aaagaaattg tgggatctaa gtttagggtg aggtgtttta ttgaacctct agggttggtc 900
atagcacctg ccatatagca ggtattcaat aaatgatgtg taatgggtgg ttagactgat 960
ggaaaggcag gccttcccag tgacggtaaa ggacctgggg caaaagagac ctgtggttga 1020
gtcctgatcc ccacacatta gcacactgac tagacgaagt tataagcttc aatttcttcc 1080
tgtgtaaaat ggagataata cctcataggg ttatgaggat taaataagaa aatgcttagt 1140
tcagtgccta atacacagga agcacaccgt aaatattagt tattattatt actaatactg 1200
tgatcatatc ttccatccaa agacttctct gaggagcagg gggcatccag agataaaaag 1260
cctgcaggtg ggaaatctgt tagccttcta gggcgttagg tttctgtgga actctgcacc 1320
tcttccctgt cctagttctt aagaacagaa actctccagg gacctctggt gaggtagccg 1380
tgggaagatg aggtgcagaa gtaagctggg acctgtgagc ctcaatttcg gcctcttctg 1440
cgctgagacc caagcggatc ttgcttggcc tgtatgcgtt actgggggaa atggacgtgg 1500
gcctgagcgc ggcaggtgcg agggcgctgc cccgggccg accaccctgc ggggacactg 1560
tagctgtcat tccttcttct gcaggcgggt aggggaagcg gtggccaaag tgggagtcga 1620
ccgctcagca cagtctgtct gagtgttgac caggaaagtc caggctcttt ctaaatctcg 1680
ccgccagacc tggtgacgca ttcgcatgta tttaaggcgt ttgcacgcag aacgttatca 1740
cagaatgtag ccacctttct taacggtccg ggaaaccaga ggtctctcca gctactcagg 1800
gtagaggaat ttctcctatc tccatgtgac atcttctgat ttagaagaac taatgttaga 1860
tttctcttgg gcctttccac ctacagctat agtcttccct ttgtttagct aaaattgagg 1920
caggtaggaa aatattattg gggcataag cctattagtg tgtaaacgta tttttatgaa 1980
gtgtgcctcc agggagccat taaaaactga cctctcaacc acagaaatag atgagatttt 2040
gagaacattg agaagctgcc ttttgcaaag taaatttgca atggtccttg acgaaggggg 2100
gtcgggggcg gggagaagtc cagccgagag aggagctcat tccacgctat atttttgcag 2160
ttgaaaagct gcctaatcat cgctaaccgc ttcccgcata agagttctgg gaagacttca 2220
gaaacaaggc aaatgaagac ttttcactgc ctccttcggg ctgtcgcttc cggaagccga 2280
agtcctagca cgcagagcag caggagaggg ttactttcag gcaattccac tgagcaaaat 2340
aaatcactta atggcataac gttctggctt aaaaaattgg aatttatcag aggcaaaaat 2400
atccttcaag aaactatgga cactccgcgc cctattcatt tccatggcag cagagtatct 2460
gcatcttgag ccacctatac agattcatgc ctcgtatcgc tctcacctcc tttcttttg 2520
aagtaaagcc ctttcccaag aaggcggcca gaaagtggac cccaccgggg gaaaaagaaa 2580
aatgaaacgc aaatcagctt ggcactgctt gcgtcttcca aaacgcggtg ggacaaggct 2640
attgagtcta tagctaattc tttcatgtat ataaaatgta tacatatgta tatattttta 2700
tatacataaa agaattcata tatatgtata tagctatgtg gagccctgaa gcaattctcc 2760
atgcttttgt ctccctcaag ttccccaggt ggaggcagtc ataagcatta taagccgcct 2820
tagtgaccac cagggacgga aaccgttaat tatcacgttt cctttcatct ccagggggccc 2880
tttggcccgt gacacaagag gcttcggtat tggcgctttc ccagaactgg cccagaggag 2940
ccagttcaga gtgtgaggtc gggtctgcat tgaacgtaca caccgaggtc tatcagactc 3000
ccccgatttt agcgaagggt gctgactgct gtgctgctag aggctagcaa gctccctgtg 3060
cgcagctgat gagtttcagc aactcgccac ctgggcgctt ttctttaaat tttgggagta 3120

aactgggaaa ataaaaaaat ctccacgtcc actggctctc tccccttctc caacttcctc 3180
tttcgactcg tttgtgggag ttttctcctc tttgctggga ctataatgtg atgcgcaatc 3240
gtttgtgaat gaacaaaagt caccggcaag cagggagacg gggacagatc gctgacggca 3300
gattgagggt ggcagcaaag gcccggcctc caaggataat ggggagccgt tttccctcac 3360
gcctggtctc tatggccccc ttcgtcttcc aggtaaaatg aatgttcctt catccatcat 3420
ccgcagagta ccctcaggcg tgcgtagaat ctgctgatga aacctattag cgccgactgg 3480
gcagctttgt ggagccaccc gaggctctcc attgtggcct ttgtctgcag aatttaagca 3540
tttacataat gcattagcac ggaactcagc acccggtggg gacatcgcgt gccaagcctg 3600
gcgcggccaa cgcttcagcg gctccctcac ccggcagctc cctaggacca ccctcgagga 3660
ggcattggag tcgggctgca ggcgcacggg caaagaactt agcatctcat ccaagtactt 3720
cgccttcctt ggccgtctcc gggaggttat gcttaaaaac ataaaaataa aaataaaaat 3780
aaaaataaag ggaggcggac aaagtttcgg tgggtgaact gaagctgggt ccatgtgacc 3840
ctgaagccgg agaaataaac ttaacatgaa tcttgctttc ctggcgggcg ttgggacccc 3900
gccgtttttc atgccaaccg ttggaagctt cgtactcaac ggccacaggt gcctaggagc 3960
gcagagaggc ctcgggttca aatcaccggc gcgcagggac tggactcgcg ggtagcgacc 4020
cccccaaccc cccccccccc gccctacaca cacaccctcg cgccggctga aagcatggag 4080
gattcagggc atttgaaaaa agaggggctg ggcgcggtgg ctcacgcctg taatcccagc 4140
ggtttgggag gtccagaagg gcggatcact tgaggtcagg agttcgagac cagcctggcc 4200
aacaccagcc tggccaacat ggtgagaccc cgtgtctact aaaaatacaa aaattagcca 4260
ggcgtggtgc ctgtaatatc agctacttgg gaggctgagg cacgagaatc gcttgaacct 4320
gggaggcgga ggttgcagtg agcccagatc gcgccaccgc cctcctgctc tgggtgatag 4380
agcaaggcac tgtctcaaaa caaaacaaaa caaaacgaaa gattcggtca ggaaagaatc 4440
tgcaggcatt cgaggcgctc gcactttgca aagtaaatgc aatctcttta ttaagccgaa 4500
gtccctcata tctatccttt tagaggaagg tggtccaact cagaaatctc tcccaagagg 4560
actttccacc gaagactacc gcgaagtgcc aggaactcgc cccagtcccg acaggtgcag 4620
gacctttcgt gccgccacac cttgggactc tacctcccta aataggccac ttaaaagcca 4680
gtagtgcaac cgggatcccg cggcgataaa gaatcactgt gcagaaccct ggagctggga 4740
gtcggcccgc cccccctccca aagaaaccgg gatcccgcgt cctccccgcc gctagcgcag 4800
cgcgccagcg gcgcccaatc agtgagcttt ccgggtctgt gacggccttc ggctccgccc 4860
cctcgacggc cataaaaagt cgcagcgaag cctgcacctc cgagcaccgc gcgcggccct 4920
gcccccggca cggcccccag gtgcgctcct tctccggctg cttgtagcac tggtctcact 4980
gtccccgccg tcagccaccg gttccttatc cgtctcattc cccattgtgg cttggctgag 5040
ccggtcgcca ggcctcgctg tcctcctttg ccttcctctc tcctcagcgg ccgtactttg 5100
cgccgtacct cacctggcct gcaggtgagc agcagcgcag cacccctgcc cggcgagctt 5160
aacttgccca gcccggcccc tgccggagtg gcaccggcac ctctccaaga cgccctcttc 5220
cctgcaggat gaagaacccc atgctggagg tggtgtcttt actactggag aagctgctcc 5280
tcatctccaa cttcacgctc tttagttcgg gcgccgcggg cgaagacaaa gggaggaaca 5340
gtttttatga aaccagctct ttccaccgag gcgacgtgct ggaggtgccc cggacccacc 5400
tgacccacta tggcatctac ctaggagaca accgtgttgc ccacatgatg cccgacatcc 5460
tgttggccct gacagacgac atggggcgca cgcagaaggt ggtctccaac aagcgtctca 5520
tcctgggcgt tattgtcaaa gtggccagca tccgcgtgga cacagtggag gacttcgcct 5580
acggagctaa catcctggtc aatcacctgg acgagtccct ccagaaaaag gcactgctca 5640
acgaggaggt ggcgcggagg gctgaaaagc tgctgggctt tac 5683

<210> 9
<211> 2347
<212> DNA
<213> Homo Sapiens

<400> 9

```
ctgaggctta agccagggag gtcgagactg cagtgagctc taattgcgcc actgcactcc 60
agcctgggcc acagagcaag accctgctca aaaaataaca agaaaggtgg aggaaatagg 120
tagagatcag aggaaagtca tggccccctc ctgaccatcc tgagaccctc ccaactccac 180
caattcctgg agagtccctc acttgcctta tttcacagat tgggaaactg agtccagggg 240
cactcagacc acctgaggcc ccagctggag atgaagagat ggattcaaac caccccttgt 300
ggagtatagt tttgcccctt ccagagtgga gaccgtgcct cagggcttcc cccaccccgca 360
ccaaggttgg ccctctcagg cctcatcttt cccacctgca aaatgggctt ttgtgagtat 420
gaaggagggc ttggcttgca gtgagcgctg tccacccgct ggccagtgtg attagtcatt 480
gttgttgttc cccagtggcc tctgcctggc agctgctgga aaaacatcga gggagggaga 540
tacagggctg gggaggtgaa aagctggaca cagacgcccc tagccccttg ggatcaggaa 600
cagatggagg ccgggtgccc tgaagtggag cagggaggac tgcctggagg agggacactt 660
gagctagaat gtatagaatt acagggcatc tccggcagag gccacagcag aggcaaaagt 720

ccagaggagg tgctttgggg accaccaaga tggacacagt gggtctgggt ttctgtgggg 780
tgatgggctg gggcctcgag cgccaggcta aagggcctgg ctttctccga gggcggtagg 840
gagccatggc aggtgtgtga gctggagggg taccccagac aaaggtgaag tggtatttct 900
ccccttattg aagtcacaac ctgacactcc ctctgaagtt ccccacatcc caccttcccc 960
ttgccacatc ttggcaagcc cacctccttc cctccgtctc taggcctgcc aagaggccct 1020
gtgggcccct tatggcccca cccagctgcc ccagaagctc gtctgagata agttcccagg 1080
gctgccccac tttcaggtgc cccacagact cgggggcggg tgaagcagga aggatcgagt 1140
cacgagggcc ccagaaaaca tgacgggagg aaccacagca cttccccacc tcctcagctt 1200
ccgccttctc agggagagag accttcagcc cgaggaggag ggccgagctt ccctccatgt 1260
ggcaagagac cctaacgtcg ctgaggatca tctaaagagg gtgttgaagg atgcatagga 1320
gttagccagg agacaaccca tttgttaatt taacaaactc ataggcatca ctcatcaaca 1380
agaacaattt tcatcattca atcaggactt tctaatgcct ctgagggatc atttgaggat 1440
gtttaattat tatttttatt tgagacaggg tctcactctg ttacccaggc tggagtgcag 1500
tgacacaatc atagctcact gcagcctcaa cctcccagtt tcaagctatc cttctacctc 1560
agcctctgat atagctgaga cacaggccaa tgccaccatg atgcctggct ttcttttttt 1620
tttttttttt ttttactttt gagacagagt cttgctcagt cacccaggct ggagtgcagt 1680
ggtgcgattt cagctcacta taacctccgc ctcctgagtt taagcaattc tcctgcctca 1740
gcctcctgag tagctgggat tacagacatg caccaccaca cccagctaat ttttgtattt 1800
gtagtagaga tggggtttca ccatgttgtc caggctggtc tcaaatgcct gaccttaagt 1860
gatctgctgg cctcggcctc ccaaagggct gggattaccg gcgtaagcca ccgcccctcg 1920
accatccagc tttttttttt tttttttcag tttttggtag aaatgtggtc tcatgcctgt 1980
atcccagcac tttgggaagg ccgaggtggg cagatcactt gaggtcagga attcgagact 2040
agcctgacca acatggagaa accccgtctc tactaaaaat acaaaattag ccgggcatgg 2100
tggctcctgt ctgtaatccc agctacttgg gaggctgagg caagagaatc acttgaacct 2160
ggtaagcggg gtttgcggtg agccaagatc gtgccattgc actccagcct gggcaacaag 2220
agtgaaattc tgtctcaaaa aaataaaata aaataaaaat acaaaattag ccaggcatgg 2280
tggcacgcgc ctgtaatcct agctactcag gaggctgagg caggagaatc gcttgaacct 2340
ggtaggc 2347
```

<210> 10
<211> 6709
<212> DNA
<213> Homo Sapiens

<400> 10

```
actcttaaac tcctcaactc acagagggtt ggagagctgg caccatgagc ttccagttcc     60
tacgggtgag aaaagaggtg tctgtgtgga gggcaggtgc gaccccgggg gctagcagag    120
aggagaggct gcgggaggac tcgctgcgaa gggcgagggg tggcgctggg tgggacgggc    180
gcctggggcc gtgcagggtg gctcctgggt gctgcccggg ctgcctgtcg cccagtactg    240
gcgcaggaag acgggtccgc gcagcgtctg gcaacagtgg cctgctccac gcctggaggc    300
gaccaagttc tgagttaaag gcgccgggct tagccgggaa gtgtatggcg acccagaaca    360
cggagcggag aaggcctcag ggacacgag gctaccgcct tagtcctcgg gcctgcgctc     420
cgagcggtta gggtgcgtac ggatgggctc cgggatgtta gtggagaggt gacaggagtc    480
taccatccca cggccaagcg tgtgaggcct ccggcgggcg ggggtccccc ccgggggcagg   540
gcagggggag agtcaggcat gtctctaacg ggctccctag tagaaccgct aggaaagtgg    600
ccttcaaccc taggcacctt tgtggctttc aggtagttgg gcgactaaaa attcactttt    660
gcctgctgtg aaatgggaca ggtggttgtc aggttagaga ctgtgcccac gaagtctcgt    720
ttggcgggcc gggactgacg tggacccggg ccctcctcgg aagcttggga acctgtgagc    780
ccctcagctc ctcgcctcac tttgcctgtt tgaaaaaagg agttgtttga gaggaagagt    840
taaggctcaa aaggttctaa acgaagagca tgcggcctga cgcggagcgc aaagaagcga    900
gcagtgaact gaatctggtg ggactgagac cagaggcgtc tgtatgcggc ggccgcttcg    960
cgccctatta aagcaatctg ttatttacga taatcatttt tgtaattatt ttggagtggc   1020
tgtgacttga tgtctgtggt gccccagggc atgcccttac tggacataga tacttcaggg   1080
tgccctttga cgccagtgtc ggtccagtgg gtgccagtgc cgcgaaggga gggtggcggg   1140
cgtgaatgcc ccctcctcct gggtttgccg caggccccgg cctttccacc ttgcttgatc   1200
ctggcgggtc tgccacttac tgaggtccct accggcgctc acgttgtccc gagaggcggg   1260
acaaacgtcc ggcgcgtggc gcggagtctg cgaaagcgct cggggggcctc catcttctgc  1320
tcgggagtcc cctccctggg gcgggaagag gagtcgcaga tctgcccgaa tcttgctcta   1380
cccgcctggc cgccttcttc cgtctggggt gggggcaggg gtggcggatg actcaggcct   1440
ggggccggga gccctgtttc caaaggcacc acctcgcgcc tctttggaag tggcgcgctg   1500
ttggggcgct cccgctgttg cagaggcctg gtttgcggtt tcataggggc gtgggtccag   1560
ggcttccagg gagtaagtga gtttaggctc cctagggggc ggtagagggg aacccccgac   1620
```

aaccttccat cctcttctcc aggcggctgc gaggcctccg gggttccaga ggggtcccct 1680
tctgccggtg ccggctgtct tgagcgcggc cacgcagtcc gcgtcttcac gtgggcaagg 1740
ggcggggacg ttggagggag gctgtcttgg atgctggagc cttcggttcc cggctctgct 1800
gagcagaaaa gttgaagtca aggaagggtt gaggcttcct cacaagggcg cgcgcgtgcc 1860
tgggtgccgg cccagaagcc gagaatgtgc gtattgggga agggtctccc gtgccgagaa 1920
gacagaggat ttgttctagc tgccttccgt acagagggcg cggaggttgc gctccagttc 1980
gaacgcttac ccattggaaa gagggcagcg ccggggtcca gggaagctcc ttgggaatga 2040
atggcctttg ccaagcggtt ccggatcctc tgggtccttt gggcccacgg cacggtgctg 2100
cgcgagccct cagtgcccat cggctccctt cgcctcctgc gtagacgctc ccaggcgggg 2160
aggcatatcg gttcctccgg gcagctttgg ctagtgttgc tgtgggaaag gagagccagg 2220
gcctgggatg gggatgagc accttcttgc ccattccggg ccccagcgtg caggaggtaa 2280
acttgccagc acagacaaga cagcttgttc aagctgcacc tcaggccggg tcagagaata 2340
aaaccgaggg ctagaaggcc cagaatgtcg gacagcccag cggcacccgt cagggagtcc 2400
caggcgcccg aaagaggcgc cgcacctctg gcgagtctag gacccatctt cctggacctg 2460
tgctctggag tgcctgcggg cctgggtcta atttctgctt ctggcaggtg tccccctccc 2520
ccgccgctaa taccaggagc gctgcttctg cggtaactta ttttaccccc agaagcctgt 2580
tttgggaccg aagtgtcagg gtcctgtgtg tctttttatg cactgttctc cttagtgcaa 2640
agccctgcag atatgcttgg ccgaaaagtc tcagtggttt caacttccag attttgttcc 2700
cgcgtccggt cggaaaatca cctgggattt ggctgctgtc taaggccggg ggaaagtttc 2760
ccttggagga cgcctattat tattattttc ttttaggccc acctgggtct aaataaatgc 2820
taaagttcaa acgaacccga gaaggaacag cgaggcagtg gatgccgcca ttccgtggat 2880
gggagagtca tgtctactgc aggtgcggac accgtactga taggcattga ttattgcaaa 2940
atcgtattca gtatcaaata gaaatagtct tgtcctctaa tccccacaga atgtttaaat 3000
ccagcccagg gagccagttg gggcgtccca acaaatacgc cctcgagaat tagcgatgtt 3060
ccccttacaa tgaaagtcaa ttgctgcata ataactgggc caaagaattt cgttagttta 3120
aattttaaaa attagtattg cactttgctt tcaagtttgg gtggcaagta ccgaagggga 3180
gagggaagca gcgtttgtgt ggaaggaagt ttcgttttct gtctaaacat gtgggacctg 3240
ggtgtctacc tctccagtcg agagctctct gcaggctctc tcccaacttc ctgcacctcc 3300
tcaccctcac cttacccgtg gcgctcgggt tttattcctg cccggcgctt ctttcccagg 3360
gcgagtagag gcttctgagt gaccggcccg ccagaggcag ctgcagagcc ggcgttccgc 3420
agggcagggc agggccgggc cgctgggctg gtccagtgcg cgcgggttcc ccctggcctc 3480
agccagccgc gggagatgcc acttcgggcg acagcggcga gacgccgctg tccgcaactc 3540
ctgggcgggt aggaccgtct gctgcctctg cggcccttgg ggcagactcc ccaggagtcc 3600
cttcctctct cctcccgccc gggggccggg accgcgctgt gcccacggag ggaactgggc 3660
gtctctggcg cactggggct ccagctgcat gatcccagct cagaccttaa gcctcagtgg 3720
ggtgtaggtg ggatgggatg tgtttttagg agatgaggag accccggttg ttgagtcgct 3780
ccccaaatgc gcagtttctt aactccaggt gacatccgtc ttttggagga acgagtgaag 3840
gccgtttagg cgaaagaggg gtgggtttca gtatttggat cccttccccc agctctgcca 3900
atctcaggtg tgtttacggt ggaggtgaca gaaggaaggc gcgtcggggg acccaggcct 3960
cgcaggtctt cggctgtcaa cgaggcaccg cccactgact ccgcgcttcg tccctcacag 4020
cgtggggctc ggcgctgcgc caggcccggg tgcggggcgg agctaggctg ggactggctg 4080
ggggccgccc cgcccggcgc ctgggcctcc gcgccggccc ccggggagga gttatgataa 4140
tttccttctc attaaggcgc tcgggtcccc cggctatcgc caggacacac tgttcggcg 4200
cggctttccc cgtccgcgga gcggtcttga cactcgcggc ggcagcatct acgctcgcag 4260
agccgccgat gcgtgtccag tgacccggac agcaaggccc gcgcgcggcg gggcggcgg 4320
cagacgcctg gtcaccgtga ccccgatttt ggatttaccg cttgggggct gggggatcc 4380
tggatttaac tggcgactgt tttgggggac gccggacgcc atgttgtgga aaataaccga 4440
taatgtcaag tacgaagagg actgcgaggt gagctggggc tccggggtgc agccccgccc 4500
cgccgaggac agtccgggag gcaggggcca ctggaccgag gtcggggacg agggcatagg 4560
agccctggcc tctcggtggg acgggatcca cttctccgga cacccottag tccatttctg 4620
cggggcccct ttcctgtata gggccttttc ctaaatagcc tcccctcggg aacgaggcct 4680
ggaaagagaa tggcaaatcc cacccacaca gcttaccggg cgctttccta agcggcaaac 4740
agccctgctt cccgttttct cggaaaagtg ccccgtctgc aaccctcaga cgtggctttt 4800
acgcacgaag tctctgtcca aaggggtcag atgaggctac ctgctagcga caccttggcg 4860
gacactcctc caaggccggc ttgtcacctg ccccgctacc tcctcgggga agcacgaaaa 4920
caaccgaagt ttggctttca aaagaagtgg gggctgggga gtggtggtgg gggtgcccga 4980
ggccagatgg ggccgtaaga gctcgcgagt gctccagcct gtcggacagg ttgagctaga 5040
gtttcggaga gtgggaggga agaaggaggc ggcgagcggg aagaggaaga agacgcaggc 5100
agcgctaggc gagctcaggg gcgccgggag cgcggagctc gggctacgga ctcgcgggag 5160
ttcactgcgc ctccgggccc tggagggctg cccctgcccg caggcccggg cgcttccgcc 5220
aggaggcgac agcgccatgt tcctccaggt tcccggcgcc ccgagacccc tgggcagatg 5280
gggacgcatc ctttagaaac tgagtcgggc cgcccagggg cgagggaacg tgcgcggca 5340
aggctgccca atttccaggg ttcttcatgc cccctctgcg ccccgacgtg cgagaacact 5400

gcccttggca gtgcagggcg cccccactta ttactcccct cggctgggcc cggccagcag 5460
ggagggccgc cctgtgcgcg cgctccctct tcacttccca gggcggcgca gggtggcggg 5520
ccctgctttc cgagcgccgc ccgctcggag gtctttgtcc cgagggcgtg gaagggaggg 5580
tcccgggggc gggggaggtg cgcatttccc gcgccgcgca ctctatccgc gcctgccgcg 5640
ctgccacctc cagcagtccc tgcgtcatgg gcgggctcca cgagatagct ctgcaccggg 5700
cgtccggctc cttcgccccg ggctctggct ccttcgcccc gggctccggc cacggacttt 5760
tctggcccaa gacccagggt tcggacttgg cgcctccaag cgcctcgggc ttgggagcag 5820
cgcctagacc ttcgccgccg ggctttgaga actcgttccc ccaggtcttt caccagactc 5880
tcctccctcc ccgcactctt tgcttacaac gaaatcctcg ggcgcattg cccccgggta 5940
ccttccgacc ctgggcaagc cccgccgggc ggggtgcggt tggtcccccg gggccctctg 6000
cgtagcccgg cgatgccggc cagttcgcag tagcggggtt tcgcactaac ggggtctcct 6060
gttttttttt ttccctccag gatcgccacg acgggagcag caatgggaat ccgcgggtcc 6120
cccacctctc ctccgccggg cagcacctct acagccccgc gccacccctc tcccacactg 6180
gagtcgccga atatcagccg ccaccctact ttccccctcc ctaccagcag ctggcctact 6240
cccagtcggc cgacccctac tcgcatctgg gggaagcgta cgccgccgcc atcaaccccc 6300
tgcaccagcc ggcgcccaca ggcagccagc agcaggcctg gcccggccgc cagagccagg 6360
agggagcggg gctgccctcg caccacgggc gcccggccgg cctactgccc cacctctccg 6420
ggctggaggc gggcgcggtg agcgcccgca gggatgccta ccgccgctcc gacctgctgc 6480
tgccccacgc acacgccctg gatgccgcgg gcctggccga gaacctgggg ctccacgaca 6540
tgcctcacca gatggacgag gtgcaggtga gcggcgctgc ggctcctgac cggacctgtt 6600
caccctacgg ccttctgccc ccaccccgca ctcctctagg ctcccccgaa cttaagggaa 6660
ttttgtcctc tctcccccag aatgtcgacg accagcacct gttgctgca 6709

<210> 11
<211> 2296
<212> DNA
<213> Homo Sapiens

<400> 11

```
gtcactgcac cctgccaaca tttttcaaaa gtaaaaaata aattaaaaaa aaagtcttta      60
gtggattgct gagtcccctc tgacaggagg taccaaatgt gcttttcctg aggacagggg     120
aagagttcac tattcagcca tacctttgca tattttacaa aattcacttc taccaaggat     180
gcctctccac aaaatgagga gtagcacaaa gaacacctaa gaaaccaggc ctgacccagg     240
gcctctgatg ctggggcagg ttccccgcgt gctaatatcc caggggaccc tgttgcagtt     300
ctctgaatac cggctgtgag ctttccaact tcagctgagt cccagcatcc tcctcacacc     360
acttatcctc cttgcctgtc cctggcgcgt ttagacaact ggaatctttg ctgacatttt     420
aaatgggctg gctacttctc cagcgtggag gcacctagca caaggtgtga tagatcagta     480
tttgttgatg agtaacaaag ttattctggc aaattggact tttcccctga aacgttttgg     540
cttttacatt tttaatgatg ggtcaagaaa cagcccccaa gaagaagggg cggagatagg     600
gggtaggacc ctgcctgaag cttccaagct gactctgttt gcagtcactt aattcactag     660
ggagggctgg atctgcctcc ctgtggtgga ctctccgcat ctccagaaag tgcaggggtg     720
gagaattaac tcagcccccc tcctccccat aagagccccg ccaggtcagc ctgcatgagg     780
agggcacgtg cacacctcca aaaggactgg tgcagccact tccctaagct cacagtccca     840
gtgtgagctc agggcccaga ctcggggcct ctggtgtttc aggcgtgagc caagtcccct     900
gagcaagagg cctcatgata aatcagttgg agcctggggg ccatgaaagc attggttatg     960
gcagaaacta ccagctcgtc accgtttatg ctgctttgct taaaaatgac accgcgtaag    1020
ggattttgcc aattcccaac ctcgggagat cacccaccac ctggctgtgg tccgccgctg    1080
aacaggaaag cactccgaga tgccaagaga ctaaaaggct cgtaactgtt gtttagaaat    1140
gaaaatggcc ctaaattata accagagatg gaagcgcgtc tcaggggagg gcggggcgct    1200
gtgtaatcga gcctcccatg cgcaaggcgg atacaaacgg tttatggatg tgggcgaagg    1260
ttctcagagc aaagaatgtc catttccatc agattaaaat acagtttggg aaaacaatta    1320
cctgcctgag atcctgggaa agtaaaactt cctggtttct ttgccaggaa gaacgattgt    1380
aaaacaaggg ctgagaatct cccaaacaag attttagaat tttatccaaa cagctgcagt    1440
tcacctttga ggagccccag actcacttgg gtctgatgga tgttgcaaca ccaagtttga    1500
ggtctcccca gcctggtgcc aagagtgggc cccaggggac aaacagagaa cagttgctct    1560
cttaatcgga gtagaaaaca aaactccctc cccttcccac ccccaacctc cctgctttgt    1620
tgtgaaattg tcactgcgcc tttgaggagt gattaaaatc ccagccatga ccaatttaaa    1680
gggcaggttc ccaggcaggg aagggaaagg ggttcaaggc aaaaaaaaaa aaaaaaagtc    1740
agttgaagtg agtttatgga ggatttgggg atcacaaaag tcatgggact tgagtcaatt    1800
aaaattaatg gaaaagcacc aacatgcaat ttaaaaaata ataataaaat aaagcagtaa    1860
ggatatttct ggcttgctct ctgcttccat tcttgagcct gcttgggaat cttggagcag    1920
acttcctgaa gcacagcatg agtgcccaat ggctgtttta acctattttc ctgcccttca    1980

cacacaaaga ttctgcaatg gagagtggga atgaatggtc cacccacccc tgaacatttc    2040
ctgtgagttg ggaatgcgct cagtaaggct gctggagtgg agggaaaaga aaaggtgtct    2100
gagttctcta gacatgggca gaaaaggaga gaatttgaga caggctgaga gaaagctgct    2160
ccggttggca gcattgccag attctcagac acaccgtaca ttccagcccc cgaaaaggcc    2220
atcctgagtc cctctagggc ccctggggct ctcatctcat cccaaatatt tggaactgtc    2280
tgtgcctgct ataata                                                    2296
```

<210> 12

<211> 4247

<212> DNA

<213> Homo Sapiens

<400> 12

```
ctccaaaaaa actaaaacac aaaacaacaa taattactaa aatcctacc tccgctcctc 60
taaataaaaa acctattcca aaaactccaa tctaaaaacc taaaaacgaa aaacgcgcac 120
tacaattccc aaaaatcccc gaactcaaaa acgatccccg ccccccgcct tctccgcgcg 180
acgaaaccga acttaccaat aaaaacccga aataaaacga acaaaaacga aacgaaacga 240
aactacgaaa aaataaccga cgaaccgcgc cgcgaaccaa tatctcaaaa aacgcgaaat 300
ccgaacaacc gcgcgctaaa aatctcgaaa cgaacgccgc gaaacctctc cgaaccataa 360
ataaacgact ctacgacgcg acccgaacaa aaactcgacg accctacaaa aaaattcaaa 420
ataccgacgc cgaatcccct ccaaccctcg taacaatcga tacgctatat aaaacgaaaa 480
aaaacccgaa ataaatttcg aaacgcgaac caaaaaaaac gaacccgacc cgcgacttca 540
acgctccccg accgacgcga aaaaaataaa actaaacgtc cgcgcgaact tcgaaaaaaa 600
aaaaaacaaa aaattttaac gaccaaaaac ttcccgaacg tttatctcga aactcttttc 660
gccgtctctt acctaaaata taccgaaccc gatacgactc tatcgtccca cttcctacta 720
cgatttccac ctcgaactaa actcaactaa tttattataa caactatcga caaccgcccc 780
tccgccccgt aacatcccgc aaatttcaaa accgcgacgc cgccgaacac aaactccgaa 840
tccaaaacgc gcgacccttt cctctacgac cgccgtttcc taaaaaaaac tacgcgaata 900
taaaaaactt actcaatacg ctttataaaa tccactattc ctctttcgac cgcgaactac 960
gccgataatc cgaaaaaccc gacttctcgc cccctacccc ttccttctct tcccttttct 1020
cttttcctct cctatctctc cgcccacccg ccgcaacccc acaaaaaaaa aatcgcttcc 1080
ctaattactc tcctctccta tcccttatat atccccttat cttccctcga tctacaaaaa 1140
aaacgaaata acgactaaaa aacgtcaaaa aaaaaaaaca aaaacgtaaa ctacgaactt 1200
acaaacgata cccgacttcg cgctcgacga aaactacgaa ccaacccgaa cccgcgctcc 1260
gtccctccgc gatatctaaa aatcgcccga actaaaatcg cgtcaccctc cgcccaacgc 1320
cgacgtaata aaacttacgc gatacaaaac ccccgaaaac ccaaaaaatc ccaaaacgct 1380
acgaatccaa tatcccttta tttacaaaat cttaaaccaa taaaaaatac tccgcgaacc 1440
ccaaaaaaaa acaacaaata cctcttaccc cacccgaact cctctaataa aaacccacac 1500
gcacgataac gtcactacgc ctacaaacaa cgctaaaaca caaaaaaaat aaaaaatcga 1560
aactcgaaaa cttcaccccg caacaaaact caaaacccaa tccaaacctt aaaaacccgc 1620
ctaaaaccta aaacttaata taacaaaaat aaaaaaaacc tccgataatc tacttctaca 1680
attaaaaaac gtcaactaac acctctaaac ctctaccccg taaataaaat aaaaacacac 1740
gaaaccatct aaaaaactct cactatccgt caccaccaaa tataacccaa actccgtaaa 1800
aaaaaacaat cccatcctaa aactaaaact tttaaaacat cccaaaaccc gataaaaaaa 1860
acaaaaaaaa taaaacttaa aaattaataa cctcacattc aatatccccc gcacctcctt 1920
ccctttctat accctctaat aactcttata acctcaacta cccccaaacc ctatctctct 1980
cgccctcccc caaactaaaa tactaaaatt aaactataac ttaaactaca cctataccct 2040
attaaaaaat aaaaatccca aacccatcgc gtttcttctc actactaact ccaactcaac 2100
acttaacaaa ccccccctccc caaactacta aaacaaactc gcgtaaactc gacctaaccg 2160
ccgctcccga actaccgaat aaaccgaccg tttcgccgct tcaaaaacta tctttatttt 2220
actccaactc aaacttaaaa aaacttaaac caacaacctc aaccgaaaaa acgaaacgac 2280
gtctactaat tattattaaa actactatta ccattattat aataattaac gcaatattcc 2340
caatcgataa cttttcataa aaaaaaatat cacttccttc ccatcacacc caaaataaat 2400
aaaacgaaca acaaaaaaat aaaaaaaaaa aaaaaaaaaa aaatatatac ctccaacaaa 2460
cccttaaact taaaaactat ataaccttat actaatcact acccctctct aaacaacaaa 2520
aatctataaa tccattacgc tcatcctaca cccaaaaaac taatatttcc gaaacaccta 2580
ctaaacaacg aaatacctta aaaaaaaaac aaaacaaaca aacctctaat cttctcacta 2640
aacctaacta taatttataa aaaaaacctc ctttctcttc tcactttttt ctactaaaaa 2700
cattaaacac attaaatcta acacgcccat cttataaata aaaaaaaaaa aatacaaaac 2760
caataaccaa cttaataaaa aatacaacca tacaatacta aaacccctta ccccatctat 2820
cctcttaacc cactttcatc tacttccaac ttaatatcta acaaaacaaa aacccactaa 2880
cttccccgca tccacccact acctaacccc ataactacct tctaatccta actaaacccc 2940
```

gtaaaacatc ttaaatttat cttcaataac tctcaaccac cgcccgacta ccttcacctt 3000
cctaccttta catacctacc tttacttcgc caaattaatt aaaccctaac caaaactaac 3060
cccctactct ctcaaataac aaataacaat tttttaaata aaatctcaat aaatatcaac 3120
aaatctttcc tccttcctca aaattataaa aataacaccc cttattcaaa aataataaat 3180
cctctaaacc ctccccgacc caaaccaaaa aaattaattc ataaaatata aaaaccatac 3240
cccttcccga ccctccccct ctccccgcta ctacccaact ctctaaccaa cccccctccca 3300
tccctaaacc taaataccttt aacaatcaac acaaacctac tcttattaac aaaataacaa 3360
aaacgatata ctaaaaaact acacatctac ttaaaaaaaa aaaacaaatt tacatcctct 3420
aaacacaaat aaaaaactta aaatctccct actcccatta aaaaaaaaaa aatctctaac 3480
tctcaaaaat caacgtctct caaaaatcaa actaccctaa ttctaaatcc caactctacc 3540
ataaataaac tctataaccc caaacaaatt actaaacctc tctaaacctc aaacattata 3600
tctattatat ctaaataact aaaaaaattc aacaatacaa tattatataa aatacctaac 3660
ccacaccaac caaaacacta atttaaatcg ataaaacata taacttaaaa acaaataaac 3720
ctataaaaac atcctctact aaaaacaata acaaactaac atcttcaata ctaaaatttt 3780
aaaatctaaa aattaaactt aactcaaaca aaaactataa aattaaaaaa aaatcaaact 3840
cataacacaa aaaaaacctc aaacaatcct cctacccaac accctaccgc accccctctc 3900
tacttaataa aaaactaaat cactaaaaac ccaaactcat cttctaacgc aaccacctaa 3960
cctacctcga ccctcttccc aacaataaaa taaaaaaatc gcaaaaaaac ttttataaaa 4020
taaactacta ttatcaaaat ttttctaaca ccattttcta actacacaaa actcaaaaaa 4080
caaactaaaa ataaacctcc ccaaaaaaac acaaaaactt tttaaaaaaa aacttctaaa 4140
aactacaaaa aaaaacctaa attataaaaa cacataatcc taaaaaaaaa aatacaatac 4200
tataaaaata ttatttctcc tcaaaataat ttctaaattt aaataca 4247

<210> 13
<211> 3905
<212> DNA
<213> Artificial Sequence

<220>
<223> chemically treated genomic DNA (Homo sapiens)

<400> 13

tttaaaaaag tagattcgaa gatgtttaaa gtaataatgg tgttgttaga atggtgtttt 60
tttttttttt taggggatga ttttgaagga tattataata atagatgtgt aaattttgaa 120
atatatggtt aaaatgaggt ttattatttt ttagttatta tttagatatt ttttagttat 180
ttgtttatga tagtttataa atgagaaaga agtttattta aaatattttt atgaggattt 240
taaggtaaat aataggaatc gattatttat taatttggtt tggtttcggt ttattgaaga 300
aagttttttt ttgagtgtat ttaaagtttt tatggtttag ttttgtttaa tttgagtagt 360
attttatttg tttttttttt agacgtttcg atgttttgta ttagatttaa gagagagtta 420
agtttttaa taggttatgt acgtggttat taatatttag tatacgttat ttttggttag 480
tttttttttt ttttttaag aattaaatat tttttggttt gagtttttttt tttttatgtt 540
attataaagt tgagttattt attattaggg tttatatatg agtagttaaa atatacgggt 600
ttttatattt aggttaaggg gtttttagta ggagaatatg aataattagg agtgaggtta 660
tgagatagag gaagaaaagt ttataggaat gggaagatag tttttttagat gagaggtgtg 720
cggagtgatt gttgtgagga attgtttaat aggaaagtta gttaatttga ggacgttttt 780
aggggaggaa aagggaaatt tatatggttt tttgttaatt tttaaaaata aggtagattt 840
ttatttattt agggtgtttg ttcgttatga atataggaag attaaggaat gagtatggat 900
tgtttttatt ttatgttttc ggataagaag aataataaaa aagtattggg tagttggaat 960
aattgtagag gttttaataa tgatggagat tttttggtgt atcgtggtat aggggttttt 1020
tttaaaggga tttaattaat tttaataggt attatttttt gtgatttaag atgttttttt 1080
taggagtgtt tgtatttttt aatgtttaga gaagttcgta ttattgattc gggaatattg 1140
agtttttag tttttgtaaa attattttta ggtttatttt taagtatatt ttttatttcg 1200
gggggttaaa ttttttggtt tttagtgaat atcgtggtta tatttgtggt tatttattga 1260
ttttttattt gtttagttta tttgataata ggtaaatggg gaaagaggtt ttaatatttt 1320
ttaaaagggg aagattgata attagttatt tatcgtatag aattaggtta ggttaatttt 1380
ttttaagaag aaaagtaatt tggatattgt attttggggt tttttttat tttggtatta 1440
taggttttat gaggatgtag ttttttgta tttttttttt ttttttaaa ttgttattta 1500
tttattatt ttaattaggt taaagtatta tatattgtag aagagaaagt aattttagtt 1560
ttttttaaac ggtgttattt ttatgatagt tcgtaaattt tggtttttta tgggtttagg 1620
atttgcgcgt attcgttcgg ggatttgttg gtggatttat tttttatggc ggagtagtaa 1680
ggggattttt tagaaaagt aatgggcgaa gtaattgaaa gagcgacgta gaaagtaata 1740
gttagaaacg gcggggacgc gagcggttta gataggaagg gaggcggtgg cgtagttttg 1800

gtgcgtagcg cgtcgtagcg acggaatttt tgtaaaagtt gtttgttcgc gcgttattag 1860
cggcgcgtag gtttgtggtt ttttcgtttt cgtaattttg ttttaattgt cggtttattt 1920
ttcgataaat aggatgtttt tatttgaggt tgttagtatt ttaggttttt tgaaagaaaa 1980
gaagtagagc ggttttattt tagaggtaag gacggggttt gtgttaagag gtttttttaga 2040
gaagtgaaaa ttttgtaggt gtagtcgttg ggagagtatt aagaagggta gggtggaggg 2100
gtagggggcg aagggagggg gtgaagttcg tattttattt ttatatgaaa ttgattttat 2160
tattttattt ttgtaagcgt ttagaggtag agaggttaat atttcgggga tagtttggag 2220
gcgggagatt taggtagggt tttttaaatt tttatgtgta tgaaaattag gttaattacg 2280
gggtttttga gtaaatgggg acgatgattt agtaggtttg ggttgaggtt ttagattttg 2340
tatttttaat aagtttttag gtggtagtga tgttgttagt ttaaagatta tattgtgaat 2400
agtaaggcgt cgtcgggata gtttgtttta gagtaaaatt taatagatgt gtaaatattt 2460
aaattaagtt gtatttattt ttgaggtttt ggtttaattg gagttatttt gttaatggaa 2520
aatggaaaat gaaatttttt taggttttaa agggaaggaa agtttgaaat ggttaaggta 2580
tttaaaaaat tttagttggg agtggttttg gggattcggg agagttgaag ttaggttttg 2640
agttaagaga tgggggagga agggtgggat gtggtaaata aatataaata tatatatata 2700
aattttagtt taattatagg ttttttttta ttaatgaaat atttgtattt tgtaggtaga 2760
atttatacgt gtttatattt tttagaagtt gaatggtttt gtgtaatggt atagaatttg 2820
gagtttatat ttgaatattt tagatttaat gttattttag attggtttta ggattttgtg 2880
gtttatattt aaaaatgtat gtttttgagt tatgtttaat tagtagtagt atttgagaat 2940
tgagagatta gattaagaaa tttatggtat atgaataaaa tttttagata gtagttagaa 3000
tatagaggtt tttttagagg tagtgttagt tgttagttgt aaaaataggt tttgtttagt 3060
tttgtttagg tttgaagttt tttgaagatt ttatttagat tttattaaaa ttagttaaag 3120
atttttttgtt atagaaaggt ttttaaagta tggttgggat tatagggtta ttaagatgta 3180
gatttttggg ttttttttgtt ttatttatta tggtttttta gggtgggtgt cgggaatttg 3240
tattttaata aacgttttga ggatttttat gtattgttaa gtttaggagt ttttggtttg 3300
taggtttaag tttaattttt ttattgtagt atttaaagat ttggttttaa ttttgttgtt 3360
tataggcgag ttttagtgat tttttttattg ttttttgagt atttttggag ttatttggtt 3420
gttttattta tttttttttt tttgtttttt tttagttttg gaataaaatg atttttattt 3480
ttttttttcga atttttttta gattatggtt ttttatttag taagtagtta ttttttttggt 3540
ttgttattgt tgtggtatta atgataggtt attcgatttt tattttgttg attgaatatt 3600
ttttatgttt aggttttgtt tttttgatta gatagtaagt tttttgaagt tattttatgt 3660
tatataattt ttatgtttaa gattattttt taacgtagtg ttatgtttat atggattatt 3720
tagtaaattt atcgatggtt tatttattat taatatgttt tatagagtgt tattaagtag 3780
ggatattttt tatgggttta ggattagaag aagttgggaa gagaaatggg gtagtggtga 3840
gagtgttatg agaatttggt tatatagtta ttaaagatat agaggaagtg ggaaaaaggg 3900
agtgt 3905

<210> 14
<211> 3905
<212> DNA
<213> Artificial Sequence

<220>
<223> chemically treated genomic DNA (Homo sapiens)

<400> 14

```
atattttttt tttttattt tttttgtgtt tttgatgatt gtataattag atttttatga 60
tatttttatt attattttat tttttttttt agtttttttt gattttagat ttatagagag 120
tgtttttgtt taataatatt ttgtaaaata tgttggtgat agataagtta tcggtaaatt 180
tattgaatga tttatgtgga tatgatattg cgttagaaag tagttttgaa tataaagatt 240
atatagtatg aaatggtttt agagaattta ttgtttggtt agggaggtaa agtttaagta 300
tgagaaatat ttagttaata aaatgagaat cgagtggttt attattaata ttatagtagt 360
ggtagattag gaaagtaatt gtttgttgag tgggaagtta tgatttgaag agagttcgga 420
gaggaagatg gaagttattt tattttaggg ttggggaaag atagaggaga agaaatgaat 480
gaggtagtta ggtggtttta ggagtgttta gagggtagtg aggaagttat tgaggttcgt 540
ttgtgaatag taaaattgaa gttaggtttt tgaatgttgt agtgagaagg ttgaatttaa 600
atttgtagat tagggatttt taaatttggt agtatataag aatttttaaa gcgtttgtta 660
aaatgtaggt tttcggtatt tattttgaga aattatgatg agtgaggtag agaggtttaa 720
gaatttgtat tttaatagtt ttgtagtttt agttatattt taagaatttt tttgtaatag 780
gaagttttg attggttttg atgaagttta aatgaaattt ttaggaaatt ttaggtttaa 840
gtaaaattga gtagaattta tttttatagt tgataattgg tattgttttt ggagggattt 900
ttatgtttta gttgttgttt ggagatttta tttatatatt atggattttt tagtttagtt 960
```

```
ttttagtttt tagatattat tattaattaa atatgattta gaaatatgta tttttaaatg   1020
taggttataa gattttaaag ttagtttgag ataatattgg atttagaata tttaaatatg   1080
ggttttaaat tttgtattat tatataaaat tatttaattt ttgaaaaatg tggatacgtg   1140
tgagttttat ttgtaagatg taaatgtttt attgatagga aagaatttgt ggttgggttg   1200
gggtttgtgt gtgtgtgttt atatttattt gttatatttt attttttttt ttttattttt   1260
tggtttagaa tttaatttta attttttcga atttttaagg ttatttttaa ttaggatttt   1320
ttaaatattt taattatttt aaattttttt tttttttgga atttaaagga attttatttt   1380
ttattttta ttagtaaggt ggttttagtt aaattagggt tttaggaata aatatagttt   1440
ggtttaaata tttgtatatt tattaggttt tattttaaag tagattgttt cgacgacgtt   1500
ttgttattta tagtgtggtt tttggattgg tagtattatt attatttggg aatttgttag   1560
aagtgtagaa tttgaggttt tagtttagat ttgttgaatt atcgttttta tttgtttaag   1620
agtttcgtga ttggtttgat ttttatgtat ataaaagttt aaggagtttt gtttaaattt   1680
ttcgttttta agttgttttc gaaatgttgg ttttttttgtt tttggacgtt tgtagagatg   1740
gggtagtgga attagtttta tgtgggggta gggtgcgggt tttatttttt tttttcgttt   1800
tttgtttttt tattttgttt tttttgatgt ttttttagcg gttgtatttg tagagttttt   1860
attttttttgg gaagtttttt gatatagatt tcgtttttgt ttttagggtg gggtcgtttt   1920
attttttttt ttttaaagag tttgggatgt tgatagtttt agatggaggt attttgtttg   1980
tcgaaaaaata aatcggtagt taaagtaggg ttgcgagagc gagaaaatta taggtttgcg   2040
cgtcgttgat aacgcgcggg taggtagttt ttgtagaagt ttcgtcgttg cggcgcgttg   2100
cgtattagag ttgcgttatc gttttttttt ttgtttgggt cgttcgcgtt ttcgtcgttt   2160
ttggttgttg tttttgcgt cgttttttta gttatttcgt ttattgtttt ttttaaagga   2220
tttttttgtt gtttcgttat gagggatgga tttattaata agttttcggg cggatgcgcg   2280
tagattttgg atttatagga ggttaaaatt tacgagttgt tatgaaaatg atatcgttta   2340
agagaagttg aagttgtttt tttttttgta gtatgtaatg ttttgatttg gttaaaataa   2400
ataaataaat aataatttaa aaagaaagaa agaaatatag aaagattata ttttttatgag   2460
gtttgtaatg ttaaaatgaa aagggatttt aaagtgtaat atttagattg tttttttttt   2520
tggagagagt tgatttggtt tgattttatg cggtgaataa ttaattatta attttttttt   2580
tttgagggggt attaaagttt ttttttttat ttgtttgtta ttagataagt taggtagatg   2640
agaagttagt gggtaattat aggtataatt acgatattta ttggaaatta aaagatttga   2700
tttttcgagg taaagaatgt atttgaaaat aaatttgaaa atagttttat aggagttgaa   2760
aaatttagtg ttttcgaatt agtaatacga atttttttga gtattaaaaa gtataagtat   2820
ttttaaaaag gatattttga gttatagaag atgatgtttg ttggggttga ttgagttttt   2880
ttggaagaga tttttgtgtt acggtgtatt agaaagtttt tattattgtt ggggtttttg   2940
tagttgtttt agttatttag tgttttttta ttattttttt tgttcgaaaa tatagagtag   3000
aaatagttta tatttatttt ttaatttttt tgtgtttata acgagtaagt attttagatg   3060
agtaagaatt tattttattt ttaagaatta gtagagaatt atatgagttt tttttttttt   3120
tttttagaaa cgtttttagg ttggttaatt tttttattga gtaatttttt atagtaatta   3180
tttcgtatat tttttatttg agaggttgtt tttttatttt tatgagtttt tttttttttg   3240
ttttatggtt ttatttttaa ttatttatgt tttttttgttg aaggtttttt gatttaaata   3300
tagagattcg tatattttgg ttatttatgt atagatttta ataatgaata atttaatttt   3360
atggtgatat aaaggaggaa aatttaggtt aaagaatgtt taatttttag ggaagaagga   3420
gagagttggt tagggatagc gtgtgttggg tgttaatagt tacgtgtata gtttgttggg   3480
gaatttggtt ttttttgaa tttggtgtag ggtatcggaa cgtttggggg aggagtagat   3540
aggatgttgt ttaggttgaa tagggttgga ttatgagagt tttgaatgta tttaagaagg   3600
aatttttttt agtgggtcgg ggttaggtta gattgatgaa tgatcgattt ttattattta   3660
ttttgaggtt tttatggaga tattttaaat ggattttttt tttatttata aattgttata   3720
aataaatgat taaaagatgt ttaagtggtg attagaaaat aataaatttt attttagtta   3780
tatattttag aatttatata tttgttattg tagtgttttt taaagttatt ttttgggagg   3840
gagagggagt attattttaa tagtattatt attattttaa atattttcgg atttgttttt   3900
ttgaa                                                               3905
```

<210> 15
<211> 5338
<212> DNA
<213> Artificial Sequence

<220>
<223> chemically treated genomic DNA (Homo sapiens)

<400> 15

```
ggtaattttt tttgttatta ttttttgtat aaaatatgta agatgttttt attttattag      60
tattgaaatt aaagattaaa agagagaaag gtgttggatc gaaaaggagg cggcgtaacg     120
```

gttatttttt ttagtatttc ggttttgttt ttttcgggga aggcggttat atttttattc 180
gttttgtttt tgaacgtagt aaataatttt ataaataatt atcgttgttt acgtttttta 240
ttcgtttttt cggttttatt agattttcgt tttttcgtat ttttcgggta gggtttttaa 300
taagtttagg ttgaaagaac gtttgttatt tttttatttt tcgttgaaag aaaaggaaga 360
aaaatagtag tagcgagaaa ttttcggggc gatttttttt tcgtttttaa gtattagcgt 420
atagtatttt tttttgtttt tgttgtggtt tttcgttgtt tcgggttacg tcgtttagtt 480
aagtaattcg gatttgagag tgtatagtta ggattagttt aggggggcgag gggttggttt 540
ttgggaaatt aagcgtttag gatagaggtg gaaagtgggt ttcgggagtt agaaaagaga 600
gagaagggta gacggttggg tggtaaatat aaaaatagaa ataatttagg gggatgttcg 660
ttaggttttt gcgtttgttt tttttttttt aattcggagt aggttttttt cggtttttcg 720
cgtttcgggg cgtttttttgg cggtagcggt agtagcgggt aacgcgcgga gggtttaggg 780
ggcgtatagg ggattttcgg gtatatttag agaggcgggc gcggtttttt ggcggtggcg 840
acgtagttat ttggtgagcg gagtttcgtt tttttggttc ggcgggttta cggtcgtttt 900
attaagtatc gcggtcgagt agggtaggaa gtcgttttcg gaacgcggcg tcgtcgcgtt 960
gtagtcgaag ttcgagtttt ttttcggtttc ggcgttttcg tcgtcgtcgt tattattatt 1020
atcgtcgtcg ttatcgttat ttcgggaatt tagggttgcg gtagttgttg tcgcggttgt 1080
cgtcgtcgat tgattgttgt ggtaattgag gtacgagtag ggtgtagagt cgtcgttggg 1140
gggcgcgtcg gtcgtcgtcg tcgaggaggt cgtagtcgtt gcggttgtcg cggttgtcgc 1200
ggtagaggtc gcgttgttga gtttcgcggc ggtcgcggga gtttggtaga gattagggtg 1260
gcggaagtta tatagtagtt tcggtcgaga gtaggggtgc gagaggcgc ggaaggtgtt 1320
tagtggtcgg atggaagtag cgaaggcgga cgaagtcgcg gtcgtcgcgt ttgaggttgt 1380
agtcgcggtc gtcgtcgtcg tgacgtttac gtgcgggtag tagtgtagcg gtacgtgcga 1440
gtggaagggg tagggtaggt tttcggtggc ggtcgcgtgc gttattatgt aggtgtagaa 1500
gttggggtcg gttgggtgcg gttaggatat ggttaggcgt tgtcgtttgt tttttatgcg 1560
tcggttttgg aattatattt gcggggagag acgcgtcgta gtttgggtta gggagcgttt 1620
cgtgttttta gtttttgttt taggattttt gttttttttcg gatttttgaa tggtttggtt 1680
tatttttttt cgattaagtt taatttcgag tattttgtgg ttttttagtt gggaaagtgt 1740
ggacggtagt gtgtggatcg tcgtgggtat atcgttttta acgaagaggg tttttttttt 1800
cgcgttcggt tgttgttgtt ttttaggttt ttattttttt atttttttgtt gtattttttt 1860
gttttaattt aatttttttt tttttttttcg ttatttatta gtgtcggttt cgttgagtat 1920
tcgtttttta attttaggat ttgtacgggg attttgggta gtttttgaag agaggttgtc 1980
gtttagtttt tttgagaggt cgtcgcgcga agttttgagt ttttcgaatt gtaaggattt 2040
gtttttaggg gtacgggttc gattttgata ttgaagggat gattttgttg gaatcgtttg 2100
ttttaaatga gtgggtagag taatgttttt ataaacgggg aaggggacgt ttttattttt 2160
tttaatatta tttaataaag atattattcg ttataatttt aaattaaaga aagttcgatt 2220
aaattagagg gagattttta tatttttttt ttatttcgtg gagatttttt tttttttttg 2280
tagtgtcgaa cgttttatga agggtttatt aaatcgttta atttttcggt tatttttttt 2340
agacgtataa taaaattaat aatttaaagt tatatataaa taaggataat ttcgttgtat 2400
ttttttcgt aggaggttgt ttttttttcg ttgtttaaga ggaaaatgtt taggaaatta 2460
ttgttttaaa ttaatcgatt ttaaagatat agtatttttt ttttcgtgta acgatttatg 2520
cggaaaataa atttttaagt ttaagagtaa atgaaaagtt ttattttttgg tttttgtttg 2580
aggaataaag attaattggg tttgtcgttt aggggaaagt ggggtcgtgg gtatgggcga 2640
gggggtattt ggttaggcgt tgggtataat ggagtagggg cgagtgtttt tagtattgga 2700
gttattattc gggggttttt ttagattcgt taggtcggat aatcgttcgg attcggtggt 2760
cgggaaataa ataagttaat ttttttggtg attatttttc gcggattttt agatttttag 2820
ttaaatttag ttatttagaa aggggaaagg ggaaaaagaa ataattaatt tagatgtttt 2880
cggggaggtt agagtaggta tgtattggaa ttgatatttt gatggtggtt tcgggtaggt 2940
tgagtgtcgc ggttagttcg tatcggcggg gtcgcgatat atagtttttt cggtagaatt 3000
tttttttttag gcgcgcgatt tgttcgcggg tgaacgtcgt acggtagcgt cgtatttgat 3060
tcgcgttaga gtcggagtta tttagcgtcg cgttttcgtc gttgttttcg ttgtttttat 3120
gtaggttttc gaggtttgag ttcgacgtcg acgtcgtggt gtcggtagtc gagtcgtttt 3180
ttgcgtattt tggtaaataa acgattaata gcgtatgagt ggttgtagga ttaatagttc 3240
ggcgttggcg ttgcgcgcgg atcggggaag tttcgttagg aaggagagtc gttgtcggaa 3300
ttgatggggt ttgttatgtt tataaattgt tgtcgttaat ggaattaata aagtttgggg 3360
agtttttttat aagtaaataa tagaaacgga attaggagat tttttttttta ataattagaa 3420
atttttaatt aaggaggaaa agtggtcgag ggaaaatgtt tatttttggg cgtagtttgg 3480
gaagttttgg gttttttatg gtttggagat cgtagggtag tttttttatac ggtttttaat 3540
ttttatttga gttttcgggg ttttaaatat tttaagagtt ttaatatagt agtagtttaa 3600
atttaagtta atagggggtg aaatatattt tttaattttt tttttcgttg atttaaatag 3660
agacgtcggc gaggtttttt tcgatttatt tagaagaatg ttttttttttag ttttgagggt 3720
ataggggtag gggagtttag agtagttgtt tagggtattt tttttaaaag taattcggtt 3780
gttgattagg ggtttatcgg ttcgttcggg tttttagata cgagcgtaga aatatttttt 3840
cggatttagg agcgagggcg ggtgggtttg gtgttttagg tttcgtaggt ttgagtttgg 3900

```
cgggaaagtt taaggagtag aattggaagg tacggtttta gatatgcgtt tcgttttcgt    3960
tcgtgttagt tcggtgtagt ttgtttgtgg agggtttgag aggggaaaag gtatcgggaa    4020
aggttggcgg gggtcgcgga ggagtaaaga ggatgggatt ggagagcgcg gtgcggtcgg    4080
cgcggttatt tttgttattg ttttttttaa gttgagcggc gtcgaggttt tcgggggagc    4140
gaagcgcgga gtagtttatt tttacgtcgt tgtttatgtc ggttttagcg gtcgttttttg    4200
aataatggtt cggtttttttg cggttttcgg cggcggagga gatttcggag gagacggtgt    4260
tttcgttgtt cgtgtgttgt aggttgaata aagtgtttat ttcgaatttg tttttggcgg    4320
ggagtttttt tagagcgttg tgtagggggg tagacggtag gcgcgggttt aggcgagtcg    4380
ggtgttgcga atttttttagg gtttcgagta tagtattgtt agtcgagttg gataaattgg    4440
agaatttttt gttcgtcgta gggttgtgta gtttttttttt tattagaatt atttttttttt    4500
ttattttttt tattatttta gttttttttaa aaatgttata gtggttttgt tgtttcgttt    4560
taatgatagg ttgcgtttag ggttaatttt tatttagttt tagcgaacgt ttttaaatat    4620
tattatcgtt ttcggaggga ggcggaaaaa ttgtgggatg ttagagcgag ggaatgattg    4680
gttaaaatga tttatatatt ttttcgattt cgagatgtta tttattaaaa agtagcgttc    4740
gttcgttatt aaggtacgaa tgacgttgtt cgaataatta tttattgtaa taggtttata    4800
agtaaataaa tatatttttt tgttagtggg taatgaaggt agttttagag tagataaata    4860
gatttaggta ggaggcgaga agagataagt gaggaggaag gtttcggttt tttgttcgtt    4920
tcgagttagt tttgttcgtt cgggggtttg gtttcggggg tgaaattgat agtttgatta    4980
atagagcgcg tcgcggtata tttttttttt tatttagggg tattattacg tttttagttt    5040
gttaggttgt tttttaggtt ttaattatgt agcgtttttt ttattttttt ttggatatag    5100
atacgtatta aataatagat aatggtttga tttttttagag taggttttcg ggaaggtttg    5160
gttttttttt tttttttttt tttttttttt attatttata agttgggggt ataagtgggg    5220
ggagataagg gggaggatgt aggaagagtt gcgtaaggag aagattttgc gttttttttta    5280
tttggattat ttattttttcg gatttcggaa ttaaatatgt attttattta ataaatag      5338
```

<210> 16
<211> 5338
<212> DNA
<213> Artificial Sequence

<220>
<223> chemically treated genomic DNA (Homo sapiens)

<400> 16

```
ttatttgtta aataaaatat atatttggtt tcgaaattcg aaagataagt aatttagata      60
ggagaggcgt aaggtttttt ttttacgtaa tttttttgt attttttttt ttattttttt      120
ttatttatgt ttttagtttg taaatggtaa aaaaaaaaaa aaaaaaaaaa aaaaaaatta      180
aatttttcg aggatttatt ttggaaagtt aaattattat ttattattta atacgtattt      240
gtgtttaagg aaaaatgaag aaggcgttgt atgattagga tttaaggggt aatttagtaa      300
attgagagcg taatgatgtt tttaaatgaa gggggaaatg tgtcgcggcg cgttttatta      360
attagattgt taattttatt ttcgaggtta aattttcggg cgagtagaat tggttcggag      420
cgggtaaagg atcggagttt ttttttttat ttgttttttt tcgtttttta tttggattta      480
tttgtttgtt ttgaagttgt ttttattatt tattgatagg agggtgtatt tatttgttta      540
taaatttgtt ataataaatg attattcgaa tagcgttatt cgtattttaa tgacgagcgg      600
gcgttatttt ttgatgaatg atatttcggg atcggaagga tgtatgggtt attttgatta      660
gttatttttt cgttttggta ttttataatt ttttcgtttt ttttcgaaag cgataataat      720
atttagaggc gttcgttggg gttgaatgag aattagtttt aggcgtagtt tattattagg      780
acgggatagt agggttattg tgatattttt aagaaagttg agatgatgga aagaataaga      840
aaagagatga ttttgatgga gagagggttg tatagtttta cggcgggtaa gagatttttt      900
aatttgttta attcggttgg taatgttgtg ttcgaggttt tggaaaattc gtagtattcg      960
gttcgtttaa gttcgcgttt gtcgtttgtt tttttgtata gcgttttggg agaatttttc     1020
gttaagggta aattcgaaat agatattttg tttaatttgt agtatacggg tagcgaaagt     1080
atcgtttttt tcgaaatttt tttcgtcgtc gagagtcgta agaagtcggg ttattattta     1140
gaggcggtcg ttgaggtcga tatgagtagc gacgtggagg tgggttgttt cgcgtttcgt     1200
tttttcgggg gtttcggcgt cgtttagttt aaggaaaata atggtaaagg taatcgcgtc     1260
ggtcgtatcg cgtttttag ttttattttt tttgtttttt cgcggttttc gttagttttt     1320
ttcggtgttt tttttttttt tagattttt ataggtaagt tatatcgagt tagtacgggc     1380
gggggcggga cgtatgtttg ggatcgtgtt ttttagtttt gtttttgag ttttttcgtt     1440
aggtttaggt ttgcggagtt tggaatatta ggtttattcg ttttcgtttt tgagttcgag     1500
aaaatgtttt tgcgttcgtg tttaagagtt cgagcgagtc ggtagatttt tagttagtag     1560
tcgagttgtt tttgggaaag gtattttggg taattatttt gaattttttt gtttttgtgt     1620
ttttagggtt ggagggggta tttttttggg taagtcggag gaagtttcgt cggcgttttt     1680
```

gtttaggtta gcgggagaaa gagttgaaaa gtatatttta tttttattg gtttgggttt 1740
gagttattgt tgtgttggaa tttttaaaat atttgaaatt tcgaagattt agataaaggt 1800
tagaggtcgt ataaaaagtt gttttgcggt ttttagatta tgggaaattt agagtttttt 1860
aaattgcgtt tagaggtagg tatttttttt cggttatttt ttttttttgg ttgaaaattt 1920
ttaattatta aaaaagaaat tttttaattt cgtttttatt atttgtttat gaagggtttt 1980
ttaaatttta ttgattttat taacggtagt aatttgtaag tatgatagat tttattaatt 2040
tcggtagcga tttttttttt tgacggggtt ttttcgattc gcgcgtagcg ttagcgtcgg 2100
gttgttggtt ttatagttat ttatgcgttg ttggtcgttt gtttgttagg gtacgtagag 2160
agcggttcgg ttgtcggtat tacgacgtcg gcgtcgggtt taggtttcgg aagtttgtat 2220
ggaggtagcg gaggtagcgg cgggagcgcg gcgttgggtg gtttcggttt tggcgcggat 2280
taagtgcggc gttatcgtac ggcgtttatt cgcgagtaga tcgcgcgttt ggagaaggag 2340
ttttatcggg agaattatgt gtcgcggttt cgtcggtgcg agttggtcgc ggtatttaat 2400
ttgttcgaaa ttattattaa ggtattaatt ttagtgtatg tttgttttgg ttttttcggg 2460
ggtatttggg ttgattgttt tttttttttt tttttttttt ttgggtggtt agatttagtt 2520
aagggtttgg aaattcgcgg ggggtagtta ttaaaggaat tggtttattt atttttcggt 2580
tatcgaattc gaacggttgt tcggtttgac ggatttaaga aggttttcga atggtgattt 2640
taatgttgaa agtattcgtt tttgttttat tgtgtttaac gtttggttag atgtttttttc 2700
gtttatattt acggttttat tttttttttag gcggtaagtt tagttggttt ttgtttttta 2760
agtaggaatt agaggtgaaa tttttttattt gtttttgagt ttggagattt atttttcgta 2820
taaatcgtta tacggagaaa aggatattgt atttttaaaa tcgattggtt tgagatagta 2880
gtttttttgaa tatttttttt ttgggtaacg aaaaaagggt aatttttgc ggggaaaaat 2940
gtaacgaaat tgtttttattt tatatataat tttaggttat taattttattt atacgtttgg 3000
gagagatagt cgggaggtta ggcgatttgg tgagtttttt atggagcgtt cgatattgta 3060
gaaaaaagaa aaaattttta cggggtaggg gaggagtgtg gaagtttttt tttggtttga 3120
tcgggttttt tttaatttag agttgtgacg aatgatgttt ttattagata gtgttgggag 3180
gggtggaaac gtttttttttt tcgtttatgg agatattgtt ttatttattt atttaaggta 3240
aacgatttta ataaaattat ttttttaata ttaaaatcgg attcgtgttt ttaggggtag 3300
gtttttgtag ttcggagggt ttaagatttc gcgcggcgat tttttagaaa agttgagcgg 3360
tagtttttttt ttaaaggttg tttagaattt tcgtataaat tttgggattg agagacgggt 3420
gtttagcgag atcggtattg gtgggtggcg ggagggagag gaaaggttgg attgggataa 3480
aaaagtgtag taagaagtaa gggaataaaa gtttgaggag tagtagtagt cggacgcggg 3540
ggagaggatt tttttcgttg aggacggtgt gtttacggcg gtttatatat tgtcgtttat 3600
atttttttag ttgggagatt atagggtatt cggggttggg tttggtcgga gagaagtaga 3660
ttaagttatt tagaggttcg gaaggggtag aggttttggg ataggagttg ggaatacggg 3720
gcgtttttta atttaggttg cggcgcgttt tttttcgtag gtgtggtttt agaatcggcg 3780
tatgaaggat aagcggtagc gtttggttat gttttggtcg tatttagtcg attttagttt 3840
ttatatttat atgatgacgt acgcggtcgt tatcggaagt ttgttttatt tttttattc 3900
gtacgtgtcg ttgtattatt attcgtacgt gggcgttacg gcggcggcgg tcgcggttgt 3960
agttttaggc gcggcggtcg cggtttcgtc gtttttcgtt atttttattc ggttattgga 4020
tatttttcgc gtttttttcgt atttttattt tcggtcggag ttgttgtgta gttttcgtta 4080
ttttggtttt tattaggttt tcgcggtcgt cgcggggttt aatagcgcgg tttttgtcgc 4140
ggtagtcgcg gtagtcgtag cggttgcggt tttttcggcg gcggcggtcg gcgcgttttt 4200
tagcggcggt tttgtatttt gttcgtgttt tagttgttat agtagttagt cggcggcggt 4260
agtcgcggta gtagttgtcg tagttttggg ttttcggggt ggcggtggcg gcggcggtgg 4320
tggtggtggc ggcggcggcg ggggcgtcgg ggtcggggga ggttcggatt tcggttgtag 4380
cgcggcggcg tcgcgtttcg agagcggttt tttgttttat tcggtcgcgg tgtttagtaa 4440
gacggtcgtg agttcgtcgg attagaggga cgaggtttcg tttattagat aattacgtcg 4500
ttatcgttag ggggtcgcgt tcgttttttt gagtgtgttc gggagttttt tgtgcgtttt 4560
ttgagttttt cgcgcgttgt tcgttgttgt cgttgtcgtt aggggcgttt tcgggcgcg 4620
ggaggtcgaa gggaatttgt ttcgaattgg gggagaagga gtaggcgtaa aagtttggcg 4680
ggtatttttt taaattattt ttatttttgt atttgttatt tagtcgtttg tttttttttt 4740
ttttttttgg ttttcgggat ttattttttta tttttgtttt gagcgtttgg ttttttaaag 4800
attaattttt cgtttttttaa gttagttttg gttgtgtatt tttaggttcg ggttgtttgg 4860
ttgaacggcg tggttcgaag taacggagaa ttataataaa gatagagggg gatgttgtgc 4920
gttggtgttt gggggcgggg gaggagtcgt ttcggaggtt tttcgttgtt gttgtttttt 4980
tttttttttt tttaacgagg gtgggggagg tggtaaacgt tttttagtt tgagtttatt 5040
agggattttg ttcgaagagt gcgggagaac ggaggtttga taaggtcggg aggacggatg 5100
gagagcgtgg gtagcggtgg ttgtttgtga gattgtttat tacgtttagg agtaaggcgg 5160
gtagggatgt ggtcgttttt ttcgggaagg ataaagtcga ggtgttggaa ggggtggtcg 5220
ttgcgtcgtt tttttttcgg tttaatattt tttttttttt tggtttttgg ttttagtgtt 5280
gatggaatgg aagtattttg tatattttat gtaaaaagtg atggtaaaag aggttgtt 5338

<210> 17
<211> 5332
<212> DNA
<213> Artificial Sequence

<220>
<223> chemically treated genomic DNA (Homo sapiens)

<400> 17

ttagttttaa attttgtatt cgtatttttt agataggaag ttttttttt tcgattaggg 60
attgagggag aagagattta gaatgtattt ttttgtttta ggtttttcgg gggggatatg 120
gttttagata gttatttcga aattgggttt aggtttttat agtaagtcga gtgtttttt 180
atatttttaa tttcggtttt tttattaaag tttaaaagat tttttgttt tttaggtttt 240
tttcgtttgg ttgtttttat tatttttaa ttgcggaaat attagttatt atatgttcgt 300
cggagaataa gcgtgagtgt cgcggtggat agttgtcgtt aaaggtgttt agacggatgg 360
tatttttaaa gtagggattt tagtattttt tttatatatt attatttaag aatggtagag 420
gaaagagtag gagtggggtt atagatagga gagaaaggag gttgaggtta taaaattggt 480
attttttaa ttattaattt tttttttat tattttgtta ttatttgtta tggtaaagtt 540
tttttttgt gttttttttt taataaagaa atttttagtt agtttttatt tattttttt 600
gatttgtagg aagttaaatt gttagtagat tgagtttttt aagggatatt tttgggatat 660
gttggaattt tgatttgttt aagatgatga cggttattag tataaattag aaataatgtt 720
atttttagg gtgttatttg taagtgtaat aatacggatt tatttgtaaa atttagttaa 780
attgcgtttg gataagtgat tagatttaaa cgataaattt tttttattat ttagtttttt 840
ttaagttttg cgggggtagt atagaagatt gttttagggt gaaattaagg tttagtttat 900
tatttgtgtt agtttgaggg attttggaaa tgttgatttt gagaaaaaga taaaataaaa 960
taaaataaaa taaaatttaa ttataaaata tattttattg tttataaagt aaatagaaat 1020
ataaagttgt ttttaaaatt atttttttag taaataagta tgtgatattt tgagtaggag 1080
taggagggag agaaagattg ttatttaaaa aaatatgaaa gtattaagta ataatttaaa 1140
atacggttgt ttagaaaaat atttatattt attgtagttg tttaattggt ttcgttaaag 1200
tcggtaagta tttaaattgt gttataattt tatttaaatt tcgtttcgtt ttagtaggtt 1260
gaagagtttg aatagattaa ttatttata atgatgatga atgagaaatt aatttagata 1320
taagtaagat aatttaggtt tttatttgtt taaatagttt tttaatatta ttcgttatcg 1380
cgggttatag attgaattaa ttgtttaatt ttgtgaaagt tatattttg cgtttttatc 1440
gagattattt atagcgtagt gagcgttgtt gaaaggtata cgttgttaat agggataatt 1500
atttaaaggg aagcgtttgt aaaatggaaa ataaatgtcg gggttggtaa taaatgggat 1560
taaaagtagt tattttaatt tggtttttcg agggaaagga gcgggcgtgg ttttgtagga 1620
ttaggggcgt ttttgatttt tttaagattt aggtgaggtc gagggtttc ggcgcgttag 1680
tttgcgtcgt ggttgtggtt gcggtggcga ttcgggtcgg gtttcgtttt cgtcggtttt 1740
aggttttcgt tttttcgcg taggtagcgg gcgcgtgtgg ttcgggttgg gtaagtcgag 1800
gaatagcgag ttttcggacg ttgattgtag gacgttttag tttgtgttcg ggttttcgtt 1860
tttttcgta cggggttcgt attttcgggt ttgggtttga tttatagggc gttgaggttt 1920
ttgtagttga agtcggaagg tttcgttgcg agcgcggtat aggttgttgg tagtttttgg 1980
attttggagg tttggttttt tttttaagtc gatggcgggg aaagaatttc gtttttatag 2040
tttttcgat tttcgtcgtt tgttatttgg ggacgggaag cgcgttcggg tcgttttacg 2100
tttttttggg tcggagtttt ttttatggtt ggttttttg ggggtttttg ggtttgtgag 2160
tagcgtttat ttttttaga gaagaatttt tttttttt tatcgaagtg ttttttttg 2220
tattttgaaa taattttttt tgggtgaggt tagttttttt ttgtcgtttt tttttcgtag 2280
gcgttcggga gtttcgtgag gatttcgtgt agttgagttt aggcgatagg tgttttttta 2340
ggtgtttatt tgtttttttt taatttgttt agggaaagat tagaacgatg tgcgcggga 2400
ggtggttttg tttttttcga aattggcgtt tttgggtaaa atcgcgtgtc gcgtttacgc 2460
gggttttgtt agtgtgcgtg ttttagaaga gtgggttttt taggcggtat tttttggaga 2520
agtaagtgag gtgaatttag aatagagaag cgggaacgat cgtttgatgt gtcgagtcgg 2580
aaaaagagag gagagggaga ggtttttagc gagcgcggag atagaggagt ttacgcggta 2640
tagtacgagt tttatttttg ttcgcgtaga ggtgtgtgtg tgtgtgtgtg tgtgtgtgtg 2700
tgtgtgtgtg tgtgtgtgtt tttttttgta tttttgtgtt tgaaataaaa gtttaattaa 2760
cggattttg gtgttttgag gatttatttt cgggagcgat tttgatgtat tgttgtttaa 2820
ttagtgtttt taattggtgt tttcggagat aggtaggttc ggagttggga tagagttttt 2880
tttcgttttt tttgtatatt ggaaaggtaa aatttataat atattgtggt tattttttgg 2940
gagagcgaaa gagagtggga ggtagggaag aaagggagag atgtagttgt ttgtagagga 3000
cggagttttt agtttcgggg gtcgtgggga agggtttaga ttatcgggtt cgatttagtt 3060
ttttgtgtaa gagggttcgg gtcggttgag tcgttgagga ttatgaattt gttcgtagaa 3120
aggtttaggg agtttttatt gtttgtataa atttatttta tgtggtttat tatttcgtta 3180
ataatggttg tttgttaata aggatgcgga ggttaaataa ttcgggtgga agattaaata 3240

cgttttatta aggcgtttgg aagtcgcgcg gtgtagagat ttgcgttgga gtcgtttggt 3300
ttattggagt ttggagggag atgggagtat agtttgtaag gagtttcggg gtagaagagg 3360
aggtaaggtt tagtttttttt ttttagggtg ttttgttatg ttaggatagg tttaattttt 3420
ttatatgtta aatagaaaat ttatatagaa gttagtttta atgaggtgtg gaggttaaat 3480
tttcggagta gttggttgga gggtagatat ggagtatttg gggtttgaat taaaggtttt 3540
tttttgagga ggggtggtta ttttttgttt tttagttttt atatgttatg tatttggtaa 3600
tttgtgttta tatgggatta gtattggatt agaagataga agatgtggtt tttagtttgg 3660
gttttgtttt taaattattg tagaattttt attatattat tttttagtt ttattttttt 3720
attgtgtaag agaaaggaga tggggtttga tgtagatgat gtatagtatt tttttttttg 3780
tttagttttt tattagagtt agggaattga atattttata agatgttttg tttttttttaa 3840
atttgtagg atgtagttaa aaagaatttt atttagtaaa tatttgaggg tttattttgt 3900
attagttatg gtgtagtatg tattttttaa ttgagagtag aatattagtt tatatttttt 3960
tgttattttt aaatataatg ttttataata aaagatgaaa gagaaagaat tttaaagaga 4020
atagaatagg atgggtatgt atgtataggg tagagaaatt atggaggtaa aggtaaaggt 4080
attgataaaa gttgtgaaga atttgttgaa ttggtaagga agttagtgga taaatattgt 4140
agaagtagtt ttttaataga gatattatgt aggagagtag agagaaggtt tttttttgaa 4200
ttttggtgat ttggtagaga ataaaagggt aagggttgta gaatttgatt aggaggttta 4260
agttgtatgt atttaatatt gttaattttt ggtttatata tttttttagt agttggaatt 4320
tttttttttt tttttttttt tttttttttt tttgagatgg ggttttgttt tgttgtttag 4380
gttggagtgt agtggtttta ttaaggttta ttgtagtttt aaatttttag gtttaagtga 4440
tttttttatt ttagtttttc gagtagtaag ggttataggt atatgttatt atgtttggat 4500
aattttttttt tttggttgag gggtatagat ggggttttgt tgtgttattt aggttggttt 4560
taagttttta ggtttaatga atttttttat tttggtgttt taaagtgttg agattatagg 4620
aatgagttat tgtgttcggt ttaggaagtt tatttttttag ttaattttga aatattattt 4680
tttgaattta ttaattagga aattcggttg ttaggttggg atttttttaag tttttgggtg 4740
atgggtattt agttttaagg aaggtaatga tgtttaaatt attaaattat gttttagttt 4800
ttgtgagtta ggaatttagt gagtttagtt gtgtgttttt ggttttaggt tattttattg 4860
gggttgtggt tttatttaaa gatttagttg ggtgaggata tgtttcgggt ttatttatgt 4920
ggttgtttgg taggatttag tttttttgttg gtgggttatt tggttgaagg ttttagtttt 4980
tttttggtgg ttggttagag gttttttttgt tttttattat atgagttttt ttatagggta 5040
gtttataaag tggtagttgt ttttttttag attgagtaag taagagtgag agtggagggg 5100
ggagaaagta tttaagatag aagttatatt tttttggtta tttaatttttg gaagtgattt 5160
tttattatta tgttatatta tatttgttag aagtgagtta ttagatttag tttatattaa 5220
aggaaagggg attatagaag gtattaaatt taggaggtag ggattattgg ggattgtttt 5280
agaggttgtt gtttagattt tttttatata atagttttga ggtttatatg gt 5332

<210> 18
<211> 5332
<212> DNA
<213> Artificial Sequence

<220>
<223> chemically treated genomic DNA (Homo sapiens)

<400> 18

```
attatataag ttttagagtt gttatataaa aggggtttgg gtagtagttt ttaagatagt      60
ttttagtgat ttttgttttt tggatttgat gtttttgta atttttttt ttttagtgtg      120
ggttggattt agtgatttat ttttaataaa tataatatgg tatagtgatg gaaagttatt     180
tttaagatta gatgattaaa agagtgtagt ttttgttttg ggtgtttttt tttttttta      240
tttttatttt tgtttattta gtttgaggaa agatagttgt tattttataa gttattttat     300
agagaggttt atatggtaag gaatagagaa atttttggtt aattattaag aaggaattga     360
ggtttttagt taaatagttt attagtaagg aattgaattt tgttaaataa ttatatgagt     420
gagttcggaa tatattttta tttagttgag tttttagata agattataat tttagtggaa     480
tagtttggag ttagggatat atagttaagt ttattagatt tttgatttat agaaattgag     540
atatggttta gtgatttaag tattattgtt tttttgagg ttaaatgttt attatttaga      600
agtttgaaga gttttagttt agtagtcgag ttttttgatt ggtggattta gaaagtaatg     660
ttttaaaatt agttaaagag taaatttttt aggtcgggta tagtggttta tttttgtaat     720
tttaatattt tgggatatta gggtgggagg gtttattgag tttagaagtt tgagattagt     780
ttgggtaata tagtaagatt ttatttgtat tttttaatta aaaaaaaaaa ttatttaggt     840
atggtggtat atgtttgtgg tttttgttat tcgggagatt gagatgagag gattatttga     900
gtttgggagt ttaaggttgt agtgagtttt gatggggtta ttgtatttta gtttgagtaa     960
tagagtaaga ttttatttta aaaagaaaaa gagagagaga gagagagaga gaaattttaa    1020
```

ttattgaaag aatatataaa ttaaaaattg gtaatattag atgtatataa tttgggtttt 1080
ttagttaaat tttgtaattt ttattttttt gtttttgtt aaattattag gatttaggag 1140
gggattttt ttttattttt ttatatagta tttttgttaa ggagttattt ttgtagtatt 1200
tatttattga tttttttgtt agtttaataa gtttttata gtttttgtta atgtttttat 1260
ttttattttt atagtttttt tattttgtat atatatattt attttatttt atttttttg 1320
aagtttttt tttttattt tttgttataa aatattatat ttgagaataa taggggaata 1380
tgaattgata ttttgttttt agttaaaaaa tatatattgt attatgattg gtgtagagta 1440
ggtttttaga tatttgttaa ataaaatttt ttttgattgt attttgtaag gtttagggga 1500
gatagagtat tttataaggt gtttagtttt ttaatttgg tgggaagttg gataaaagaa 1560
gaaatgttgt atattattta tattaagttt tattttttt tttttatata gtagaaaaat 1620
gaagttggga gaataatgta ataaaaattt tatagtgatt taggggtaga atttagatta 1680
gaaattatat tttttgtttt ttaatttaat gttaatttta tgtaggtata aattgttaga 1740
tgtatgatat gtaaggattg ggaggtaggg gatggttatt ttttttaaa aggggtttt 1800
tggtttagat tttagatgtt ttatgtttgt tttttagtta attatttcga gggtttgatt 1860
tttatatttt attggggttg atttttatgt ggattttttg tttgatatgt ggaagagtta 1920
gatttgtttt ggtatagtag gatattttga agagggagat tagattttgt tttttttttt 1980
atttcggggt tttttgtaga ttgtgttttt attttttttt aggttttagt gagttaggcg 2040
gttttagcgt aggtttttgt atcgcgcggt ttttaagcgt tttgatgaga cgtgtttaat 2100
tttttattcg gattatttaa ttttcgtatt tttgttaata aatagttatt attaacgaga 2160
tggtagatta tatggggtgg gtttgtatag ataatgagag tttttttaggt tttttttgcga 2220
gtaaatttat aatttttagc gatttagtcg gttcgaattt ttttatatag aaaattgagt 2280
cgaattcggt ggtttgggtt ttttttttacg atttcgggg ttggaggttt cgtttttttgt 2340
aaatagttgt attttttttt ttttttttttg tttttttattt tttttcgttt ttttaagggg 2400
tggttatagt gtgttataaa ttttattttt ttagtgtgta ggagaggcga gaggaggttt 2460
tgttttagtt tcgggtttgt ttgttttcga ggatattaat taaaggtatt aattggatag 2520
taatatatta aagtcgtttt cgggagtgag tttttagagt attaggaatt cgttagttgg 2580
gtttttgttt taaatataga aatatagagg aaaatatata tatatatata tatatatata 2640
tatatatata tatatatata tttttacgcg ggtaagagta gggttcgtgt tgtgtcgcgt 2700
gggtttttttt gttttcgcgt tcgttgggag ttttttttt ttttttttt tttcggttcg 2760
gtatattaaa cgatcgtttt cgttttttttg ttttgagttt attttattta ttttttttaaa 2820
gagtgtcgtt tagaaggttt atttttttgg gatacgtata ttaatagagt tcgcgtagac 2880
gcggtacgcg gttttattta agagcgttag tttcgggaga aataaaatta tttttcgcg 2940
tatatcgttt tggttttttt ttaaataggt tgagggaggg taagtagata tttggggagg 3000
tatttgtcgt ttggatttaa ttgtacgggg tttttacgag gttttcggac gtttgcggga 3060
ggagggcgat agaggggaat tggttttatt taggaggggt tattttagga tatagaaagg 3120
gatatttcga tgggggaagg aaaggatttt tttttgaggg aagtagacgt tgtttatagg 3180
tttaagggtt tttagaggag ttagttatgg aaagggtttc ggtttagagg gacgtgaagc 3240
gattcgggcg cgtttttcgt ttttaaatgg taagcggcgg gggtcgggga agttgtggaa 3300
acgaggtttt tttttcgtta tcggtttaga aggaaggtta agtttttaga gtttagaagt 3360
tattagtaat ttgtgtcgcg ttcgtaacga ggtttttcga ttttagttat aaaggtttta 3420
gcgttttgtg ggttagattt aggttcgggg gtacgagttt cgtacgggga gggacggaga 3480
ttcgggtata aattgggacg ttttgtagtt agcgttcggg ggttcgttgt ttttcggttt 3540
gtttagttcg ggttatacgc gttcgttgtt tgcgcgaggg aggcgggaat ttgaagtcgg 3600
cgagagcgag gttcggttcg agtcgttatc gtagttatag ttacggcgta ggttggcgcg 3660
tcgggggattt tcggttttat ttgggttttg gggaggttag aagcgttttt aattttatag 3720
ggttacgttc gttttttttt ttcggggagt tagattggaa tggttgtttt tgattttatt 3780
tgttattaat ttcggtattt gtttttttatt ttataaacgt tttttttttaa gtaattgttt 3840
ttgttaatag cgtatatttt ttagtagcgt ttattgcgtt ataaataatt tcgatgaaga 3900
cgtaaggata tgatttttat aagattggat aattgattta atttgtgatt cgcgatggcg 3960
aataatattg gaaggttatt taaatagatg aaggtttaaa ttgttttgtt tgtatttgaa 4020
ttaatttttt atttattatt attatgaagt gattggttta tttaagtttt ttagtttgtt 4080
ggaacggaac gggatttaaa tgagattgta atataattta aatgtttgtc gattttaacg 4140
aggttaattg agtagttgta ataaatataa atattttttt aaatagtcgt gttttaaatt 4200
attatttaat gtttttatgt tttttaagt ggtagttttt ttttttttt tgtttttgtt 4260
tagaatatta tatatttatt tgttgaaaag gtgattttgg aaataatttt gtgtttttgt 4320
ttgttttgta gatagtagga tatgttttgt gattgggttt tgttttgttt tgttttgttt 4380
tgttttttttt ttagagttaa tatttttagg gtttttttaag ttggtataaa tggtgagttg 4440
agttttagtt ttattttaaa gtagtttttt gtgttgtttt cgtagagttt gggaggagtt 4500
gagtgatgga agaaatttgt cgtttggatt tagttattta tttagacgta gtttagttgg 4560
gttttgtaaa tgaattcgtg ttattgtatt tataaatgat attttgaaga gtggtattgt 4620
ttttgatttg tattgatgat cgttattatt ttgaataaat tagaatttta atatatttta 4680
aaggtatttt ttagaaagtt taatttgttg gtagtttggt tttttgtaaa ttaaaaggag 4740
taggtggagg ttgattgagg attttttat tagggaaggg gtatagaaag agaattttgt 4800

```
tataataaat ggtggtagga tggtggagga gggggttgat aattagagaa atgttaattt    4860
tgtggtttta attttttttt tttttattt  gtaattttat ttttattttt ttttttatta    4920
tttttgggtg atgatgtatg gagaggatgt taagattttt attttgagaa tgttattcgt    4980
ttggatattt ttggcggtaa ttgtttatcg cgatatttac gtttattttt cggcggatat    5040
atggtggttg gtgttttcgt agttggggaa taatggaagt aattaagcgg gagaagtttg    5100
aggaataaaa gaatttttta aattttagtg aaagggtcgg ggttggggat gtgggggat     5160
attcgattta ttgtgaggat ttgagtttaa tttcggggtg attgtttaag gttatgtttt    5220
tttcgaaagg tttagggtaa ggagatgtat tttgaatttt ttttttttta atttttggtc    5280
gagggagagg gatttttgt  ttggagagtg cgggtgtagg gtttgaaatt ag            5332
```

<210> 19
<211> 4215
<212> DNA
<213> Artificial Sequence

<220>
<223> chemically treated genomic DNA (Homo sapiens)

<400> 19

ggtgttcgat taagcgtagt tcgtagtttg ggtattgtta attgatttta atttttttta 60
tggtgagagg atggattttt cgttattttt tcgtttaatt tggtatttat ttatttattt 120
atttattacg ttcgttgggc gtatttaagt ttaatttcgg ggcgtacgcg ttagcgtaga 180
tatcgtattt tttttttttt ttcggttaat tttaggtaga attttaaaat aattgttttt 240
tttttttacga tttagatgtt gcggttcgcg gataggaggt ttaagaaata gtatatttcg 300
agcggtaggt agcgaggcgg aaacggtcgt cggttttagt ggtggtttta ttggaagtcg 360
agtttaggag cggttaagcg gtcgtcgggg aaagtatcgg ggtttttttag ggtttttttc 420
gagttttat ttcgtatttt cgagggcgtg aaaattacgg gagtcgtttt atttcgcgcg 480
tttagtttcg ttttagttta gatatcgttt ttcgttagtt tttttgcgg cgtttaggga 540
ggacgcgttt cgtttttttt taattcggtt aatgggcgtt cgggtagcgc gcggtttgtt 600
ttcgttttcg ttagggaaat ttggaggagg agaaaagttt gtatagaggg tggaaaggcg 660
agagcggagt tttaagttcg gtagttcgag aggaagatga atagttttag gttagagttt 720
ttggtagagt ggatttcgag tcgtttttag gtagttagga gcggttttag cggtagtcgt 780
aaattttagt agtcgttcgt gttgttcgtg gttgggcgg agggtagtta gagttgggga 840
ttaaggtttc gcgttatttg cgcgtatagt tttatatttg aacgttgttt tttcgtagac 900
gagatcggcg ggtattgtaa agttgggatt cgtttttgaa ggaaaaaaaa tagcgagtaa 960
gaaatttagt attatttttt attgatttat ttcgttgtat tttttgtttt ttaagttttt 1020
gaaagttggt aattttgatt tcggtgttta aaaatcgata gttattgaga tcggttttga 1080
gaagtcgaag atttggtagt ttttagattg agtaggataa ggtgaaagta ggttggaggc 1140
gggtttagga tatttgaggg ttgattttgg gggttcgtga ggttgttatc gttgttgtcg 1200
ttataggtga gatggcgttg ggttgacgtt ggggttaacg ggtagagaac gtagggatgc 1260
ggttttcgtc gaagagagtt aagaagggaa gagcgcgttt tttaaattgt ttttgtaatt 1320
tgtttttagt gagtatttta ttgatttaga atttatcgag aatagtatta gcgagttatt 1380
ttttttttga gatgggtttt atttatttg gtaatggagt gagttggatt gtggggagga 1440
agaggaatgg gaaaattagt ttataaatat taatgttagt aagagtgtgt tgttggtagg 1500
acgtatcgcg agtttggaga ttttggtggt cgtagttggt aagtggttat aatttagaaa 1560
gtaggatcga gttgtttttt ttgttttatt agtgtatcgt ttttcgggcg cgggtttaat 1620
attttataag tggtaatttt cgtttacggt agttttgttt ttttttatt atttttagat 1680
ttagttttgt attttaaggt tgcgtatcgt tagttattat tatgtttatt ttcgggtta 1740
attcgttcgt tttttgagt ttcgatcggt tgaatagttt agtgattatt tcggcggtga 1800
tgtttatttt cggggtggtg ggtaatttgg tggttatcgt ggtgttgtgt aagtcgcgta 1860
aggagtagaa ggagacgatt ttttatacgt tggtatgtgg gttggttgtt atcgatttgt 1920
tgggtatttt gttggtgagt tcggtgatta tcgttacgta tatgaagggt taatggttcg 1980
ggggttagtc gttgtgcgag tatagtattt ttattttgtt tttttttagt ttgttcggtt 2040
ttagtattat ttgcgttatg agtgtcgagc gttatttggt tattaattat gtttattttt 2100
atagttatta cgtggataag cgattggcgg gttttacgtt ttttgtagtt tatgcgttta 2160
acgtgttttt ttgcgcgttg tttaatatgg gtttcggtag ttcgcggttg tagtatttag 2220
atatttggtg ttttatcgat tggattatta acgtgacggc gtacgtcgtt tatttttata 2280
tgtacgcggg ttttagtttt ttttttattt tcgttatcgt tttttgtaac gtgtttgtgt 2340
gcggcgcgtt gtttcgtatg tatcgttagt ttatgcgtcg tatttcgttg ggtatcgagt 2400
agtattacgc ggtcgcggtc gtttcggttg tttttcgggg ttatttcgtt gtttttttag 2460
ttttgtcgcg ttttagcgat tttcggcgtc gtcggagttt tcgtcgtatc gcgggcgtcg 2520
agatttagat ggttatttta tttattgtta tttttttggt ggtgtttatt tgttttattt 2580

```
cgttcgtggt gagtgatcgg ggttggggtt ttattcggtt tttttttcgt atttattttt 2640
cgcgtttatt tttcgttttt tgtttttttt ttgagttttt ggtagtgaac gtgtcgtttt 2700
taggtcgggg ttgggatttt tatattgttt tttagagtag gtttaatttt ttttgaagtt 2760
ttaattttaa cgagatttag taggtgtttt gtttttatat tttttagttt atgttttcgg 2820
aagtttgggt tttttttttt atcgagatag tttttatttt ttgttgtttg atattggtcg 2880
agtttttttaa gaaaattttt cgtttttttt gttagacgtg gaggggagtt tgttgtagtg 2940
tgatttagtt tatttttttcg tattgtgaat tgtgaattgt aaaatttgaa atgttaggga 3000
tggtgatttg ttcgttgtaa attattttta aggaaagtgg ggtttttggt gttttgatgt 3060
atgtaaggtt ttttgaattt tttttttcgt ggattgcgta gaattttaaa gttagaagag 3120
atattaagag gtgttttagt ttaatttttt tattttattg agggagtgaa tgatacgttt 3180
aaaataattt agtcgaattt atatagttaa gttaggtttt ttgtttttta ttatggtgtg 3240
ttttgggagg gtagtttaaa gtatataaat tttaatttat atataggta gggtttagat 3300
ttagtttttt ttattttgtt tagattagga aagtaaagtt ttttagggtg attaattatg 3360
gataggttga tatatatagg gtatgggatt tgaattttcg ataattgtgc gaggtatagt 3420
attattttag agaaatcgag gtttagagat ggttgttttt gtttttttaa gttaaagttt 3480
tatagtttgt aatggatttt aggtcgtttg aggttagatt tagtttatgt gttattatat 3540
tacgtttttt tcgtagaata tttttttattt tggcgtttgt aaagcgtggg ttggagtttt 3600
ggtatatttt atttttagtt tatttttttaa gaattgtttt tggattaatt tttaaaatat 3660
gataaataga aaggtttaaa ttttatttag tttttttattt tgtttgtggt taaaatggaa 3720
tgtattagaa gtagatgggt agaataatgt aatattttat ttagagtttt ttttgaaagt 3780
aattagttat gagttttttt attttttaaa atattgtatt tttggaaatt ttaaattatt 3840
aatttaaaaa gagataattg tttttttttta tgtttgttgg gaataagaga atataaaatg 3900
taatgggtta ttattttttt tttttttttta ttgatattgt ttagggattt ttggtgtaaa 3960
aatatatttt tgataaaagt gattgtaaat ggaaggaatt tagaggggggt ttagagttag 4020
aatatagtag gtttttatta atttgtataa atagaagtaa atataatagg ttataagagt 4080
ttttaagtta ttttaggatt taatataatt tatagagtta agttatattt gagttatata 4140
aatatatttt attgtttgta atggtaattt agaataaagt gagattttgg aagaaataat 4200
attgtttttg atggg 4215
```

<210> 20
<211> 4215
<212> DNA
<213> Artificial Sequence

<220>
<223> chemically treated genomic DNA (Homo sapiens)

<400> 20

```
tttattagga gtagtgttgt tttttttaaa attttatttt attttaggtt gttattatag     60
atagtaaaat atatttatgt ggtttaagtg tgatttaatt ttgtaaatta tattgggttt    120
tggaatggtt taaaggtttt tatggtttat tatatttatt tttgtttata taaattggta    180
agaatttatt gtattttggt tttgagtttt ttttaagttt tttttattta tagttatttt    240
tattaaagat atatttttat attaaagatt tttggatagt attaataagg aaaaggaagg    300
aataatagtt tattatattt tatatttttt tgtttttaat aagtataaga aaaagtagtt    360
attttttttt gagttggtag tttagaattt ttaggaatgt agtgttttgg aagatggaag    420
aatttataat tagttgtttt taagagaaat tttagataaa gtattgtatt attttgttta    480
tttgtttttg gtgtatttta ttttagttat aggtaaagta gaaaattagg tagggtttga    540
gtttttttgt ttattatatt ttaaaagtta atttaaaggt aatttttaaa agatgaatta    600
aaaatgaagt gtattaaaat tttaatttac gttttataaa cgttaaaatg agaaatgttt    660
tacgagggag acgtgatgta ataatatatg agttgggttt ggttttagac ggtttaaggt    720
ttattataag ttgtagagtt ttggtttgaa gaaataaaga tagttatttt taaatttcgg    780
tttttttaaa atggtattgt atttcgtata gttgtcgaga atttaaattt tatattttat    840
gtatattagt ttgtttataa ttagttattt tgagaaattt tgtttttttg gtttagatag    900
aatgaaagaa attgagtttg gattttgttt tgtatatgaa ttagaatttg tgtgttttaa    960
gttgtttttt tagggtatat tatggtaaag agtagggagt ttagtttgat tgtgtaggtt   1020
cggttggatt attttaagcg tattatttat ttttttagtg aagtgaggga gttagattaa   1080
aatatttttt gatatttttt ttagttttaa aattttacgt aatttacggg ggaagaaatt   1140
taagaaattt tgtatatatt aaaatattaa aaattttatt ttttttaaaa atggtttata   1200
acgaatagat tattattttt gatattttag gttttgtagt ttatagttta tagtacggag   1260
gaatgggtta agttatatta tagtaggttt ttttttacgt ttaatagagg gggcgggggg   1320
tttttttgga agattcggtt agtgttaggt agtaaggggt aaggttgttt tcggtggaga   1380
aagaaattta ggttttcggg aatataaatt gggggatgta ggggtaaagt atttgttaaa   1440
```

tttcgttagg gttgggattt taaagagggt tgggtttgtt ttgagaaata gtgtgggaat 1500
tttagtttcg atttaaaggc gatacgttta ttgttaagga tttagaggga aagtagggag 1560
cggggaatgg acgcgggagg tggatgcgag aaaaaggtcg agtagggttt tagtttcggt 1620
tatttattac gagcgggatg gagtagatga gtattattag ggaggtggta atgagtaaga 1680
tgattatttg gatttcggcg ttcgcgatgc ggcggaagtt tcggcggcgt cgaaagtcgt 1740
tgaggcgcgg taaggttggg gaggtagcgg ggtggtttcg ggaggtaatc gaggcggtcg 1800
cggtcgcgtg gtgttgttcg gtgtttagcg aggtgcggcg tatgaattgg cggtgtatgc 1860
ggagtagcgc gtcgtatata agtacgttgt agaggacggt ggcgagaatg aggaaggagt 1920
tgaagttcgc gtatatgtag gagtaggcgg cgtgcgtcgt tacgttggtg gtttagtcga 1980
tgaagtatta ggtgtttggg tattgtagtc gcgagttatc gagatttatg ttgggtagcg 2040
cgtaaaagag tacgttggac gtatagattg taaagagcgt gaggttcgtt aatcgtttgt 2100
ttacgtagtg gttgtagaaa taggtatggt tgatggttag gtagcgttcg atatttatgg 2160
cgtagatgat gttgaggtcg gataggttga agaagagtag aatgaaggtg ttgtattcgt 2220
atagcggttg gttttcgggt tattggtttt ttatgtacgt ggcgatggtt atcgggttta 2280
ttaataaagt gtttaatagg tcggtgatag ttagtttata tattagcgtg tagaaggtcg 2340
tttttttttg ttttttgcgc gatttgtata gtattacgat ggttattagg ttgtttatta 2400
tttcgaagat gaatattatc gtcgggatgg ttattgggtt gtttagtcgg tcggggttta 2460
aggaggcgga cgaattgatt tcgggagtgg atatgatagt ggttggcggt gcgtagtttt 2520
ggagtgtaag gttgggtttg gggatggtag aagagagata aagttgtcgt gagcggaaat 2580
tattatttgt aaggtgttag attcgcgttc gagaaacgat atattgataa gataagggga 2640
gtaattcgat tttatttttt ggattgtagt tatttattaa ttgcggttat taaaattttt 2700
aggttcgcga tacgttttgt taatagtata tttttgttga tattaatatt tataaattga 2760
ttttttttatt tttttttttt tttataattt aatttatttt attgttaaga tgaataagat 2820
ttattttaag ggaaaagtag ttcgttagtg ttattttcga tagattttga attaataaaa 2880
tgtttattga aaataagtta taaaagtaat ttggagagcg cgtttttttt tttttggttt 2940
ttttcggcga gggtcgtatt tttgcgtttt ttattcgttg attttaacgt tagtttaacg 3000
ttattttatt tgtagcggta gtagcggtgg tagttttacg agtttttagg gttagttttt 3060
agatgttttg gattcgtttt taatttgttt ttattttgtt ttgtttagtt tggaaattgt 3120
taaattttcg gtttttttaaa gtcggtttta gtggttgtcg atttttggat atcgaggtta 3180
gagttgttag tttttaaaaa tttgggaaat aagaggtgta gcgggatggg ttagtgaaga 3240
atggtgttgg atttttttatt cgttattttt tttttttttaa agacgagttt tagttttgta 3300
gtgttcgtcg gtttcgtttg cgggaggata gcgtttaggt gtgaggttgt gcgcgtaggt 3360
ggcgcggagt tttggttttt agttttggtt gtttttcgtt ttagttacgg gtagtacggg 3420
cggttattgg agtttgcggt tgtcgttgag gtcgtttttg gttatttggg ggcggttcgg 3480
ggtttatttt gttagaggtt ttggtttggg gttgtttatt tttttttcgg gttgtcgggt 3540
ttggagtttc gttttcgttt ttttattttt tgtataaatt tttttttttt tttaagtttt 3600
tttggcggag gcggaggtaa atcgcgcgtt gttcgggcgt ttattggtcg gattggaagg 3660
gggcggagcg cgtttttttt gggcgtcgta gggaagattg gcgggggggcg gtgtttgggt 3720
tggggcgggg ttgggcgcgc ggggtggggc ggttttcgtg gtttttacgt tttcggaggt 3780
gcggagtggg aattcggagg ggatttttggg aagtttcggt gttttttttcg gcgatcgttt 3840
agtcgttttt gaattcggtt tttagtgggg ttattattga aatcggcgat cgttttcgtt 3900
tcgttgtttg tcgttcggag tgtattattt tttgaatttt ttgttcgcgg gtcgtaatat 3960
ttagatcgtg gaagagaggg tagttgtttt aggattttat ttagggttgg tcgagaaagg 4020
agaagaaata cggtgtttgc gttagcgcgt gcgtttcggg gttagatttg ggtgcgttta 4080
gcggacgtgg taggtgggtg ggtgggtggg tattagattg ggcggggaaa taacgaagaa 4140
tttatttttt tattataaaa ggagttggag ttagttggta gtgtttagat tgcgggttgc 4200
gtttggtcgg gtatt 4215

<210> 21
<211> 5473
<212> DNA
<213> Artificial Sequence

<220>
<223> chemically treated genomic DNA (Homo sapiens)

<400> 21

```
gtaatgttaa ttagttttat tttaaagaaa aattaaatat tagagtaaat aatgattttt      60
ttgcggagaa ggagttgtaa gggttgggga gaggtaatta gtgttataaa agttttatta     120
taaagaagtt tagaattttt ttgttttgtt gtgtagtaga gtgaaatata gtagtagaat     180
aattgtgtat tattattatg ataatttaat ttgttttttt agaaaaagtt attgtttgta     240
gttaggatag taggaaaata tttagaaaat aattaaaatg taaataaaag tatgaaataa     300
```

taataaagtt tattttttaa gagaaaaaaa gaagagtatt tagcgaaagg agaatgaagt 360
atatataaag taagtaattt ttaggtattt tgttttttttg gatgttattt gtttaaattt 420
gataattttt ataggtagta tgatagattt tttaaaattt aaataaagtt tgtttttgta 480
ttttattttt aataataaag tatattgatt gatttataaa ttaaagtttt taaaatttta 540
ttaaagtaaa aatattgttt tagagttttt attggtatgt ttttgaaagt aagtgttata 600
gttaatgtaa atttttaaaa atttttttgt tatttagtat aatagagggt ggattttgtg 660
ttagggtggt atttttttgtg taatttatat ggtttgaggt tttttgtttt tttttttttt 720
agaagagttg gaattaagat attaataatt ataattgtaa taattattat tatgtttttta 780
aggggttgtt gagtttttgg agatttagt gtatagagcg aagtaggaga ggtttaaaaa 840
ggaaggtttt gttgttgatg ttgtgatgat taaggtagtt tattattatt tttttttttt 900
aacgtgattg ttttgttttt tttttttttg atttgttat aagatgtggg gaagtttttt 960
aagaattgtg tatagttcgt tatagatggt ggtttttgtg tttagagatc gaaagagtat 1020
ttgttatagc gatattttgt aatagttgta gatgtgcggg tttaaaattg ttttatgtgt 1080
ttaaattaat aataattatt attttatttt attttattat ttgagcggaa atataatttg 1140
aaatatttaa atttgttttt tttaaagtta gaaggatatg tgtatgtgaa tgtatataat 1200
ttgttttttt aaatgttttt aaagtttatt tttgttatta tatttgtttt tataattaat 1260
aaagaaaatt tgttttagtt ttaattttat tgggtatggt acgtgttgga gggtaggttt 1320
tggaaatata aatattaata tatgtggttt gttttaaaat ttaatgtatt tagggttagt 1380
ttcgttgttt tggttaaatg aatgaataaa gagtgattaa aatgcgttta tttttttcgtt 1440
attttatata tagtttgaga tcgatcgtta atagtagatt gtaaattttt gtatttaata 1500
gttattaatt tttttttatag tttgtatatt tataaattta attgggatat tcgtttgata 1560
aatttagatt ttgtagagta gatttttaag gaaatttaat tttagagaat aaaatatttt 1620
ttggaatttt taaggtattt agagataaaa cgtttttttgt gggatttttt tttaagtttt 1680
ataaaagtag gtttattagg attaaattat attaaagtta gtgcgaggta agggggggtgt 1740
ggtttataga aattttggga gaaagtttag tatagttttt tttttttggg aatatatgtt 1800
aggatggaaa aaaaaaaaag gaggggagtg ttataggatt aagatttatt ttggaaaata 1860
agattttag taaagatatt tttttttttt ttattttata ttagatatta gaagtattat 1920
ttttcgagtg tgttttttaa gttttttttt aattttaggt ttttttgtt ttttgggttt 1980
tcgatttttt ggtagttggg tatttgtatt cgggttttcg ataggtttag tcgttggttt 2040
agtttttttc gtatgaaggc gtcgggatag tgggtttcgt taaaaagttt ttttagttta 2100
ttgagttgtt ttagggtgaa attggttcga tttcgttttt gtttgatttt ggtttggttt 2160
tcgtttttta tttttttcgt attttttttg cgatttttta gtttcgggga tattggaggg 2220
ggtattttag cggggttata cgtgtattta tacgaatata tatatatacg tatatatata 2280
cggacgaaaa tagtatagta aatttgtta ttagatttgt cgtaaaaaag aatagagaat 2340
tttttttttt cgtggaattt tggttcggtt ttttaattta ttcggaggaa gaatatttcg 2400
gagaagcgta aaggttaagt ttgagcggtc gtttggggta aggtgggtag ttatttatta 2460
tttttttttat aaacgtattt cgatttatcg ttttattatt tatttttttag ttatcggttt 2520
ttttagtaa atattaaatg gtttttattt atttttcgtat ttatttgttt cgtagtattt 2580
atagaaatta aaggcgtagc gttaggtttt ttatagagta aagtttggat ataagggcgc 2640
ggtaaggtta tttattggtt ttttatatcg gttattttta gaaggttggt tcgtttttag 2700
gttagttttt attatcgttg gttttttgtt tatcgtaaat ttgttggttt aatttaggaa 2760
taattgggtc gaaaggtatt agcgagagta atagatttcg gtgttgtgtc gtatagggag 2820
tcgtattcgt agacgttttt cgttgttttt gggttcgggt taaatttgt ataaggtttt 2880
ttggatagtt aggtaatttt cgtttcgttt gttcgatcgg ggtcgtacga gtataggcgt 2940
ttacgttatg ttggttgttt aaagggttcg tcgtttaagt cgggttagaa ggtaggaggc 3000
ggaaaattag ttttcggtgg cgggcgaaag taatcgtttt ttttgttttt ttttcgtttt 3060
ttttcgtgga aacgtagatt cgattttaaa cgtttaattt atagagatta ataggtttaa 3120
gcggaatatt cgcgattttc ggtttttatt ggttgtttaa agttttttta tgtaattagt 3180
agttcggatg ttttaataaaa tatgaatttt tttgttttgg gtttgtatat attcgttgtt 3240
attttttttgt tttttttttt tttttaaaaa taggagtttt tttagtgtga gtatatatag 3300
ttattatata tatatttttt tggaattttt atttttttgcg ttcgaattag agatttcggg 3360
ttttaagaag ttaaagttga gtttagatag tcgaggttgt ttgtagtttt ttttagcgta 3420
ggatttaggt tagtatgggt gttaaaaatt attttcggaa gagtcggttt gtagagaaat 3480
taatatttgt tttgagtttg gaaattaata attttaggaa ttgcgttcgt cgggggttta 3540
gatttgaatt tcgtcgttag gcgcgttggc gaatttgtag ttcgggtttg tagttgtgga 3600
tttatggtcg ggaggttgta ttgggtattt tgacggtaat tttattattt tataaattta 3660
ggaagatttg gtgttggttt ggaggaagaa gagtcgttag atttcggttt tgtcgggtat 3720
tttagttacg gtgattttaa atattgtcgt atttttttagt ttttagagtt agttttgtgg 3780
atatttgttt ttttttttagg aaggaatatt atagtaaata ttatgggtta atttaatagt 3840
aaagtatttt tattgatatt gtattgaaat ttaaatatga aaatatattt tttaaatttt 3900
gttaatgagg tgggaaggtt ttgtttatta tatgttattt atatagtgga tgttatttaa 3960
agttttgggg tttcgaggag atgtttaaat tttaagttga tttatagatt taataaaatt 4020
tttattttg tttttaaatt tttaattatt gatttaaata ggaatagatc gtgttataaa 4080

```
taatttaatt gtttgtttaa atatgtgatt ttacgtatgt tatatatttt tttaattgga    4140
aaaatatggt agtatattag ttattgtagg tattaagtgt taaattaatt ttattgttga    4200
atttttttga attggtatag aacgttttgt tattgagata aagacgtttt ttaatattga    4260
gttagtatta aaggattttt gttttaagag aatcgagtat tgggtgattg aaaatattaa    4320
aggaattgtg taaatataga ttttttatttt tttcgtcgtt tttttttttc gtttttaata    4380
tattaagaaa tcgaaacgtt tcgttcggga gaagtagcgt agtttggatt ttagttttcg    4440
ttggtttttt tttattttga aagagaatgg gaagttttgg tttttaagat ttaaagattt    4500
aggggttttg cggagttcga ggggttttgg cggttttaaa tatttaggcg atcggagggt    4560
taagtttttt atttttgttt tttttttttt tcggtaaatt ttgcgttaga ggttagtttt    4620
agtttcgtcg tttggtttag tatttttttt tgtagttcgg tttcgcgaat ttttacgttc    4680
gtttgcgggt tattcgggtt gcgggtttat tttttcgttg gtgaaaaatg tatttcggtg    4740
gaatgggaat atttgggaag ggcgcgtata aattttatac gttttttttt acgttatttt    4800
tataatatat taggatttta tttatgatcg agtatattat cggattttta ttttttttatt    4860
attattttag ttgataaagt tggggtttag ggtttttttc gtgttttttt tttttttttcg    4920
ttgggtttcg gagtttttttt tcgttcgaga ggagttcgga aggcgggaag gggaatcgtt    4980
gtcgggggtt agttaggtcg ttatttttcg ttagtcgcgg gttgttcggt tttttgtttt    5040
tttcggcggc gtttatgttt agttttcgga cgggagagcg ttttttttta gtttttttcgt    5100
ttgttttttt tttttttata tttttttcgt ttttttcgtc gtcgttttcg ttttttttcgt    5160
cgtcgttttc gtcggtcgtt cggattgtcg ggttgttgcg gtcgtcgcgg ttcgtttcgg    5220
tgtagtcggt cggtttttttg gtttcgcgta gcggttcgtt ttttagtatt tttcggtacg    5280
tgatcgtttt tttttttttt tttattttt ggttaaaaga tttggagacg aacgtcgtaa    5340
gttttttttat cgtcgtcgta cgttagttcg gcgtttaatt tttgttagta gagtttcgtt    5400
tttttttttt tttttttttt tttattgttt tagttttttt aataataata tattaatggg    5460
ataggaggtg ggg                                                       5473
```

<210> 22
<211> 5473
<212> DNA
<213> Artificial Sequence

<220>
<223> chemically treated genomic DNA (Homo sapiens)

<400> 22

```
ttttattttt tgttttattg atgtgttatt attggggggg ttggagtagt aaaaaaagaa     60
gaaggaaaaa aagagcgggg ttttgttggt agaggttgag cgtcgggttg acgtgcggcg    120
gcgatggaag aatttacggc gttcgttttt aagtttttg attagaaagt gaaggagaag    180
aaggaggcga ttacgtatcg ggaggtgttg gagagcgggt cgttgcgcgg ggttaaggag    240
tcgatcggtt gtatcgaggc gggtcgcgac gatcgtagta gttcggtagt tcgggcggtc    300
ggcggaggcg gcggcggagg aggcggaggc ggcggcggag gaggcggagg aggtgtagga    360
ggaggaggag taggcggagg agttggagga gggcgttttt tcgttcggga gttggatatg    420
ggcgtcgtcg agagaagtag ggagtcgggt agttcgcgat tgacggaggg taacgatttg    480
attgattttc ggtagcggtt tttttttcg tttttcggat tttttcggg cgggagagga    540
tttcgaggtt tagcggggag gggagaggga tacggagagg gttttggatt ttagttttgt    600
tagttagagt gatagtggaa gggtgggggt tcggtggtat gttcggttat gggtggggtt    660
ttagtgtgtt gtgggggtgg cgtgggggga agcgtgtggg gtttgtgcgc gtttttttta    720
gatgttttta ttttatcgaa atgtattttt tattagcggg aggatgggtt cgtagttcgg    780
atggttcgta ggcggacgtg gaagttcgcg gggtcgggtt gtaggagggg gtgttgaatt    840
agacggcggg attaaagtta gtttttagcg tagagtttgt cgggagggag aaggggtaga    900
ggtggaaagt ttaattttc ggtcgtttag gtgtttgggg tcgttaagat ttttcgaatt    960
tcgtagagtt tttgaatttt tggattttag gggttaaaat tttttatttt ttttttaaggt   1020
agaaggaagt taacgaaagt tgaggtttag gttacgttgt tttttcggg cgaggcgttt   1080
cggtttttg gtgtgttggg ggcggggggg gggggcggcg ggggaggtgg ggatttatat   1140
ttatataatt tttttagtgt ttttaattat ttagtattcg gttttttag agtaaaaatt   1200
ttttggtgtt agtttagtgt taggaaacgt ttttatttta atggtagagc gttttatatt   1260
aatttaaagg agtttaataa taaaattgat ttggtatttg gtgtttgtag taattggtgt   1320
attgttatat ttttttaatt aagaaaatat gtggtatgcg tggaattata tatttaagta   1380
agtaattaag ttgtttatga tacggtttgt ttttgtttag attagtaatt ggaaatttgg   1440
ggataaaaat agagatttta ttaagtttat ggattaattt gaaatttgga tattttttcg   1500
aagttttaaa attttgagtg gtatttattg tgtagatgat atgtaataga taaagttttt   1560
ttattttatt ggtagaattt gaagggtgtg tttttatatt taaattttaa tataatatta   1620
gtaaaaatat tttgttatta aattggttta taatgtttat tataatgttt tttttttaaag   1680
```

aaaggataaa tgtttataaa attaatttg agaattagaa aatacggtaa tatttaagat 1740
tatcgtagtt agaatgttcg gtagggtcga gatttagcga tttttttttt tttaaattag 1800
tattaggttt ttttaaattt gtaaagtggt ggagttatcg ttaaggtgtt tagtgtagtt 1860
tttcggttat gagtttataa ttataggttc gaattatagg ttcgttagcg cgtttgacgg 1920
cgaggtttag gtttgaattt tcggcgggcg tagtttttgg gattgttgat ttttaagttt 1980
agggtaagta ttaatttttt tgtagatcgg ttttttcgga aatgattttt aatatttatg 2040
ttggtttgga ttttgcgttg ggaagaattg taaatagttt cgattattta aatttagttt 2100
tgattttta aaattcggag tttttgattc gggcgtagag gataaaggtt ttagggggat 2160
atatatatgg tggttgtata tgtttatatt agaagagttt ttgtttttaa agggaggaga 2220
aggataggaa agtggtaacg gatgtgtgta aatttagagt agaaaaattt atattttatt 2280
aaatattcga gttgttggtt gtatgaaaag gttttgagta attaatagaa atcgaggatc 2340
gcgaatattt cgtttgaatt tgttgatttt tgtgggttaa gcgtttaggg tcgagtttgc 2400
gtttttacga gggagggcga agagagaata gaaagagcgg ttgttttcgt tcgttatcgg 2460
aggttggttt ttcgtttttt gtttttttggt tcggtttggg cggcgagttt tttgggtagt 2520
taatatggcg tgggcgtttg tgttcgtgcg atttcggtcg ggtaggcggg acggagatta 2580
tttggttgtt taggggattt tatgtagggt ttggttcgag tttaggggta gcgagggggcg 2640
tttgcggatg cggttttttg tgcggtataa tatcggggtt tgttgttttc gttgatattt 2700
ttcggtttaa ttgtttttaa attagattag taagtttgcg gtaggtagag agttagcggt 2760
ggtgagagtt ggtttgagaa cgagttagtt ttttgggaat ggtcggtgtg agaggttagt 2820
gggtggtttt gtcgcgtttt tatgtttaga ttttgttttg tgagaggttt ggcgttgcgt 2880
ttttgatttt tgtgagtgtt gcggagtaag tgagtgcgaa agtgagtgga aattatttgg 2940
tgtttgttgg ggaaggtcgg tgattggggg gtgaatagtg aggcggtgag tcgaggtacg 3000
tttgtgaagg aggtggtgga tgattgttta ttttgtttta ggcggtcgtt tagatttgat 3060
ttttgcgttt tttcgggata tttttttttc gggtgggttg agaagtcgga ttaggatttt 3120
acgagggggga aggattttt atttttttttt gcgataaatt tggtaatagg atttgttgtg 3180
ttgttttcgt tcgtgtgtgt gtgcgtgtgt gtgtgtgttc gtgtggatgt acgtgtggtt 3240
tcgttggggt gttttttta gtgttttcgg agttgaaaga tcgtaaagag gatgcgaaag 3300
ggatggagga cgaaggttag attaaaatta agtagaggcg aagtcggatt aattttattt 3360
tggaataatt taatgagttg gagaggtttt ttgacgagat ttattatttc gacgttttta 3420
tgcgagagga attgagttag cgattgggtt tgtcggaggt tcgagtgtag gtatttagtt 3480
gttaggaggt cgagggttta gggagtagag agagtttggg gttggaaaga ggtttggggg 3540
atatattcgg gaggtggtgt ttttagtgtt tggtgtaaga tgagaaggaa agagatattt 3600
ttattagagg ttttattttt taaagtagat tttagtttta tagtattttt tttttttttt 3660
ttttttttat tttaatatat gtttttagaa gaagaaagtt gtgttgggtt tttttttaga 3720
gtttttataa attatatttt ttttgtttcg tattggtttt aatgtggttt aattttaatg 3780
ggtttatttt tataaggttt aaaaaaaaat tttatagaaa acgttttatt tttggatgtt 3840
ttaaaagttt taaagagtat tttgtttttt ggagttggat ttttttgaaa gtttgttttg 3900
tagggtttag gtttgttaag cggatatttt aattaggttt atagatgtgt agattataaa 3960
gaaaattagt aattgttaag tgtagaaatt tatagtttgt tgttagcggt cgattttaaa 4020
ttgtgtgtga aatagcggag aaataaacgt attttggtta ttttttgttt atttatttaa 4080
ttaaggtaac gaaattgatt ttaaatatat tagattttaa aataaattat atatattagt 4140
atttatattt ttaaagtttg tttttagta cgtgttatat ttaatgaagt taggattgaa 4200
atagattttt tttattggtt gtggaggtaa atgtaatagt aaaaataggt tttggggggta 4260
tttaaaggag taaattgtgt atatttatat atatatattt ttttagtttt aggggggaata 4320
aatttaaatg ttttagattg tattttcgtt tagataataa aatagaataa aatgatagtt 4380
attattggtt tgaatatatg gagtagtttt aggttcgtat atttgtagtt gttataaagt 4440
atcgttgtaa tagatatttt ttcggttttt gggtatagag attattattt gtagcgaatt 4500
atgtataatt tttaaggggt ttttttatat tttgtaatag gattaggaaa aaaaaaaata 4560
gagtagttac gttgaagagg aaaagtaata ataggttgtt ttggttatta tagtattagt 4620
agtagagttt ttttttttaa gtttttttttg tttcgttttg tgtattgaga tttttagagg 4680
tttagtaatt ttttaaaaat atgatgataa ttattatagt tatgattatt agtattttaa 4740
ttttaatttt tttaggggaa aaaaaggtaa gaagttttag gttatgtgaa ttatataaag 4800
gatgttattt tggtataaaa tttatttttt gttgtattaa atagtagaga aatttttgaa 4860
gatttatatt gattatgata tttgttttta aaaatatatt aatgaaagtt ttgaagtagt 4920
atttttattt taatggggtt ttaaaaattt taatttgtga gttaattaat atgttttatt 4980
attgaaagtg gaatataaaa gtaagtttta tttgaatttt aaaaaatttg ttatattgtt 5040
tgtaaaagtt gttaagttta agtaggtaat atttaggaga ataaagtatt taagagttat 5100
ttgttttata tatattttat tttttttcg ttgagtgttt tttttttttt ttttttaagaa 5160
gtgaatttta ttgttgtttt atatttttat ttatatttta gttatttttt agatgttttt 5220
ttattatttt aattgtaaat agtggttttt tttaaagaag taaattagat tattatgata 5280
atggtgtata attattttgt tattgtgttt tattttgttg tatagtagaa tagagaaatt 5340
ttaaattttt ttgtagtgag gtttttatga tattaattat tttttttaa tttttataat 5400
ttttttttcg taaaggagtt attatttgtt ttggtgtttg gttttttttt aagatgaagt 5460

taattaatat tat 5473

<210> 23
<211> 5328
<212> DNA
<213> Artificial Sequence

<220>
<223> chemically treated genomic DNA (Homo sapiens)

<400> 23

```
ttgataagag aaaaggtgtt gagggtaggt cgcgttgttt tatttatagt ttattgtttg 60
ttgtagagtt tttgttagga ttggaaagtt aatttagtgt tgattaaaag tattatagag 120
ttaaaggatt ggtaatgatt gttaatttta ttttagtttt atgtattttt aattgttgta 180
attaaagata cgttatttaa atataggtga agggtttttt ttttttaagt tgggttgggg 240
gggggtgggt gggtggatta taattttatt tagattttt atttatttg ggtgttatgt 300
agttttaaat tagattttat tattattatt atttagagtc gtttttgtt atttatattt 360
ggttttgtag aatcggaagt gtgaaatgtg ttatttatat aaaagttttt gtttaaagaa 420
agggtgggac gggggagtgt agaggagtaa ttatttaggg gtgatttaaa tatttaggtt 480
tttttttttt tttttttaaa ttaatagaaa ggttttcgat atagttaaaa gtatttgaaa 540
ttgggggtaa ggttaatagg ttaaagggat tagtgttagt tttttaagta tttttaaaaa 600
gattatgtga atagatggaa aaatatattt ttaattaaat aatttttttt atattatagt 660
tgtagtgttg gtttagaaat cgtgtaattt tttattcgat aggagttttt ttatgagaat 720
attaaaggat aaaaagtttt gtattttcgt agttggttaa aattgataat tattttttta 780
tgcgcgcgcg cgtatatata tatatatata tatatatata tatatattaa tatatatata 840
tatatatata tatatatata tatatatata tatttttaaa aaagaattta gtttttaatt 900
tttttttatt tacgaagatt aaagttttgt attcgatttc gcgagcggtt ttcgtttttc 960
ggggttggaa ttcgatttag atgtgaagcg gtattgagtc gttgcgtagg ttcgtgttta 1020
gcgtttttttg ttggatatag taggagttgc gtagcgttag ttttagaaat cgttggaatt 1080
ttgttttggg gttagagaaa atttttttat tgttttttaa aaaagaaacg ggatgcgaaa 1140
ataaattttt atattttttga aaaataatat aatattaatt taataaaaaa ttaaagaggc 1200
gatattataa ttttaattta ataaaaaata aagattgttt tttttatatt ttagttattt 1260
tttattcggt ttggttttgt aatttttttg aaaggttttg cgatggtaaa ataggtaaag 1320
aaaagtgacg aaaaataaag tattttttttg tagtcgtgga gaattaagta aagtatattt 1380
ttttcgtttt gtttatattt taaaaaatta aacggaagat gttgttgttt gttgttttga 1440
cgttgttagt agcgttttga atgtttttag aggttttgta agtatcgtaa ttgagttttc 1500
gagtaaacga aataatttgt ttttatgtgt ttattttttag agtataatta aaagattagt 1560
ttttttttg agtgtttttt atttttttaga ttaattttgg ttaaagtttt ttttttttag 1620
atattggtta agaaatatat attggttaag aaataggtgt ttggtatttt ttttttttta 1680
ttttagtcgt aggatttcgg ggaaggtgtg ttaatggtag ggaaggtttt ttttgaaatt 1740
tagtatttcg ttgattagga atttaaatta ttttagggaa gggggagtat ttttatattt 1800
ttaaaaaatt atttgtttat ataataaaga gttttgtttt ttttattatt attatttttt 1860
tttttttttt ttggttagta agtgagggta aaagtagttt ttgaattatt ttagaaagag 1920
aaattgtaaa aatggtatat tttttgaatt taaaataata gggaaatgtg ggtttgttta 1980
attgtttaaa attaaataga aataattatt gtgttaaatg aataagatgt tttaagattt 2040
atattttttag ggttatggtt attgatttta attttatttt taagaagttt agagatcgcg 2100
tttttaaagt ttgtttattg acggaaatcg gtttaatttt ttataaaaga atggtttttt 2160
aaaatattga atattatggt tatgtaaggt ttatgatatt tttatgttta ttttttttat 2220
tttttaaaat tagcgaattc gagataatat tttaagggga attgttattt agtgcgatga 2280
gagatgattt ttttatttgg ttttagaatt agatttcggt ttaggttttt tagtaagtag 2340
gtttagatgt aatggaagga gagaagaatt aagtttttttg ataaaatttt tatttcgaat 2400
tttatggaaa agataggtaa gcgggatttt agttatagta tttttatttt ttgttttatt 2460
aaaaggtttg tttttattat agaaataata tagtcggtgt tagttttgtg aattcgcgtt 2520
aatcgataag gtaagaaacg gtgcgttgat agtcgaggtt cgaaggttgt ttagtggttt 2580
tcgtagttcg tggggtaggg aggttaaagt tagtaaaatg ttggaggcga cgttagaaag 2640
agagtatatg tttgttattc ggaagtgatg tcgaacgtgt aatagtagag ttttggagat 2700
ttttatttcg tttcgtattt agggatttta gtttgtgggg tagtaggttg ttgggtgaag 2760
gtaggaggga gaagtcgtat ttggttgagg gcgggtgggc gtgtcgggag cgagtgttta 2820
atttttttagg gtttatggag aaagtataat ttttagttga ggaaagagag ttaaataagt 2880
gggtggttta ggtttgggaa tgttatttag tttttttcgac gtagtgttat ttttgtggtt 2940
ttaggggggtt ttagtatttg ttcgaatttt atttttttaag gtcgttagta ggaaggtagt 3000
tttttttattt ttgagggttg aggagggggt agcgagtgat ttgatttttt agtagtaggg 3060
```

aaaatcgtag cggttggtaa cgggtcgcgt tttttcgcgg cggtaaagcg tttttaggg 3120
gcggggcggg tatttttggt tttttgtttt agcgtttagg tttgagttcg cgcgggttgg 3180
ttgggcgttt tgttttgtaa gtaattaatt ttgttggatt ttgaggatta tttaggcgga 3240
tgggtttatt tagtttaggt ttattgagat tttgttaatt ttttagattt tttttttttt 3300
tattttttat atttttatg atgaatttag gaaagaattc gaattttgaa aaatagtata 3360
tgtggtttga agtttgatag ttatttgggt gtgagggata tgattttatt aataaagatt 3420
aagatttaag gttgagtttt tatttattaa atttgttttt ttattttgtt tttaaatttt 3480
ggagattttt tagtttcgtt gttttaattt tgatatagat aaaggcgtta gggagaaagt 3540
ttaggcgggt ttgaagtagt tgaggtatag attgatattt atttttttat tatttagacg 3600
ggtagataag tattgttaga ttgggggttt taatttaggg tttagcggtt acgtgggttg 3660
ttgcggggtg ttgaaattat aagtataatt tgatattatt gagtttacgg tgtttgttaa 3720
taatttgtga attaggtgta gagattgtta ttgtagatta taatagtttt tgaaggattt 3780
attatagttt atcgtgttaa tgatttttaa attaaggagg ggaggtgggg cgagacgaaa 3840
aaggagtggg gagagttagt aaaaagggaa gaagggtggg agggaagata aaagttagag 3900
ttttagtttc gaaggagttt ttaaagatta ttaggttagc gatttgagaa ttttattggg 3960
atgggtttgg aaatttaatt ttatttgagt taggattgga agagggggcgt tgggggtaag 4020
tagaagtggg tttttgggaa ggtcgatttt ttaggtattt tttttgaggt cggaatagtt 4080
tttgggagag ggttttagat gttaatggga ggttttgagg atgttagttc gtttaggatg 4140
ggtgtatatt ttgatttaga gtatttttta gggtaagtcg tagttgtaga tttttgaaaa 4200
aagtggtaat agtttcgttt agggatgatg gtgattgtag gatgattttt ttttttttaat 4260
ggtttgtttt aatttttttt tatgttatag gtgaaagttt tttttttttt tttttgatag 4320
tatttttgtt tgttttttat atgttacgta gatttaattt tttttttttaa tttgtttttt 4380
tgggtgttaa atatattagt tttttgagat tttattattt ttgtggtttg tattgagttt 4440
aattgtaatt ttggaaaggg aatttgtgga taatatagta gtagtatgtt ttataagatt 4500
ttagaagaaa gaagattaaa ttttatttat gcggtgtggt aggggttagg ttaaggaaa 4560
gaggtttttt ttgtttggta aattgttttt gtagtgaaag ggtagtttag tttttattgg 4620
agtgttattt aatattggtt agagaaaggt taaggaaagt gtaggtttgt tttttttata 4680
aaagttgtgg ttataattaa acgaagtaat tatagttgga gaatttaaaa taattgtttt 4740
ttttttaaaa tgagggtaat aattttagtt attggaaaat tgggatttta aggaaaattt 4800
tttttttttgg tataattaat tgtataagat agtgtggtta attagagata tatgtattta 4860
gatttaagta atgatttata ggtgtaaaag gtttagtggt tttttgggtt atttggagtt 4920
tttattgatt attttgtttt ttaaaaaatt tttttttaat tttaaaaata aaagttatag 4980
atttaatgta attgaaaatt attgatggta gttttttagg tttttagttt atgtttttaa 5040
agggaattta tatttaaaat aattaaatta tttaatggtt atattatatt tttttttgga 5100
taatagttgt agtgtaggga tagaatttta atttatatta aaattgtttg ataatttaga 5160
aatggaaatg aaattgtgaa ttaatttttt tagaaaatat ttgagttaga atataagaag 5220
gatgtttata tataatattg taagtaatat gatgattatt atatgtagtt tgaaagattt 5280
atataaatga ttatatatta tttttgttgt tataatttaa agtaaggg 5328

<210> 24
<211> 5328
<212> DNA
<213> Artificial Sequence

<220>
<223> chemically treated genomic DNA (Homo sapiens)

<400> 24

```
tttttatttt aaattatagt aatagaagta gtatataatt atttgtgtag gttttttaag     60
ttgtatataa taattattat gttatttata gtattatata tgaatatttt ttttatattt    120
tagtttaaat atttttttgga gaaattaatt tataatttta tttttatttt tgaattatta    180
agtaatttta atgtaagtta agattttatt tttgtattat agttgttgtt taggagaaaa    240
tgtagtgtga ttattaaatg atttaattat tttaaatata aatttttttt aaaaatataa    300
attgaaaatt tgaagagttg ttattagtgg tttttagttg tattaaattt atgattttta    360
tttttaaaat tagaagaaaa tttttttaaaa ggtaaaatga ttagtaggaa ttttaggtag    420
tttaaaaagt tattggattt tttatatttg taaattattg tttgagttta aatatatatg    480
tttttgattg attatattat tttatgtaat taattatatt aaaggaaaag gttttttttta    540
gaattttaat tttttagtgg ttagggttat tatttttatt ttagagaaaa aatagttgtt    600
ttaaattttt taattgtgat tatttcgttt agttgtgatt ataattttttg tgggaaaaat    660
aaatttatat tttttttaat ttttttttag ttagtattaa atgatatttt agtgaaggtt    720
gggttatttt tttattgtaa ggataattta ttaagtaggg aaaatttttt tttttttagtt    780
tggtttttgt tatatcgtat aaatggaatt tagttttttt tttttttggaa ttttatggaa    840
```

tatattgtta ttgtgttatt tataagtttt ttttttagaa ttatagttgg atttaatata 900
aattataaaa gtgataaaat tttagagggt tgatgtgttt ggtatttaaa aaaataagtt 960
ggggaaagaa attagattta cgtaatatgt gagggataga taggaatatt attagaggaa 1020
aaggaagaaa gtttttattt gtggtatgaa aagaagttaa agtaaattat tggaaaaaaa 1080
aagttatttt ataattatta ttatttttaa acgaaattat tattattttt tttaaagatt 1140
tgtagttgcg gtttgttttg gaaagtgttt taaattaaag tgtatattta ttttgggcgg 1200
gttggtattt ttaaggtttt ttattgatat ttagaatttt tttttaaaag ttatttcggt 1260
tttaaaagaa atgtttgaga agtcggtttt tttaggggtt tatttttgtt tgtttttagc 1320
gttttttttt tagttttggt ttagatggag ttgggttttt aaatttattt taatggaatt 1380
tttagatcgt tggtttgata atttttaaag gtttttttcgg agttggggtt ttgattttta 1440
tttttttttt tattttttttt ttttttttgt tggttttttt tattttttttt tcgtttcgtt 1500
ttattttttt tttttaattt gaagattatt gatacgatag gttgtaatga gtttttttaga 1560
aattgttatg atttatagtg ataattttttg tatttaattt atagattatt gataagtatc 1620
gtgggtttaa tggtgttaag ttgtatttgt ggttttaata tttcgtagta atttacgtag 1680
tcgttgggtt ttggattagg atttttagtt tggtagtgtt tatttgttcg tttgagtgat 1740
ggagagatga gtattagttt atattttaat tgttttaagt tcgtttgggt tttttttttg 1800
gcgtttttgt ttgtgttagg gttggagtaa cgaaattgaa agatttttag agtttgaaaa 1860
tagagtgaaa gagtaaattt aataaatgag agtttagttt tgaattttag tttttattaa 1920
tagagttatg ttttttatat ttaaataatt attaaatttt aaattatatg tattgttttt 1980
tagagttcgg gttttttttt gggtttatta tgaaaggtgt gagggatgaa ggggaaaagg 2040
gtttgaggag ttggtagggt tttagtgggt ttaagttaag tgggtttatt cgtttggata 2100
gtttttagaa tttagtaaga ttggttattt ataaagtagg gcgtttagtt aattcgcgcg 2160
ggtttaggtt tggacgttgg gataggagat taaaaatgtt cgtttcgttt ttaaaggcg 2220
ttttgtcgtc gcggagaaac gcggttcgtt gttagtcgtt gcggtttttt ttgttattaa 2280
ggaattaagt tattcgttgt tttttttttta gtttttaaag atggaggggt tgtttttttg 2340
ttggcggttt tggggagtgg gattcgggta ggtgttgggg ttttttggga ttatagaaat 2400
aatattgcgt cgggagagtt gggtggtatt tttaggtttg ggttatttat ttgtttagtt 2460
tttttttttt aattaggggt tatgtttttt ttatgggttt tagaaagttg ggtattcgtt 2520
ttcggtacgt ttattcgttt ttagttaggt gcggtttttt ttttttgttt ttatttaata 2580
atttgttatt tttataggttg ggatttttgg gtgcggaacg gggtggaaat ttttaggatt 2640
ttgttattgt acgttcgata ttattttcga atagtaggta tatattttttt ttttggcgtc 2700
gtttttaata ttttgttggt tttggttttt ttattttacg ggttgcggga attattgagt 2760
agttttcgga tttcggttgt tagcgtatcg ttttttattt tatcggttgg cgcgggttta 2820
taaaattagt atcgattata ttatttttgt aatggggata agttttttgg tagagtaaaa 2880
ggtgggggtg ttgtggttgg aatttcgttt gtttgttttt tttatagaat tcggggtaga 2940
gattttatta aaaggtttga tttttttttt tttttattgt atttgaattt atttattaaa 3000
aaatttaagt cgagatttaa ttttggaatt agatggagaa gttatttttt atcgtattaa 3060
atagtaattt tttttgaaat gttatttcga attcgttaat tttgggaggt gggaagaata 3120
aatataaagg tattataaat tttgtatagt tatagtattt aatattttaa aaagttattt 3180
ttttatgaaa aattaagtcg attttcgtta ataagtaaat tttaaaaacg cgattttttaa 3240
attttttaag ggtaaagtta aaattaataa ttataatttt gaagatgtaa attttaaaat 3300
attttgttta tttagtatag taattatttt tatttgattt taaataatta agtaaattta 3360
tattttttta ttgttttaaa tttagaggat gtgttatttt tatagttttt ttttttgaaa 3420
taatttaaag gttattttttg tttttatttg ttgattaaaa agaagaagaa gaaataataa 3480
taataaaaaa ggtaaagttt tttattatgt agataggtag ttttttggaa gtgtaaaaat 3540
attttttttt ttttaggata atttggattt ttggttaacg aaatgttgaa ttttaaagaa 3600
aatttttttt attattggta tattttttttc gaaattttac ggttaagatg aaagaaaaaa 3660
gatattaggt atttgttttt tagttagtat gtgtttttta gttagtattt gggaaggaaa 3720
aattttgatt aggattggtt taggggatgg gaaatattta aagaagagat tggtttttta 3780
attatatttt ggaaataaat atataaaagt aagttatttc gtttattcga aggtttagtt 3840
acgatatttg taggattttt gagggtattt aagacgttgt tgataacgtt aaaatagtaa 3900
gtagtagtat ttttcgttta atttttttgaa atgtggataa aacggaggaa gtatattttg 3960
tttgattttt tacggttata gaaaaatgtt ttgtttttcg ttattttttt ttatttgttt 4020
tattatcgta gaattttttta aaagagttat aaaattaaat cgggtggggg atggttggga 4080
tgtgaggagg atagttttta tttttttattg aattaaaatt gtagtatcgt tttttttagtt 4140
ttttattgag ttaatattat attatttttt aaggatataa aaatttgttt tcgtatttcg 4200
tttttttttt ggagggtaat aaaagagttt ttttttaattt tagagtagga ttttagcgat 4260
ttttgggatt ggcgttgcgt agtttttatt gtgtttaata gggggcgttg ggtacgaatt 4320
tgcgtagcgg tttagtgtcg ttttatattt gagtcgggtt ttagtttcgg aaggcgagag 4380
tcgttcgcgg ggtcgagtat agagttttag ttttcgtgga taaaaagaga ttaaaggtta 4440
aatttttttt tggggatatg tgtgtgtgtg tgtgtgtgtg tgtgtgtgtg tgtgtgtgtt 4500
ggtgtgtgtg tgtgtatatg tgtgtatgtg tgtgtgcgcg cgcgcgtatg gggggggtggt 4560
tattaatttt aattaattac gaaaatgtag agttttttat tttttgatgt ttttataaag 4620

```
aaatttttat cggatgagaa attatacggt ttttgagtta atattgtagt tatagtgtgg    4680
gggaaattat ttaattaaaa atatattttt ttatttgttt atatgatttt tttagaagta    4740
tttaaaaagt tgatattgat ttttttggtt tgttaatttt gtttttagtt ttaaatattt    4800
ttaattatat cgaaggtttt tttattaatt tgaaggagaa aaaaaggaga tttaaatatt    4860
taaattattt ttaaatagtt atttttttgt attttttcgt tttatttttt ttttaaatag    4920
gggtttttat gtgagtgata tattttatat tttcgatttt gtaaagttag gtataaatga    4980
taaggagcgg ttttaaataa taataataat aaaatttaat ttgggattat ataatattta    5040
agtagagtag aagatttaaa taaaattgta atttatttat ttattttttt ttaatttaat    5100
ttaaaaaaaa aaaatttttt atttgtattt aaatagcgtg tttttaatta tagtagttgg    5160
ggatgtatga ggttgaaatg aaattagtag ttattattag ttttttaatt ttgtggtgtt    5220
tttgattagt attaaattag tttttttagtt ttaataaaga ttttatagta agtagtgggt    5280
tgtaaataag atagcgcgat ttgtttttaa tattttttttt tttgttaa               5328
```

<210> 25
<211> 4324
<212> DNA
<213> Artificial Sequence

<220>
<223> chemically treated genomic DNA (Homo sapiens)

<400> 25

```
gtttttagt attattattt taaaggtatt tttatttttt ttattattag ggaaggagtg 60
tgtagttttg atttagtttt ttagtggagt aaagggaatt tagaggaagg gggattttg 120
tagaaattgt tagttatata agtaagtttt ttttggtttt tcgttttatt aaaagtgaaa 180
aattgtttat aggagtgttt atgtttttg tttttttatg tattattaga gttaagaggg 240
gatttcgtta gtaaggtatt ttttgttttg tttcgtagat tatagttttc gatttttatt 300
ttattgtttg tgtaatgtgt agaattggag tttaaagtag cgggtttagg attatttttt 360
aggttcggcg ggtttataga ttttttttt ggttgggaag agggttttc gtgaaagtag 420
agtttgataa gttttagtat tgttatggtt aagattatta atattattat tgttgtgttt 480
ataaatatga agattacggt agaggaggag tcgaaagttt gaggagtggt agaggaagtc 540
gtagaattaa atttggaaat tacgtttttt gatggggtga tagtggtttt tgattcggtt 600
tgaagggata tttgaagggt agatatcgtg ttttgtgatt tttatcgagg aattttagga 660
atagatgtta ttgaattgtt ttgtttaggg ataagtggtg ttttttttag tttttcgtcg 720
attttgattg gttatgtttt ttgcggtacg gggttggttg tagtggtcgg cgggcgtttg 780
gtgggtattt cggtttttttt aagggtcggt tgtttttttt tattggttat ataagagcgg 840
tcgttttttt ttagttcgaa gttcgtagta tattcgtagg taaagttttt taagtcgttt 900
aggtagttag ggagttttgc gtatttgtta gtacggaggt atttttcggg gtagggatat 960
aatatatcgt tcgagagttt gttttagcga gcgtcgattt cgttcgcgat gtaagtaatt 1020
gagatcggga gttgttttcg gtagagcgta tttatttcgg ttttaggtgg attgaagttt 1080
agagcggcgt tgtgtagttg gaagggcgcg cgatagttta agttagaggc ggtttcgggg 1140
cgcggcgtag gatataagat tttaaattgg tatttgtata ggtagtcgtt ggcgcgtagg 1200
tggtatcgta ttttttttta gtttgcgggt tcgattttat cggtggtttg gagtatcgcg 1260
tattttcgcg cggtgtagga gcgttggggt ttttttattt attgtagcgt gtcgttttcg 1320
agatcgtcgg ggtcggagga tagttaggag aaatttcgta aaggttcgtt ttttagggtg 1380
tagtgggaac gtttgcgttt tagtgcgatt tagaatagta ggtttttgga gtttttttcg 1440
ggttttgggt ttgttcgtag gagcgcgagt atagcgcgta gttcggcgtt cgtacgtacg 1500
gtgttgagcg ttttatttcg taggatgtag gtttttttcgg tcgtttgtcg ttttatggta 1560
gcgtggtgta ggttgtagta ggttttcgag gtcgagtagt tagtacggtc ggtagtgggg 1620
tgttcgtcgt cgttcggttc gggttagagc gtttgttaga ggaggtatag ggcgaacgtc 1680
ggttttattt tttgaggttt cgtacgtaga gttgttttta atttggattt gtttcgttcg 1740
aggacgtaga gttgtgtttg gagcgcggtt atcgtttttt atagttggtt tttttaattc 1800
ggtttttttt ttttttttt tcgttttcgt tttcgacgga ttttttaatt gttaagaatt 1860
ttttcgcggg tttagcggtt aatagttttt tttaggtcgc gataggaaat gtgtgcggag 1920
cgttgttagg ttcggtgtag ttttgagtta ttttggggtt aggaatatag ttggcgtcgg 1980
gtatagggga cgtagtcgat tagaaggtta aatattttgt ttagagtaag aggagaaatt 2040
cgttattggg atgaagtgtg tggttttttt tcgtttaggg agttttatta ggatcgtaga 2100
atttataata cgtaagcgaa tataggtagg aaggatttga gaaaggaatt ggtttatgat 2160
tataagatta cgttcgttta ttgtgttttt gttcggagtt gtggagttag cgttagaata 2220
attcgtagac ggttttagtg tttgggggt ttgtgtttgt gggggaaaaa tatgttgtga 2280
aataatttag taattgtttt tgttattaga ggtagtatta tttaatcgta tattttttta 2340
tgggttttg ttttttatt atgttaagag attttaggag ttttaattat taaagaattt 2400
```

```
ggagttgttt gggtatttta aatgggaaat tttgtttttt ttattagtat ttgttttaga    2460
aatttatttt ttatttttac gtgttttttt ttgagttaga ttatattgat ttattataga    2520
aaggaaatgt atttttagga ttaaatgaaa aataatgatt atagatttta atagttaggt    2580
tgttagtttt ttttgttatt gtattggttt aaaaattttt taagtttata ggttatatat    2640
aaatgagttc gtggttttat aaatattcga gaataattgt tttatgtgtg attttagttt    2700
tgaattattt tagtattatt tttttttatg attgagtggt agtatttata atagtaagtt    2760
gtaaatattt tttttaagga aaattagagt tttttaagaa attaaaatgt ttattttatt    2820
ataatgaata ttgtatattg ttaaaaaata ttttgtaatt tgaaagatgg gtgagttaat    2880
tttttttagaa ttttagtggg tgttttgttt taatataaat attttaattt agtgggaagt    2940
ggtaaggaat taaagatgaa taaagaagta aaggttaaaa attgtattgt tatgaaagga    3000
tttttttgtta gtaatttaga taatttagta ttagtttaga aaacgtatat tttattttgg    3060
tttataatgg ttaaatacgt tgttattaga aatatataga gagttttagt taagtttttt    3120
tagagatgtt tgatgttttt aaatattttt aattagaatg aaagttgtta ttttatggat    3180
aatgtatata agtttatttt gttttattgt atttaggttg atgaggggtg ggagggaagg    3240
ttttaggatt atatttgtat gtttttattt tatttttatt ataattttta tttttttttt    3300
tttttggaaa ttttatgtat tttaataaat aatagtaagt ttatgagtta ttattatatt    3360
tattttgttt ttgttgagta tggagttttg tattaggatt ttttttttgt tttatttttt    3420
aatttaaaga atatttttta gttttttttt tagatgtttg agaattattt taaacgtggt    3480
tttgcgttta aattaggtaa tttagtgttt tataagaaag aaatatgaaa atttagaaat    3540
taaattgaat ggtttttaaa aaatatttag ttatttgggt tagtttttta tagattattg    3600
taattttatg attgtgtttg taaatgtgtt tatgtagtgt aatattttta ataaaatttt    3660
ttttaaattt aagtagttaa tggaaatttt ttagatagtt ttaaaaatcg tttgggatga    3720
gatgaggtgt ttatagtaag aaagaatgaa tagttggaaa gtttaagtaa atatatgatt    3780
ttattttttg atattttgtt taggggagtg ggattatagt agagaatata ttggttataa    3840
cgattatatg agagtattga ttatgattat gattatatta tggaatgata taagagttag    3900
aaaaaattag tttaaagtag gacgtaaata gttttgtaat tagtttgaaa tagatagttt    3960
atttgattaa attagaaatg gaggaaagtt ttttattttt aataatattt attaagataa    4020
taatatagaa ttattttatt tttattggat tttaaatgtt taaaaatttt ataatattaa    4080
atgtgaattt attggttttt ttatatgtta tagattagag taattatttt ggaaaaattt    4140
gatatttttt tttattaaaa ttgaatattt gtaaatttta gtatttttat gataaggtat    4200
aaaattaaga gaaatttttg tatatgtata atagaagata tgtaaaaaat gtttatagtg    4260
atattattta tattaataaa acgttgaatt aattaaaata tttaaaaata gtataaaaat    4320
aaat                                                                 4324
```

<210> 26
<211> 4324
<212> DNA
<213> Artificial Sequence

<220>
<223> chemically treated genomic DNA (Homo sapiens)

<400> 26

```
gtttattttt atattgtttt tggatatttt gattgattta acgttttgtt aatatgaata      60
gtgttattat aaatattttt tatatatttt ttgttgtata tgtgtaaaaa ttttttttag     120
ttttatattt tattatggaa gtgttagagt ttgtaaatat ttaattttag tgaaaaaaag     180
tgttaaattt ttttaaagtg gttgttttaa tttgtaatat gtaaaagagt taatagattt     240
atatttggta ttataagatt tttaggtatt tggaatttag tggggtaaa atggttttat      300
attattattt taataaatat tattaaaaaa tagaaatttt tttttatttt taatttggtt     360
agataaattg tttattttaa attaattata aaattattta cgttttattt tgagttaatt     420
ttttttaatt tttgtgttat tttatgatat ggttatggtt atggttagta tttttatata     480
gtcgttatgg ttaatgtgtt ttttgttata attttatttt tttagataga atgttaggga     540
gtgagattat gtatttattt aagtttttta gttatttatt ttttttttatt gtagatattt    600
tattttattt taaacgattt ttaaagttat ttaagaaatt tttattgatt atttaagttt     660
gaaaaagatt ttattaaaaa tattgtatta tatggatata tttataaata tagttataag     720
attgtaatgg tttatagaga gttggtttaa gtgattaagt atttttttaaa aattatttaa    780
tttgattttt ggatttttat gtttttttttt tataagatat taagttattt aatttaaacg    840
taaagttacg tttaaggtga tttttagata tttgaaggaa ggattaagga atgtttttta     900
aattgggaaa tagggtagga gagaaatttt agtgtaaagt tttatattta gtaaaagtaa     960
ggtaagtatg atgatgattt ataggtttat tgttgtttgt taagatatat ggaattttta    1020
ggaggaaaga agaatgagag ttatggtaaa aataagatgg aagtatatag atgtgatttt    1080
gaaatttttt tttttatttt ttattaattt gaatgtagta aaataaagtg agtttatatg    1140
```

```
tattatttat ggaatggtaa ttttatttt gattaaagat atttaaaagt attagatatt 1200
tttaagaggg tttagttgaa attttttatg tatttttgat ggtaacgtat ttggttatta 1260
tgaattaaga tagaatgtac gtttttaaa ttgatattga attgtttaag ttgttaataa 1320
gaaatttttt tataatagta tagtttttaa ttttatttt tttatttatt tttaattttt 1380
tgttattttt tattgagttg aaatgtttat attaaataa aatatttatt ggaattttaa 1440
agaaattggt ttatttattt tttaaattat aagatatttt ttgatagtat ataatattta 1500
ttataatgga ataaatattt taattttta aggaatttta atttttttta aaggagatgt 1560
ttataattta ttattataaa tgttgttatt taattatagg agaaaatgat attaaaatag 1620
tttaaggtta aaattatata tagggtagtt gttttcgaat atttataagg ttacgaattt 1680
atttatgtat aatttataaa tttgaagaat ttttgaatta atatagtggt aaggaaagtt 1740
aataatttaa ttgttaagat ttatggttat tattttttat ttagtttag aaatatattt 1800
tttttttgta atgaattaat atggtttaat ttaaagagaa atacgtggaa gtgaaaaata 1860
aatttttaaa gtaagtgttg atggaagaag tagaattttt tatttaaaat atttaaataa 1920
ttttaaattt tttagtagtt aggatttttg aaattttttg atataataga gaagtaaaga 1980
tttatagaga gatatgcggt taaatagtgt tgtttttggt ggtaaaagta gttgttaagt 2040
tgttttataa tatatttttt ttttataggt ataggttttt taaatattgg agtcgtttac 2100
gaattatttt gacgttaatt ttatagtttc ggatagaaat ataatagacg ggcgtggttt 2160
tataattata agttagtttt tttttaagt tttttttgtt tatgttcgtt tacgtgttat 2220
gggtttgcg gttttgatga aattttttga acggagaaaa attatatatt ttattttagt 2280
ggcgaatttt tttttttgtt ttaagtaggg tgtttgattt tttagtcgat tgcgtttttt 2340
gtattcggcg ttagttgtgt ttttgatttt agaataattt agggttgtat cgggtttggt 2400
agcgtttcgt atatattttt tgtcgcggtt taagggaaat tgttggtcgt tgggttcgcg 2460
gggggatttt tggtagttgg ggggttcgtc gggagcgagg gcggaggggga agggaggggg 2520
aatcgggttg gggaagttag ttgtagaggg cggtgatcgc gttttagata tagttttgcg 2580
ttttcgagcg ggatagattt aagttgggag tagttttgcg tgcggggttt tagagaatga 2640
ggtcggcgtt cgttttgtgt tttttttggt aggcgttttg gttcgggtcg ggcggcggcg 2700
aatattttat tgtcgatcgt gttggttgtt cggtttcggg ggtttgttat agtttgtatt 2760
acgttattat gaagcggtag gcggtcgagg aggtttgtat tttgcgaggt ggggcgttta 2820
gtatcgtgcg tgcgggcgtc gagttgcgcg ttgtgttcgc gtttttgcgg gtaggtttag 2880
ggttcggagg gggttttaaa gatttgttgt tttgggtcgt attggagcgt aggcgttttt 2940
attgtatttt ggagaacgag tttttgcggg gtttttttttg gttgttttc gatttcggcg 3000
gtttcgaaag cgatacgttg tagtgggtgg aggagtttta acgtttttgt atcgcgcgga 3060
gatgcgcggt attttaggtt atcggtgggg tcgagttcgt aggttggaag gagatgcgat 3120
gttatttgcg cgttaacggt tatttgtgta agtattagtt tgaggttttg tgttttgcgt 3180
cgcgtttcgg ggtcgttttt aatttgagtt atcgcgcgtt tttttagttg tatagcgtcg 3240
ttttggattt tagtttattt gggatcgagg tgagtgcgtt ttgtcgggga tagttttcga 3300
ttttagttat ttgtatcgcg gacgaaatcg gcgttcgttg ggataaattt tcgggcgatg 3360
tgttgtgttt ttgtttcggg aggtattttc gtgttggtaa atgcgtagag tttttttaatt 3420
gtttagacga tttgggaggt tttgtttgcg aatgtgttac gggtttcgag ttggggaagg 3480
acggtcgttt ttgtgtgatt agtggggaag gatagtcgat ttttgggggg atcggggtgt 3540
ttattaggcg ttcgtcggtt attgtaatta gtttcgtgtc gtagagaata tggttaatta 3600
gggtcgacga gaagttggga gagatattat ttgtttttga ataagataat ttagtaatat 3660
ttatttttga gatttttcga tggggattat agagtacgat gtttattttt taaatgtttt 3720
tttaagtcga gttaaaggtt attattattt tattagggag cgtgattttt aagtttaatt 3780
ttacgatttt ttttgttatt ttttaggttt tcgatttttt ttttgtcgtg gtttttatat 3840
ttgtgagtat agtagtagta gtgttggtga ttttgattat gatagtattg gggtttgtta 3900
agttttgttt ttacgaaagt tttttttttt agttaaggaa ggagtttatg ggttcgtcgg 3960
gtttggagag tgattttgag ttcgttgttt tgggttttag ttttgtatat tgtataaata 4020
atggggtgaa agtcggggat tgtgatttgc gggatagagt agagggtgtt ttgttggcgg 4080
agtttttttt tggttttagt gatgtatagg gaaatagggg atatgggtat ttttgtgaat 4140
agtttttat ttttgatgaa acggggaatt aagaggaatt tatttgtgta attgataatt 4200
tttgtagaaa tttttttttt tttaaatttt ttttatttta ttgaggagtt aaattagaat 4260
tgtatatttt tttttttgatg atagaggaag tggaagtgtt tttaggatgg tgatattggg 4320
ggat 4324
```

<210> 27

<211> 5683

<212> DNA

<213> Artificial Sequence

<220>

<223> chemically treated genomic DNA (Homo sapiens)

<400> 27

ataatgttaa atgtaaattt tttgttttgt ttaatgtttt tattttatgg ttggtaaata 60
aaaataataa aatagtattg ataataataa tagaaataat taattgaagt tgtagttttt 120
aatttttta aagataatag gttaaagtga ggtttttata ataagatgtt agttattagg 180
aaggaagtta gggaaaattt tttttaggg ggtaattgat ttttattggt ttaatattgt 240
atgtagtatt gagttaggag gttttatgga gaagtgtttg tttttttttt tttgttgtta 300
gaataaagtt tataaataat tttgtttttt ttatattttt ggagttagtt agttagggtt 360
tttatttatg tttaggggat tagatttgga tatttgtaaa agaattgaat atagatggat 420
agttaagaaa aaaataaatg ttaagattat tattatttat agtttaatga tttagtaatt 480
taaaaaaatt taagaataat aaaataattt ttaagaagga taagaatttt aaaatatgta 540
gtttagaaaa ttaaattttt taggtttaga gtttaagtaa aagaaaattt gagataatga 600
agtttggaat attgggtttg tattttgttt ttgattttgt gtttttgggt aagatattga 660
atttttttat tttagattta ttttatattt taaggagggt gtaggatttt gtagtttttt 720
tagtgatggt atattattaa ttagaatttt aattagtttt atttttatta ttttagtaaa 780
atgatgttaa aaattagatt tcgtgatatg agagaagagg ggaaattaat tatagattat 840
aaagaaattg tgggatttaa gtttagggtg aggtgtttta ttgaattttt agggttggtt 900
atagtatttg ttatatagta ggtatttaat aaatgatgtg taatgggtgg ttagattgat 960
ggaaaggtag gttttttag tgacggtaaa ggatttgggg taaaagagat ttgtggttga 1020
gttttgattt ttatatatta gtatattgat tagacgaagt tataagtttt aatttttttt 1080
tgtgtaaaat ggagataata ttttataggg ttatgaggat taaataagaa aatgtttagt 1140
ttagtgttta atatatagga agtatatcgt aaatattagt tattattatt attaatattg 1200
tgattatatt ttttatttaa agattttttt gaggagtagg gggtatttag agataaaaag 1260
tttgtaggtg ggaaatttgt tagtttttta gggcgttagg tttttgtgga atttgtatt 1320
ttttttttgt tttagttttt aagaatagaa atttttagg gatttttggt gaggtagtcg 1380
tgggaagatg aggtgtagaa gtaagttggg atttgtgagt tttaatttcg gttttttttg 1440
cgttgagatt taagcggatt ttgtttggtt tgtatgcgtt attgggggaa atggacgtgg 1500
gtttgagcgc ggtaggtgcg agggcgttgt ttcggggtcg attattttgc ggggatattg 1560
tagttgttat tttttttttt gtaggcgggt aggggaagcg gtggttaaag tgggagtcga 1620
tcgtttagta tagtttgttt gagtgttgat taggaaagtt taggtttttt ttaaatttcg 1680
tcgttagatt tggtgacgta ttcgtatgta tttaaggcgt ttgtacgtag aacgttatta 1740
tagaatgtag ttattttttt taacggttcg ggaaattaga ggttttttta gttatttagg 1800
gtagaggaat tttttttatt tttatgtgat atttttgat ttagaagaat taatgttaga 1860
tttttttgg gtttttttat ttatagttat agtttttttt ttgtttagtt aaaattgagg 1920
taggtaggaa aatattattg ggggtataag tttattagtg tgtaaacgta tttttatgaa 1980
gtgtgttttt agggagttat taaaaattga ttttttaatt atagaaatag atgagatttt 2040
gagaatattg agaagttgtt ttttgtaaag taaatttgta atggtttttg acgaaggggg 2100
gtcgggggcg gggagaagtt tagtcgagag aggagtttat tttacgttat atttttgtag 2160
ttgaaaagtt gtttaattat cgttaatcgt ttttcgtata agagttttgg gaagatttta 2220
gaaataaggt aaatgaagat tttttattgt ttttttcggg ttgtcgtttt cggaagtcga 2280
agttttagta cgtagagtag taggagaggg ttatttttag gtaattttat tgagtaaaat 2340
aaattattta atggtataac gttttggttt aaaaaattgg aatttattag aggtaaaaat 2400
attttttaag aaattatgga tatttcgcgt tttatttatt tttatggtag tagagtattt 2460
gtattttgag ttatttatat agatttatgt ttcgtatcgt ttttattttt tttttttttg 2520
aagtaaagtt ttttttaag aaggcggtta gaaagtggat tttatcgggg gaaaaagaaa 2580
aatgaaacgt aaattagttt ggtattgttt gcgtttttta aaacgcggtg ggataaggtt 2640
attgagttta tagttaattt ttttatgtat ataaaatgta tatatatgta tatattttta 2700
tatatataaa agaatttata tatatgtata tagttatgtg gagttttgaa gtaatttttt 2760
atgtttttgt ttttttaag tttttaggt ggaggtagtt ataagtatta taagtcgttt 2820
tagtgattat tagggacgga aatcgttaat tattacgttt ttttttattt ttagggttt 2880
tttggttcgt gatataagag gtttcggtat tggcgttttt ttagaattgg tttagaggag 2940
ttagtttaga gtgtgaggtc gggtttgtat tgaacgtata tatcgaggtt tattagattt 3000
tttcgatttt agcgaagggt gttgattgtt gtgttgttag aggttagtaa gttttttgtg 3060
cgtagttgat gagttttagt aattcgttat ttgggcgttt ttttttaaat tttgggagta 3120
aattgggaaa ataaaaaaat ttttacgttt attggttttt tttttttttt taattttttt 3180
tttcgattcg tttgtgggag tttttttttt tttgttggga ttataatgtg atgcgtaatc 3240
gtttgtgaat gaataaaagt tatcggtaag tagggagacg gggatagatc gttgacggta 3300
gattgagggt ggtagtaaag gttcggtttt taaggataat ggggagtcgt tttttttac 3360
gtttggtttt tatggttttt ttcgtttttt aggtaaaatg aatgtttttt tatttattat 3420
tcgtagagta tttttaggcg tgcgtagaat ttgttgatga aatttattag cgtcgattgg 3480
gtagttttgt ggagttattc gaggtttttt attgtggttt ttgtttgtag aatttaagta 3540
tttatataat gtattagtac ggaatttagt attcggtggg gatatcgcgt gttaagtttg 3600
gcgcggttaa cgttttagcg gttttttat tcggtagttt tttaggatta ttttcgagga 3660

```
ggtattggag tcgggttgta ggcgtacggg taaagaattt agtattttat ttaagtattt 3720
cgtttttttt ggtcgttttc gggaggttat gtttaaaaat ataaaaataa aaataaaaat 3780
aaaaataaag ggaggcggat aaagtttcgg tgggtgaatt gaagttgggt ttatgtgatt 3840
ttgaagtcgg agaaataaat ttaatatgaa ttttgttttt ttggcgggcg ttgggatttc 3900
gtcgtttttt atgttaatcg ttggaagttt cgtatttaac ggttataggt gtttaggagc 3960
gtagagaggt ttcgggttta aattatcggc gcgtagggat tggattcgcg ggtagcgatt 4020
tttttaattt tttttttttc gttttatata tatattttcg cgtcggttga aagtatggag 4080
gatttagggt atttgaaaaa agaggggttg ggcgcggtgg tttacgtttg taattttagc 4140
ggtttgggag gtttagaagg gcggattatt tgaggttagg agttcgagat tagtttggtt 4200
aatattagtt tggttaatat ggtgagattt cgtgtttatt aaaaatataa aaattagtta 4260
ggcgtggtgt ttgtaatatt agttatttgg gaggttgagg tacgagaatc gtttgaattt 4320
gggaggcgga ggttgtagtg agtttagatc gcgttatcgt ttttttgttt tgggtgatag 4380
agtaaggtat tgttttaaaa taaaataaaa taaaacgaaa gattcggtta ggaaagaatt 4440
tgtaggtatt cgaggcgttc gtattttgta aagtaaatgt aatttttta ttaagtcgaa 4500
gtttttata tttatttttt tagaggaagg tggtttaatt tagaaatttt ttttaagagg 4560
atttttatc gaagattatc gcgaagtgtt aggaattcgt tttagtttcg ataggtgtag 4620
gatttttcgt gtcgttatat tttgggattt tatttttttta aataggttat ttaaaagtta 4680
gtagtgtaat cgggatttcg cggcgataaa gaattattgt gtagaatttt ggagttggga 4740
gtcggttcgt tttttttta aagaaatcgg gatttcgcgt ttttttcgtc gttagcgtag 4800
cgcgttagcg gcgtttaatt agtgagtttt tcgggtttgt gacggttttc ggtttcgttt 4860
tttcgacggt tataaaaagt cgtagcgaag tttgtatttt cgagtatcgc gcgcggtttt 4920
gttttcggta cggtttttag gtgcgttttt ttttcggttg tttgtagtat tggttttatt 4980
gttttcgtcg ttagttatcg gtttttttatt cgttttattt tttattgtgg tttggttgag 5040
tcggtcgtta ggtttcgttg tttttttttg ttttttttt tttttagcgg tcgtattttg 5100
cgtcgtattt tatttggttt gtaggtgagt agtagcgtag tatttttgtt cggcgagttt 5160
aatttgttta gttcggtttt tgtcggagtg gtatcggtat ttttttaaga cgtttttttt 5220
tttgtaggat gaagaatttt atgttggagg tggtgttttt attattggag aagttgtttt 5280
ttatttttaa ttttacgttt tttagttcgg gcgtcgcggg cgaagataaa gggaggaata 5340
gtttttatga aattagtttt ttttatcgag gcgacgtgtt ggaggtgttt cggatttatt 5400
tgatttatta tggtatttat ttaggagata atcgtgttgt ttatatgatg ttcgatattt 5460
tgttggtttt gatagacgat atggggcgta cgtagaaggt ggtttttaat aagcgtttta 5520
ttttgggcgt tattgttaaa gtggttagta ttcgcgtgga tatagtggag gatttcgttt 5580
acggagttaa tattttggtt aattatttgg acgagttttt ttagaaaaag gtattgttta 5640
acgaggaggt ggcgcggagg gttgaaaagt tgttgggttt tat 5683
```

<210> 28
<211> 5683
<212> DNA
<213> Artificial Sequence

<220>
<223> chemically treated genomic DNA (Homo sapiens)

<400> 28

```
gtaaagttta gtagtttttt agtttttcgc gttatttttt cgttgagtag tgtttttttt      60
tggagggatt cgtttaggtg attgattagg atgttagttt cgtaggcgaa gtttttttatt     120
gtgtttacgc ggatgttggt tattttgata ataacgttta ggatgagacg tttgttggag     180
attatttttt gcgtgcgttt tatgtcgttt gttagggtta ataggatgtc gggtattatg     240
tgggtaatac ggttgttttt taggtagatg ttatagtggg ttaggtgggt tcggggtatt     300
tttagtacgt cgtttcggtg gaaagagttg gttttataaa aattgttttt tttttttgttt     360
tcgttcgcgg cgttcgaatt aaagagcgtg aagttggaga tgaggagtag tttttttagt     420
agtaaagata ttatttttag tatggggttt tttattttgt agggaagagg gcgttttgga     480
gaggtgtcgg tgttatttcg gtaggggtcg ggttgggtaa gttaagttcg tcgggtaggg     540
gtgttgcgtt gttgtttatt tgtaggttag gtgaggtacg gcgtaaagta cggtcgttga     600
ggagagagga aggtaaagga ggatagcgag gtttggcgat cggtttagtt aagttataat     660
ggggaatgag acggataagg aatcggtggt tgacggcggg gatagtgaga ttagtgttat     720
aagtagtcgg agaaggagcg tatttggggg tcgtgtcggg ggtagggtcg cgcgcggtgt     780
tcggaggtgt aggtttcgtt gcgatttttt atggtcgtcg agggggcgga gtcgaaggtc     840
gttatagatt cggaaagttt attgattggg cgtcgttggc gcgttgcgtt agcggcgggg     900
aggacgcggg atttcggttt ttttgggagg ggggcgggtc gatttttagt tttagggttt     960
tgtatagtga tttttttatcg tcgcgggatt tcggttgtat tattggtttt taagtggttt    1020
atttagggag gtagagtttt aaggtgtggc ggtacgaaag gttttgtatt tgtcgggatt    1080
```

```
ggggcgagtt tttggtattt cgcggtagtt ttcggtggaa agtttttttg ggagagattt   1140
ttgagttgga ttattttttt ttaaaaggat agatatgagg gatttcggtt taataaagag   1200
attgtattta ttttgtaaag tgcgagcgtt tcgaatgttt gtagattttt ttttgatcga   1260
atttttcgtt ttgttttgtt ttgttttgag atagtgtttt gttttattat ttagagtagg   1320
agggcggtgg cgcgatttgg gtttattgta attttcgttt tttaggttta agcgattttc   1380
gtgttttagt tttttaagta gttgatatta taggtattac gtttggttaa tttttgtatt   1440
tttagtagat acggggtttt attatgttgg ttaggttggt gttggttagg ttggtttcga   1500
atttttgatt ttaagtgatt cgtttttttg gatttttttaa atcgttggga ttataggcgt   1560
gagttatcgc gtttagtttt ttttttttta aatgttttga atttttttatg tttttagtcg   1620
gcgcgagggt gtgtgtgtag ggcggggggg gggggggttgg gggggtcgtt attcgcgagt   1680
ttagtttttg cgcgtcggtg atttgaattc gaggtttttt tgcgttttta ggtatttgtg   1740
gtcgttgagt acgaagtttt taacggttgg tatgaaaaac ggcggggttt taacgttcgt   1800
taggaaagta agatttatgt taagtttatt ttttcggttt tagggttata tggatttagt   1860
tttagtttat ttatcgaaat tttgttcgtt ttttttttatt tttattttta tttttatttt   1920
tatgttttta agtataattt ttcggagacg gttaaggaag gcgaagtatt tggatgagat   1980
gttaagtttt ttgttcgtgc gtttgtagtt cgattttaat gtttttttcga gggtggtttt   2040
agggagttgt cgggtgaggg agtcgttgaa gcgttggtcg cgttaggttt ggtacgcgat   2100
gtttttatcg ggtgttgagt ttcgtgttaa tgtattatgt aaatgtttaa attttgtaga   2160
taaaggttat aatggagagt ttcgggtggt tttataaagt tgtttagtcg gcgttaatag   2220
gttttattag tagattttac gtacgtttga gggtattttg cggatgatgg atgaaggaat   2280
atttatttta tttggaagac gaaggggggtt atagagatta ggcgtgaggg aaaacggttt   2340
tttattattt ttggaggtcg ggtttttgtt gttatttttta atttgtcgtt agcgatttgt   2400
tttcgttttt ttgtttgtcg gtgattttttg tttatttata aacgattgcg tattatatta   2460
tagttttagt aaagaggaga aaatttttat aaacgagtcg aaagaggaag ttggagaagg   2520
ggagagagtt agtggacgtg gagatttttt tatttttttta gtttattttt aaaatttaaa   2580
gaaaagcgtt taggtggcga gttgttgaaa tttattagtt gcgtataggg agtttgttag   2640
tttttagtag tatagtagtt agtattttc gttaaaatcg ggggagtttg atagatttcg   2700
gtgtgtacgt ttaatgtaga ttcgatttta tattttgaat tggtttttttt gggttagttt   2760
tgggaaagcg ttaatatcga agtttttttgt gttacgggtt aaagggtttt tggagatgaa   2820
aggaaacgtg ataattaacg gtttcgtttt ttggtggtta ttaaggcggt ttataatgtt   2880
tatgattgtt tttatttggg gaatttgagg gagataaaag tatggagaat tgttttaggg   2940
ttttatatag ttatatatat atatatgaat ttttttatgt atataaaaat atatatatat   3000
gtatatattt tatatatatg aaagaattag ttatagattt aatagtttg ttttatcgcg   3060
ttttggaaga cgtaagtagt gttaagttga tttgcgtttt attttttttt ttttttcggt   3120
ggggtttatt ttttggtcgt ttttttggga aagggtttta ttttaaaaag aaaggaggtg   3180
agagcgatac gaggtatgaa tttgtatagg tggtttaaga tgtagatatt ttgttgttat   3240
ggaaatgaat agggcgcgga gtgtttatag tttttttgaag gatattttttg tttttgataa   3300
attttaattt tttaagttag aacgttatgt tattaagtga tttattttgt ttagtggaat   3360
tgtttgaaag taattttttt ttgttgtttt gcgtgttagg atttcggttt tcggaagcga   3420
tagttcgaag gaggtagtga aaagttttta tttgttttgt ttttgaagtt tttttagaat   3480
ttttatgcgg gaagcggtta gcgatgatta ggtagttttt taattgtaaa aatatagcgt   3540
ggaatgagtt ttttttttcgg ttggattttt tttcgttttc gatttttttt cgttaaggat   3600
tattgtaaat ttattttgta aaaggtagtt ttttaatgtt tttaaaattt tatttatttt   3660
tgtggttgag aggttagttt ttaatggttt tttggaggta tattttataa aaatacgttt   3720
atatattaat aggtttatgt ttttaataat atttttttat ttgttttaat tttagttaaa   3780
taaagggaag attatagttg taggtggaaa ggtttaagag aaatttaata ttagtttttt   3840
taaattagaa gatgttatat ggagatagga gaaatttttt tattttgagt agttggagag   3900
atttttggtt tttcggatcg ttaagaaagg tggttatatt ttgtgataac gttttgcgtg   3960
taaacgtttt aaatatatgc gaatgcgtta ttaggtttgg cggcgagatt tagaaagagt   4020
ttggattttt ttggttaata tttagataga ttgtgttgag cggtcgattt ttattttggt   4080
tatcgttttt tttattcgtt tgtagaagaa ggaatgatag ttatagtgtt ttcgtagggt   4140
ggtcggtttc ggggtagcgt tttcgtattt gtcgcgttta ggtttacgtt tattttttttt   4200
agtaacgtat ataggttaag taagattcgt ttgggtttta gcgtagaaga ggtcgaaatt   4260
gaggtttata ggttttagtt tatttttgta ttttattttt ttacggttat tttattagag   4320
gtttttggag agtttttgtt tttaagaatt aggataggga agaggtgtag agttttatag   4380
aaatttaacg ttttagaagg ttaatagatt ttttatttgt aggtttttta tttttggatg   4440
ttttttgttt tttagagaag tttttggatg gaagatatga ttatagtatt agtaataata   4500
ataattaata tttacggtgt gtttttttgtg tattaggtat tgaattaagt attttttttat   4560
ttaattttta taattttatg aggtattatt tttattttat ataggaagaa attgaagttt   4620
ataatttcgt ttagttagtg tgttaatgtg tggggattag gatttaatta taggtttttt   4680
ttgttttagg tttttttatcg ttattgggaa ggtttgtttt tttattagtt taattattta   4740
ttatatatta tttattgaat atttgttata tggtaggtgt tatgattaat tttagaggtt   4800
taataaaata ttttatttta aatttagatt ttataatttt tttgtaattt gtaattaatt   4860
```

```
tttttttttt ttttatgtta cgaggtttaa tttttaatat tattttattg ggatagtgag    4920
aatagagttg gttaaggttt tgattaatag tatgttatta ttaggaaggt tataaaattt    4980
tatatttttt ttagggtgta ggatgaattt ggggtgggga agtttaatgt tttatttaag    5040
ggtatagagt taggagtaga atgtaggttt agtgttttag attttattat tttagatttt    5100
tttttatttg agttttaggt ttgagaaatt tggtttttta aattgtatgt tttaaaattt    5160
ttgttttttt tagggattat tttgttgttt ttggattttt ttagattatt aaattattgg    5220
gttataaata atgataattt taatatttgt ttttttttta gttatttatt tatatttagt    5280
tttttttataa atgtttaggt ttggtttttt ggatataaat aggggtttta attagttaat    5340
tttaaaggtg tggggaaaat aaagttgttt gtgaattttg ttttgatagt agaggaaaag    5400
aagtagatat tttttttatgg agttttttaa tttagtgtta tatgtagtgt taggttaata    5460
gggattaatt attttttggg aggagatttt ttttggtttt tttttggtg attagtattt    5520
tattatgagg attttatttt agtttattgt ttttagaaag attaaaagtt gtagttttag    5580
ttggttgttt ttgttgttgt tgttaatgtt gttttgttgt ttttgtttgt taattataaa    5640
ataagaatat tgaataaaat aaagaattta tatttagtat tat                      5683
```

<210> 29

<211> 2347

<212> DNA

<213> Artificial Sequence

<220>

<223> chemically treated genomic DNA (Homo sapiens)

<400> 29

```
ttgaggttta agttagggag gtcgagattg tagtgagttt taattgcgtt attgtatttt     60
agtttgggtt atagagtaag attttgttta aaaaataata agaaaggtgg aggaaatagg    120
tagagattag aggaaagtta tggttttttt ttgattattt tgagattttt ttaattttat    180
taatttttgg agagtttttt atttgtttta ttttatagat tgggaaattg agtttagggg    240
tatttagatt atttgaggtt ttagttggag atgaagagat ggatttaaat tatttttttgt   300
ggagtatagt tttgtttttt ttagagtgga gatcgtgttt tagggttttt tttattcgta    360
ttaaggttgg tttttttagg ttttattttt tttatttgta aaatgggttt ttgtgagtat    420
gaaggagggt ttggtttgta gtgagcgttg tttattcgtt ggttagtgtg attagttatt    480
gttgttgttt tttagtggtt tttgtttggt agttgttgga aaaatatcga gggagggaga    540
tatagggttg gggaggtgaa aagttggata tagacgtttt tagtttttttg ggattaggaa   600
tagatggagg tcgggtgttt tgaagtggag tagggaggat tgtttggagg agggatattt    660
gagttagaat gtatagaatt atagggtatt ttcggtagag gttatagtag aggtaaaagt    720
ttagaggagg tgttttgggg attattaaga tggatatagt gggtttgggt ttttgtgggg    780
tgatgggttg gggtttcgag cgttaggtta aagggtttgg ttttttttcga gggcggtagg   840
gagttatggt aggtgtgtga gttggagggg tattttagat aaaggtgaag tggtattttt    900
tttttttattg aagttataat ttgatatttt ttttgaagtt ttttatattt tattttttttt  960
ttgttatatt ttggtaagtt tattttttttt ttttcgtttt taggtttgtt aagaggtttt  1020
gtgggttttt tatggtttta tttagttgtt ttagaagttc gtttgagata agtttttagg   1080
gttgttttat ttttaggtgt tttatagatt cgggggcggg tgaagtagga aggatcgagt   1140
tacgagggtt ttagaaaata tgacgggagg aattatagta ttttttttatt tttttagttt  1200
tcgttttttt agggagagag atttttagtt cgaggaggag ggtcgagttt tttttttatgt  1260
ggtaagagat tttaacgtcg ttgaggatta tttaaagagg gtgttgaagg atgtatagga   1320
gttagttagg agataattta tttgttaatt taataaattt ataggtatta tttattaata   1380
agaataattt ttattattta attaggattt tttaatgttt ttgagggatt atttgaggat   1440
gtttaattat tatttttatt tgagataggg ttttattttg ttatttaggt tggagtgtag   1500
tgatataatt atagtttatt gtagttttaa tttttttagtt ttaagttatt tttttatttt  1560
agtttttgat atagttgaga tataggttaa tgttattatg atgtttggtt tttttttttt   1620
tttttttttt ttttattttt gagatagagt tttgtttagt tatttaggtt ggagtgtagt   1680
ggtgcgattt tagtttatta taattttcgt tttttgagtt taagtaattt ttttgtttta   1740
gtttttttgag tagttgggat tatagatatg tattattata tttagttaat ttttgtattt  1800
gtagtagaga tggggtttta ttatgttgtt taggttggtt ttaaatgttt gattttaagt   1860
gatttgttgg tttcggtttt ttaaagggtt gggattatcg gcgtaagtta tcgtttttcg   1920
attatttagt tttttttttt ttttttttag tttttggtag aaatgtggtt ttatgtttgt   1980
atttagtat tttgggaagg tcgaggtggg tagattattt gaggttagga attcgagatt    2040
agtttgatta atatggagaa atttcgtttt tattaaaaat ataaaattag tcgggtatgg   2100
tggtttttgt ttgtaatttt agttatttgg gaggttgagg taagagaatt atttgaattt   2160
ggtaagcggg gtttgcggtg agttaagatc gtgttattgt attttagttt gggtaataag   2220
agtgaaattt tgttttaaaa aaataaaata aaataaaaat ataaaattag ttaggtatgg   2280

tggtacgcgt ttgtaatttt agttatttag gaggttgagg taggagaatc gtttgaattt   2340
ggtaggt                                                               2347
```

<210> 30
<211> 2347
<212> DNA
<213> Artificial Sequence

<220>
<223> chemically treated genomic DNA (Homo sapiens)

<400> 30

```
gtttattagg tttaagcgat tttttgttt tagtttttg agtagttagg attataggcg 60
cgtgttatta tgtttggtta attttgtatt tttattttat tttatttttt tgagatagaa 120
ttttattttt gttgtttagg ttggagtgta atggtacgat tttggtttat cgtaaatttc 180
gtttattagg tttaagtgat tttttgttt tagtttttta agtagttggg attatagata 240
ggagttatta tgttcggtta attttgtatt tttagtagag acggggtttt tttatgttgg 300
ttaggttagt ttcgaatttt tgattttaag tgatttgttt atttcggttt ttttaaagtg 360
ttgggatata ggtatgagat tatattttta ttaaaaattg aaaaaaaaaa aaaaaaagtt 420
ggatggtcga ggggcggtgg tttacgtcgg taattttagt tttttgggag gtcgaggtta 480
gtagattatt taaggttagg tatttgagat tagtttggat aatatggtga aattttattt 540
ttattataaa tataaaaatt agttgggtgt ggtggtgtat gtttgtaatt ttagttattt 600
aggaggttga ggtaggagaa ttgtttaaat ttaggaggcg gaggttatag tgagttgaaa 660
tcgtattatt gtattttagt ttgggtgatt gagtaagatt ttgttttaaa agtaaaaaaa 720
aaaaaaaaaa aaagaaaagt taggtattat ggtggtattg gtttgtgttt tagttatatt 780
agaggttgag gtagaaggat agtttgaaat tgggaggttg aggttgtagt gagttatgat 840
tgtgttattg tattttagtt tgggtaatag agtgagattt tgttttaaat aaaaataata 900
attaaatatt tttaaatgat tttttagagg tattagaaag ttttgattga atgatgaaaa 960
ttgtttttgt tgatgagtga tgtttatgag tttgttaaat taataaatgg gttgtttttt 1020
ggttaatttt tatgtatttt ttaatatttt ttttagatga tttttagcga cgttagggtt 1080
ttttgttata tggagggaag ttcggttttt tttttcgggt tgaaggtttt tttttttgag 1140
aaggcggaag ttgaggaggt ggggaagtgt tgtggttttt ttcgttatgt tttttggggt 1200
tttcgtgatt cgattttttt tgttttattc gttttcgagt ttgtggggta tttgaaagtg 1260
gggtagtttt gggaatttat tttagacgag tttttggggt agttgggtgg ggttataagg 1320
ggtttatagg gtttttttggt aggtttagag acggagggaa ggaggtgggt ttgttaagat 1380
gtggtaaggg gaaggtggga tgtggggaat tttagaggga gtgttaggtt gtgattttaa 1440
taaggggaga aatattattt tatttttgtt tggggtattt ttttagttta tatatttgtt 1500
atggtttttt atcgttttcg gagaaagtta ggttttttag tttggcgttc gaggttttag 1560
tttattattt tatagaaatt tagatttatt gtgtttattt tggtggtttt taaagtattt 1620
tttttggatt tttgtttttg ttgtggtttt tgtcggagat gttttgtaat tttatatatt 1680
ttagtttaag tgtttttttt ttaggtagtt tttttttgttt tattttaggg tattcggttt 1740
ttatttgttt ttgattttaa ggggttaggg gcgtttgtgt ttagtttttt attttttttag 1800
ttttgtattt tttttttttcg atgttttttt agtagttgtt aggtagaggt tattggggaa 1860
taataataat gattaattat attggttagc gggtggatag cgtttattgt aagttaagtt 1920
ttttttttata tttataaaag tttattttgt aggtgggaaa gatgaggttt gagagggtta 1980
attttggtgc gggtggggga agttttgagg tacggttttt attttggaag gggtaaaatt 2040
atattttata aggggtggtt tgaatttatt tttttatttt tagttggggt tttaggtggt 2100
ttgagtgttt ttggatttag tttttaatt tgtgaaataa ggtaagtgag ggatttttta 2160
ggaattggtg gagttgggag ggttttagga tggttaggag gggggttatga ttttttttttg 2220
attttattt attttttta ttttttttgt tattttttga gtagggtttt gttttgtggt 2280
ttaggttgga gtgtagtggc gtaattagag tttattgtag tttcgatttt tttggtttaa 2340
gttttag 2347
```

<210> 31

<211> 6709

<212> DNA

<213> Artificial Sequence

<220>

<223> chemically treated genomic DNA (Homo sapiens)

<400> 31

```
atttttaaat tttttaattt atagagggtt ggagagttgg tattatgagt ttttagtttt      60
tacgggtgag aaaagaggtg tttgtgtgga gggtaggtgc gatttcgggg gttagtagag     120
aggagaggtt gcgggaggat tcgttgcgaa gggcgagggg tggcgttggg tgggacgggc     180
gtttggggtc gtgtagggtg gtttttgggt gttgttcggg ttgtttgtcg tttagtattg     240
gcgtaggaag acgggttcgc gtagcgtttg gtaatagtgg tttgttttac gtttggaggc     300
gattaagttt tgagttaaag gcgtcgggtt tagtcgggaa gtgtatggcg atttagaata     360
cggagcggag aaggttttag gggatacgag gttatcgttt tagttttcgg gtttgcgttt     420
cgagcggtta gggtgcgtac ggatgggttt cgggatgtta gtggagaggt gataggagtt     480
tattatttta cggttaagcg tgtgaggttt tcggcgggcg ggggtttttt tcggggtagg     540
gtagggggag agttaggtat gtttttaacg ggttttttag tagaatcgtt aggaaagtgg     600
tttttaattt taggtatttt tgtggttttt aggtagttgg gcgattaaaa atttattttt     660
gtttgttgtg aaatgggata ggtggttgtt aggttagaga ttgtgtttac gaagtttcgt     720
ttggcgggtc gggattgacg tggattcggg ttttttttcgg aagtttggga atttgtgagt     780
tttttagttt ttcgttttat tttgtttgtt tgaaaaaagg agttgtttga gaggaagagt     840
taaggtttaa aaggttttaa acgaagagta tgcggtttga cgcggagcgt aaagaagcga     900
gtagtgaatt gaatttggtg ggattgagat tagaggcgtt tgtatgcggc ggtcgtttcg     960
cgttttatta aagtaatttg ttatttacga taattatttt tgtaattatt ttggagtggt    1020
tgtgatttga tgtttgtggt gttttagggt atgtttttat tggatataga tattttaggg    1080
tgtttttttga cgttagtgtc ggtttagtgg gtgttagtgt cgcgaaggga gggtggcggg    1140
cgtgaatgtt tttttttttt gggtttgtcg taggtttcgg ttttttttatt ttgtttgatt    1200
ttggcgggtt tgttatttat tgaggttttt atcggcgttt acgttgtttc gagaggcggg    1260
ataaacgttc ggcgcgtggc gcggagtttg cgaaagcgtt cggggggtttt tattttttgt    1320
tcgggagttt tttttttggg gcgggaagag gagtcgtaga tttgttcgaa ttttgtttta    1380
ttcgtttggt cgtttttttt cgtttggggt gggggtaggg gtggcggatg atttaggttt    1440
ggggtcggga gttttgtttt taaaggtatt atttcgcgtt tttttggaag tggcgcgttg    1500
ttggggcgtt ttcgttgttg tagaggtttg gtttgcggtt ttatagggc gtgggtttag     1560
ggtttttagg gagtaagtga gtttaggttt tttagggggc ggtagagggg aattttcgat    1620
aatttttttat tttttttttt aggcggttgc gaggttttcg ggttttaga ggggtttttt    1680
tttgtcggtg tcggttgttt tgagcgcggt tacgtagttc gcgtttttac gtgggtaagg    1740
ggcggggacg ttggagggag gttgttttgg atgttggagt tttcggtttt cggttttgtt    1800
gagtagaaaa gttgaagtta aggaagggtt gaggtttttt tataagggcg cgcgcgtgtt    1860
tgggtgtcgg tttagaagtc gagaatgtgc gtattgggga agggttttttc gtgtcgagaa   1920
gatagaggat ttgttttagt tgtttttcgt atagagggcg cggaggttgc gttttagttc    1980
gaacgtttat ttattggaaa gagggtagcg tcgggggttta gggaagtttt ttgggaatga   2040
atggtttttg ttaagcggtt tcggattttt tgggtttttt gggtttacgg tacggtgttg    2100
cgcgagtttt tagtgtttat cggttttttt cgtttttttgc gtagacgttt ttaggcgggg   2160
aggtatatcg gtttttttcgg gtagttttgg ttagtgttgt tgtgggaaag gagagttagg   2220
gtttgggatg gggatgagt atttttttgt ttatttcggg ttttagcgtg taggaggtaa     2280
atttgttagt atagataaga tagtttgttt aagttgtatt ttaggtcggg ttagagaata    2340
aaatcgaggg ttagaaggtt tagaatgtcg gatagtttag cggtattcgt tagggagttt    2400
taggcgttcg aaagaggcgt cgtattttttg gcgagtttag gatttatttt tttggatttg   2460
tgttttggag tgtttgcggg tttgggttta atttttgttt ttggtaggtg tttttttttt    2520
tcgtcgttaa tattaggagc gttgttttttg cggtaattta ttttattttt agaagtttgt   2580
tttgggatcg aagtgttagg gttttgtgtg ttttttttatg tattgttttt tttagtgtaa   2640
agttttgtag atatgtttgg tcgaaaagtt ttagtggttt taatttttag atttgttttt    2700
cgcgttcggt cggaaaatta tttgggattt ggttgttgtt taaggtcggg ggaaagtttt    2760
ttttggagga cgtttattat tattattttt ttttaggttt atttgggttt aaataaatgt    2820
taaagtttaa acgaattcga gaaggaatag cgaggtagtg gatgtcgtta tttcgtggat    2880
gggagagtta tgtttattgt aggtgcggat atcgtattga taggtattga ttattgtaaa    2940
atcgtattta gtattaaata gaaatagttt tgttttttaa tttttataga atgtttaaat    3000
ttagtttagg gagttagttg gggcgtttta ataaatacgt tttcgagaat tagcgatgtt    3060
tttttttataa tgaaagttaa ttgttgtata ataattgggt taaagaattt cgttagttta   3120
aattttaaaa attagtattg tattttgttt ttaagtttgg gtggtaagta tcgaagggga    3180
gagggaagta gcgtttgtgt ggaaggaagt ttcgtttttt gtttaaatat gtgggatttg    3240
ggtgtttatt tttttagtcg agagtttttt gtaggttttt ttttaatttt ttgtattttt    3300
ttatttttat tttattcgtg gcgttcgggt tttattttttg ttcggcgttt tttttttagg   3360
gcgagtagag gtttttgagt gatcggttcg ttagaggtag ttgtagagtc ggcgtttcgt    3420
agggtagggt agggtcgggt cgttgggttg gtttagtgcg cgcgggtttt ttttggtttt    3480
agttagtcgc gggagatgtt atttcgggcg atagcggcga gacgtcgttg ttcgtaattt    3540
ttgggcgggt aggatcgttt gttgtttttg cggtttttgg ggtagatttt ttaggagttt    3600
tttttttttt tttttcgttc gggggtcggg atcgcgttgt gtttacggag ggaattgggc    3660
gtttttggcg tattggggtt ttagttgtat gattttagtt tagattttaa gttttagtgg    3720
```

ggtgtaggtg ggatgggatg tgtttttagg agatgaggag atttcggttg ttgagtcgtt 3780
ttttaaatgc gtagtttttt aattttaggt gatattcgtt ttttggagga acgagtgaag 3840
gtcgtttagg cgaaagaggg gtgggtttta gtatttggat tttttttttt agttttgtta 3900
attttaggtg tgtttacggt ggaggtgata gaaggaaggc gcgtcggggg atttaggttt 3960
cgtaggtttt cggttgttaa cgaggtatcg tttattgatt tcgcgtttcg tttttttatag 4020
cgtggggttc ggcgttgcgt taggttcggg tgcggggcgg agttaggttg ggattggttg 4080
ggggtcgttt cgttcggcgt ttgggttttc gcgtcggttt tcggggagga gttatgataa 4140
tttttttttt attaaggcgt tcgggttttt cggttatcgt taggatatat tgttcgggcg 4200
cggttttttt cgttcgcgga gcggttttga tattcgcggc ggtagtattt acgttcgtag 4260
agtcgtcgat gcgtgtttag tgattcggat agtaaggttc gcgcgcggcg gggcgcggcg 4320
tagacgtttg gttatcgtga tttcgatttt ggatttatcg tttgggggtt ggggggattt 4380
tggatttaat tggcgattgt tttgggggac gtcggacgtt atgttgtgga aaataatcga 4440
taatgttaag tacgaagagg attgcgaggt gagttggggt ttcggggtgt agtttcgttt 4500
cgtcgaggat agttcgggag gtaggggtta ttggatcgag gtcggggacg agggtatagg 4560
agttttggtt tttcggtggg acgggattta ttttttcgga tatttttttag tttatttttg 4620
cggggttttt tttttgtata gggttttttt ttaaatagtt tttttcggg aacgaggttt 4680
ggaaagagaa tggtaaattt tatttatata gtttatcggg cgttttttta agcggtaaat 4740
agttttgttt ttcgtttttt cggaaaagtg tttcgtttgt aatttttaga cgtggttttt 4800
acgtacgaag tttttgttta aaggggttag atgaggttat ttgttagcga tattttggcg 4860
gatatttttt taaggtcggt ttgttatttg tttcgttatt ttttcgggga agtacgaaaa 4920
taatcgaagt ttggttttta aaagaagtgg gggttgggga gtggtggtgg gggtgttcga 4980
ggttagatgg ggtcgtaaga gttcgcgagt gttttagttt gtcggatagg ttgagttaga 5040
gtttcggaga gtgggaggga agaaggaggc ggcgagcggg aagaggaaga agacgtaggt 5100
agcgttaggc gagtttaggg gcgtcgggag cgcggagttc gggttacgga ttcgcgggag 5160
tttattgcgt tttcgggttt tggagggttg tttttgttcg taggttcggg cgttttcgtt 5220
aggaggcgat agcgttatgt tttttttaggt tttcggcgtt tcgagatttt tgggtagatg 5280
gggacgtatt ttttagaaat tgagtcgggt cgtttagggg cgagggaacg tgcgcgggta 5340
aggttgttta atttttaggg tttttttatgt tttttttgcg tttcgacgtg cgagaatatt 5400
gtttttggta gtgtagggcg tttttattta ttattttttt cggttgggtt cggttagtag 5460
ggagggtcgt tttgtgcgcg cgtttttttt ttattttttta gggcggcgta gggtggcggg 5520
ttttgttttt cgagcgtcgt tcgttcggag gtttttgttt cgagggcgtg gaagggaggg 5580
tttcgggggc gggggaggtg cgtatttttc gcgtcgcgta ttttattcgc gtttgtcgcg 5640
ttgttatttt tagtagtttt tgcgttatgg gcgggtttta cgagatagtt ttgtatcggg 5700
cgttcggttt tttcgtttcg ggttttggtt ttttcgtttc gggtttcggt tacggatttt 5760
tttggtttaa gatttagggt tcggatttgg cgtttttaag cgtttcgggt ttgggagtag 5820
cgtttagatt ttcgtcgtcg ggttttgaga attcgttttt ttaggttttt tattagattt 5880
tttttttttt tcgtattttt tgtttataac gaaattttcg gggcgtattg ttttcgggta 5940
tttttcgatt ttgggtaagt ttcgtcgggc ggggtgcggt tggtttttcg gggtttttttg 6000
cgtagttcgg cgatgtcggt tagttcgtag tagcggggtt tcgtattaac ggggtttttt 6060
gttttttttt tttttttttag gatcgttacg acgggagtag taatgggaat tcgcgggttt 6120
tttatttttt tttcgtcggg tagtattttt atagtttcgc gttatttttt ttttatattg 6180
gagtcgtcga atattagtcg ttattttatt tttttttttt ttattagtag ttggtttatt 6240
tttagtcggt cgatttttat tcgtatttgg gggaagcgta cgtcgtcgtt attaattttt 6300
tgtattagtc ggcgtttata ggtagttagt agtaggtttg gttcggtcgt tagagttagg 6360
agggagcggg gttgttttcg tattacgggc gttcggtcgg tttattgttt tattttttcg 6420
ggttggaggc gggcgcggtg agcgttcgta gggatgttta tcgtcgtttc gatttgttgt 6480
tgttttacgt atacgttttg gatgtcgcgg gtttggtcga gaatttgggg ttttacgata 6540
tgttttatta gatggacgag gtgtaggtga gcggcgttgc ggtttttgat cggatttgtt 6600
tattttacgg ttttttgttt ttatttcgta tttttttagg ttttttcgaa tttaagggaa 6660
ttttgttttt tttttttttag aatgtcgacg attagtattt gttgttgta 6709

<210> 32

<211> 6709

<212> DNA

<213> Artificial Sequence

<220>

<223> chemically treated genomic DNA (Homo sapiens)

<400> 32

```
tgtagtaata ggtgttggtc gtcgatattt tgggggagag aggataaaat ttttttaagt      60
tcgggggagt ttagaggagt gcggggtggg ggtagaaggt cgtagggtga ataggttcgg     120
```

```
ttaggagtcg tagcgtcgtt tatttgtatt tcgtttattt ggtgaggtat gtcgtggagt 180
tttaggtttt cggttaggtt cgcggtattt agggcgtgtg cgtggggtag tagtaggtcg 240
gagcggcggt aggtattttt gcgggcgttt atcgcgttcg tttttagttc ggagaggtgg 300
ggtagtaggt cggtcgggcg ttcgtggtgc gagggtagtt tcgttttttt ttggttttgg 360
cggtcgggtt aggtttgttg ttggttgttt gtgggcgtcg gttggtgtag gggttgatg 420
gcggcggcgt acgttttttt tagatgcgag taggggtcgg tcgattggga gtaggttagt 480
tgttggtagg gagggggaaa gtagggtggc ggttgatatt cggcgatttt agtgtgggag 540
aggggtggcg cggggttgta gaggtgttgt tcggcggagg agaggtgggg gattcgcgga 600
tttttattgt tgttttcgtc gtggcgattt tggagggaaa aaaaaaaata ggagatttcg 660
ttagtgcgaa atttcgttat tgcgaattgg tcggtatcgt cgggttacgt agagggtttc 720
gggggattaa tcgtatttcg ttcggcgggg tttgtttagg gtcggaaggt attcgggggt 780
aatgcgtttc gaggatttcg ttgtaagtaa agagtgcggg gagggaggag agtttggtga 840
aagatttggg ggaacgagtt tttaaagttc ggcggcgaag gtttaggcgt tgtttttaag 900
ttcgaggcgt ttggaggcgt taagttcgaa ttttgggttt tgggttagaa aagttcgtgg 960
tcggagttcg gggcgaagga gttagagttc ggggcgaagg agtcggacgt tcggtgtaga 1020
gttatttcgt ggagttcgtt tatgacgtag ggattgttgg aggtggtagc gcggtaggcg 1080
cggatagagt gcgcggcgcg ggaaatgcgt atttttttcg ttttcgggat tttttttttt 1140
acgttttcgg gataaagatt ttcgagcggg cggcgttcgg aaagtagggt tcgttatttt 1200
gcgtcgtttt gggaagtgaa gagggagcgc gcgtataggg cggttttttt tgttggtcgg 1260
gtttagtcga ggggagtaat aagtggggc gttttgtatt gttaagggta gtgttttcgt 1320
acgtcggggc gtagaggggg tatgaagaat tttggaaatt gggtagtttt gttcgcgtac 1380
gtttttttcgt ttttgggcgg ttcgatttag tttttaaagg atgcgttttt atttgtttag 1440
gggtttcggg gcgtcgggaa tttggaggaa tatggcgttg tcgtttttttg gcggaagcgt 1500
tcgggtttgc gggtaggggt agttttttag ggttcggagg cgtagtgaat tttcgcgagt 1560
tcgtagttcg agtttcgcgt tttcggcgtt tttgagttcg tttagcgttg tttgcgtttt 1620
tttttttttt tcgttcgtcg tttttttttt tttttttatt tttcgaaatt ttagtttaat 1680
ttgttcgata ggttggagta ttcgcgagtt tttacggttt tatttggttt cgggtatttt 1740
tattattatt ttttagtttt tatttttttt gaaagttaaa tttcggttgt tttcgtgttt 1800
tttcgaggag gtagcggggt aggtgataag tcggttttgg aggagtgttc gttaaggtgt 1860
cgttagtagg tagttttatt tgattttttt ggatagagat ttcgtgcgta aaagttacgt 1920
ttgagggttg tagacggggt atttttttcga gaaaacggga agtagggttg tttgtcgttt 1980
aggaaagcgt tcggtaagtt gtgtgggtgg gatttgttat tttttttttta ggtttcgttt 2040
tcgaggggag gttatttagg aaaaggtttt atataggaaa ggggtttcgt agaaatggat 2100
taagggtgt tcggagaagt ggatttcgtt ttatcgagag gttagggttt ttatgtttttc 2160
gttttcgatt tcggtttagt ggtttttgtt tttcggattg ttttcggcgg ggcggggttg 2220
tatttcggag ttttagttta tttcgtagtt tttttcgtat ttgatattat cggttatttt 2280
ttataatatg gcgttcggcg ttttttaaaa tagtcgttag ttaaatttag gatttttttta 2340
gtttttaagc ggtaaattta aaatcggggt tacggtgatt aggcgtttgt cgtcgttttc 2400
gtcgcgcgcg ggttttgttg ttcgggttat tggatacgta tcggcggttt tgcgagcgta 2460
gatgttgtcg tcgcgagtgt taagatcgtt tcgcggacgg ggaaagtcgc gttcgaatag 2520
tgtgttttgg cgatagtcgg gggattcgag cgttttaatg agaaggaaat tattataatt 2580
tttttttcggg ggtcggcgcg gaggtttagg cgtcgggcgg ggcggttttt agttagtttt 2640
agtttagttt cgtttcgtat tcgggtttgg cgtagcgtcg agttttacgt tgtgagggac 2700
gaagcgcgga gttagtgggc ggtgtttcgt tgatagtcga agatttgcga ggtttgggtt 2760
tttcgacgcg tttttttttt gttattttta tcgtaaatat atttgagatt ggtagagttg 2820
ggggaaggga tttaaatatt gaaatttatt tttttttcgt ttaaacggtt tttattcgtt 2880
tttttaaaag acggatgtta tttggagtta agaaattgcg tatttgggga gcgatttaat 2940
aatcggggtt tttttatttt ttaaaaatat attttatttt atttatattt tattgaggtt 3000
taaggtttga gttgggatta tgtagttgga gttttagtgc gttagagacg tttagttttt 3060
ttcgtgggta tagcgcggtt tcggttttcg ggcgggagga gagaggaagg gatttttggg 3120
gagtttgttt taagggtcgt agaggtagta gacggtttta ttcgtttagg agttgcggat 3180
agcggcgttt cgtcgttgtc gttcgaagtg gtatttttcg cggttggttg aggttagggg 3240
gaattcgcgc gtattggatt agtttagcgg ttcggttttg ttttgttttg cggaacgtcg 3300
gttttgtagt tgtttttggc gggtcggtta tttagaagtt tttattcgtt ttgggaaaga 3360
agcgtcgggt aggaataaaa ttcgagcgtt acgggtaagg tgagggtgag gaggtgtagg 3420
aagttgggag agagtttgta gagagtttttc gattggagag gtagatattt aggttttata 3480
tgtttagata gaaaacgaaa ttttttttta tataaacgtt gttttttttt tttttcggt 3540
atttgttatt taaatttgaa agtaaagtgt aatattaatt tttaaaattt aaattaacga 3600
aattttttgg tttagttatt atgtagtaat tgatttttat tgtaagggga atatcgttaa 3660
ttttcgaggg cgtatttgtt gggacgtttt aattggtttt ttgggttgga tttaaatatt 3720
ttgtggggat tagaggataa gattattttt atttgatatt gaatacgatt ttgtaataat 3780
taatgtttat tagtacggtg ttcgtatttg tagtagatat gatttttttta tttacggaat 3840
ggcggtattt attgtttcgt tgtttttttt cgggttcgtt tgaattttag tatttattta 3900
```

gatttaggtg ggtttaaaag aaaataataa taataggcgt tttttaaggg aaattttttt 3960
tcggttttag atagtagtta aattttaggt gatttttcga tcggacgcgg gaataaaatt 4020
tggaagttga aattattgag atttttcggt taagtatatt tgtagggttt tgtattaagg 4080
agaatagtgt ataaaaagat atataggatt ttgatatttc ggttttaaaa taggttttg 4140
ggggtaaaat aagttatcgt agaagtagcg tttttggtat tagcggcggg ggaggggat 4200
atttgttaga agtagaaatt agatttaggt tcgtaggtat tttagagtat aggtttagga 4260
agatgggttt tagattcgtt agaggtgcgg cgttttttttc gggcgtttgg gatttttga 4320
cgggtgtcgt tgggttgttc gatattttgg gtttttagt tttcggtttt atttttgat 4380
tcggtttgag gtgtagtttg aataagttgt tttgtttgtg ttggtaagtt tatttttgt 4440
acgttggggt tcggaatggg taagaaggtg tttattttt atttaggtt ttggtttttt 4500
tttttatag taatattagt taaagttgtt cggaggaatc gatatgtttt ttcgtttggg 4560
agcgtttacg taggaggcga agggagtcga tgggtattga gggttcgcgt agtatcgtgt 4620
cgtgggttta aaggatttag aggattcgga atcgtttggt aaaggttatt tatttttaag 4680
gagttttttt ggatttcggc gttgtttttt ttttaatggg taagcgttcg aattggagcg 4740
taattttcgc gtttttgta cggaaggtag ttagaataaa ttttttgttt tttcggtacg • 4800
ggagattttt ttttaatacg tatatttcg gtttttgggt cggtatttag gtacgcgcgc 4860
gtttttgtga ggaagtttta atttttttt gattttaatt tttttgttta gtagagtcgg 4920
gaatcgaagg ttttagtatt taagatagtt tttttaac gttttcgttt tttgtttacg 4980
tgaagacgcg gattgcgtgg tcgcgtttaa gatagtcggt atcggtagaa ggggattttt 5040
ttggaatttc ggaggtttcg tagtcgtttg gagaagagga tggaaggttg tcgggggttt 5100
tttttatcg ttttttaggg agtttaaatt tatttatttt ttggaagttt tggatttacg 5160
tttttatgaa atcgtaaatt aggttttgt aatagcggga gcgttttaat agcgcgttat 5220
ttttaaagag gcgcgaggtg gtgttttgg aaataggggtt ttcggtttta ggtttgagtt 5280
attcgttatt tttgttttta ttttagacgg aagaaggcgg ttaggcgggt agagtaagat 5340
tcgggtagat ttgcgatttt tttttcgtt ttagggaggg gatttcgag tagaagatgg 5400
aggttttcga gcgttttcgt agatttcgcg ttacgcgtcg gacgtttgtt tcgtttttcg 5460
ggataacgtg agcgtcggta gggattttag taagtggtag attcgttagg attaagtaag 5520
gtggaaaggt cggggtttgc ggtaaattta ggaggagggg gtatttacgt tcgttatttt 5580
tttttcgcgg tattggtatt tattggatcg atattggcgt taaagggtat tttgaagtat 5640
ttatgtttag taagggtatg ttttggggta ttatagatat taagttatag ttattttaaa 5700
ataattataa aaatgattat cgtaaataat agattgtttt aatagggcgc gaagcggtcg 5760
tcgtatatag acgtttttgg ttttagtttt attagattta gtttattgtt cgtttttttg 5820
cgtttcgcgt taggtcgtat gtttttcgtt tagaattttt tgagttttaa ttttttttt 5880
taaataattt tttttttaa ataggtaaag tgaggcgagg agttgagggg tttataggtt 5940
tttaagtttt cgaggagggt tcgggtttac gttagtttcg gttcgttaaa cgagatttcg 6000
tgggtatagt ttttaatttg ataattattt gttttatttt atagtaggta aaagtgaatt 6060
tttagtcgtt taattatttg aaagttataa aggtgtttag ggttgaaggt tattttttta 6120
gcggttttat tagggagttc gttagagata tgtttgattt ttttttgtt ttgtttcggg 6180
ggggattttc gttcgtcgga ggttttatac gtttggtcgt gggatggtag atttttgtta 6240
tttttttatt aatatttcgg agtttattcg tacgtatttt aatcgttcgg agcgtaggtt 6300
cgaggattaa ggcggtagtt tcgtgttttt tgaggttttt ttcgtttcgt gttttgggtc 6360
gttatatatt tttcggttaa gttcggcgtt tttaatttag aatttggtcg tttttaggcg 6420
tggagtaggt tattgttgtt agacgttgcg cggattcgtt tttttgcgtt agtattgggc 6480
gataggtagt tcgggtagta tttaggagtt attttgtacg gttttaggcg ttcgttttat 6540
ttagcgttat ttttcgtttt tcgtagcgag ttttttcgta gttttttttt tttgttagtt 6600
ttcggggtcg tatttgtttt ttatatagat atttttttt ttattcgtag gaattggaag 6660
tttatggtgt tagtttttta atttttgtg agttgaggag tttaagagt 6709

<210> 33

<211> 2296

<212> DNA

<213> Artificial Sequence

<220>

<223> chemically treated genomic DNA (Homo sapiens)

<400> 33

```
gttattgtat tttgttaata ttttttaaaa gtaaaaaata aattaaaaaa aaagttttta      60
gtggattgtt gagtttttttt tgataggagg tattaaatgt gttttttttg aggataggggg    120
aagagtttat tatttagtta tatttttgta tattttataa aatttatttt tattaaggat      180
gtttttttat aaaatgagga gtagtataaa gaatatttaa gaaattaggt ttgatttagg      240
gtttttgatg ttggggtagg tttttcgcgt gttaatattt taggggattt tgttgtagtt      300

ttttgaatat cggttgtgag ttttttaatt ttagttgagt tttagtattt tttttatatt      360
atttattttt tttgtttgtt tttggcgcgt ttagataatt ggaattttttg ttgatatttt     420
aaatgggttg gttatttttt tagcgtggag gtatttagta taaggtgtga tagattagta      480
tttgttgatg agtaataaag ttatttttggt aaattggatt tttttttttga aacgttttgg    540
tttttatatt tttaatgatg ggttaagaaa tagtttttaa gaagaagggg cggagatagg      600
gggtaggatt ttgtttgaag tttttaagtt gattttgttt gtagttattt aatttattag      660
ggagggttgg atttgttttt ttgtggtgga tttttcgtat ttttagaaag tgtaggggtg      720
gagaattaat ttagtttttt ttttttttat aagagtttcg ttaggttagt ttgtatgagg      780
agggtacgtg tatattttta aaaggattgg tgtagttatt tttttaagtt tatagtttta      840
gtgtgagttt agggtttaga ttcggggttt ttggtgtttt aggcgtgagt taagtttttt      900
gagtaagagg ttttatgata aattagttgg agtttggggg ttatgaaagt attggttatg      960
gtagaaatta ttagttcgtt atcgtttatg ttgttttgtt taaaaatgat atcgcgtaag     1020
ggattttgtt aatttttaat ttcgggagat tatttattat ttggttgtgg ttcgtcgttg     1080
aataggaaag tatttcgaga tgttaagaga ttaaaaggtt cgtaattgtt gtttagaaat     1140
gaaaatggtt ttaaattata attagagatg gaagcgcgtt ttaggggagg gcggggcgtt     1200
gtgtaatcga gtttttttatg cgtaaggcgg atataaacgg tttatggatg tgggcgaagg    1260
tttttagagt aaagaatgtt tatttttatt agattaaaat atagtttggg aaaataatta     1320
tttgtttgag attttgggaa agtaaaattt tttggttttt ttgttaggaa gaacgattgt     1380
aaaataaggg ttgagaattt tttaaataag atttagaat tttatttaaa tagttgtagt      1440
ttatttttga ggagttttag atttatttgg gtttgatgga tgttgtaata ttaagtttga     1500
ggttttttta gtttggtgtt aagagtgggt tttaggggat aaatagagaa tagttgtttt     1560
tttaatcgga gtagaaaata aaattttttt ttttttttat ttttaatttt tttgttttgt     1620
tgtgaaattg ttattgcgtt tttgaggagt gattaaaatt ttagttatga ttaatttaaa     1680
gggtaggttt ttaggtaggg aagggaaagg ggtttaaggt aaaaaaaaaa aaaaaaagtt     1740
agttgaagtg agtttatgga ggatttgggg attataaaag ttatgggatt tgagttaatt     1800
aaaattaatg gaaaagtatt aatatgtaat ttaaaaaata ataataaaat aaagtagtaa     1860
ggatattttt ggtttgtttt ttgtttttat ttttgagttt gtttgggaat tttggagtag     1920
attttttgaa gtatagtatg agtgtttaat ggttgtttta atttattttt ttgtttttta     1980
tatataaaga ttttgtaatg gagagtggga atgaatggtt tatttatttt tgaatatttt     2040
ttgtgagttg ggaatgcgtt tagtaaggtt gttggagtgg agggaaaaga aaaggtgttt     2100
gagtttttta gatatgggta gaaaaggaga gaatttgaga taggttgaga gaaagttgtt     2160
tcggttggta gtattgttag atttttagat atatcgtata ttttagtttt cgaaaaggtt     2220
attttgagtt tttttagggt ttttggggtt tttattttat tttaaatatt tggaattgtt     2280
tgtgtttgtt ataata                                                     2296
```

<210> 34
<211> 2296
<212> DNA
<213> Artificial Sequence

<220>
<223> chemically treated genomic DNA (Homo sapiens)

<400> 34

```
tattatagta ggtatagata gttttaaata tttgggatga gatgagagtt ttaggggttt      60
tagagggatt taggatggtt ttttcggggg ttggaatgta cggtgtgttt gagaatttgg     120
taatgttgtt aatcggagta gttttttttt agtttgtttt aaattttttt tttttttgtt     180
tatgtttaga gaatttagat attttttttt ttttttttat tttagtagtt ttattgagcg     240
tatttttaat ttataggaaa tgtttagggg tgggtggatt atttattttt atttttttatt     300
gtagaatttt tgtgtgtgaa gggtaggaaa ataggttaaa atagttattg ggtatttatg     360
ttgtgtttta ggaagtttgt tttaagattt ttaagtaggt ttaagaatgg aagtagagag     420
taagttagaa atatttttat tgttttattt tattattatt ttttaaattg tatgttggtg     480
ttttttttatt aattttaatt gatttaagtt ttatgatttt tgtgattttt aaattttttta     540
taaatttatt ttaattgatt tttttttttt tttttttgttt tgaatttttt tttttttttt     600
gtttgggaat ttgtttttta aattggttat ggttgggatt ttaattattt tttaaaggcg     660
tagtgataat tttataataa agtagggagg ttgggggtgg gaaggggagg gagttttgtt     720
ttttatttcg attaagagag taattgtttt ttgtttgttt tttggggttt atttttggta     780
ttaggttggg gagattttaa atttggtgtt gtaatattta ttagatttaa gtgagtttgg     840
ggttttttaa aggtgaattg tagttgtttg gataaaattt taaaatttttg tttgggagat     900
ttttagtttt tgttttataa tcgttttttt tggtaaagaa attaggaagt tttatttttt     960
taggatttta ggtaggtaat tgttttttta aattgtattt taatttgatg gaaatggata    1020
tttttttgttt tgagaatttt cgtttatatt tataaatcgt ttgtattcgt tttgcgtatg    1080

ggaggttcga ttatatagcg tttcgttttt ttttgagacg cgtttttatt tttggttata    1140
atttagggtt atttttattt ttaaataata gttacgagtt ttttagtttt ttggtatttc    1200
ggagtgtttt tttgtttagc ggcggattat agttaggtgg tgggtgattt ttcgaggttg    1260
ggaattggta aaatttttta cgcggtgtta tttttaagta aagtagtata aacggtgacg    1320
agttggtagt ttttgttata attaatgttt ttatggtttt taggttttaa ttgatttatt    1380
atgaggtttt ttgtttaggg gatttggttt acgtttgaaa tattagaggt ttcgagtttg    1440
ggttttgagt ttatattggg attgtgagtt tagggaagtg gttgtattag ttttttttgga    1500
ggtgtgtacg tgtttttttt atgtaggttg atttggcggg gtttttatgg ggaggagggg    1560
ggttgagtta atttttttatt tttgtatttt ttggagatgc ggagagttta ttatagggag    1620
gtagatttag ttttttttag tgaattaagt gattgtaaat agagttagtt tggaagtttt    1680
aggtagggtt ttattttttta ttttcgtttt ttttttttgg gggttgtttt ttgatttatt    1740
attaaaaatg taaaagttaa aacgttttag gggaaaagtt taatttgtta gaataatttt    1800
gttatttatt aataaatatt gatttattat attttgtgtt aggtgttttt acgttggaga    1860
agtagttagt ttatttaaaa tgttagtaaa gattttagtt gtttaaacgc gttagggata    1920
ggtaaggagg ataagtggtg tgaggaggat gttgggattt agttgaagtt ggaaagttta    1980
tagtcggtat ttagagaatt gtaatagggt tttttgggat attagtacgc ggggaatttg    2040
ttttagtatt agaggttttg ggttaggttt ggttttttag gtgtttttttg tgttattttt    2100
tattttgtgg agaggtattt ttggtagaag tgaattttgt aaaatatgta aaggtatggt    2160
tgaatagtga attttttttt tgtttttagg aaaagtatat ttggtatttt ttgttagagg    2220
ggatttagta atttattaaa gatttttttt ttaatttatt ttttattttt gaaaaatgtt    2280
ggtagggtgt agtgat                                                    2296
```

<210> 35

<211> 4247

<212> DNA

<213> Artificial Sequence

<220>

<223> chemically treated genomic DNA (Homo sapiens)

<400> 35

```
ttttaaaaaa attaaaatat aaaataataa taattattaa aaattttatt ttcgtttttt      60
taaataaaaa atttatttta aaaattttaa tttaaaaatt taaaaacgaa aaacgcgtat     120
tataattttt aaaaattttc gaatttaaaa acgattttcg tttttcgttt ttttcgcgcg     180
acgaaatcga atttattaat aaaaattcga aataaaacga ataaaaacga aacgaaacga     240
aattacgaaa aaataatcga cgaatcgcgt cgcgaattaa tattttaaaa aacgcgaaat     300
tcgaataatc gcgcgttaaa aatttcgaaa cgaacgtcgc gaaatttttt cgaattataa     360
ataaacgatt ttacgacgcg attcgaataa aaattcgacg attttataaa aaaatttaaa     420
atatcgacgt cgaatttttt ttaattttcg taataatcga tacgttatat aaaacgaaaa     480
aaaattcgaa ataaatttcg aaacgcgaat taaaaaaaac gaattcgatt cgcgatttta     540
acgtttttcg atcgacgcga aaaaaataaa attaaacgtt cgcgcgaatt tcgaaaaaaa     600
aaaaaataaa aaattttaac gattaaaaat ttttcgaacg tttatttcga aatttttttc     660
gtcgtttttt atttaaaata tatcgaattc gatacgattt tatcgtttta tttttttatta     720
cgattttttat ttcgaattaa atttaattaa tttattataa taattatcga taatcgtttt     780
ttcgtttcgt aatatttcgt aaattttaaa atcgcgacgt cgtcgaatat aaatttcgaa     840
tttaaaacgc gcgatttttt tttttacgat cgtcgttttt taaaaaaaat tacgcgaata     900
taaaaaattt atttaatacg ttttataaaa tttattattt ttttttcgat cgcgaattac     960
gtcgataatt cgaaaaattc gatttttcgt tttttatttt tttttttttt tttttttttt    1020
tttttttttt tttatttttt cgtttattcg tcgtaatttt ataaaaaaaa aatcgttttt    1080
ttaattattt ttttttttta tttttttatat atttttttat ttttttttcga tttataaaaa    1140
aaacgaaata acgattaaaa aacgttaaaa aaaaaaaata aaaacgtaaa ttacgaattt    1200
ataaacgata ttcgatttcg cgttcgacga aaattacgaa ttaattcgaa ttcgcgtttc    1260
gtttttttcgc gatatttaaa aatcgttcga attaaaatcg cgttattttt cgtttaacgt    1320
cgacgtaata aaatttacgc gatataaaat tttcgaaaat ttaaaaaatt ttaaaacgtt    1380
acgaatttaa tatttttttta tttataaaat tttaaattaa taaaaaatat ttcgcgaatt    1440
ttaaaaaaaa ataataaata tttttttattt tattcgaatt tttttaataa aaatttatac    1500
gtacgataac gttattacgt ttataaataa cgttaaaata taaaaaaaat aaaaaatcga    1560
aattcgaaaa ttttatttcg taataaaatt taaaatttaa tttaaatttt aaaaattcgt    1620
ttaaaattta aaatttaata taataaaaat aaaaaaaatt ttcgataatt tatttttata    1680
attaaaaaac gttaattaat attttttaaat ttttatttcg taaataaaat aaaaatatac    1740
gaaattattt aaaaaatttt tattattcgt tattattaaa tataatttaa atttcgtaaa    1800
aaaaaataat tttattttaa aattaaaatt tttaaaatat ttttaaaattc gataaaaaaa    1860
```

ataaaaaaaa taaaatttaa aaattaataa ttttatattt aatatttttc gtattttttt 1920
tttttttttat atttttttaat aatttttata attttaatta tttttaaatt ttattttttt 1980
cgttttttttt taaattaaaa tattaaaatt aaattataat ttaaattata tttatatttt 2040
attaaaaaat aaaaatttta aatttatcgc gttttttttt attattaatt ttaatttaat 2100
atttaataaa tttttttttt taaattatta aaataaattc gcgtaaattc gatttaatcg 2160
tcgttttcga attatcgaat aaatcgatcg tttcgtcgtt ttaaaaatta tttttatttt 2220
attttaattt aaatttaaaa aaatttaaat taataatttt aatcgaaaaa acgaaacgac 2280
gtttattaat tattattaaa attattatta ttattattat aataattaac gtaatatttt 2340
taatcgataa ttttttataa aaaaaaatat tattttttttt ttattatatt taaaataaat 2400
aaaacgaata ataaaaaaat aaaaaaaaaa aaaaaaaaaa aaatatatat ttttaataaa 2460
tttttaaatt taaaaattat ataattttat attaattatt atttttttttt aaataataaa 2520
aatttataaa tttattacgt ttattttata tttaaaaaat taatattttc gaaatattta 2580
ttaaataacg aaatatttta aaaaaaaaat aaaataaata aatttttaat ttttttatta 2640
aatttaatta taatttataa aaaaaatttt tttttttttt ttattttttt ttattaaaaa 2700
tattaaatat attaaattta atacgtttat tttataaata aaaaaaaaaa aatataaaat 2760
taataattaa tttaataaaa aatataatta tataatatta aaatttttta ttttatttat 2820
tttttttaatt tatttttatt tatttttaat ttaatattta ataaaataaa aatttattaa 2880
tttttttcgta tttatttatt atttaatttt ataattattt tttaatttta attaaatttc 2940
gtaaaatatt ttaaatttat ttttaataat ttttaattat cgttcgatta tttttatttt 3000
tttattttta tatatttatt tttatttcgt taaattaatt aaattttaat taaaattaat 3060
tttttatttt tttaaataat aaataataat tttttaaata aaattttaat aaatattaat 3120
aaattttttt tttttttta aaattataaa aataatattt tttatttaaa aataataaat 3180
tttttaaatt tttttcgatt taaattaaaa aaattaattt ataaaatata aaaattatat 3240
tttttttcga ttttttttttt tttttcgtta ttatttaatt ttttaattaa ttttttttta 3300
tttttaaatt taaatatttt aataattaat ataaatttat ttttattaat aaaataataa 3360
aaacgatata ttaaaaaatt atatatttat ttaaaaaaaa aaaataaatt tatattttttt 3420
aaatataaat aaaaaattta aaattttttt atttttatta aaaaaaaaaa aatttttaat 3480
ttttaaaaat taacgttttt taaaaattaa attattttaa ttttaaattt taattttatt 3540
ataaataaat tttataattt taaataaatt attaaatttt tttaaatttt aaatattata 3600
tttattatat ttaaataatt aaaaaaattt aataatataa tattatataa aatatttaat 3660
ttatattaat taaaatatta atttaaatcg ataaaatata taatttaaaa ataaataaat 3720
ttataaaaat atttttttatt aaaaataata ataaattaat attttttaata ttaaaatttt 3780
aaaatttaaa aattaaattt aatttaaata aaaattataa aattaaaaaa aaattaaatt 3840
tataatataa aaaaaatttt aaataatttt tttatttaat attttatcgt attttttttt 3900
tatttaataa aaaattaaat tattaaaaat ttaaatttat tttttaacgt aattatttaa 3960
tttatttcga ttttttttttt aataataaaa taaaaaaatc gtaaaaaaat ttttataaaa 4020
taaattatta ttattaaaat tttttttaata ttatttttta attatataaa atttaaaaaa 4080
taaattaaaa ataaattttt ttaaaaaaat ataaaaattt tttaaaaaaa aattttttaaa 4140
aattataaaa aaaaatttaa attataaaaa tatataattt taaaaaaaaa aatataatat 4200
tataaaaata ttattttttt ttaaaataat ttttaaattt aaatata 4247

<210> 36
<211> 4247
<212> DNA
<213> Artificial Sequence

<220>
<223> chemically treated genomic DNA (Homo sapiens)

<400> 36

```
tgtatttaaa tttagaaatt attttgagga gaaataatat ttttatagta ttgtattttt      60
tttttttagga ttatgtgttt ttataattta ggtttttttt tgtagttttt agaagttttt    120
ttttaaaaag tttttgtgtt tttttgggga ggtttatttt tagtttgttt tttgagtttt     180
gtgtagttag aaaatggtgt tagaaaaatt ttgataatag tagtttattt tataaaagtt     240
tttttgcgat ttttttattt tattgttggg aagagggtcg aggtaggtta ggtggttgcg     300
ttagaagatg agtttgggtt tttagtgatt tagtttttta ttaagtagag aggggggtgcg    360
gtagggtgtt gggtaggagg attgtttgag gttttttttg tgttatgagt ttgatttttt     420
tttaatttta tagtttttgt ttgagttaag tttaattttt agattttaaa attttagtat     480
tgaagatgtt agtttgttat tgtttttagt agaggatgtt tttataggtt tatttgtttt     540
taagttatat gttttatcga tttaaattag tgttttggtt ggtgtgggtt aggtatttta     600
tataatattg tattgttgaa ttttttttagt tatttagata taatagatat aatgtttgag    660
gtttagagag gtttagtaat ttgtttgggg ttatagagtt tatttatggt agagttggga     720
```

```
tttagaatta gggtagtttg atttttgaga gacgttgatt tttgagagtt agagattttt 780
tttttttaa tgggagtagg gagattttaa gttttttatt tgtgtttaga ggatgtaaat 840
ttgtttttt tttttaagta gatgtgtagt tttttagtat atcgttttg ttattttgtt 900
aataagagta ggtttgtgtt gattgttaag gtatttaggt ttagggatgg gagggggttg 960
gttagagagt tgggtagtag cggggagagg gggagggtcg ggaaggggta tggtttttat 1020
attttatgaa ttaatttttt tggtttgggt cggggagggt ttagaggatt tattattttt 1080
gaataagggg tgttattttt ataattttga ggaaggagga aagatttgtt gatatttatt 1140
gagattttat ttaaaaaatt gttatttgtt atttgagaga gtaggggggtt agttttggtt 1200
agggtttaat taatttggcg aagtaaaggt aggtatgtaa aggtaggaag gtgaaggtag 1260
tcgggcggtg gttgagagtt attgaagata aatttaagat gttttacggg gtttagttag 1320
gattagaagg tagttatggg gttaggtagt gggtggatgc ggggaagtta gtgggttttt 1380
gttttgttag atattaagtt ggaagtagat gaaagtgggt taagaggata gatggggtaa 1440
ggggttttag tattgtatgg ttgtattttt tattaagttg gttattggtt ttgtattttt 1500
tttttttat ttataagatg ggcgtgttag atttaatgtg tttaatgttt ttagtagaaa 1560
aaagtgagaa gagaaaggag gttttttttta taaattatag ttaggtttag tgagaagatt 1620
agaggtttgt ttgttttgtt tttttttaa ggtatttcgt tgtttagtag gtgtttcgga 1680
aatattagtt ttttgggtgt aggatgagcg taatggattt atagattttt gttgtttaga 1740
gaggggtagt gattagtata aggttatata gtttttaagt ttaagggttt gttggaggta 1800
tatattttt tttttttttt tttttttatt tttttgttgt tcgttttatt tattttgggt 1860
gtgatgggaa ggaagtgata ttttttttta tgaaaagtta tcgattggga atattgcgtt 1920
aattattata ataatggtaa tagtagtttt aataataatt agtagacgtc gtttcgtttt 1980
ttcggttgag gttgttggtt taagtttttt taagtttgag ttggagtaaa ataaagatag 2040
tttttgaagc ggcgaaacgg tcggtttatt cggtagttcg ggagcggcgg ttaggtcgag 2100
tttacgcgag tttgttttag tagtttgggg agggggggttt gttaagtgtt gagttggagt 2160
tagtagtgag aagaaacgcg atggtttgg gatttttatt ttttaatagg gtataggtgt 2220
agtttaagtt atagtttaat tttagtattt tagtttgggg gagggcgaga gagatagggt 2280
ttgggggtag ttgaggttat aagagttatt agagggtata gaaagggaag gaggtgcggg 2340
ggatattgaa tgtgaggtta ttaattttta agttttattt tttttgtttt ttttatcggg 2400
ttttgggatg ttttaaaagt tttagtttta ggatgggatt gtttttttttt acggagtttg 2460
ggttatattt ggtggtgacg gatagtgaga gtttttttaga tggtttcgtg tgttttttatt 2520
ttatttacgg ggtagaggtt tagaggtgtt agttgacgtt ttttaattgt agaagtagat 2580
tatcggaggt ttttttttatt tttgttatat taagttttag gttttaggcg ggtttttaag 2640
gtttggattg ggttttgagt tttgttgcgg ggtgaagttt tcgagtttcg attttttatt 2700
tttttgtgt tttagcgttg tttgtaggcg tagtgacgtt atcgtgcgtg tgggttttta 2760
ttagaggagt tcgggtgggg taagaggtat ttgttgtttt tttttggggt tcgcggagta 2820
tttttattg gtttaagatt ttgtaaataa agggatattg gattcgtagc gttttgggat 2880
tttttgggtt ttcgggggtt ttgtatcgcg taagttttat tacgtcggcg ttgggcggag 2940
ggtgacgcga ttttagttcg ggcgattttt agatatcgcg gagggacgga gcgcgggttc 3000
gggttggttc gtagttttcg tcgagcgcga agtcgggtat cgtttgtaag ttcgtagttt 3060
acgtttttgt tttttttttt tgacgttttt tagtcgttat ttcgtttttt ttgtagatcg 3120
agggaagata aggggatata taagggatag gagaggagag taattaggga agcgattttt 3180
tttttgtggg gttgcggcgg gtgggcggag agataggaga ggaaaagaga aaagggaaga 3240
gaaggaaggg gtagggggcg agaagtcggg tttttcggat tatcggcgta gttcgcggtc 3300
gaaagaggaa tagtggattt tataaagcgt attgagtaag tttttttatat tcgcgtagtt 3360
tttttagga aacggcggtc gtagaggaaa gggtcgcgcg ttttggattc ggagtttgtg 3420
ttcggcggcg tcgcggtttt gaaatttgcg ggatgttacg gggcggaggg gcggttgtcg 3480
atagttgtta taataaatta gttgagttta gttcgaggtg gaaatcgtag taggaagtgg 3540
gacgatagag tcgtatcggg ttcggtatat tttaggtaag agacggcgaa aagagtttcg 3600
agataaacgt tcgggaagtt tttggtcgtt aaaatttttt gttttttttt ttttcgaagt 3660
tcgcgcggac gtttagtttt atttttttcg cgtcggtcgg ggagcgttga agtcgcgggt 3720
cgggttcgtt tttttggtt cgcgtttcga aatttatttc gggtttttt tcgttttata 3780
tagcgtatcg attgttacga gggttggagg ggattcggcg tcggtatttt gaattttttt 3840
gtagggtcgt cgagttttg ttcgggtcgc gtcgtagagt cgtttattta tggttcggag 3900
aggtttcgcg gcgttcgttt cgagattttt agcgcgcggt tgttcggatt tcgcgttttt 3960
tgagatattg gttcgcggcg cggttcgtcg gttatttttt cgtagtttcg tttcgtttcg 4020
tttttgttcg ttttatttcg ggttttttatt ggtaagttcg gtttcgtcgc gcggagaagg 4080
cgggggggcgg ggatcgtttt tgagttcggg gatttttggg aattgtagtg cgcgtttttc 4140
gtttttaggt ttttagattg gagtttttgg aataggtttt ttatttagag gagcggaggt 4200
aggattttta gtaattattg ttgttttgtg ttttagtttt tttggag 4247
```

<210> 37

<211> 3905

<212> DNA
<213> Artificial Sequence

<220>
<223> chemically treated genomic DNA (Homo sapiens)

<400> 37

```
tttaaaaag tagatttgaa gatgtttaaa gtaataatgg tgttgttaga atggtgtttt 60
tttttttttt taggggatga ttttgaagga tattataata atagatgtgt aaattttgaa 120
atatatggtt aaaatgaggt ttattatttt ttagttatta tttagatatt ttttagttat 180
ttgtttatga tagtttataa atgagaaaga agtttattta aaatattttt atgaggattt 240
taaggtaaat aataggaatt gattatttat taatttggtt tggttttggt ttattgaaga 300
aagtttttt ttgagtgtat ttaaagtttt tatggtttag ttttgtttaa tttgagtagt 360
attttatttg tttttttttt agatgttttg atgttttgta ttagatttaa gagagagtta 420
agtttttaa taggttatgt atgtggttat taatatttag tatatgttat ttttggttag 480
tttttttttt ttttttaag aattaaatat tttttggttt gagtttttt tttttatgtt 540
attataaagt tgagttattt attattaggg tttatatatg agtagttaaa atatatgggt 600
ttttatattt aggttaaggg gtttttagta ggagaatatg aataattagg agtgaggtta 660
tgagatagag gaagaaaagt ttataggaat gggaagatag ttttttagat gagaggtgtg 720
tggagtgatt gttgtgagga attgtttaat aggaaagtta gttaatttga ggatgttttt 780
aggggaggaa aagggaaatt tatatggttt tttgttaatt tttaaaaata aggtagattt 840
ttatttattt agggtgtttg tttgttatga atataggaag attaaggaat gagtatggat 900
tgtttttatt ttatgttttt ggataagaag aataataaaa aagtattggg tagttggaat 960
aattgtagag gttttaataa tgatggagat tttttggtgt attgtggtat aggggttttt 1020
tttaaaggga tttaattaat tttaataggt attatttttt gtgatttaag atgttttttt 1080
taggagtgtt tgtatttttt aatgtttaga gaagtttgta ttattgattt gggaatattg 1140
agttttttag ttttgtaaa attattttta ggtttatttt taagtatatt ttttattttg 1200
gggggttaaa tttttggtt tttagtgaat attgtggtta tatttgtggt tatttattga 1260
tttttattt gtttagttta tttgataata ggtaaatggg gaaagaggtt ttaatatttt 1320
ttaaaagggg aagattgata attagttatt tattgtatag aattaggtta ggttaatttt 1380
ttttaagaag aaaagtaatt tggatattgt attttggggt ttttttttat tttggtatta 1440
taggttttat gaggatgtag ttttttgta ttttttttt tttttttaaa ttgttattta 1500
tttatttatt ttaattaggt taaagtatta tatattgtag aagagaaagt aattttagtt 1560
tttttaaat ggtgttattt ttatgatagt ttgtaaattt tggtttttta tgggtttagg 1620
atttgtgtgt atttgtttgg ggatttgttg gtggatttat tttttatggt ggagtagtaa 1680
ggggattttt tagaaaaagt aatgggtgaa gtaattgaaa gagtgatgta gaaagtaata 1740
gttagaaatg gtggggatgt gagtggttta gataggaagg gaggtggtgg tgtagttttg 1800
gtgtgtagtg tgttgtagtg atggaatttt tgtaaaagtt gtttgtttgt gtgttattag 1860
tggtgtgtag gtttgtggtt tttttgtttt tgtaatttg ttttaattgt tggtttattt 1920
tttgataaat aggatgtttt tatttgaggt tgttagtatt ttaggttttt tgaaagaaaa 1980
gaagtagagt ggttttattt tagaggtaag gatggggttt gtgttaagag gtttttaga 2040
gaagtgaaaa ttttgtaggt gtagttgttg ggagagtatt aagaagggta gggtggaggg 2100
gtagggggtg aagggagggg gtgaagtttg tattttattt ttatatgaaa ttgattttat 2160
tattttattt ttgtaagtgt ttagaggtag agaggttaat attttgggga tagtttggag 2220
gtgggagatt taggtagggt tttttaaatt tttatgtgta tgaaaattag gttaattatg 2280
gggtttttga gtaaatgggg atgatgattt agtaggtttg ggttgaggtt ttagattttg 2340
tatttttaat aagttttag gtggtagtga tgttgttagt ttaaagatta tattgtgaat 2400
agtaaggtgt tgttgggata gtttgtttta gagtaaaatt taatagatgt gtaaatattt 2460
aaattaagtt gtatttattt ttgaggtttt ggtttaattg gagttatttt gttaatggaa 2520
aatggaaaat gaaatttttt taggttttaa agggaaggaa agtttgaaat ggttaaggta 2580
tttaaaaaat tttagttggg agtggttttg gggatttggg agagttgaag ttaggttttg 2640
agttaagaga tgggggagga agggtgggat gtggtaaata aatataaata tatatatata 2700
aattttagtt taattatagg tttttttta ttaatgaaat atttgtattt tgtaggtaga 2760
atttatatgt gtttatattt tttagaagtt gaatggtttt gtgtaatggt atagaatttg 2820
gagtttatat ttgaatattt tagatttaat gttattttag attggtttta ggattttgtg 2880
gtttatattt aaaaatgtat gtttttgagt tatgtttaat tagtagtagt atttgagaat 2940
tgagagatta gattaagaaa tttatggtat atgaataaaa tttttagata gtagttagaa 3000
tatagaggtt tttttagagg tagtgttagt tgttagttgt aaaaataggt tttgtttagt 3060
tttgtttagg tttgaagttt tttgaagatt ttatttagat tttattaaaa ttagttaaag 3120
atttttgtt atagaaaggt ttttaaagta tggttgggat tatagggtta ttaagatgta 3180
gatttttggg tttttttgtt ttatttatta tggtttttta gggtgggtgt tgggaatttg 3240
tattttaata aatgttttga ggatttttat gtattgttaa gtttaggagt ttttggtttg 3300
taggtttaag tttaattttt ttattgtagt atttaaagat ttggttttaa ttttgttgtt 3360
```

```
tataggtgag ttttagtgat ttttttattg ttttttgagt atttttggag ttatttggtt     3420
gttttattta tttttttttt tttgtttttt tttagttttg gaataaaatg attttttattt    3480
ttttttttga attttttttta gattatggtt ttttatttag taagtagtta tttttttggt    3540
ttgttattgt tgtggtatta atgataggtt atttgatttt tattttgttg attgaatatt     3600
ttttatgttt aggttttgtt tttttgatta gatagtaagt tttttgaagt tattttatgt     3660
tatataattt ttatgtttaa gattattttt taatgtagtg ttatgtttat atggattatt     3720
tagtaaattt attgatggtt tatttattat taatatgttt tatagagtgt tattaagtag     3780
ggatattttt tatgggttta ggattagaag aagttgggaa gagaaatggg gtagtggtga     3840
gagtgttatg agaatttggt tatatagtta ttaaagatat agaggaagtg ggaaaaaggg     3900
agtgt                                                                  3905
```

<210> 38
<211> 3905
<212> DNA
<213> Artificial Sequence

<220>
<223> chemically treated genomic DNA (Homo sapiens)

<400> 38

```
atatttttt ttttttattt tttttgtgtt tttgatgatt gtataattag atttttatga 60
tatttttatt attatttttat ttttttttt agtttttttt gattttagat ttatagagag 120
tgtttttgtt taataatatt ttgtaaaata tgttggtgat agataagtta ttggtaaatt 180
tattgaatga tttatgtgga tatgatattg tgttagaaag tagttttgaa tataaagatt 240
atatagtatg aaatggtttt agagaattta ttgtttggtt agggaggtaa agtttaagta 300
tgagaaatat ttagttaata aaatgagaat tgagtggttt attattaata ttatagtagt 360
ggtagattag gaaagtaatt gtttgttgag tgggaagtta tgatttgaag agagtttgga 420
gaggaagatg gaagttattt tattttaggg ttggggaaag atagaggaga agaaatgaat 480
gaggtagtta ggtggtttta ggagtgttta gagggtagtg aggaagttat tgaggtttgt 540
ttgtgaatag taaaattgaa gttaggtttt tgaatgttgt agtgagaagg ttgaatttaa 600
atttgtagat tagggatttt taaatttggt agtatataag aatttttaaa gtgtttgtta 660
aaatgtaggt ttttggtatt tattttgaga aattatgatg agtgaggtag agaggtttaa 720
gaatttgtat tttaatagtt ttgtagtttt agttatattt taagaatttt tttgtaatag 780
gaagtttttg attggttttg atgaagttta aatgaaattt ttaggaaatt ttaggtttaa 840
gtaaaattga gtagaattta tttttatagt tgataattgg tattgttttt ggagggattt 900
ttatgtttta gttgttgttt ggagatttta tttatatatt atggattttt tagtttagtt 960
ttttagtttt tagatattat tattaattaa atatgattta gaaatatgta tttttaaatg 1020
taggttataa gattttaaag ttagtttgag ataatattgg atttagaata tttaaatatg 1080
ggttttaaat tttgtattat tatataaaat tatttaattt ttgaaaaatg tggatatgtg 1140
tgagttttat ttgtaagatg taaatgtttt attgatagga aagaatttgt ggttgggttg 1200
gggtttgtgt gtgtgtgttt atatttattt gttatatttt attttttttt ttttattttt 1260
tggtttagaa tttaatttta atttttttga atttttaagg ttatttttaa ttaggatttt 1320
ttaaatattt taattatttt aaattttttt ttttttttgga atttaaagga attttatttt 1380
ttatttttta ttagtaaggt ggttttagtt aaattagggt tttaggaata aatatagttt 1440
ggtttaaata tttgtatatt tattaggttt tattttaaag tagattgttt tgatgatgtt 1500
ttgttattta tagtgtggtt tttggattgg tagtattatt attatttggg aatttgttag 1560
aagtgtagaa tttgaggttt tagtttagat ttgttgaatt attgttttta tttgtttaag 1620
agttttgtga ttggtttgat ttttatgtat ataaaagttt aaggagtttt gtttaaattt 1680
tttgttttta agttgttttt gaaatgttgg tttttttgtt tttggatgtt tgtagagatg 1740
gggtagtgga attagtttta tgtgggggta gggtgtgggt tttattttttt tttttttgttt 1800
tttgtttttt tattttgttt tttttgatgt ttttttagtg gttgtatttg tagagttttt 1860
attttttttgg gaagtttttt gatatagatt ttgtttttgt ttttagggtg gggttgtttt 1920
attttttttt ttttaaagag tttgggatgt tgatagtttt agatggaggt attttgtttg 1980
ttgaaaaata aattggtagt taaagtaggg ttgtgagagt gagaaaatta taggtttgtg 2040
tgttgttgat aatgtgtggg taggtagttt ttgtagaagt tttgttgttg tggtgtgttg 2100
tgtattagag ttgtgttatt gttttttttt ttgtttgggt tgtttgtgtt tttgttgttt 2160
ttggttgttg tttttgtgt tgttttttta gttattttgt ttattgtttt ttttaaagga 2220
ttttttttgtt gttttgttat gagggatgga tttattaata agtttttggg tggatgtgtg 2280
tagattttgg atttatagga ggttaaaatt tatgagttgt tatgaaaatg atattgttta 2340
agagaagttg aagttgtttt ttttttgta gtatgtaatg ttttgatttg gttaaaataa 2400
ataaataaat aataatttaa aaagaaagaa agaaatatag aaagattata tttttatgag 2460
gtttgtaatg ttaaaatgaa aagggatttt aaagtgtaat atttagattg tttttttttt 2520
```

```
tggagagagt tgatttggtt tgattttatg tggtgaataa ttaattatta attttttttt   2580
tttgaggggt attaaagttt ttttttttat ttgtttgtta ttagataagt taggtagatg   2640
agaagttagt gggtaattat aggtataatt atgatattta ttggaaatta aaagatttga   2700
tttttttgagg taaagaatgt atttgaaaat aaatttgaaa atagttttat aggagttgaa   2760
aaatttagtg tttttgaatt agtaatatga atttttttga gtattaaaaa gtataagtat   2820
ttttaaaaag gatattttga gttatagaag atgatgtttg ttggggttga ttgagttttt   2880
ttggaagaga tttttgtgtt atggtgtatt agaaagtttt tattattgtt ggggtttttg   2940
tagttgtttt agttatttag tgttttttta ttattttttt tgtttgaaaa tatagagtag   3000
aaatagttta tatttatttt ttaatttttt tgtgtttata atgagtaagt attttagatg   3060
agtaagaatt tattttattt ttaagaatta gtagagaatt atatgagttt tttttttttt   3120
tttttagaaa tgtttttagg ttggttaatt tttttattga gtaatttttt atagtaatta   3180
ttttgtatat tttttatttg agaggttgtt tttttatttt tatgagtttt ttttttttttg   3240
ttttatggtt ttatttttaa ttatttatgt ttttttgttg aaggtttttt gatttaaata   3300
tagagatttg tatattttgg ttatttatgt atagatttta ataatgaata atttaatttt   3360
atggtgatat aaaggaggaa aatttaggtt aaagaatgtt taatttttag ggaagaagga   3420
gagagttggt tagggatagt gtgtgttggg tgttaatagt tatgtgtata gtttgttggg   3480
gaatttggtt ttttttttgaa tttggtgtag ggtattggaa tgtttggggg aggagtagat   3540
aggatgttgt ttaggttgaa tagggttgga ttatgagagt tttgaatgta tttaagaagg   3600
aatttttttt agtgggttgg ggttaggtta gattgatgaa tgattgattt ttattattta   3660
ttttgaggtt tttatggaga tattttaaat ggattttttt tttatttata aattgttata   3720
aataaatgat taaaagatgt ttaagtggtg attagaaaat aataaatttt attttagtta   3780
tatattttag aatttatata tttgttattg tagtgttttt taaagttatt ttttgggagg   3840
gagagggagt attattttaa tagtattatt attattttaa atatttttgg atttgttttt   3900
ttgaa                                                               3905
```

<210> 39
<211> 5338
<212> DNA
<213> Artificial Sequence

<220>
<223> chemically treated genomic DNA (Homo sapiens)

<400> 39

```
ggtaatttt tttgttatta tttttgtat aaaatatgta agatgtttt attttattag    60
tattgaaatt aaagattaaa agagagaaag gtgttggatt gaaaaggagg tggtgtaatg   120
gttattttt ttagtatttt ggttttgttt tttttgggga aggtggttat attttattt   180
gttttgtttt tgaatgtagt aaataatttt ataaataatt attgttgttt atgtttttta   240
tttgtttttt tggttttatt agatttttgt ttttttgtat tttttgggta gggtttttaa   300
taagtttagg ttgaaagaat gtttgttatt ttttttattt ttgttgaaag aaaaggaaga   360
aaaatagtag tagtgagaaa tttttggggt gatttttttt ttgtttttaa gtattagtgt   420
atagtatttt tttttgtttt tgttgtggtt ttttgttgtt ttgggttatg ttgtttagtt   480
aagtaatttg gatttgagag tgtatagtta ggattagttt aggggggtgag gggttggttt   540
ttgggaaatt aagtgtttag gatagaggtg gaaagtgggt tttgggagtt agaaaagaga   600
gagaagggta gatggttggg tggtaaatat aaaaatagaa ataatttagg gggatgtttg   660
ttaggttttt gtgtttgttt tttttttttt aatttggagt aggttttttt tggtttttttg   720
tgttttgggg tgtttttttgg tggtagtggt agtagtgggt aatgtgtgga gggtttaggg   780
ggtgtatagg ggatttttgg gtatatttag agaggtgggt gtggtttttt ggtggtggtg   840
atgtagttat ttggtgagtg gagttttgtt tttttggttt ggtgggttta tggttgtttt   900
attaagtatt gtggttgagt agggtaggaa gttgtttttg gaatgtggtg ttgttgtgtt   960
gtagttgaag tttgagtttt ttttggtttt ggtgtttttg ttgttgttgt tattattatt  1020
attgttgttg ttattgttat tttgggaatt tagggttgtg gtagttgttg ttgtggttgt  1080
tgttgttgat tgattgttgt ggtaattgag gtatgagtag ggtgtagagt tgttgttggg  1140
gggtgtgttg gttgttgttg ttgaggaggt tgtagttgtt gtggttgttg tggttgttgt  1200
ggtagaggtt gtgttgttga gttttgtggt ggttgtggga gtttggtaga gattagggtg  1260
gtggaagtta tatagtagtt ttggttgaga gtaggggtgt gagagggtgt ggaaggtgtt  1320
tagtggttgg atggaagtag tgaagggtga tgaagttgtg gttgttgtgt ttgaggttgt  1380
agttgtggtt gttgttgttg tgatgtttat gtgtgggtag tagtgtagtg gtatgtgtga  1440
gtggaagggg tagggtaggt ttttggtggt ggttgtgtgt gttattatgt aggtgtagaa  1500
gttggggttg gttgggtgtg gttaggatat ggttaggtgt tgttgtttgt tttttatgtg  1560
ttggttttgg aattatattt gtggggagag atgtgttgta gtttgggtta gggagtgttt  1620
tgtgttttta gtttttgttt taggattttt gtttttttttg gatttttgaa tggtttggtt  1680
```

```
tatttttttt tgattaagtt taattttgag tattttgtgg ttttttagtt gggaaagtgt    1740
ggatggtagt gtgtggattg ttgtgggtat attgttttta atgaagaggg tttttttttt   1800
tgtgtttggt tgttgttgtt ttttaggttt ttattttttt atttttgtt gtattttttt    1860
gttttaattt aattttttttt tttttttttg ttatttatta gtgttggttt tgttgagtat  1920
ttgtttttta attttaggat ttgtatgggg attttgggta gtttttgaag agaggttgtt   1980
gtttagtttt tttgagaggt tgttgtgtga agttttgagt tttttgaatt gtaaggattt   2040
gtttttaggg gtatgggttt gattttgata ttgaagggat gattttgttg gaattgtttg   2100
ttttaaatga gtgggtagag taatgttttt ataaatgggg aaggggatgt ttttattttt   2160
tttaatatta tttaataaag atattatttg ttataatttt aaattaaaga aagtttgatt   2220
aaattagagg gagattttta tatttttttt ttattttgtg gagatttttt tttttttttg   2280
tagtgttgaa tgttttatga agggtttatt aaattgttta atttttggt tatttttttt    2340
agatgtataa taaaattaat aatttaaagt tatatataaa taaggataat tttgttgtat   2400
tttttttttgt aggaggttgt tttttttttg ttgtttaaga ggaaaatgtt taggaaatta  2460
ttgttttaaa ttaattgatt ttaaagatat agtatttttt tttttgtgta atgatttatg   2520
tggaaaataa atttttaagt ttaagagtaa atgaaaagtt ttatttttgg tttttgtttg   2580
aggaataaag attaattggg tttgttgttt aggggaaagt ggggttgtgg gtatgggtga   2640
gggggtattt ggttaggtgt tgggtataat ggagtagggg tgagtgtttt tagtattgga   2700
gttattattt ggggggtttt ttagatttgt taggttggat aattgtttgg atttggtggt   2760
tgggaaataa ataagttaat tttttggtg attatttttt gtggattttt agattttag     2820
ttaaatttag ttatttagaa aggggaaagg ggaaaaagaa ataattaatt tagatgtttt   2880
tggggaggtt agagtaggta tgtattggaa ttgatatttt gatggtggtt ttgggtaggt   2940
tgagtgttgt ggttagtttg tattggtggg gttgtgatat atagtttttt tggtagaatt   3000
ttttttttag gtgtgtgatt tgtttgtggg tgaatgttgt atggtagtgt tgtatttgat   3060
ttgtgttaga gttggagtta tttagtgttg tgtttttgtt gttgttttg ttgtttttat    3120
gtaggttttt gaggtttgag tttgatgttg atgttgtggt gttggtagtt gagttgtttt   3180
ttgtgtattt tggtaaataa atgattaata gtgtatgagt ggttgtagga ttaatagttt   3240
ggtgttggtg ttgtgtgtgg attggggaag ttttgttagg aaggagagtt gttgttggaa   3300
ttgatggggt ttgttatgtt tataaattgt tgttgttaat ggaattaata aagtttgggg   3360
agttttttat aagtaaataa tagaaatgga attaggagat ttttttttta ataattagaa   3420
atttttaatt aaggaggaaa agtggttgag ggaaaatgtt tatttttggg tgtagtttgg   3480
gaagttttgg gtttttttatg gtttggagat tgtagggtag ttttttatat ggtttttaat  3540
ttttatttga gtttttgggg ttttaaatat tttaagagtt ttaatatagt agtagtttaa   3600
atttaagtta ataggggggtg aaatatattt tttaattttt tttttgttg atttaaatag   3660
agatgttggt gaggtttttt ttgatttatt tagaagaatg tttttttttag ttttgagggt  3720
ataggggtag gggagtttag agtagttgtt tagggtattt tttttaaaag taatttggtt   3780
gttgattagg ggtttattgg tttgtttggg tttttagata tgagtgtaga aatatttttt   3840
tggatttagg agtgagggtg ggtgggtttg gtgttttagg ttttgtaggt ttgagtttgg   3900
tgggaaagtt taaggagtag aattggaagg tatggtttta gatatgtgtt ttgtttttgt   3960
ttgtgttagt ttggtgtagt ttgtttgtgg agggtttgag aggggaaaag gtattgggaa   4020
aggttggtgg gggttgtgga ggagtaaaga ggatgggatt ggagagtgtg gtgtggttgg   4080
tgtggttatt tttgttattg tttttttttaa gttgagtggt gttgaggttt ttggggggagt 4140
gaagtgtgga gtagtttatt tttatgttgt tgtttatgtt ggttttagtg gttgtttttg   4200
aataatggtt tggtttttttg tggtttttgg tggtggagga gattttggag gagatggtgt  4260
ttttgttgtt tgtgtgttgt aggttgaata aagtgtttat tttgaatttg tttttggtgg   4320
ggagtttttt tagagtgttg tgtagggggg tagatggtag gtgtgggttt aggtgagttg   4380
ggtgttgtga atttttttagg gttttgagta tagtattgtt agttgagttg gataaattgg  4440
agaatttttt gtttgttgta gggttgtgta gttttttttt tattagaatt attttttttt   4500
ttattttttt tattatttta gtttttttaa aaatgttata gtggttttgt tgttttgttt   4560
taatgatagg ttgtgtttag ggttaatttt tatttagttt tagtgaatgt ttttaaatat   4620
tattattgtt tttggaggga ggtggaaaaa ttgtgggatg ttagagtgag ggaatgattg   4680
gttaaaatga tttatatatt tttttgattt tgagatgtta tttattaaaa agtagtgttt   4740
gtttgttatt aaggtatgaa tgatgttgtt tgaataatta tttattgtaa taggtttata   4800
agtaaataaa tatatttttt tgttagtggg taatgaaggt agttttagag tagataaata   4860
gatttaggta ggaggtgaga agagataagt gaggaggaag gttttggttt tttgtttgtt   4920
ttgagttagt tttgtttgtt tggggggtttg gttttggggg tgaaattgat agtttgatta  4980
atagagtgtg ttgtggtata tttttttttt tatttagggg tattattatg tttttagttt   5040
gttaggttgt tttttaggtt ttaattatgt agtgtttttt ttattttttt ttggatatag   5100
atatgtatta aataatagat aatggtttga ttttttagag taggtttttg ggaaggtttg   5160
gtttttttttt tttttttttt tttttttttt attatttata agttgggggt ataagtgggg  5220
ggagataagg gggaggatgt aggaagagtt gtgtaaggag aagattttgt gttttttttta  5280
tttggattat ttattttttg gattttggaa ttaaatatgt attttattta ataaatag     5338
```

<210> 40
<211> 5338
<212> DNA
<213> Artificial Sequence

<220>
<223> chemically treated genomic DNA (Homo sapiens)

<400> 40

ttatttgtta aataaaatat atatttggtt ttgaaatttg aaagataagt aatttagata 60
ggagaggtgt aaggtttttt ttttatgtaa tttttttgt attttttttt ttattttttt 120
ttatttatgt ttttagtttg taaatggtaa aaaaaaaaaa aaaaaaaaaa aaaaaaatta 180
aatttttttg aggatttatt ttggaaagtt aaattattat ttattattta atatgtattt 240
gtgtttaagg aaaaatgaag aaggtgttgt atgattagga tttaaggggt aatttagtaa 300
attgagagtg taatgatgtt tttaaatgaa gggggaaatg tgttgtggtg tgttttatta 360
attagattgt taattttatt tttgaggtta aatttttggg tgagtagaat tggtttggag 420
tgggtaaagg attggagttt tttttttat ttgttttttt ttgtttttta tttggattta 480
tttgtttgtt ttgaagttgt ttttattatt tattgatagg agggtgtatt tatttgttta 540
taaatttgtt ataataaatg attatttgaa tagtgttatt tgtattttaa tgatgagtgg 600
gtgttatttt ttgatgaatg atattttggg attggaagga tgtatgggtt attttgatta 660
gttatttttt tgttttggta ttttataatt tttttgtttt tttttgaaag tgataataat 720
atttagaggt gtttgttggg gttgaatgag aattagtttt aggtgtagtt tattattagg 780
atgggatagt agggttattg tgatattttt aagaaagttg agatgatgga aagaataaga 840
aaagagatga ttttgatgga gagagggttg tatagtttta tggtgggtaa gagatttttt 900
aatttgttta atttggttgg taatgttgtg tttgaggttt tggaaaattt gtagtatttg 960
gtttgtttaa gtttgtgttt gttgtttgtt tttttgtata gtgttttggg agaatttttt 1020
gttaagggta aatttgaaat agatattttg tttaatttgt agtatatggg tagtgaaagt 1080
attgtttttt ttgaaatttt ttttgttgtt gagagttgta agaagttggg ttattattta 1140
gaggtggttg ttgaggttga tatgagtagt gatgtggagg tgggttgttt tgtgttttgt 1200
tttttggggg gttttggtgt tgtttagttt aaggaaaata atggtaaagg taattgtgtt 1260
ggttgtattg tgtttttttag ttttattttt tttgtttttt tgtggttttt gttagttttt 1320
tttggtgttt tttttttttt tagatttttt ataggtaagt tatattgagt tagtatgggt 1380
gggggtggga tgtatgtttg ggattgtgtt ttttagtttt gtttttttgag ttttttttgtt 1440
aggtttaggt ttgtggagtt tggaatatta ggtttatttg tttttgtttt tgagtttgag 1500
aaaatgtttt tgtgtttgtg tttaagagtt tgagtgagtt ggtagatttt tagttagtag 1560
ttgagttgtt tttgggaaag gtattttggg taattatttt gaattttttt gtttttgtgt 1620
ttttagggtt ggagggggta tttttttggg taagttggag gaagttttgt tggtgttttt 1680
gtttaggtta gtgggagaaa gagttgaaaa gtatatttta ttttttattg gtttgggttt 1740
gagttattgt tgtgttggaa tttttaaaat atttgaaatt ttgaagattt agataaaggt 1800
tagaggttgt ataaaaagtt gttttgtggt ttttagatta tgggaaattt agagtttttt 1860
aaattgtgtt tagaggtagg tatttttttt tggttatttt tttttttttgg ttgaaaattt 1920
ttaattatta aaaaagaaat tttttaattt tgtttttatt atttgtttat gaagggtttt 1980
ttaaatttta ttgattttat taatggtagt aatttgtaag tatgatagat tttattaatt 2040
ttggtagtga tttttttttt tgatggggtt tttttgattt gtgtgtagtg ttagtgttgg 2100
gttgttggtt ttatagttat ttatgtgttg ttggttgttt gtttgttagg gtatgtagag 2160
agtggtttgg ttgttggtat tatgatgttg gtgttgggtt taggttttgg aagtttgtat 2220
ggaggtagtg gaggtagtgg tgggagtgtg gtgttgggtg gttttggttt tggtgtggat 2280
taagtgtggt gttattgtat ggtgtttatt tgtgagtaga ttgtgtgttt ggagaaggag 2340
ttttattggg agaattatgt gttgtggttt tgttggtgtg agttggttgt ggtatttaat 2400
ttgtttgaaa ttattattaa ggtattaatt ttagtgtatg tttgttttgg ttttttttggg 2460
ggtatttggg ttgattgttt tttttttttt tttttttttt ttgggtggtt agatttagtt 2520
aagggtttgg aaatttgtgg ggggtagtta ttaaaggaat tggtttattt atttttttggt 2580
tattgaattt gaatggttgt ttggtttgat ggatttaaga aggtttttga atggtgattt 2640
taatgttgaa agtatttgtt tttgttttat tgtgtttaat gtttggttag atgttttttt 2700
gtttatattt atggttttat tttttttttag gtggtaagtt tagttggttt ttgtttttta 2760
agtaggaatt agaggtgaaa tttttttattt gtttttgagt ttggagattt atttttttgta 2820
taaattgtta tatggagaaa aggatattgt atttttaaaa ttgattggtt tgagatagta 2880
gtttttttgaa tatttttttt ttgggtaatg aaaaaagggt aatttttttgt ggggaaaaat 2940
gtaatgaaat tgtttttatt tatatataat tttaggttat taattttatt atatgtttgg 3000
gagagatagt tgggaggtta ggtgatttgg tgagtttttt atggagtgtt tgatattgta 3060
gaaaaaagaa aaaattttta tgggtagggg gaggagtgtg gaagtttttt tttggtttga 3120
ttgggttttt tttaatttag agttgtgatg aatgatgttt ttattagata gtgttgggag 3180
gggtggaaat gttttttttt ttgtttatgg agatattgtt ttatttattt atttaaggta 3240

aatgatttta ataaaattat ttttttaata ttaaaattgg atttgtgttt ttaggggtag 3300
gtttttgtag tttggagggt ttaagatttt gtgtggtgat tttttagaaa agttgagtgg 3360
tagttttttt ttaaaggttg tttagaattt ttgtataaat tttgggattg agagatgggt 3420
gtttagtgag attggtattg gtgggtggtg ggagggagag gaaaggttgg attgggataa 3480
aaaagtgtag taagaagtaa gggaataaaa gtttgaggag tagtagtagt tggatgtggg 3540
ggagaggatt ttttttgttg aggatggtgt gtttatggtg gtttatatat tgttgtttat 3600
attttttttag ttgggagatt atagggtatt tggggttggg tttggttgga gagaagtaga 3660
ttaagttatt tagaggtttg gaaggggtag aggttttggg ataggagttg ggaatatggg 3720
gtgtttttta atttaggttg tggtgtgttt ttttttgtag gtgtggtttt agaattggtg 3780
tatgaaggat aagtggtagt gtttggttat gttttggttg tatttagttg attttagttt 3840
ttatatttat atgatgatgt atgtggttgt tattggaagt ttgttttatt ttttttattt 3900
gtatgtgttg ttgtattatt atttgtatgt gggtgttatg gtggtggtgg ttgtggttgt 3960
agttttaggt gtggtggttg tggttttgtt gttttttgtt atttttattt ggttattgga 4020
tatttttttgt gtttttttgt atttttattt ttggttggag ttgttgtgta gtttttgtta 4080
ttttggtttt tattaggttt ttgtggttgt tgtggggttt aatagtgtgg tttttgttgt 4140
ggtagttgtg gtagttgtag tggttgtggt ttttttggtg gtggtggttg gtgtgttttt 4200
tagtggtggt tttgtatttt gtttgtgttt tagttgttat agtagttagt tggtggtggt 4260
agttgtggta gtagttgttg tagttttggg tttttggggt ggtggtggtg gtggtggtgg 4320
tggtggtggt ggtggtggtg ggggtgttgg ggttggggga ggtttggatt ttggttgtag 4380
tgtggtggtg ttgtgttttg agagtggttt tttgttttat ttggttgtgg tgtttagtaa 4440
gatggttgtg agtttgttgg attagaggga tgaggttttg tttattagat aattatgttg 4500
ttattgttag ggggttgtgt ttgttttttt gagtgtgttt gggagttttt tgtgtgtttt 4560
ttgagttttt tgtgtgttgt ttgttgttgt tgttgttgtt agggggtgtt ttgggggtgtg 4620
ggaggttgaa gggaatttgt tttgaattgg gggagaagga gtaggtgtaa aagtttggtg 4680
ggtatttttt taaattattt ttatttttgt atttgttatt tagttgtttg tttttttttt 4740
tttttttttgg tttttgggat ttattttttta tttttgtttt gagtgtttgg ttttttaaag 4800
attaattttt tgtttttttaa gttagttttg gttgtgtatt tttaggtttg ggttgtttgg 4860
ttgaatggtg tggtttgaag taatggagaa ttataataaa gatagagggg gatgttgtgt 4920
gttggtgttt gggggtgggg gaggagttgt tttggaggtt ttttgttgtt gttgtttttt 4980
tttttttttt tttaatgagg gtgggggagg tggtaaatgt ttttttagtt tgagtttatt 5040
agggatttttg tttgaagagt gtgggagaat ggaggtttga taaggttggg aggatggatg 5100
gagagtgtgg gtagtggtgg ttgtttgtga gattgtttat tatgtttagg agtaaggtgg 5160
gtagggatgt ggttgttttt tttgggaagg ataaagttga ggtgttggaa ggggtggttg 5220
ttgtgttgtt tttttttttgg tttaatattt tttttttttt tggtttttgg ttttagtgtt 5280
gatggaatgg aagtattttg tatattttat gtaaaaagtg atggtaaaag aggttgtt 5338

<210> 41
<211> 5332
<212> DNA
<213> Artificial Sequence

<220>
<223> chemically treated genomic DNA (Homo sapiens)

<400> 41

ttagttttaa attttgtatt tgtatttttt agataggaag tttttttttt ttgattaggg 60
attgagggag aagagattta gaatgtattt ttttgtttta ggtttttggg gggggatatg 120
gttttagata gttattttga aattgggttt aggtttttat agtaagttga gtgttttttt 180
atatttttaa ttttggtttt tttattaaag tttaaaagat ttttttgttt tttaggtttt 240
ttttgtttgg ttgtttttat tattttttaa ttgtggaaat attagttatt atatgtttgt 300
tggagaataa gtgtgagtgt tgtggtggat agttgttgtt aaaggtgttt agatggatgg 360
tatttttaaa gtagggattt tagtattttt tttatatatt attatttaag aatggtagag 420
gaaagagtag gagtggggtt atagatagga gagaaaggag gttgaggtta taaaattggt 480
atttttttaa ttattaattt tttttttttat tattttgtta ttatttgtta tggtaaagtt 540
ttttttttgt gttttttttt taataaagaa atttttagtt agtttttatt tatttttttt 600
gatttgtagg aagttaaatt gttagtagat tgagtttttt aagggatatt tttgggatat 660
gttggaattt tgatttgttt aagatgatga tggttattag tataaattag aaataatgtt 720
atttttttagg gtgttatttg taagtgtaat aatatggatt tatttgtaaa atttagttaa 780
attgtgtttg gataagtgat tagatttaaa tgataaattt tttttattat ttagtttttt 840
ttaagttttg tggggggtagt atagaagatt gttttagggt gaaattaagg tttagtttat 900
tatttgtgtt agtttgaggg attttggaaa tgttgatttt gagaaaaaga taaaataaaa 960
taaaataaaa taaaatttaa ttataaaata tattttattg tttataaagt aaatagaaat 1020

```
ataaagttgt ttttaaaatt attttttag taaataagta tgtgatattt tgagtaggag 1080
taggagggag agaaagattg ttatttaaaa aaatatgaaa gtattaagta ataatttaaa 1140
atatggttgt ttagaaaaat atttatattt attgtagttg tttaattggt tttgttaaag 1200
ttggtaagta tttaaattgt gttataattt tatttaaatt ttgttttgtt ttagtaggtt 1260
gaagagtttg aatagattaa ttattttata atgatgatga atgagaaatt aatttagata 1320
taagtaagat aatttaggtt tttatttgtt taaatagttt tttaatatta tttgttattg 1380
tgggttatag attgaattaa ttgtttaatt ttgtgaaagt tatattttg tgtttttatt 1440
gagattattt atagtgtagt gagtgttgtt gaaaggtata tgttgttaat agggataatt 1500
atttaaaggg aagtgtttgt aaaatggaaa ataaatgttg gggttggtaa taaatgggat 1560
taaaagtagt tattttaatt tggtttttg agggaaagga gtgggtgtgg ttttgtagga 1620
ttaggggtgt ttttgatttt tttaagattt aggtgaggtt gagggttttt ggtgtgttag 1680
tttgtgttgt ggttgtggtt gtggtggtga tttgggttgg gttttgtttt tgttggtttt 1740
aggtttttgt tttttttgtg taggtagtgg gtgtgtgtgg tttgggttgg gtaagttgag 1800
gaatagtgag tttttggatg ttgattgtag gatgttttag tttgtgtttg ggtttttgtt 1860
tttttttgta tggggtttgt atttttgggt ttgggtttga tttatagggt gttgaggttt 1920
ttgtagttga agttggaagg ttttgttgtg agtgtggtat aggttgttgg tagtttttgg 1980
attttggagg tttggttttt tttttaagtt gatggtgggg aaagaatttt gtttttatag 2040
tttttttgat ttttgttgtt tgttatttgg ggatgggaag tgtgtttggg ttgttttatg 2100
tttttttggg ttggagtttt ttttatggtt ggttttttttg ggggtttttg ggtttgtgag 2160
tagtgtttat tttttttaga gaagaatttt tttttttttt tattgaagtg ttttttttttg 2220
tattttgaaa taattttttt tgggtgaggt tagttttttt ttgttgtttt ttttttgtag 2280
gtgtttggga gttttgtgag gattttgtgt agttgagttt aggtgatagg tgtttttttta 2340
ggtgtttatt tgtttttttt taatttgttt agggaaagat tagaatgatg tgtgtgggga 2400
ggtggttttg tttttttga aattggtgtt tttgggtaaa attgtgtgtt gtgtttatgt 2460
gggttttgtt agtgtgtgtg ttttagaaga gtgggttttt taggtggtat tttttggaga 2520
agtaagtgag gtgaatttag aatagagaag tgggaatgat tgtttgatgt gttgagttgg 2580
aaaaagagag gagagggaga ggtttttagt gagtgtggag atagaggagt ttatgtggta 2640
tagtatgagt tttattttg tttgtgtaga ggtgtgtgtg tgtgtgtgtg tgtgtgtgtg 2700
tgtgtgtgtg tgtgtgtgtt tttttttgta tttttgtgtt tgaaataaaa gtttaattaa 2760
tggattttg gtgttttgag gatttatttt tgggagtgat tttgatgtat tgttgtttaa 2820
ttagtgtttt taattggtgt ttttggagat aggtaggttt ggagttggga tagagttttt 2880
ttttgttttt tttgtatatt ggaaaggtaa aatttataat atattgtggt tatttttttgg 2940
gagagtgaaa gagagtggga ggtagggaag aaagggagag atgtagttgt ttgtagagga 3000
tggagttttt agttttgggg gttgtgggga agggtttaga ttattgggtt tgatttagtt 3060
ttttgtgtaa gagggtttgg gttggttgag ttgttgagga ttatgaattt gtttgtagaa 3120
aggtttaggg agtttttatt gtttgtataa atttatttta tgtggtttat tattttgtta 3180
ataatggttg tttgttaata aggatgtgga ggttaaataa tttgggtgga agattaaata 3240
tgttttatta aggtgtttgg aagttgtgtg gtgtagagat ttgtgttgga gttgtttggt 3300
ttattggagt ttggagggag atgggagtat agtttgtaag gagttttggg gtagaagagg 3360
aggtaaggtt tagttttttt ttttagggtg ttttgttatg ttaggatagg tttaattttt 3420
ttatatgtta aatagaaaat ttatatagaa gttagtttta atgaggtgtg gaggttaaat 3480
ttttggagta gttggttgga gggtagatat ggagtatttg gggtttgaat taaaggtttt 3540
tttttgagga ggggtggtta tttttttgttt tttagttttt atatgttatg tatttggtaa 3600
tttgtgttta tatgggatta gtattggatt agaagataga agatgtggtt tttagtttgg 3660
gttttgtttt taaattattg tagaattttt attatattat ttttttagtt ttatttttttt 3720
attgtgtaag agaaaggaga tggggtttga tgtagatgat gtatagtatt ttttttttttg 3780
tttagttttt tattagagtt agggaattga atattttata agatgttttg ttttttttaa 3840
attttgtagg atgtagttaa aaagaatttt atttagtaaa tatttgaggg tttattttgt 3900
attagttatg gtgtagtatg tattttttaa ttgagagtag aatattagtt tatatttttt 3960
tgttattttt aaatataatg ttttataata aaagatgaaa gagaaagaat tttaaagaga 4020
atagaatagg atgggtatgt atgtataggg tagagaaatt atggaggtaa aggtaaaggt 4080
attgataaaa gttgtgaaga atttgttgaa ttggtaagga agttagtgga taaatattgt 4140
agaagtagtt ttttaataga gatattatgt aggagagtag agagaaggtt ttttttttgaa 4200
ttttggtgat ttggtagaga ataaaagggt aagggttgta gaatttgatt aggaggttta 4260
agttgtatgt atttaatatt gttaattttt ggtttatata tttttttagt agttggaatt 4320
tttttttttt tttttttttt tttttttttt tttgagatgg ggttttgttt tgttgtttag 4380
gttggagtgt agtggtttta ttaaggttta ttgtagtttt aaatttttag gtttaagtga 4440
tttttttatt ttagtttttt gagtagtaag ggttataggt atatgttatt atgtttggat 4500
aattttttttt tttggttgag gggtatagat ggggttttgt tgtgttattt aggttggttt 4560
taagttttta ggtttaatga atttttttat tttggtgttt taaagtgttg agattatagg 4620
aatgagttat tgtgtttggt ttaggaagtt tattttttag ttaattttga aatattattt 4680
tttgaattta ttaattagga aatttggttg ttaggttggg attttttaag tttttgggtg 4740
atgggtattt agttttaagg aaggtaatga tgtttaaatt attaaattat gttttagttt 4800
```

```
ttgtgagtta ggaatttagt gagtttagtt gtgtgttttt ggttttaggt tattttattg    4860
gggttgtggt tttatttaaa gatttagttg ggtgaggata tgttttgggt ttatttatgt    4920
ggttgtttgg taggatttag ttttttgttg gtgggttatt tggttgaagg ttttagtttt    4980
tttttggtgg ttggttagag gtttttttgt tttttattat atgagttttt ttatagggta    5040
gtttataaag tggtagttgt tttttttttag attgagtaag taagagtgag agtggagggg    5100
ggagaaagta tttaagatag aagttatatt tttttggtta tttaattttg gaagtgattt    5160
tttattatta tgttatatta tatttgttag aagtgagtta ttagatttag tttatattaa    5220
aggaaagggg attatagaag gtattaaatt taggaggtag ggattattgg ggattgtttt    5280
agaggttgtt gtttagattt tttttatata atagttttga ggtttatatg gt            5332
```

<210> 42
<211> 5332
<212> DNA
<213> Artificial Sequence

<220>
<223> chemically treated genomic DNA (Homo sapiens)

<400> 42

attatataag ttttagagtt gttatataaa aggggtttgg gtagtagttt ttaagatagt 60
ttttagtgat ttttgttttt tggatttgat gttttttgta attttttttt ttttagtgtg 120
ggttggattt agtgatttat ttttaataaa tataatatgg tatagtgatg gaaagttatt 180
tttaagatta gatgattaaa agagtgtagt ttttgttttg ggtgtttttt ttttttttta 240
tttttatttt tgtttattta gtttgaggaa agatagttgt tattttataa gttattttat 300
agagaggttt atatggtaag gaatagagaa atttttggtt aattattaag aaggaattga 360
ggtttttagt taaatagttt attagtaagg aattgaattt tgttaaataa ttatatgagt 420
gagtttggaa tatattttta tttagttgag tttttagata agattataat tttagtggaa 480
tagtttggag ttagggatat atagttaagt ttattagatt tttgatttat agaaattgag 540
atatggttta gtgatttaag tattattgtt ttttttgagg ttaaatgttt attatttaga 600
agtttgaaga gttttagttt agtagttgag ttttttgatt ggtggattta gaaagtaatg 660
ttttaaaatt agttaaagag taaatttttt aggttgggta tagtggttta tttttgtaat 720
tttaatattt tgggatatta gggtgggagg gtttattgag tttagaagtt tgagattagt 780
ttgggtaata tagtaagatt ttatttgtat tttttaatta aaaaaaaaaa ttatttaggt 840
atggtggtat atgtttgtgg tttttgttat ttgggagatt gagatgagag gattatttga 900
gtttgggagt ttaaggttgt agtgagtttt gatggggtta ttgtatttta gtttgagtaa 960
tagagtaaga ttttatttta aaaagaaaaa gagagagaga gagagagaga gaaattttaa 1020
ttattgaaag aatatataaa ttaaaaattg gtaatattag atgtatataa tttgggtttt 1080
ttagttaaat tttgtaattt ttattttttt gttttttgtt aaattattag gatttaggag 1140
gggatttttt ttttattttt ttatatagta tttttgttaa ggagttattt ttgtagtatt 1200
tatttattga tttttttgtt agtttaataa gttttttata gttttttgtta atgtttttat 1260
ttttattttt atagtttttt tattttgtat atatatattt attttatttt attttttttg 1320
aagttttttt ttttttattt tttgttataa aatattatat ttgagaataa taggggaata 1380
tgaattgata ttttgttttt agttaaaaaa tatatattgt attatgattg gtgtagagta 1440
ggtttttaga tatttgttaa ataaaatttt ttttgattgt attttgtaag gtttagggga 1500
gatagagtat tttataaggt gtttagtttt ttaattttgg tgggaagttg gataaaagaa 1560
gaaatgttgt atattattta tattaagttt tatttttttt tttttatata gtagaaaaat 1620
gaagttggga gaataatgta ataaaaattt tatagtgatt taggggtaga atttagatta 1680
gaaattatat tttttgtttt ttaatttaat gttaatttta tgtaggtata aattgttaga 1740
tgtatgatat gtaaggattg ggaggtaggg gatggttatt tttttttaaa aggggttttt 1800
tggtttagat tttagatgtt ttatgtttgt tttttagtta attattttga gggtttgatt 1860
tttatatttt attggggttg atttttatgt ggattttttg tttgatatgt ggaagagtta 1920
gatttgtttt ggtatagtag gatattttga agagggagat tagattttgt tttttttttt 1980
attttggggt tttttgtaga ttgtgttttt attttttttt aggttttagt gagttaggtg 2040
gttttagtgt aggtttttgt attgtgtggt ttttaagtgt tttgatgaga tgtgtttaat 2100
tttttatttg gattatttaa tttttgtatt tttgttaata aatagttatt attaatgaga 2160
tggtagatta tatggggtgg gtttgtatag ataatgagag ttttttaggt ttttttgtga 2220
gtaaatttat aatttttagt gatttagttg gtttgaattt ttttatatag aaaattgagt 2280
tgaatttggt ggtttgggtt tttttttatg atttttgggg ttggaggttt tgttttttgt 2340
aaatagttgt attttttttt tttttttttg ttttttattt ttttttgttt ttttaagggg 2400
tggttatagt gtgttataaa ttttattttt ttagtgtgta ggagaggtga gaggaggttt 2460
tgttttagtt ttgggtttgt ttgtttttga ggatattaat taaaggtatt aattggatag 2520
taatatatta aagttgtttt tgggagtgag tttttagagt attaggaatt tgttagttgg 2580

gtttttgttt taaatataga aatatagagg aaaatatata tatatatata tatatatata 2640
tatatatata tatatatata tttttatgtg ggtaagagta gggtttgtgt tgtgttgtgt 2700
gggttttttt gtttttgtgt ttgttgggag tttttttttt tttttttttt ttttggtttg 2760
gtatattaaa tgattgtttt tgtttttttg ttttgagttt atttatttta tttttttaaa 2820
gagtgttgtt tagaaggttt atttttttgg gatatgtata ttaatagagt ttgtgtagat 2880
gtggtatgtg gttttattta agagtgttag ttttgggaga aataaaatta tttttttgtg 2940
tatattgttt tggttttttt ttaaataggt tgagggaggg taagtagata tttggggagg 3000
tatttgttgt ttggatttaa ttgtatgggg tttttatgag gtttttggat gtttgtggga 3060
ggagggtgat agaggggaat tggttttatt taggaggggt tattttagga tatagaaagg 3120
gatattttga tgggggaagg aaaggatttt tttttgaggg aagtagatgt tgtttatagg 3180
tttaagggtt tttagaggag ttagttatgg aaagggtttt ggtttagagg gatgtgaagt 3240
gatttgggtg tgtttttttgt ttttaaatgg taagtggtgg gggttgggga agttgtggaa 3300
atgaggtttt ttttttgtta ttggtttaga aggaaggtta agtttttaga gtttagaagt 3360
tattagtaat ttgtgttgtg tttgtaatga ggtttttttga ttttagttat aaaggtttta 3420
gtgttttgtg ggttagattt aggtttgggg gtatgagttt tgtatgggga gggatggaga 3480
tttgggtata aattgggatg ttttgtagtt agtgtttggg ggtttgttgt tttttggttt 3540
gtttagtttg ggttatatgt gtttgttgtt tgtgtgaggg aggtgggaat ttgaagttgg 3600
tgagagtgag gtttggtttg agttgttatt gtagttatag ttatggtgta ggttggtgtg 3660
ttggggattt ttggttttat ttgggttttg gggaggttag aagtgttttt aattttatag 3720
ggttatgttt gttttttttt tttggggagt tagattggaa tggttgtttt tgattttatt 3780
tgttattaat tttggtattt gtttttttatt ttataaatgt tttttttttaa gtaattgttt 3840
ttgttaatag tgtatatttt ttagtagtgt ttattgtgtt ataaataatt ttgatgaaga 3900
tgtaaggata tgatttttat aagattggat aattgattta atttgtgatt tgtgatggtg 3960
aataatattg gaaggttatt taaatagatg aaggtttaaa ttgttttgtt tgtatttgaa 4020
ttaatttttt atttattatt attatgaagt gattggttta tttaagtttt ttagtttgtt 4080
ggaatggaat gggatttaaa tgagattgta atataattta aatgtttgtt gattttaatg 4140
aggttaattg agtagttgta ataaatataa atattttttt aaatagttgt gttttaaatt 4200
attatttaat gtttttatgt tttttaagt ggtagttttt tttttttttt tgtttttgtt 4260
tagaatatta tatatttatt tgttgaaaag gtgattttgg aaataatttt gtgtttttgt 4320
ttgttttgta gatagtagga tatgttttgt gattgggttt tgttttgttt tgttttgttt 4380
tgtttttttt ttagagttaa tatttttagg gtttttttaag ttggtataaa tggtgagttg 4440
agttttagtt ttattttaaa gtagtttttt gtgttgtttt tgtagagttt gggaggagtt 4500
gagtgatgga agaaatttgt tgtttggatt tagttattta tttagatgta gtttagttgg 4560
gttttgtaaa tgaatttgtg ttattgtatt tataaatgat attttgaaga gtggtattgt 4620
ttttgatttg tattgatgat tgttattatt ttgaataaat tagaatttta atatatttta 4680
aaggtatttt ttagaaagtt taatttgttg gtagtttggt tttttgtaaa ttaaaaggag 4740
taggtggagg ttgattgagg attttttat tagggaaggg gtatagaaag agaattttgt 4800
tataataaat ggtggtagga tggtggagga ggggggttgat aattagagaa atgttaattt 4860
tgtggtttta attttttttt tttttattt gtaattttat ttttattttt ttttttatta 4920
tttttgggtg atgatgtatg gagaggatgt taagattttt attttgagaa tgttatttgt 4980
ttggatattt ttggtggtaa ttgtttattg tgatatttat gtttattttt tggtggatat 5040
atggtggttg gtgtttttgt agttggggaa taatggaagt aattaagtgg gagaagtttg 5100
aggaataaaa gaatttttta aattttagtg aaagggttgg ggttggggat gtggggggat 5160
atttgattta ttgtgaggat ttgagtttaa ttttgggtg attgtttaag gttatgtttt 5220
ttttgaaagg tttagggtaa ggagatgtat tttgaatttt ttttttttta atttttggtt 5280
gagggagagg gattttttgt ttggagagtg tgggtgtagg gtttgaaatt ag 5332

<210> 43
<211> 4215
<212> DNA
<213> Artificial Sequence

<220>
<223> chemically treated genomic DNA (Homo sapiens)

<400> 43

```
ggtgtttgat taagtgtagt ttgtagtttg ggtattgtta attgatttta atttttttta      60
tggtgagagg atggattttt tgttattttt ttgtttaatt tggtatttat ttatttattt     120
atttattatg tttgttgggt gtatttaagt ttaattttgg ggtgtatgtg ttagtgtaga     180
tattgtattt tttttttttt tttggttaat tttaggtaga attttaaaat aattgttttt     240
tttttatga tttagatgtt gtggtttgtg gataggaggt ttaagaaata gtatattttg     300
agtggtaggt agtgaggtgg aaatggttgt tggttttagt ggtggtttta ttggaagttg     360
```

```
agtttaggag tggttaagtg gttgttgggg aaagtattgg ggttttttag ggtttttttt    420
gagtttttat tttgtatttt tgagggtgtg aaaattatgg gagttgtttt attttgtgtg    480
tttagttttg ttttagttta gatattgttt tttgttagtt ttttttgtgg tgtttaggga    540
ggatgtgttt tgtttttttt taatttggtt aatgggtgtt tgggtagtgt gtggtttgtt    600
tttgtttttg ttagggaaat ttggaggagg agaaaagttt gtatagaggg tggaaaggtg    660
agagtggagt tttaagtttg gtagtttgag aggaagatga atagttttag gttagagttt    720
ttggtagagt ggattttgag ttgtttttag gtagttagga gtggttttag tggtagttgt    780
aaattttagt agttgtttgt gttgtttgtg gttggggtgg agggtagtta gagttgggga    840
ttaaggtttt gtgttatttg tgtgtatagt tttatatttg aatgttgttt ttttgtagat    900
gagattggtg ggtattgtaa agttgggatt tgtttttgaa ggaaaaaaaa tagtgagtaa    960
gaaatttagt attatttttt attgatttat tttgttgtat tttttgtttt ttaagttttt   1020
gaaagttggt aattttgatt ttggtgttta aaaattgata gttattgaga ttggttttga   1080
gaagttgaag atttggtagt ttttagattg agtaggataa ggtgaaagta ggttggaggt   1140
gggtttagga tatttgaggg ttgattttgg gggtttgtga ggttgttatt gttgttgttg   1200
ttataggtga gatggtgttg ggttgatgtt ggggttaatg ggtagagaat gtagggatgt   1260
ggtttttgtt gaagagagtt aagaagggaa gagtgtgttt tttaaattgt ttttgtaatt   1320
tgtttttagt gagtatttta ttgatttaga atttattgag aatagtatta gtgagttatt   1380
ttttttttga gatgggtttt atttattttg gtaatggagt gagttggatt gtggggagga   1440
agaggaatgg gaaaattagt ttataaatat taatgttagt aagagtgtgt tgttggtagg   1500
atgtattgtg agtttggaga ttttggtggt tgtagttggt aagtggttat aatttagaaa   1560
gtaggattga gttgtttttt ttgttttatt agtgtattgt tttttgggtg tgggtttaat   1620
attttataag tggtaatttt tgtttatggt agttttgttt ttttttttatt atttttagat   1680
ttagttttgt attttaaggt tgtgtattgt tagttattat tatgtttatt tttggggtta   1740
atttgtttgt tttttttgagt tttgattggt tgaatagttt agtgattatt ttggtggtga   1800
tgtttatttt tggggtggtg ggtaatttgg tggttattgt ggtgttgtgt aagttgtgta   1860
aggagtagaa ggagatgatt ttttatatgt tggtatgtgg gttggttgtt attgatttgt   1920
tgggtatttt gttggtgagt ttggtgatta ttgttatgta tatgaagggt taatggtttg   1980
ggggttagtt gttgtgtgag tatagtattt ttattttgtt ttttttttagt ttgtttggtt   2040
ttagtattat ttgtgttatg agtgttgagt gttatttggt tattaattat gtttattttt   2100
atagttatta tgtggataag tgattggtgg gtttttatgtt ttttgtagtt tatgtgttta   2160
atgtgttttt ttgtgtgttg tttaatatgg gttttggtag tttgtggttg tagtatttag   2220
atatttggtg ttttattgat tggattatta atgtgatggt gtatgttgtt tatttttata   2280
tgtatgtggg ttttagtttt tttttttattt ttgttattgt tttttgtaat gtgtttgtgt   2340
gtggtgtgtt gttttgtatg tattgttagt ttatgtgttg tattttgttg ggtattgagt   2400
agtattatgt ggttgtggtt gttttggttg tttttttgggg ttattttgtt gtttttttag   2460
ttttgttgtg ttttagtgat ttttggtgtt gttggagttt ttgttgtatt gtgggtgttg   2520
agatttagat ggttattttta tttattgtta ttttttttggt ggtgtttatt tgttttattt   2580
tgtttgtggt gagtgattgg ggttggggtt ttatttggtt tttttttttgt atttattttt   2640
tgtgtttatt ttttgttttt tgtttttttt ttgagttttt ggtagtgaat gtgttgtttt   2700
taggttgggg ttgggatttt tatattgttt tttagagtag gtttaatttt ttttgaagtt   2760
ttaattttaa tgagatttag taggtgtttt gtttttatat tttttagttt atgtttttgg   2820
aagtttgggt tttttttttt attgagatag tttttatttt ttgttgtttg atattggttg   2880
agtttttttaa gaaaattttt tgtttttttt gttagatgtg gaggggagtt tgttgtagtg   2940
tgatttagtt tattttttttg tattgtgaat tgtgaattgt aaaatttgaa atgttaggga   3000
tggtgatttg tttgttgtaa attattttta aggaaagtgg ggtttttggt gttttgatgt   3060
atgtaaggtt ttttgaattt tttttttttgt ggattgtgta gaattttaaa gttagaagag   3120
atattaagag gtgttttagt ttaatttttt tattttattg agggagtgaa tgatatgttt   3180
aaaataattt agttgaattt atatagttaa gttaggtttt ttgtttttta ttatggtgtg   3240
ttttgggagg gtagtttaaa gtatataaat tttaatttat atatagggta gggtttagat   3300
ttagtttttt ttattttgtt tagattagga aagtaaagtt ttttagggtg attaattatg   3360
gataggttga tatatatagg gtatgggatt tgaatttttg ataattgtgt gaggtatagt   3420
attattttag agaaattgag gtttagagat ggttgttttt gttttttttaa gttaaagttt   3480
tatagtttgt aatggatttt aggttgtttg aggttagatt tagtttatgt gttattatat   3540
tatgtttttt ttgtagaata tttttttattt tggtgtttgt aaagtgtggg ttggagtttt   3600
ggtatatttt attttttagtt tatttttttaa gaattgtttt tggattaatt tttaaaatat   3660
gataaataga aaggtttaaa ttttatttag tttttttattt tgtttgtggt taaaatggaa   3720
tgtattagaa gtagatgggt agaataatgt aatattttat ttagagtttt ttttgaaagt   3780
aattagttat gagttttttt atttttttaaa atattgtatt tttggaaatt ttaaattatt   3840
aatttaaaaa gagataattg tttttttttta tgtttgttgg gaataagaga atataaaatg   3900
taatgggtta ttattttttt tttttttttta ttgatattgt ttagggattt ttggtgtaaa   3960
aatatatttt tgataaaagt gattgtaaat ggaaggaatt tagaggggggt ttagagttag   4020
aatatagtag gtttttatta atttgtataa atagaagtaa atataatagg ttataagagt   4080
ttttaagtta ttttaggatt taatataatt tatagagtta agttatattt gagttatata   4140
```

aatatatttt attgtttgta atggtaattt agaataaagt gagattttgg aagaaataat 4200
attgtttttg atggg 4215

<210> 44
<211> 4215
<212> DNA
<213> Artificial Sequence

<220>
<223> chemically treated genomic DNA (Homo sapiens)

<400> 44

tttattagga gtagtgttgt tttttttaaa attttatttt attttaggtt gttattatag 60
atagtaaaat atatttatgt ggtttaagtg tgatttaatt ttgtaaatta tattgggttt 120
tggaatggtt taaaggtttt tatggtttat tatatttatt tttgtttata taaattggta 180
agaatttatt gtattttggt tttgagtttt ttttaagttt tttttattta tagttatttt 240
tattaaagat atatttttat attaaagatt tttggatagt attaataagg aaaaggaagg 300
aataatagtt tattatattt tatatttttt tgtttttaat aagtataaga aaaagtagtt 360
attttttttt gagttggtag tttagaattt ttaggaatgt agtgttttgg aagatggaag 420
aatttataat tagttgtttt taagagaaat tttagataaa gtattgtatt attttgttta 480
tttgttttg gtgtatttta ttttagttat aggtaaagta gaaaattagg tagggtttga 540
gttttttttgt ttattatatt ttaaaagtta atttaaaggt aatttttaaa agatgaatta 600
aaaatgaagt gtattaaaat tttaatttat gttttataaa tgttaaaatg agaaatgttt 660
tatgagggag atgtgatgta ataatatatg agttgggttt ggttttagat ggtttaaggt 720
ttattataag ttgtagagtt ttggtttgaa gaaataaaga tagttatttt taaattttgg 780
tttttttaaa atggtattgt attttgtata gttgttgaga atttaaattt tatattttat 840
gtatattagt ttgtttataa ttagttattt tgagaaattt tgtttttttg gtttagatag 900
aatgaaagaa attgagtttg gattttgttt tgtatatgaa ttagaatttg tgtgttttaa 960
gttgtttttt tagggtatat tatggtaaag agtagggagt ttagtttgat tgtgtaggtt 1020
tggttggatt attttaagtg tattatttat tttttagtg aagtgaggga gttagattaa 1080
aatattttt gatattttt ttagttttaa aattttatgt aatttatggg ggaagaaatt 1140
taagaaattt tgtatatatt aaaatattaa aaattttatt tttttaaaa atggtttata 1200
atgaatagat tattattttt gatattttag gttttgtagt ttatagttta tagtatggag 1260
gaatgggtta agttatatta tagtaggttt ttttttatgt ttaatagagg gggtgggggg 1320
tttttttgga agatttggtt agtgttaggt agtaaggggt aagggttgtt ttggtggaga 1380
aagaaattta ggtttttggg aatataaatt gggggatgta ggggtaaagt atttgttaaa 1440
ttttgttagg gttgggattt taaagagggt tgggtttgtt ttgagaaata gtgtgggaat 1500
tttagttttg atttaaaggt gatatgttta ttgttaagga tttagaggga aagtagggag 1560
tggggaatgg atgtgggagg tggatgtgag aaaaaggttg agtagggttt tagttttggt 1620
tatttattat gagtgggatg gagtagatga gtattattag ggaggtggta atgagtaaga 1680
tgattatttg gattttggtg tttgtgatgt ggtggaagtt ttggtggtgt tgaaagttgt 1740
tgaggtgtgg taaggttggg gaggtagtgg ggtggttttg ggaggtaatt gaggtggttg 1800
tggttgtgtg gtgttgtttg gtgtttagtg aggtgtggtg tatgaattgg tggtgtatgt 1860
ggagtagtgt gttgtatata agtatgttgt agaggatggt ggtgagaatg aggaaggagt 1920
tgaagtttgt gtatatgtag gagtaggtgg tgtgtgttgt tatgttggtg gtttagttga 1980
tgaagtatta ggtgtttggg tattgtagtt gtgagttatt gagatttatg ttgggtagtg 2040
tgtaaaagag tatgttggat gtatagattg taaagagtgt gaggtttgtt aattgtttgt 2100
ttatgtagtg gttgtagaaa taggtatggt tgatggttag gtagtgtttg atatttatgg 2160
tgtagatgat gttgaggttg gataggttga agaagagtag aatgaaggtg ttgtatttgt 2220
atagtggttg gtttttgggt tattggtttt ttatgtatgt ggtgatggtt attgggttta 2280
ttaataaagt gtttaatagg ttggtgatag ttagtttata tattagtgtg tagaaggttg 2340
tttttttttg tttttgtgt gatttgtata gtattatgat ggttattagg ttgtttatta 2400
ttttgaagat gaatattatt gttgggatgg ttattgggtt gtttagttgg ttggggttta 2460
aggaggtgga tgaattgatt ttgggagtgg atatgatagt ggttggtggt gtgtagtttt 2520
ggagtgtaag gttgggtttg gggatggtag aagagagata aagttgttgt gagtggaaat 2580
tattatttgt aaggtgttag atttgtgttt gagaaatgat atattgataa gataagggga 2640
gtaatttgat tttattttt ggattgtagt tatttattaa ttgtggttat taaaattttt 2700
aggtttgtga tatgttttgt taatagtata tttttgttga tattaatatt tataaattga 2760
tttttttatt tttttttttt tttataattt aatttatttt attgttaaga tgaataagat 2820
ttattttaag ggaaaagtag tttgttagtg ttatttttga tagattttga attaataaaa 2880
tgtttattga aaataagtta taaaagtaat ttggagagtg tgtttttttt tttttggttt 2940
tttttggtga gggttgtatt tttgtgtttt ttatttgttg attttaatgt tagtttaatg 3000

ttattttatt tgtagtggta gtagtggtgg tagttttatg agtttttagg gttagttttt 3060
agatgttttg gatttgtttt taatttgttt ttattttgtt ttgtttagtt tggaaattgt 3120
taaatttttg gtttttttaaa gttggtttta gtggttgttg atttttggat attgaggtta 3180
gagttgttag tttttaaaaa tttgggaaat aagaggtgta gtgggatggg ttagtgaaga 3240
atggtgttgg attttttatt tgttattttt ttttttttaa agatgagttt tagttttgta 3300
gtgtttgttg gttttgtttg tgggaggata gtgtttaggt gtgaggttgt gtgtgtaggt 3360
ggtgtggagt tttggttttt agttttggtt gtttttttgtt ttagttatgg gtagtatggg 3420
tggttattgg agtttgtggt tgttgttgag gttgttttttg gttatttggg ggtggtttgg 3480
ggtttatttt gttagaggtt ttggtttggg gttgtttatt ttttttttgg gttgttgggt 3540
ttggagtttt gtttttgttt ttttattttt tgtataaatt tttttttttt tttaagtttt 3600
tttggtggag gtggaggtaa attgtgtgtt gtttgggtgt ttattggttg gattggaagg 3660
gggtggagtg tgtttttttt gggtgttgta gggaagattg gtggggggtg gtgtttgggt 3720
tggggtgggg ttgggtgtgt ggggtggggt ggtttttgtg gtttttatgt ttttggaggt 3780
gtggagtggg aatttggagg ggattttggg aagttttggt gtttttttttg gtgattgttt 3840
agttgttttt gaatttggtt tttagtgggg ttattattga aattggtgat tgtttttgtt 3900
ttgttgtttg ttgtttggag tgtattattt tttgaatttt ttgtttgtgg gttgtaatat 3960
ttagattgtg gaagagaggg tagttgtttt aggattttat ttagggttgg ttgagaaagg 4020
agaagaaata tggtgtttgt gttagtgtgt gtgttttggg gttagatttg ggtgtgttta 4080
gtggatgtgg taggtgggtg ggtgggtggg tattagattg ggtggggaaa taatgaagaa 4140
tttatttttt tattataaaa ggagttggag ttagttggta gtgtttagat tgtgggttgt 4200
gtttggttgg gtatt 4215

<210> 45
<211> 5473
<212> DNA
<213> Artificial Sequence

<220>
<223> chemically treated genomic DNA (Homo sapiens)

<400> 45

```
gtaatgttaa ttagttttat tttaaagaaa aattaaatat tagagtaaat aatgattttt   60
ttgtggagaa ggagttgtaa gggttgggga gaggtaatta gtgttataaa agttttatta  120
taaagaagtt tagaattttt ttgttttgtt gtgtagtaga gtgaaatata gtagtagaat  180
aattgtgtat tattattatg ataatttaat ttgttttttt agaaaaagtt attgtttgta  240
gttaggatag taggaaaata tttagaaaat aattaaaatg taaataaaag tatgaaataa  300
taataaagtt tatttttttaa gagaaaaaaa gaagagtatt tagtgaaagg agaatgaagt  360
atatataaag taagtaattt ttaggtattt tgtttttttg gatgttattt gtttaaattt  420
gataattttt ataggtagta tgatagattt tttaaaattt aaataaagtt tgtttttgta  480
ttttattttt aataataaag tatattgatt gatttataaa ttaaagtttt taaaatttta  540
ttaaagtaaa aatattgttt tagagttttt attggtatgt ttttgaaagt aagtgttata  600
gttaatgtaa atttttaaaa atttttttgt tatttagtat aatagagggt ggattttgtg  660
ttagggtggt atttttttgtg taatttatat ggtttgaggt tttttgtttt tttttttttt  720
agaagagttg gaattaagat attaataatt ataattgtaa taattattat tatgttttta  780
aggggttgtt gagtttttgg agattttagt gtatagagtg aagtaggaga ggtttaaaaa  840
ggaaggtttt gttgttgatg ttgtgatgat taaggtagtt tattattatt tttttttttt  900
aatgtgattg ttttgttttt tttttttttg attttgttat aagatgtggg gaagtttttt  960
aagaattgtg tatagttttgt tatagatggt ggtttttgtg tttagagatt gaaagagtat 1020
ttgttatagt gatattttgt aatagttgta gatgtgtggg tttaaaattg ttttatgtgt 1080
ttaaattaat aataattatt attttatttt attttattat ttgagtggaa atataatttg 1140
aaatatttaa atttgttttt tttaaagtta gaaggatatg tgtatgtgaa tgtatataat 1200
ttgttttttt aaatgttttt aaagtttatt tttgttatta tatttgtttt tataattaat 1260
aaagaaaatt tgttttagtt ttaattttat tgggtatggt atgtgttgga gggtaggttt 1320
tggaaatata aatattaata tatgtggttt gttttaaaat ttaatgtatt tagggttagt 1380
tttgttgttt tggttaaatg aatgaataaa gagtgattaa aatgtgttta tttttttgtt 1440
attttatata tagtttgaga ttgattgtta atagtagatt gtaaattttt gtatttaata 1500
gttattaatt tttttttatag tttgtatatt tataaattta attgggatat ttgtttgata 1560
aatttagatt ttgtagagta gatttttaag gaaatttaat tttagagaat aaaatatttt 1620
ttggaatttt taaggtattt agagataaaa tgttttttgt gggatttttt tttaagtttt 1680
ataaaagtag gtttattagg attaaattat attaaagtta gtgtgaggta aggggggtgt 1740
ggtttataga aattttggga gaaagtttag tatagttttt tttttttggg aatatatgtt 1800
aggatggaaa aaaaaaaaag gaggggagtg ttataggatt aagatttatt ttggaaaata 1860
```

```
agatttttag taaagatatt tttttttttt ttattttata ttagatatta gaagtattat   1920
tttttgagtg tgttttttaa gttttttttt aattttaggt tttttttgtt ttttgggttt   1980
ttgatttttt ggtagttggg tatttgtatt tgggtttttg ataggtttag ttgttggttt   2040
agtttttttt gtatgaaggt gttgggatag tgggttttgt taaaaagttt ttttagttta   2100
ttgagttgtt ttagggtgaa attggtttga ttttgttttt gtttgatttt ggtttggttt   2160
ttgtttttta ttttttttgt attttttttg tgatttttta gttttgggga tattggaggg   2220
ggtattttag tggggttata tgtgtattta tatgaatata tatatatatg tatatatata   2280
tggatgaaaa tagtatagta aattttgtta ttagatttgt tgtaaaaaag aatagagaat   2340
tttttttttt tgtggaattt tggtttggtt ttttaattta tttggaggaa gaatattttg   2400
gagaagtgta aaggttaagt ttgagtggtt gtttggggta aggtgggtag ttatttatta   2460
ttttttttat aaatgtattt tgatttattg ttttattatt tattttttag ttattggttt   2520
tttttagtaa atattaaatg gtttttattt atttttgtat ttatttgttt tgtagtattt   2580
atagaaatta aaggtgtagt gttaggtttt ttatagagta aagtttggat ataagggtgt   2640
ggtaaggtta tttattggtt ttttatattg gttattttta gaaggttggt ttgtttttag   2700
gttagttttt attattgttg gttttttgtt tattgtaaat ttgttggttt aatttaggaa   2760
taattgggtt gaaaggtatt agtgagagta atagattttg gtgttgtgtt gtatagggag   2820
ttgtatttgt agatgttttt tgttgttttt gggtttgggt taaattttgt ataaggtttt   2880
ttggatagtt aggtaatttt tgttttgttt gtttgattgg ggttgtatga gtataggtgt   2940
ttatgttatg ttggttgttt aaagggtttg ttgtttaagt tgggttagaa ggtaggaggt   3000
ggaaaattag tttttggtgg tgggtgaaag taattgtttt ttttgttttt tttttgtttt   3060
tttttgtgga aatgtagatt tgattttaaa tgtttaattt atagagatta ataggtttaa   3120
gtggaatatt tgtgattttt ggtttttatt ggttgtttaa agttttttta tgtaattagt   3180
agtttggatg tttaataaaa tatgaatttt tttgttttgg gtttgtatat atttgttgtt   3240
atttttttgt tttttttttt tttttaaaaa taggagtttt tttagtgtga gtatatatag   3300
ttattatata tatatttttt tggaattttt attttttgtg tttgaattag agattttggg   3360
ttttaagaag ttaaagttga gtttagatag ttgaggttgt ttgtagtttt ttttagtgta   3420
ggatttaggt tagtatgggt gttaaaaatt atttttggaa gagttggttt gtagagaaat   3480
taatatttgt tttgagtttg gaaattaata attttaggaa ttgtgtttgt tgggggttta   3540
gatttgaatt ttgttgttag gtgtgttggt gaatttgtag tttgggtttg tagttgtgga   3600
tttatggttg ggaggttgta ttgggtattt tgatggtaat tttattattt tataaattta   3660
ggaagatttg gtgttggttt ggaggaagaa gagttgttag attttggttt tgttgggtat   3720
tttagttatg gtgattttaa atattgttgt attttttagt ttttagagtt agttttgtgg   3780
atatttgttt tttttttagg aaggaatatt atagtaaata ttatgggtta atttaatagt   3840
aaagtatttt tattgatatt gtattgaaat ttaaatatga aaatatattt tttaaatttt   3900
gttaatgagg tgggaaggtt ttgtttatta tatgttattt atatagtgga tgttatttaa   3960
agttttgggg ttttgaggag atgtttaaat tttaagttga tttatagatt taataaaatt   4020
tttatttttg tttttaaatt tttaattatt gatttaaata ggaatagatt gtgttataaa   4080
taatttaatt gtttgtttaa atatgtgatt ttatgtatgt tatatatttt tttaattgga   4140
aaaatatggt agtatattag ttattgtagg tattaagtgt taaattaatt ttattgttga   4200
atttttttga attggtatag aatgttttgt tattgagata aagatgtttt ttaatattga   4260
gttagtatta aaggattttt gttttaagag aattgagtat tgggtgattg aaaatattaa   4320
aggaattgtg taaatataga tttttatttt ttttgttgtt tttttttttt gtttttaata   4380
tattaagaaa ttgaaatgtt ttgtttggga gaagtagtgt agtttggatt ttagtttttg   4440
ttggtttttt tttattttga aagagaatgg gaagttttgg tttttaagat ttaaagattt   4500
aggggttttg tggagtttga ggggttttgg tggttttaaa tatttaggtg attggagggt   4560
taagtttttt atttttgttt tttttttttt ttggtaaatt ttgtgttaga ggttagtttt   4620
agttttgttg tttggtttag tatttttttt tgtagtttgg ttttgtgaat ttttatgttt   4680
gtttgtgggt tatttgggtt gtgggtttat ttttttgttg gtgaaaaatg tattttggtg   4740
gaatgggaat atttgggaag ggtgtgtata aattttatat gttttttttt atgttatttt   4800
tataatatat taggatttta tttatgattg agtatattat tggattttta tttttttatt   4860
attattttag ttgataaagt tggggtttag ggtttttttt gtgttttttt tttttttttg   4920
ttgggttttg gagttttttt ttgtttgaga ggagtttgga aggtgggaag gggaattgtt   4980
gttgggggtt agttaggttg ttattttttg ttagttgtgg gttgtttggt tttttgtttt   5040
ttttggtggt gtttatgttt agtttttgga tgggagagtg ttttttttta gtttttttgt   5100
ttgttttttt tttttttata ttttttttgt tttttttgtt gttgtttttg ttttttttgt   5160
tgttgttttt gttggttgtt tggattgttg ggttgttgtg gttgttgtgg tttgttttgg   5220
tgtagttggt tggttttttg gttttgtgta gtggtttgtt ttttagtatt ttttggtatg   5280
tgattgtttt tttttttttt tttatttttt ggttaaaaga tttggagatg aatgttgtaa   5340
gtttttttat tgttgttgta tgttagtttg gtgtttaatt tttgttagta gagttttgtt   5400
tttttttttt tttttttttt tttattgttt tagttttttt aataataata tattaatggg   5460
ataggaggtg ggg                                                      5473
```

<210> 46
<211> 5473
<212> DNA
<213> Artificial Sequence

<220>
<223> chemically treated genomic DNA (Homo sapiens)

<400> 46

ttttattttt tgttttattg atgtgttatt attgggggg ttggagtagt aaaaaaagaa 60
gaaggaaaaa aagagtgggg ttttgttggt agaggttgag tgttgggttg atgtgtggtg 120
gtgatggaag aatttatggt gtttgttttt aagtttttg attagaaagt gaaggagaag 180
aaggaggtga ttatgtattg ggaggtgttg gagagtgggt tgttgtgtgg ggttaaggag 240
ttgattggtt gtattgaggt gggttgtgat gattgtagta gtttggtagt ttgggtggtt 300
ggtggaggtg gtggtggagg aggtggaggt ggtggtggag gaggtggagg aggtgtagga 360
ggaggaggag taggtggagg agttggagga gggtgttttt ttgtttggga gttggatatg 420
ggtgttgttg agagaagtag ggagttgggt agtttgtgat tgatggaggg taatgatttg 480
attgattttt ggtagtggtt tttttttttg tttttggat ttttttgggg tgggagagga 540
ttttgaggtt tagtggggag gggagaggga tatggagagg gttttggatt ttagttttgt 600
tagttagagt gatagtggaa gggtgggggt ttggtggtat gtttggttat gggtggggtt 660
ttagtgtgtt gtgggggtgg tgtgggggga agtgtgtggg gtttgtgtgt gttttttta 720
gatgttttta ttttattgaa atgtattttt tattagtggg aggatgggtt tgtagtttgg 780
atggtttgta ggtggatgtg gaagtttgtg gggttgggtt gtaggagggg gtgttgaatt 840
agatggtggg attaaagtta gtttttagtg tagagtttgt tgggagggag aaggggtaga 900
ggtggaaagt ttaatttttt ggttgtttag gtgtttgggg ttgttaagat tttttgaatt 960
ttgtagagtt tttgaatttt tggattttag gggttaaaat tttttatttt tttttaaggt 1020
agaaggaagt taatgaaagt tgaggtttag gttatgttgt tttttttggg tgaggtgttt 1080
tggtttttg gtgtgttggg ggtgggggg gggggtggtg ggggaggtgg ggatttatat 1140
ttatataatt tttttagtgt ttttaattat ttagtatttg gttttttttag agtaaaaatt 1200
ttttggtgtt agtttagtgt taggaaatgt ttttatttta atggtagagt gttttatatt 1260
aatttaaagg agtttaataa taaaattgat ttggtatttg gtgtttgtag taattggtgt 1320
attgttatat tttttaatt aagaaaatat gtggtatgtg tggaattata tatttaagta 1380
agtaattaag ttgtttatga tatggtttgt ttttgtttag attagtaatt ggaaatttgg 1440
ggataaaaat agagatttta ttaagtttat ggattaattt gaaatttgga tattttttg 1500
aagttttaaa attttgagtg gtatttattg tgtagatgat atgtaataga taaagttttt 1560
ttattttatt ggtagaattt gaagggtgtg tttttatatt taaattttaa tataatatta 1620
gtaaaaatat tttgttatta aattggttta taatgtttat tataatgttt ttttttaaag 1680
aaaggataaa tgtttataaa attaattttg agaattagaa aatatggtaa tatttaagat 1740
tattgtagtt agaatgtttg gtagggttga gatttagtga tttttttttt tttaaattag 1800
tattaggttt ttttaaattt gtaaagtggt ggagttattg ttaaggtgtt tagtgtagtt 1860
ttttggttat gagtttataa ttataggttt gaattatagg tttgttagtg tgtttgatgg 1920
tgaggtttag gtttgaattt ttggtgggtg tagtttttgg gattgttgat ttttaagttt 1980
agggtaagta ttaattttttt tgtagattgg ttttttttgga aatgattttt aatatttatg 2040
ttggtttgga ttttgtgttg ggaagaattg taaatagttt tgattattta aatttagttt 2100
tgatttttta aaatttggag tttttgattt gggtgtagag gataaaggtt ttaggggat 2160
atatatatgg tggttgtata tgtttatatt agaagagttt ttgtttttaa agggaggaga 2220
aggataggaa agtggtaatg gatgtgtgta aatttagagt agaaaaattt atattttatt 2280
aaatatttga gttgttggtt gtatgaaaag gttttgagta attaatagaa attgaggatt 2340
gtgaatattt tgtttgaatt tgttgatttt tgtgggttaa gtgtttaggg ttgagtttgt 2400
gtttttatga gggagggtga agagagaata gaaagagtgg ttgtttttgt ttgttattgg 2460
aggttggttt tttgtttttt gtttttttggt ttggtttggg tggtgagttt tttgggtagt 2520
taatatggtg tgggtgtttg tgtttgtgtg attttggttg ggtaggtggg atggagatta 2580
tttggttgtt taggggattt tatgtagggt ttggtttgag tttaggggta gtgaggggtg 2640
tttgtggatg tggtttttg tgtggtataa tattggggtt tgttgttttt gttgatattt 2700
tttggtttaa ttgtttttaa attagattag taagtttgtg gtaggtagag agttagtggt 2760
ggtgagagtt ggtttgagaa tgagttagtt ttttgggaat ggttggtgtg agaggttagt 2820
gggtggtttt gttgtgtttt tatgtttaga ttttgttttg tgagaggttt ggtgttgtgt 2880
ttttgatttt tgtgagtgtt gtggagtaag tgagtgtgaa agtgagtgga aattatttgg 2940
tgtttgttgg ggaaggttgg tgattggggg gtgaatagtg aggtggtgag ttgaggtatg 3000
tttgtgaagg aggtggtgga tgattgttta ttttgtttta ggtggttgtt tagatttgat 3060
ttttgtgttt ttttgggata tttttttttt gggtggttg agaagttgga ttaggatttt 3120
atgaggggga aggatttttt attttttttt gtgataaatt tggtaatagg atttgttgtg 3180
ttgtttttgt ttgtgtgtgt gtgtgtgtgt gtgtgtgttt gtgtggatgt atgtgtggtt 3240

ttgttggggt gttttttta gtgtttttgg agttgaaaga ttgtaaagag gatgtgaaag 3300
ggatggagga tgaaggttag attaaaatta agtagaggtg aagttggatt aattttattt 3360
tggaataatt taatgagttg gagaggtttt ttgatgagat ttattatttt gatgttttta 3420
tgtgagagga attgagttag tgattgggtt tgttggaggt ttgagtgtag gtatttagtt 3480
gttaggaggt tgagggttta gggagtagag agagtttggg gttggaaaga ggtttggggg 3540
atatatttgg gaggtggtgt ttttagtgtt tggtgtaaga tgagaaggaa agagatattt 3600
ttattagagg ttttattttt taaagtagat tttagtttta tagtattttt tttttttttt 3660
tttttttat tttaatatat gtttttagaa gaagaaagtt gtgttgggtt ttttttaga 3720
gtttttataa attatatttt ttttgttttg tattggtttt aatgtggttt aattttaatg 3780
ggtttatttt tataaggttt aaaaaaaaat tttatagaaa atgttttatt tttggatgtt 3840
ttaaaagttt taaagagtat tttgtttttt ggagttggat tttttgaaa gtttgttttg 3900
tagggtttag gtttgttaag tggatatttt aattaggttt atagatgtgt agattataaa 3960
gaaaattagt aattgttaag tgtagaaatt tatagtttgt tgttagtggt tgattttaaa 4020
ttgtgtgtga aatagtggag aaataaatgt attttggtta tttttgttt atttatttaa 4080
ttaaggtaat gaaattgatt ttaaatatat tagattttaa aataaattat atatattagt 4140
atttatattt ttaaagtttg tttttagta tgtgttatat ttaatgaagt taggattgaa 4200
atagattttt tttattggtt gtggaggtaa atgtaatagt aaaaataggt tttggggta 4260
tttaaaggag taaattgtgt atatttatat atatatattt ttttagtttt aggggaata 4320
aatttaaatg ttttagattg tatttttgtt tagataataa aatagaataa aatgatagtt 4380
attattggtt tgaatatatg gagtagtttt aggtttgtat atttgtagtt gttataaagt 4440
attgttgtaa tagatatttt tttggtttt gggtatagag attattattt gtagtgaatt 4500
atgtataatt tttaaggggt tttttatat tttgtaatag gattaggaaa aaaaaaaata 4560
gagtagttat gttgaagagg aaaagtaata ataggttgtt ttggttatta tagtattagt 4620
agtagagttt tttttttaa gtttttttg ttttgttttg tgtattgaga tttttagagg 4680
tttagtaatt ttttaaaaat atgatgataa ttattatagt tatgattatt agtattttaa 4740
ttttaatttt tttaggggaa aaaaaggtaa gaagttttag gttatgtgaa ttatataaag 4800
gatgttattt tggtataaaa tttattttt gttgtattaa atagtagaga aatttttgaa 4860
gatttatatt gattatgata tttgttttta aaaatatatt aatgaaagtt ttgaagtagt 4920
atttttattt taatggggtt ttaaaaattt taatttgtga gttaattaat atgttttatt 4980
attgaaagtg gaatataaaa gtaagtttta tttgaatttt aaaaaatttg ttatattgtt 5040
tgtaaaagtt gttaagttta agtaggtaat atttaggaga ataaagtatt taagagttat 5100
ttgttttata tatattttat ttttttttg ttgagtgttt tttttttttt tttttaagaa 5160
gtgaatttta ttgttgtttt atatttttat ttatatttta gttatttttt agatgttttt 5220
ttattatttt aattgtaaat agtggttttt tttaaagaag taaattagat tattatgata 5280
atggtgtata attattttgt tattgtgttt tattttgttg tatagtagaa tagagaaatt 5340
ttaaattttt ttgtagtgag gtttttatga tattaattat ttttttttaa tttttataat 5400
ttttttttg taaaggagtt attatttgtt ttggtgtttg gttttttttt aagatgaagt 5460
taattaatat tat 5473

<210> 47
<211> 5328
<212> DNA
<213> Artificial Sequence

<220>
<223> chemically treated genomic DNA (Homo sapiens)

<400> 47

ttgataagag aaaaggtgtt gagggtaggt tgtgttgttt tatttatagt ttattgtttg 60
ttgtagagtt tttgttagga ttggaaagtt aatttagtgt tgattaaaag tattatagag 120
ttaaaggatt ggtaatgatt gttaatttta ttttagtttt atgtattttt aattgttgta 180
attaaagata tgttatttaa atataggtga agggtttttt tttttaagt tgggttgggg 240
gggggtgggt gggtggatta taattttatt tagattttt attttatttg ggtgttatgt 300
agttttaaat tagattttat tattattatt atttagagtt gtttttgtt atttatattt 360
ggttttgtag aattggaagt gtgaaatgtg ttatttatat aaaagttttt gtttaaagaa 420
agggtgggat gggggagtgt agaggagtaa ttatttaggg gtgatttaaa tatttaggtt 480
tttttttttt ttttttaaa ttaatagaaa ggtttttgat atagttaaaa gtatttgaaa 540
ttgggggtaa ggttaatagg ttaaagggat tagtgttagt tttttaagta tttttaaaaa 600
gattatgtga atagatggaa aaatatattt ttaattaaat aatttttttt atattatagt 660
tgtagtgttg gtttagaaat tgtgtaattt tttatttgat aggagttttt ttatgagaat 720
attaaaggat aaaaagtttt gtatttttgt agttggttaa aattgataat tatttttta 780
tgtgtgtgtg tgtatatata tatatatata tatatatata tatatattaa tatatatata 840

tatatatata tatatatata tatatatata tatttttaaa aaagaattta gtttttaatt 900
tttttttatt tatgaagatt aaagttttgt atttgatttt gtgagtggtt tttgtttttt 960
ggggttggaa tttgatttag atgtgaagtg gtattgagtt gttgtgtagg tttgtgttta 1020
gtgtttttttg ttggatatag taggagttgt gtagtgttag ttttagaaat tgttggaatt 1080
ttgttttggg gttagagaaa atttttttat tgttttttaa aaaagaaatg ggatgtgaaa 1140
ataaattttt atatttttga aaaataatat aatattaatt taataaaaaa ttaaagaggt 1200
gatattataa ttttaattta ataaaaaata aagattgttt tttttatatt ttagttattt 1260
tttatttggt ttggttttgt aatttttttg aaaggttttg tgatggtaaa ataggtaaag 1320
aaaagtgatg aaaaataaag tatttttttg tagttgtgga gaattaagta aagtatattt 1380
tttttgtttt gtttatattt taaaaaatta aatggaagat gttgttgttt gttgttttga 1440
tgttgttagt agtgttttga atgtttttag aggttttgta agtattgtaa ttgagttttt 1500
gagtaaatga aataatttgt ttttatgtgt ttatttttag agtataatta aaagattagt 1560
tttttttttg agtgtttttt atttttttaga ttaatttttgg ttaaagtttt ttttttttag 1620
atattggtta agaaatatat attggttaag aaataggtgt ttggtatttt ttttttttta 1680
ttttagttgt aggattttgg ggaaggtgtg ttaatggtag ggaaggtttt ttttgaaatt 1740
tagtattttg ttgattagga atttaaatta ttttagggaa gggggagtat ttttatattt 1800
ttaaaaaatt atttgtttat ataataaaga gttttgtttt ttttattatt attatttttt 1860
tttttttttt ttggttagta agtgagggta aaagtagttt ttgaattatt ttagaaagag 1920
aaattgtaaa aatggtatat tttttgaatt taaaataata gggaaatgtg ggtttgttta 1980
attgtttaaa attaaataga aataattatt gtgttaaatg aataagatgt tttaagattt 2040
atatttttag ggttatggtt attgatttta attttatttt taagaagttt agagattgtg 2100
tttttaaagt ttgtttattg atggaaattg gtttaatttt ttataaaaga atggtttttt 2160
aaaatattga atattatggt tatgtaaggt ttatgatatt tttatgttta tttttttat 2220
tttttaaaat tagtgaattt gagataatat tttaagggga attgttattt agtgtgatga 2280
gagatgattt ttttatttgg ttttagaatt agattttggt ttaggttttt tagtaagtag 2340
gtttagatgt aatggaagga gagaagaatt aagtttttttg ataaaatttt tattttgaat 2400
tttatggaaa agataggtaa gtgggatttt agttatagta tttttatttt ttgttttatt 2460
aaaaggtttg tttttattat agaaataata tagttggtgt tagttttgtg aatttgtgtt 2520
aattgataag gtaagaaatg gtgtgttgat agttgaggtt tgaaggttgt ttagtggttt 2580
ttgtagtttg tggggtaggg aggttaaagt tagtaaaatg ttggaggtga tgttagaaag 2640
agagtatatg tttgttattt ggaagtgatg ttgaatgtgt aatagtagag ttttggagat 2700
ttttattttg ttttgtattt agggatttta gtttgtgggg tagtaggttg ttgggtgaag 2760
gtaggaggga gaagttgtat ttggttgagg gtgggtgggt gtgttgggag tgagtgttta 2820
atttttagg gtttatggag aaagtataat ttttagttga ggaaagagag ttaaataagt 2880
gggtggttta ggtttgggaa tgttatttag tttttttgat gtagtgttat ttttgtggtt 2940
ttaggggtt ttagtatttg tttgaatttt atttttaag gttgttagta ggaaggtagt 3000
tttttttattt ttgagggttg aggaggggt agtgagtgat ttgatttttt agtagtaggg 3060
aaaattgtag tggttggtaa tgggttgtgt ttttttgtgg tggtaaagtg ttttttaggg 3120
gtggggtggg tatttttggt tttttgtttt agtgtttagg tttgagtttg tgtgggttgg 3180
ttgggtgttt tgttttgtaa gtaattaatt ttgttggatt ttgaggatta tttaggtgga 3240
tgggtttatt tagtttaggt ttattgagat tttgttaatt ttttagattt tttttttttt 3300
tatttttttat atttttttatg atgaatttag gaaagaattt gaattttgaa aaatagtata 3360
tgtggtttga agtttgatag ttatttgggt gtgagggata tgattttatt aataaagatt 3420
aagatttaag gttgagtttt tatttattaa atttgttttt ttattttgtt tttaaatttt 3480
ggagattttt tagttttgtt gttttaattt tgatatagat aaaggtgtta gggagaaagt 3540
ttaggtgggt ttgaagtagt tgaggtatag attgatattt attttttttat tatttagatg 3600
ggtagataag tattgttaga ttgggggttt taatttaggg tttagtggtt atgtgggttg 3660
ttgtggggtg ttgaaattat aagtataatt tgatattatt gagtttatgg tgtttgttaa 3720
taatttgtga attaggtgta gagattgtta ttgtagatta taatagtttt tgaaggattt 3780
attatagttt attgtgttaa tgatttttaa attaaggagg ggaggtgggg tgagatgaaa 3840
aaggagtggg gagagttagt aaaaagggaa gaagggtggg agggaagata aaagttagag 3900
ttttagtttt gaaggagttt ttaaagatta ttaggttagt gatttgagaa ttttattggg 3960
atgggtttgg aaatttaatt ttatttgagt taggattgga agaggggtgt tgggggtaag 4020
tagaagtggg tttttgggaa ggttgatttt ttaggtattt tttttgaggt tggaatagtt 4080
tttgggagag ggttttagat gttaatggga ggttttgagg atgttagttt gtttaggatg 4140
ggtgtatatt ttgatttaga gtattttttta gggtaagttg tagttgtaga tttttgaaaa 4200
aagtggtaat agttttgttt agggatgatg gtgattgtag gatgattttt tttttttaat 4260
ggtttgtttt aattttttttt tatgttatag gtgaaagttt tttttttttt tttttgatag 4320
tatttttgtt tgttttttat atgttatgta gatttaattt tttttttaa tttgtttttt 4380
tgggtgttaa atatattagt tttttgagat tttattattt ttgtggtttg tattgagttt 4440
aattgtaatt ttggaaaggg aatttgtgga taatatagta gtagtatgtt ttataagatt 4500
ttagaagaaa gaagattaaa ttttatttat gtggtgtggt aggggttagg ttaagggaaa 4560
gaggtttttt ttgtttggta aattgttttt gtagtgaaag ggtagtttag tttttattgg 4620

```
agtgttattt aatattggtt agagaaaggt taaggaaagt gtaggtttgt ttttttttata    4680
aaagttgtgg ttataattaa atgaagtaat tatagttgga gaatttaaaa taattgtttt    4740
ttttttaaaa tgagggtaat aattttagtt attggaaaat tgggatttta aggaaaattt    4800
tttttttttgg tataattaat tgtataagat agtgtggtta attagagata tatgtattta    4860
gatttaagta atgatttata ggtgtaaaag gtttagtggt tttttgggtt atttggagtt    4920
tttattgatt attttgtttt ttaaaaaatt ttttttttaat tttaaaaata aaagttatag    4980
atttaatgta attgaaaatt attgatggta gtttttttagg tttttagttt atgtttttaa    5040
agggaattta tatttaaaat aattaaatta tttaatggtt atattatatt ttttttttgga    5100
taatagttgt agtgtaggga tagaatttta atttatatta aaattgtttg ataatttaga    5160
aatggaaatg aaattgtgaa ttaatttttt tagaaaatat ttgagttaga atataagaag    5220
gatgtttata tataatattg taagtaatat gatgattatt atatgtagtt tgaaagattt    5280
atataaatga ttatatatta tttttgttgt tataatttaa agtaaggg                  5328
```

<210> 48
<211> 5328
<212> DNA
<213> Artificial Sequence

<220>
<223> chemically treated genomic DNA (Homo sapiens)

<400> 48

```
tttttatttt aaattatagt aatagaagta gtatataatt atttgtgtag gtttttttaag     60
ttgtatataa taattattat gttatttata gtattatata tgaatatttt ttttatattt    120
tagtttaaat atttttgga gaaattaatt tataatttta tttttatttt tgaattatta    180
agtaatttta atgtaagtta agattttatt tttgtattat agttgttgtt taggagaaaa    240
tgtagtgtga ttattaaatg atttaattat tttaaatata aattttttttt aaaaatataa    300
attgaaaatt tgaagagttg ttattagtgg tttttagttg tattaaattt atgattttta    360
tttttaaaat tagaagaaaa tttttaaaa ggtaaaatga ttagtaggaa ttttaggtag    420
tttaaaaagt tattggattt tttatatttg taaattattg tttgagttta aatatatatg    480
tttttgattg attatattat tttatgtaat taattatatt aaaggaaaag gtttttttta    540
gaattttaat tttttagtgg ttagggttat tatttttatt ttagagaaaa aatagttgtt    600
ttaaattttt taattgtgat tattttgttt agttgtgatt ataattttttg tgggaaaaat    660
aaatttatat ttttttaat ttttttttag ttagtattaa atgatatttt agtgaaggtt    720
gggttatttt tttattgtaa ggataattta ttaagtaggg aaaatttttt tttttttagtt    780
tggtttttgt tatattgtat aaatggaatt tagttttttt tttttttggaa ttttatggaa    840
tatattgtta ttgtgttatt tataagtttt ttttttagaa ttatagttgg atttaatata    900
aattataaaa gtgataaaat tttagagggt tgatgtgttt ggtatttaaa aaaataagtt    960
ggggaaagaa attagattta tgtaatatgt gagggataga taggaatatt attagaggaa   1020
aaggaagaaa gtttttattt gtggtatgaa aagaagttaa agtaaattat tggaaaaaaa   1080
aagttatttt ataattatta ttatttttaa atgaaattat tattattttt tttaaagatt   1140
tgtagttgtg gtttgttttg gaaagtgttt taaattaaag tgtatattta ttttgggtgg   1200
gttggtattt ttaaggtttt ttattgatat ttagaatttt tttttaaaag ttattttggt   1260
tttaaaagaa atgtttgaga agttggtttt tttaggggtt tatttttgtt tgttttttagt   1320
gtttttttttt tagttttggt ttagatggag ttgggttttt aaatttattt taatggaatt   1380
tttagattgt tggtttgata atttttaaag gtttttttgg agttggggtt ttgattttta   1440
tttttttttt tatttttttt ttttttttgt tggttttttt tatttttttt ttgttttgtt   1500
ttattttttt tttttaattt gaagattatt gatatgatag gttgtaatga gtttttttaga   1560
aattgttatg atttatagtg ataattttttg tatttaattt atagattatt gataagtatt   1620
gtgggtttaa tggtgttaag ttgtatttgt ggttttaata ttttgtagta atttatgtag   1680
ttgttgggtt ttggattagg atttttagtt tggtagtgtt tatttgtttg tttgagtgat   1740
ggagagatga gtattagttt atattttaat tgttttaagt ttgtttgggt tttttttttg   1800
gtgtttttgt ttgtgttagg gttggagtaa tgaaattgaa agattttttag agtttgaaaa   1860
tagagtgaaa gagtaaattt aataaatgag agtttagttt tgaattttag tttttattaa   1920
tagagttatg tttttttatat ttaaataatt attaaatttt aaattatatg tattgttttt   1980
tagagtttgg gtttttttttt gggtttatta tgaaaggtgt gagggatgaa ggggaaaagg   2040
gtttgaggag ttggtagggt tttagtgggt ttaagttaag tgggtttatt tgtttggata   2100
gtttttagaa tttagtaaga ttggttattt ataaagtagg gtgtttagtt aatttgtgtg   2160
ggtttaggtt tggatgttgg gataggagat taaaaatgtt tgttttgttt ttaaaggtg    2220
ttttgttgtt gtggagaaat gtggtttgtt gttagttgtt gtggtttttt ttgttattaa   2280
ggaattaagt tatttgttgt tttttttttta gtttttaaag atggaggggt tgtttttttg   2340
ttggtggttt tggggagtgg gatttgggta ggtgttgggg tttttttggga ttatagaaat   2400
```

aatattgtgt tgggagagtt gggtggtatt tttaggtttg ggttatttat ttgtttagtt 2460
tttttttttt aattaggggt tatgtttttt ttatgggttt tagaaagttg ggtatttgtt 2520
tttggtatgt ttatttgttt ttagttaggt gtggtttttt tttttgtttt ttatttaata 2580
atttgttatt ttataggttg ggattttggg gtgtggaatg gggtggaaat ttttaggatt 2640
ttgttattgt atgtttgata ttatttttga atagtaggta tatatttttt ttttggtgtt 2700
gtttttaata ttttgttggt tttggttttt ttattttatg ggttgtggga attattgagt 2760
agtttttgga ttttggttgt tagtgtattg tttttttattt tattggttgg tgtgggttta 2820
taaaattagt attgattata ttatttttgt aatggggata agtttttttgg tagagtaaaa 2880
ggtgggggtg ttgtggttgg aattttgttt gtttgttttt tttatagaat ttgggtaga 2940
gattttatta aaaggtttga tttttttttt tttttattgt atttgaattt atttattaaa 3000
aaatttaagt tgagatttaa ttttggaatt agatggagaa gttatttttt attgtattaa 3060
atagtaattt tttttgaaat gttattttga atttgttaat tttgggaggt gggaagaata 3120
aatataaagg tattataaat tttgtatagt tatagtattt aatattttaa aaagttattt 3180
ttttatgaaa aattaagttg atttttgtta ataagtaaat ttttaaaaatg tgatttttaa 3240
attttttaag ggtaaagtta aaattaataa ttataatttt gaagatgtaa attttaaaat 3300
attttgttta tttagtatag taattatttt tatttgattt taaataatta agtaaattta 3360
tatttttta ttgttttaaa tttagaggat gtgttatttt tatagttttt ttttttgaaa 3420
taatttaaag gttattttttg tttttatttg ttgattaaaa agaagaagaa gaaataataa 3480
taataaaaaa ggtaaagttt tttattatgt agataggtag ttttttggaa gtgtaaaaat 3540
attttttttt ttttaggata atttggattt ttggttaatg aaatgttgaa ttttaaagaa 3600
aattttttttt attattggta tatttttttt gaaattttat ggttaagatg aaagaaaaaa 3660
gatattaggt atttgttttt tagttagtat gtgtttttta gttagtattt gggaaggaaa 3720
aattttgatt aggattggtt taggggatgg gaaatattta aagaagagat tggtttttta 3780
attatatttt ggaaataaat atataaaagt aagttatttt gtttatttga aggtttagtt 3840
atgatatttg taggattttt gagggtattt aagatgttgt tgataatgtt aaaatagtaa 3900
gtagtagtat tttttgttta atttttttgaa atgtggataa aatggaggaa gtatattttg 3960
tttgattttt tatggttata gaaaaatgtt ttgtttttttg ttattttttt ttatttgttt 4020
tattattgta gaatttttta aaagagttat aaaattaaat tgggtggggg atggttggga 4080
tgtgaggagg atagttttta tttttttattg aattaaaatt gtagtattgt ttttttagtt 4140
ttttattgag ttaatattat attatttttt aaggatataa aaatttgttt ttgtattttg 4200
tttttttttt ggagggtaat aaaagagttt tttttaattt tagagtagga ttttagtgat 4260
ttttgggatt ggtgttgtgt agtttttatt gtgtttaata ggggggtgttg ggtatgaatt 4320
tgtgtagtgg tttagtgttg ttttatattt gagttgggtt ttagttttgg aaggtgagag 4380
ttgtttgtgg ggttgagtat agagttttag tttttgtgga taaaaagaga ttaaaggtta 4440
aatttttttt tggggatatg tgtgtgtgtg tgtgtgtgtg tgtgtgtgtg tgtgtgtgtt 4500
ggtgtgtgtg tgtgtatatg tgtgtatgtg tgtgtgtgtg tgtgtgtatg gggggggtggt 4560
tattaatttt aattaattat gaaaatgtag agttttttat tttttgatgt ttttataaag 4620
aaattttttat tggatgagaa attatatggt ttttgagtta atattgtagt tatagtgtgg 4680
gggaaattat ttaattaaaa atatattttt ttatttgttt atatgatttt tttagaagta 4740
tttaaaaagt tgatattgat ttttttggtt tgttaatttt gtttttagtt ttaaatattt 4800
ttaattatat tgaaggtttt tttattaatt tgaaggagaa aaaaaggaga tttaaatatt 4860
taaattattt ttaaatagtt atttttttgt attttttttgt tttatttttt ttttaaatag 4920
gggtttttat gtgagtgata tattttatat ttttgatttt gtaaagttag gtataaatga 4980
taaggagtgg ttttaaataa taataataat aaaatttaat ttgggattat ataatattta 5040
agtagagtag aagatttaaa taaaattgta atttatttat ttattttttt ttaatttaat 5100
ttaaaaaaaa aaaatttttt atttgtattt aaatagtgtg tttttaatta tagtagttgg 5160
ggatgtatga ggttgaaatg aaattagtag ttattattag ttttttaatt ttgtggtgtt 5220
tttgattagt attaaattag tttttttagtt ttaataaaga ttttatagta agtagtgggt 5280
tgtaaataag atagtgtgat ttgtttttaa tatttttttt tttgttaa 5328

<210> 49
<211> 4324
<212> DNA
<213> Artificial Sequence

<220>
<223> chemically treated genomic DNA (Homo sapiens)

<400> 49

```
gtttttttagt attattattt taaaggtatt tttatttttt ttattattag ggaaggagtg       60
tgtagttttg atttagtttt ttagtggagt aaagggaatt tagaggaagg gggatttttg      120
tagaaattgt tagttatata agtaagtttt ttttggtttt ttgttttatt aaaagtgaaa      180
```

aattgtttat aggagtgttt atgtttttg tttttttatg tattattaga gttaagaggg 240
gattttgtta gtaaggtatt ttttgttttg ttttgtagat tatagttttt gatttttatt 300
ttattgtttg tgtaatgtgt agaattggag tttaaagtag tgggtttagg attattttt 360
aggtttggtg ggtttataga tttttttttt ggttgggaag aggggttttt gtgaaagtag 420
agtttgataa gttttagtat tgttatggtt aagattatta atattattat tgttgtgttt 480
ataaatatga agattatggt agaggaggag ttgaaagttt gaggagtggt agaggaagtt 540
gtagaattaa atttggaaat tatgtttttt gatggggtga tagtggtttt tgatttggtt 600
tgaagggata tttgaagggt agatattgtg ttttgtgatt tttattgagg aattttagga 660
atagatgtta ttgaattgtt ttgtttaggg ataagtggtg ttttttttag tttttgttg 720
attttgattg gttatgtttt ttgtggtatg gggttggttg tagtggttgg tgggtgtttg 780
gtgggtattt tggtttttt aagggttggt tgtttttttt tattggttat ataagagtgg 840
ttgttttttt ttagtttgaa gtttgtagta tatttgtagg taaagttttt taagttgttt 900
aggtagttag ggagttttgt gtatttgtta gtatggaggt atttttggg gtagggatat 960
aatatattgt ttgagagttt gttttagtga gtgttgattt tgtttgtgat gtaagtaatt 1020
gagattggga gttgttttg gtagagtgta tttattttgg ttttaggtgg attgaagttt 1080
agagtggtgt tgtgtagttg gaagggtgtg tgatagttta agttagaggt ggttttgggg 1140
tgtggtgtag gatataagat tttaaattgg tatttgtata ggtagttgtt ggtgtgtagg 1200
tggtattgta ttttttttta gtttgtgggt ttgattttat tggtggtttg gagtattgtg 1260
tatttttgtg tggtgtagga gtgttggggt ttttttattt attgtagtgt gttgtttttg 1320
agattgttgg ggttggagga tagttaggag aaattttgta aaggtttgtt ttttagggtg 1380
tagtgggaat gtttgtgttt tagtgtgatt tagaatagta ggtttttgga gttttttttg 1440
ggttttgggt ttgtttgtag gagtgtgagt atagtgtgta gtttggtgtt tgtatgtatg 1500
gtgttgagtg ttttattttg taggatgtag gttttttttgg ttgtttgttg ttttatggta 1560
gtgtggtgta ggttgtagta ggtttttgag gttgagtagt tagtatggtt ggtagtgggg 1620
tgtttgttgt tgtttggttt gggttagagt gtttgttaga ggaggtatag ggtgaatgtt 1680
ggttttattt tttgaggttt tgtatgtaga gttgttttta atttggattt gttttgtttg 1740
aggatgtaga gttgtgtttg gagtgtggtt attgtttttt atagttggtt tttttaattt 1800
ggtttttttt tttttttttt ttgtttttgt ttttgatgga ttttttaatt gttaagaatt 1860
tttttgtggg tttagtggtt aatagttttt tttaggttgt gataggaaat gtgtgtggag 1920
tgttgttagg tttggtgtag ttttgagtta ttttggggtt aggaatatag ttggtgttgg 1980
gtatagggga tgtagttgat tagaaggtta aatattttgt ttagagtaag aggagaaatt 2040
tgttattggg atgaagtgtg tggttttttt ttgtttaggg agttttatta ggattgtaga 2100
atttataata tgtaagtgaa tataggtagg aaggatttga gaaaggaatt ggtttatgat 2160
tataagatta tgtttgttta ttgtgttttt gtttggagtt gtggagttag tgttagaata 2220
atttgtagat ggttttagtg tttggggggt ttgtgtttgt gggggaaaaa tatgttgtga 2280
aataatttag taattgtttt tgttattaga ggtagtatta tttaattgta tattttttta 2340
tgggttttttg ttttttttatt atgttaagag atttttaggag ttttaattat taaagaattt 2400
ggagttgttt gggtatttta aatgggaaat tttgtttttt ttattagtat ttgttttaga 2460
aatttatttt ttatttttat gtgttttttt ttgagttaga ttatattgat ttattataga 2520
aaggaaatgt atttttagga ttaaatgaaa aataatgatt atagatttta atagttaggt 2580
tgttagtttt ttttgttatt gtattggttt aaaaattttt taagtttata ggttatatat 2640
aaatgagttt gtggttttat aaatatttga gaataattgt tttatgtgtg attttagttt 2700
tgaattattt tagtattatt tttttttatg attgagtggt agtatttata atagtaagtt 2760
gtaaatattt tttttaagga aaattagagt tttttaagaa attaaaatgt ttattttatt 2820
ataatgaata ttgtatattg ttaaaaaata ttttgtaatt tgaaagatgg gtgagttaat 2880
tttttttagaa ttttagtggg tgttttgttt taatataaat attttaattt agtgggaagt 2940
ggtaaggaat taaagatgaa taaagaagta aaggttaaaa attgtattgt tatgaaagga 3000
tttttttgtta gtaatttaga taatttagta ttagtttaga aaatgtatat tttattttgg 3060
tttataatgg ttaaatatgt tgttattaga aatatataga gagttttagt taagtttttt 3120
tagagatgtt tgatgttttt aaatattttt aattagaatg aaagttgtta ttttatggat 3180
aatgtatata agtttatttt gttttattgt atttaggttg atgaggggtg ggagggaagg 3240
ttttaggatt atatttgtat gtttttattt tatttttatt ataattttta tttttttttt 3300
tttttggaaa ttttatgtat tttaataaat aatagtaagt ttatgagtta ttattatatt 3360
tattttgttt ttgttgagta tggagttttg tattaggatt tttttttttgt tttattttttt 3420
aatttaaaga atatttttta gttttttttt tagatgtttg agaattattt taaatgtggt 3480
tttgtgttta aattaggtaa tttagtgttt tataagaaag aaatatgaaa atttagaaat 3540
taaattgaat ggtttttaaa aaatatttag ttatttgggt tagtttttta tagattattg 3600
taattttatg attgtgtttg taaatgtgtt tatgtagtgt aatattttta ataaaatttt 3660
ttttaaattt aagtagttaa tggaaatttt ttagatagtt ttaaaaattg tttgggatga 3720
gatgaggtgt ttatagtaag aaagaatgaa tagttggaaa gtttaagtaa atatatgatt 3780
ttattttttg atattttgtt taggggagtg ggattatagt agagaatata ttggttataa 3840
tgattatatg agagtattga ttatgattat gattatatta tggaatgata taagagttag 3900
aaaaaattag tttaaagtag gatgtaaata gttttgtaat tagtttgaaa tagatagttt 3960

```
atttgattaa attagaaatg gaggaaagtt tttatttttt aataatattt attaagataa    4020
taatatagaa ttattttatt tttattggat tttaaatgtt taaaaatttt ataatattaa    4080
atgtgaattt attggttttt ttatatgtta tagattagag taattatttt ggaaaaattt    4140
gatatttttt tttattaaaa ttgaatattt gtaaatttta gtatttttat gataaggtat    4200
aaaattaaga gaaatttttg tatatgtata atagaagata tgtaaaaaat gtttatagtg    4260
atattattta tattaataaa atgttgaatt aattaaaata tttaaaaata gtataaaaat    4320
aaat                                                                4324
```

<210> 50
<211> 4324
<212> DNA
<213> Artificial Sequence

<220>
<223> chemically treated genomic DNA (Homo sapiens)

<400> 50

gtttattttt atattgtttt tggatatttt gattgattta atgttttgtt aatatgaata 60
gtgttattat aaatattttt tatatatttt ttgttgtata tgtgtaaaaa tttttttttag 120
ttttatattt tattatggaa gtgttagagt ttgtaaatat ttaattttag tgaaaaaaag 180
tgttaaattt ttttaaagtg gttgttttaa tttgtaatat gtaaaagagt taatagattt 240
atatttggta ttataagatt tttaggtatt tggaatttag tggggtaaa atggttttat 300
attattattt taataaatat tattaaaaaa tagaaatttt tttttatttt taatttggtt 360
agataaattg tttattttaa attaattata aaattattta tgttttattt tgagttaatt 420
ttttttaatt tttgtgttat tttatgatat ggttatggtt atggttagta tttttatata 480
gttgttatgg ttaatgtgtt ttttgttata attttatttt tttagataga atgttaggga 540
gtgagattat gtatttattt aagtttttta gttatttatt tttttttatt gtagatattt 600
tattttattt taaatgattt ttaaagttat ttaagaaatt tttattgatt atttaagttt 660
gaaaaagatt ttattaaaaa tattgtatta tatggatata tttataaata tagttataag 720
attgtaatgg tttatagaga gttggtttaa gtgattaagt atttttttaaa aattatttaa 780
tttgattttt ggatttttat gtttttttttt tataagatat taagttattt aatttaaatg 840
taaagttatg tttaaggtga tttttagata tttgaaggaa ggattaagga atgtttttta 900
aattgggaaa tagggtagga gagaaatttt agtgtaaagt tttatattta gtaaaagtaa 960
ggtaagtatg atgatgattt ataggtttat tgttgtttgt taagatatat ggaattttta 1020
ggaggaaaga agaatgagag ttatggtaaa aataagatgg aagtatatag atgtgatttt 1080
gaaatttttt tttttatttt ttattaattt gaatgtagta aaataaagtg agtttatatg 1140
tattatttat ggaatggtaa tttttatttt gattaaagat atttaaaagt attagatatt 1200
tttaagaggg tttagttgaa attttttatg tatttttgat ggtaatgtat ttggttatta 1260
tgaattaaga tagaatgtat gttttttaaa ttgatattga attgtttaag ttgttaataa 1320
gaaatttttt tataatagta tagtttttaa tttttatttt tttatttatt tttaattttt 1380
tgttattttt tattgagttg aaatgtttat attaaaataa aatatttatt ggaattttaa 1440
agaaattggt ttatttattt ttttaaattat aagatatttt ttgatagtat ataatattta 1500
ttataatgga ataaatattt taatttttta aggaatttta attttttttta aaggagatgt 1560
ttataattta ttattataaa tgttgttatt taattatagg agaaaatgat attaaaatag 1620
tttaaggtta aaattatata tagggtagtt gtttttgaat atttataagg ttatgaattt 1680
atttatgtat aatttataaa tttgaagaat ttttgaatta atatagtggt aaggaaagtt 1740
aataatttaa ttgttaagat ttatggttat tattttttat ttagttttag aaatatattt 1800
ttttttttgta atgaattaat atggtttaat ttaaagagaa atatgtggaa gtgaaaaata 1860
aatttttaaa gtaagtgttg atggaagaag tagaattttt tatttaaaat atttaaataa 1920
ttttaaattt tttagtagtt aggatttttg aaattttttg atataataga gaagtaaaga 1980
tttatagaga gatatgtggt taaatagtgt tgtttttggt ggtaaaagta gttgttaagt 2040
tgttttataa tatattttttt ttttataggt ataggttttt taaatattgg agttgtttat 2100
gaattatttt gatgttaatt ttatagtttt ggatagaaat ataatagatg ggtgtggttt 2160
tataattata agttagtttt tttttttaagt ttttttttgtt tatgtttgtt tatgtgttat 2220
gggttttgtg gttttgatga aattttttga atggagaaaa attatatatt ttattttagt 2280
ggtgaatttt tttttttgtt ttaagtaggg tgtttgattt tttagttgat tgtgtttttt 2340
gtatttggtg ttagttgtgt ttttgatttt agaataattt agggttgtat tgggtttggt 2400
agtgttttgt atatattttt tgttgtggtt taagggaaat tgttggttgt tgggtttgtg 2460
gggggatttt tggtagttgg ggggtttgtt gggagtgagg gtggaggga agggaggggg 2520
aattgggttg gggaagttag ttgtagaggg tggtgattgt gttttagata tagttttgtg 2580
tttttgagtg ggatagattt aagttgggag tagttttgtg tgtggggttt tagagaatga 2640
ggttggtgtt tgttttgtgt ttttttttggt aggtgttttg gtttgggttg ggtggtggtg 2700

```
aatattttat tgttgattgt gttggttgtt tggttttggg ggtttgttat agtttgtatt 2760
atgttattat gaagtggtag gtggttgagg aggtttgtat tttgtgaggt ggggtgttta 2820
gtattgtgtg tgtgggtgtt gagttgtgtg ttgtgtttgt gtttttgtgg gtaggtttag 2880
ggtttggagg gggttttaaa gatttgttgt tttgggttgt attggagtgt aggtgttttt 2940
attgtatttt ggagaatgag tttttgtggg gttttttttg gttgtttttt gattttggtg 3000
gttttgaaag tgatatgttg tagtgggtgg aggagtttta atgtttttgt attgtgtgga 3060
gatgtgtggt attttaggtt attggtgggg ttgagtttgt aggttggaag gagatgtgat 3120
gttatttgtg tgttaatggt tatttgtgta agtattagtt tgaggttttg tgttttgtgt 3180
tgtgttttgg ggttgttttt aatttgagtt attgtgtgtt tttttagttg tatagtgttg 3240
ttttggattt tagtttattt gggattgagg tgagtgtgtt ttgttgggga tagtttttga 3300
ttttagttat ttgtattgtg gatgaaattg gtgtttgttg ggataaattt ttgggtgatg 3360
tgttgtgttt ttgttttggg aggtattttt gtgttggtaa atgtgtagag ttttttaatt 3420
gtttagatga tttgggaggt tttgtttgtg aatgtgttat gggttttgag ttggggaagg 3480
atggttgttt ttgtgtgatt agtggggaag gatagttgat ttttgggggg attggggtgt 3540
ttattaggtg tttgttggtt attgtaatta gttttgtgtt gtagagaata tggttaatta 3600
gggttgatga gaagttggga gagatattat ttgtttttga ataagataat ttagtaatat 3660
ttatttttga gatttttttga tggggattat agagtatgat gtttattttt taaatgtttt 3720
tttaagttga gttaaaggtt attattattt tattagggag tgtgattttt aagtttaatt 3780
ttatgatttt ttttgttatt ttttaggttt ttgatttttt ttttgttgtg gtttttatat 3840
ttgtgagtat agtagtagta gtgttggtga ttttgattat gatagtattg gggtttgtta 3900
agttttgttt ttatgaaagt tttttttttt agttaaggaa ggagtttatg ggtttgttgg 3960
gtttggagag tgattttgag tttgttgttt tgggttttag ttttgtatat tgtataaata 4020
atggggtgaa agttggggat tgtgatttgt gggatagagt agagggtgtt ttgttggtgg 4080
agtttttttt tggttttagt gatgtatagg gaaatagggg atatgggtat ttttgtgaat 4140
agtttttttat ttttgatgaa atggggaatt aagaggaatt tatttgtgta attgataatt 4200
tttgtagaaa tttttttttt tttaaatttt ttttatttta ttgaggagtt aaattagaat 4260
tgtatatttt ttttttgatg atagaggaag tggaagtgtt tttaggatgg tgatattggg 4320
ggat 4324
```

<210> 51

<211> 5683

<212> DNA

<213> Artificial Sequence

<220>

<223> chemically treated genomic DNA (Homo sapiens)

<400> 51

```
ataatgttaa atgtaaattt tttgttttgt ttaatgtttt tattttatgg ttggtaaata      60
aaaataataa aatagtattg ataataataa tagaaataat taattgaagt tgtagttttt     120
aattttttta aagataatag gttaaagtga ggtttttata ataagatgtt agttattagg     180
aaggaagtta gggaaaattt tttttagggg ggtaattgat ttttattggt ttaatattgt     240
atgtagtatt gagttaggag gttttatgga gaagtgtttg tttttttttt tttgttgtta     300
gaataaagtt tataaataat tttgtttttt ttatattttt ggagttagtt agttagggtt     360
tttatttatg tttaggggat tagatttgga tatttgtaaa agaattgaat atagatggat     420
agttaagaaa aaaataaatg ttaagattat tattatttat agtttaatga tttagtaatt     480
taaaaaaatt taagaataat aaaataattt ttaagaagga taagaatttt aaaatatgta     540
gtttagaaaa ttaaattttt taggtttaga gtttaagtaa aagaaaattt gagataatga     600
agtttggaat attgggtttg tattttgttt ttgattttgt gtttttgggt aagatattga     660
atttttttat tttagattta ttttatattt taaggagggt gtaggatttt gtagtttttt     720
tagtgatggt atattattaa ttagaatttt aattagtttt atttttatta ttttagtaaa     780
atgatgttaa aaattagatt ttgtgatatg agagaagagg ggaaattaat tatagattat     840
aaagaaattg tgggatttaa gtttagggtg aggtgtttta ttgaattttt agggttggtt     900
atagtatttg ttatatagta ggtatttaat aaatgatgtg taatgggtgg ttagattgat     960
ggaaaggtag gtttttttag tgatggtaaa ggatttgggg taaaagagat ttgtggttga    1020
gttttgattt ttatatatta gtatattgat tagatgaagt tataagtttt aatttttttt    1080
tgtgtaaaat ggagataata ttttataggg ttatgaggat taaataagaa aatgtttagt    1140
ttagtgttta atatatagga agtatattgt aaatattagt tattattatt attaatattg    1200
tgattatatt ttttatttaa agattttttt gaggagtagg gggtatttag agataaaaag    1260
tttgtaggtg ggaaatttgt tagtttttta gggtgttagg tttttgtgga attttgtatt    1320
tttttttgt tttagttttt aagaatagaa atttttttagg gatttttggt gaggtagttg    1380
tgggaagatg aggtgtagaa gtaagttggg atttgtgagt tttaattttg gttttttttg    1440
```

tgttgagatt taagtggatt ttgtttggtt tgtatgtgtt attgggggaa atggatgtgg 1500
gtttgagtgt ggtaggtgtg agggtgttgt tttggggttg attattttgt ggggatattg 1560
tagttgttat tttttttttt gtaggtgggt aggggaagtg gtggttaaag tgggagttga 1620
ttgtttagta tagtttgttt gagtgttgat taggaaagtt taggtttttt ttaaattttg 1680
ttgttagatt tggtgatgta tttgtatgta tttaaggtgt ttgtatgtag aatgttatta 1740
tagaatgtag ttattttttt taatggtttg ggaaattaga ggttttttta gttatttagg 1800
gtagaggaat tttttttatt tttatgtgat atttttttgat ttagaagaat taatgttaga 1860
ttttttttgg gtttttttat ttatagttat agtttttttt ttgtttagtt aaaattgagg 1920
taggtaggaa aatattattg ggggtataag tttattagtg tgtaaatgta tttttatgaa 1980
gtgtgttttt agggagttat taaaaattga tttttttaatt atagaaatag atgagatttt 2040
gagaatattg agaagttgtt ttttgtaaag taaatttgta atggtttttg atgaaggggg 2100
gttgggggtg gggagaagtt tagttgagag aggagtttat tttatgttat atttttgtag 2160
ttgaaaagtt gtttaattat tgttaattgt tttttgtata agagttttgg gaagatttta 2220
gaaataaggt aaatgaagat tttttattgt tttttttggg ttgttgtttt tggaagttga 2280
agttttagta tgtagagtag taggagaggg ttatttttag gtaattttat tgagtaaaat 2340
aaattattta atggtataat gttttggttt aaaaaattgg aatttattag aggtaaaaat 2400
atttttttaag aaattatgga tattttgtgt tttatttatt tttatggtag tagagtattt 2460
gtattttgag ttatttatat agatttatgt tttgtattgt ttttattttt tttttttttg 2520
aagtaaagtt ttttttttaag aaggtggtta gaaagtggat tttattgggg gaaaaagaaa 2580
aatgaaatgt aaattagttt ggtattgttt gtgtttttta aaatgtggtg ggataaggtt 2640
attgagttta tagttaattt ttttatgtat ataaaatgta tatatatgta tatattttta 2700
tatatataaa agaatttata tatatgtata tagttatgtg gagttttgaa gtaatttttt 2760
atgtttttgt ttttttttaag ttttttaggt ggaggtagtt ataagtatta taagttgttt 2820
tagtgattat tagggatgga aattgttaat tattatgttt ttttttattt ttaggggttt 2880
tttggtttgt gatataagag gttttggtat tggtgttttt ttagaattgg tttagaggag 2940
ttagtttaga gtgtgaggtt gggtttgtat tgaatgtata tattgaggtt tattagattt 3000
ttttgatttt agtgaagggt gttgattgtt gtgttgttag aggttagtaa gttttttgtg 3060
tgtagttgat gagttttagt aatttgttat ttgggtgttt ttttttaaat tttgggagta 3120
aattgggaaa ataaaaaaat ttttatgttt attggttttt tttttttttt taattttttt 3180
ttttgatttg tttgtgggag tttttttttt tttgttggga ttataatgtg atgtgtaatt 3240
gtttgtgaat gaataaaagt tattggtaag tagggagatg gggatagatt gttgatggta 3300
gattgagggt ggtagtaaag gtttggtttt taaggataat ggggagttgt tttttttttat 3360
gtttggtttt tatggttttt tttgtttttt aggtaaaatg aatgtttttt tatttattat 3420
ttgtagagta tttttaggtg tgtgtagaat ttgttgatga aatttattag tgttgattgg 3480
gtagttttgt ggagttattt gaggtttttt attgtggttt ttgtttgtag aatttaagta 3540
tttatataat gtattagtat ggaatttagt atttggtggg gatattgtgt gttaagtttg 3600
gtgtggttaa tgttttagtg gttttttttat ttggtagttt tttaggatta tttttgagga 3660
ggtattggag ttgggttgta ggtgtatggg taaagaattt agtattttat ttaagtattt 3720
tgtttttttt ggttgttttt gggaggttat gtttaaaaat ataaaaataa aaataaaaat 3780
aaaaataaag ggaggtggat aaagttttgg tgggtgaatt gaagttgggt ttatgtgatt 3840
ttgaagttgg agaaataaat ttaatatgaa ttttgttttt ttggtgggtg ttgggatttt 3900
gttgtttttt atgttaattg ttggaagttt tgtatttaat ggttataggt gtttaggagt 3960
gtagagaggt tttgggttta aattattggt gtgtagggat tggatttgtg ggtagtgatt 4020
tttttaattt tttttttttt gttttatata tatatttttg tgttggttga aagtatggag 4080
gatttagggt atttgaaaaa agagggggttg ggtgtggtgg tttatgtttg taattttagt 4140
ggtttgggag gtttagaagg gtggattatt tgaggttagg agtttgagat tagtttggtt 4200
aatattagtt tggttaatat ggtgagattt tgtgtttatt aaaaatataa aaattagtta 4260
ggtgtggtgt ttgtaatatt agttatttgg gaggttgagg tatgagaatt gtttgaattt 4320
gggaggtgga ggttgtagtg agtttagatt gtgttattgt ttttttgttt tgggtgatag 4380
agtaaggtat tgttttaaaa taaaataaaa taaaatgaaa gatttggtta ggaaagaatt 4440
tgtaggtatt tgaggtgttt gtatttgtta aagtaaatgt aattttttta ttaagttgaa 4500
gtttttttata tttatttttt tagaggaagg tggtttaatt tagaaatttt ttttaagagg 4560
atttttttatt gaagattatt gtgaagtgtt aggaatttgt tttagttttg ataggtgtag 4620
gattttttgt gttgttatat tttgggattt tattttttta aataggttat ttaaaagtta 4680
gtagtgtaat tgggattttg tggtgataaa gaattattgt gtagaatttt ggagttggga 4740
gttggtttgt tttttttttta aagaaattgg gattttgtgt tttttttgtt gttagtgtag 4800
tgtgttagtg gtgtttaatt agtgagtttt ttgggtttgt gatggttttt ggttttgttt 4860
ttttgatggt tataaaaagt tgtagtgaag tttgtatttt tgagtattgt gtgtggtttt 4920
gtttttggta tggtttttag gtgtgttttt tttttggttg tttgtagtat tggttttatt 4980
gtttttgttg ttagttattg gtttttttatt tgttttattt tttattgtgg tttggttgag 5040
ttggttgtta ggttttgttg tttttttttg tttttttttt tttttagtgg ttgtattttg 5100
tgttgtattt tatttggttt gtaggtgagt agtagtgtag tatttttgtt tggtgagttt 5160
aatttgttta gtttggtttt tgttggagtg gtattggtat tttttaaga tgtttttttt 5220

```
tttgtaggat gaagaatttt atgttggagg tggtgttttt attattggag aagttgtttt    5280
ttatttttaa ttttatgttt tttagtttgg gtgttgtggg tgaagataaa gggaggaata    5340
gtttttatga aattagtttt ttttattgag gtgatgtgtt ggaggtgttt tggatttatt    5400
tgatttatta tggtatttat ttaggagata attgtgttgt ttatatgatg tttgatattt    5460
tgttggtttt gatagatgat atggggtgta tgtagaaggt ggtttttaat aagtgtttta    5520
ttttgggtgt tattgttaaa gtggttagta tttgtgtgga tatagtggag gattttgttt    5580
atggagttaa tattttggtt aattatttgg atgagttttt ttagaaaaag gtattgttta    5640
atgaggaggt ggtgtggagg gttgaaaagt tgttgggttt tat                      5683
```

<210> 52
<211> 5683
<212> DNA
<213> Artificial Sequence

<220>
<223> chemically treated genomic DNA (Homo sapiens)

<400> 52

gtaaagttta gtagtttttt agtttttgt gttatttttt tgttgagtag tgttttttt 60
tggagggatt tgtttaggtg attgattagg atgttagttt tgtaggtgaa gttttttatt 120
gtgtttatgt ggatgttggt tattttgata ataatgttta ggatgagatg tttgttggag 180
attattttt gtgtgtgttt tatgttgttt gttagggtta ataggatgtt gggtattatg 240
tgggtaatat ggttgttttt taggtagatg ttatagtggg ttaggtgggt ttggggtatt 300
tttagtatgt tgttttggtg gaaagagttg gttttataaa aattgttttt tttttgttt 360
ttgtttgtgg tgtttgaatt aaagagtgtg aagttggaga tgaggagtag ttttttagt 420
agtaaagata ttatttttag tatgggttt tttattttgt agggaagagg gtgttttgga 480
gaggtgttgg tgttattttg gtaggggttg ggttgggtaa gttaagtttg ttgggtaggg 540
gtgttgtgtt gttgtttatt tgtaggttag gtgaggtatg gtgtaaagta tggttgttga 600
ggagagagga aggtaaagga ggatagtgag gtttggtgat tggtttagtt aagttataat 660
ggggaatgag atggataagg aattggtggt tgatggtggg gatagtgaga ttagtgttat 720
aagtagttgg agaaggagtg tatttggggg ttgtgttggg ggtagggttg tgtgtggtgt 780
ttggaggtgt aggttttgtt gtgatttttt atggttgttg agggggtgga gttgaaggtt 840
gttatagatt tggaaagttt attgattggg tgttgttggt gtgttgtgtt agtggtgggg 900
aggatgtggg attttggttt ttttgggagg ggggtgggtt gatttttagt tttagggttt 960
tgtatagtga tttttttattg ttgtgggatt ttggttgtat tattggtttt taagtggttt 1020
atttagggag gtagagtttt aaggtgtggt ggtatgaaag gttttgtatt tgttgggatt 1080
ggggtgagtt tttggtattt tgtggtagtt tttggtggaa agttttttttg ggagagattt 1140
ttgagttgga ttattttttt ttaaaaggat agatatgagg gattttggtt taataaagag 1200
attgtattta ttttgtaaag tgtgagtgtt ttgaatgttt gtagattttt ttttgattga 1260
atttttgtt ttgttttgtt ttgttttgag atagtgtttt gttttattat ttagagtagg 1320
agggtggtgg tgtgatttgg gtttattgta atttttgttt tttaggttta agtgattttt 1380
gtgttttagt tttttaagta gttgatatta taggtattat gtttggttaa tttttgtatt 1440
tttagtagat atgggtttt attatgttgg ttaggttggt gttggttagg ttggttttga 1500
atttttgatt ttaagtgatt tgttttttttg gatttttttaa attgttggga ttataggtgt 1560
gagttattgt gtttagtttt tttttttta aatgttttga atttttttatg tttttagttg 1620
gtgtgagggt gtgtgtgtag ggtgggggg gggggttgg gggggttgtt atttgtgagt 1680
ttagtttttg tgtgttggtg atttgaattt gaggtttttt tgtgtttttta ggtatttgtg 1740
gttgttgagt atgaagtttt taatggttgg tatgaaaaat ggtggggttt taatgtttgt 1800
taggaaagta agatttatgt taagtttatt tttttggttt tagggttata tggatttagt 1860
tttagtttat ttattgaaat tttgtttgtt tttttttatt tttatttttta tttttatttt 1920
tatgtttttta agtataattt tttggagatg gttaaggaag gtgaagtatt tggatgagat 1980
gttaagtttt ttgtttgtgt gtttgtagtt tgattttaat gtttttttga gggtggtttt 2040
agggagttgt tgggtgaggg agttgttgaa gtgttggttg tgttaggttt ggtatgtgat 2100
gtttttattg ggtgttgagt tttgtgttaa tgtattatgt aaatgtttaa attttgtaga 2160
taaaggttat aatggagagt tttgggtggt tttataaagt tgtttagttg gtgttaatag 2220
gttttattag tagattttat gtatgtttga gggtattttg tggatgatgg atgaaggaat 2280
atttatttta tttggaagat gaaggggtt atagagatta ggtgtgaggg aaaatggttt 2340
tttattattt ttggaggttg ggtttttgtt gttattttta atttgttgtt agtgatttgt 2400
ttttgttttt ttgtttgttg gtgattttg tttatttata aatgattgtg tattatatta 2460
tagttttagt aaagaggaga aaatttttat aaatgagttg aaagaggaag ttggagaagg 2520
ggagagagtt agtggatgtg gagattttttt tatttttta gtttattttt aaaatttaaa 2580
gaaaagtgtt taggtggtga gttgttgaaa tttattagtt gtgtataggg agtttgttag 2640

```
tttttagtag tatagtagtt agtattttt gttaaaattg ggggagtttg atagatttg    2700
gtgtgtatgt ttaatgtaga tttgatttta tattttgaat tggtttttt gggttagttt   2760
tgggaaagtg ttaatattga agtttttgt gttatgggtt aaagggtttt tggagatgaa   2820
aggaaatgtg ataattaatg gtttttgttt ttggtggtta ttaaggtggt ttataatgtt  2880
tatgattgtt tttatttggg gaatttgagg gagataaaag tatggagaat tgttttaggg  2940
ttttatatag ttatatatat atatatgaat ttttttatgt atataaaaat atatatatat  3000
gtatatattt tatatatatg aaagaattag ttatagattt aatagttttg ttttattgtg  3060
ttttggaaga tgtaagtagt gttaagttga tttgtgtttt atttttttttt tttttttggt 3120
ggggtttatt ttttggttgt ttttttggga aagggtttta ttttaaaaag aaaggaggtg   3180
agagtgatat gaggtatgaa tttgtatagg tggtttaaga tgtagatatt ttgttgttat  3240
ggaaatgaat agggtgtgga gtgtttatag tttttttgaag gatattttttg tttttgataa 3300
atttaattt tttaagttag aatgttatgt tattaagtga tttattttgt ttagtggaat   3360
tgtttgaaag taattttttt ttgttgtttt gtgtgttagg attttggttt ttggaagtga  3420
tagtttgaag gaggtagtga aaagttttta tttgttttgt ttttgaagtt tttttagaat  3480
ttttatgtgg gaagtggtta gtgatgatta ggtagttttt taattgtaaa aatatagtgt  3540
ggaatgagtt ttttttttgg ttggattttt ttttgttttt gatttttttt tgttaaggat  3600
tattgtaaat ttattttgta aaaggtagtt ttttaatgtt tttaaaattt tatttatttt  3660
tgtggttgag aggttagttt ttaatggttt tttggaggta tattttataa aaatatgttt  3720
atatattaat aggtttatgt ttttaataat atttttttat ttgttttaat tttagttaaa  3780
taaagggaag attatagttg taggtggaaa ggtttaagag aaatttaata ttagtttttt  3840
taaattagaa gatgttatat ggagatagga gaaatttttt tattttgagt agttggagag  3900
atttttggtt ttttggattg ttaagaaagg tggttatatt ttgtgataat gttttgtgtg  3960
taaatgtttt aaatatatgt gaatgtgtta ttaggtttgg tggtgagatt tagaaagagt  4020
ttggattttt ttggttaata tttagataga ttgtgttgag tggttgattt ttattttggt  4080
tattgttttt tttatttgtt tgtagaagaa ggaatgatag ttatagtgtt tttgtagggt  4140
ggttggtttt ggggtagtgt ttttgtattt gttgtgttta ggtttatgtt tattttttttt 4200
agtaatgtat ataggttaag taagatttgt ttgggtttta gtgtagaaga ggttgaaatt  4260
gaggtttata ggttttagtt tatttttgta ttttattttt ttatggttat tttattagag  4320
gtttttggag agtttttgtt ttttaagaatt aggataggga agaggtgtag agttttatag 4380
aaatttaatg ttttagaagg ttaatagatt ttttatttgt aggtttttta tttttggatg  4440
tttttttgttt tttagagaag tttttggatg gaagatatga ttatagtatt agtaataata 4500
ataattaata tttatggtgt gtttttttgtg tattaggtat tgaattaagt atttttttat 4560
ttaattttta taattttatg aggtatattt tttattttat ataggaagaa attgaagttt  4620
ataattttgt ttagttagtg tgttaatgtg tggggattag gatttaatta taggtttttt  4680
ttgttttagg tttttttattg ttattgggaa ggtttgtttt tttattagtt taattattta 4740
ttatatatta tttattgaat atttgttata tggtaggtgt tatgattaat tttagaggtt  4800
taataaaata ttttatttta aatttagatt ttataatttt tttgtaattt gtaattaatt  4860
tttttttttt ttttatgtta tgaggtttaa tttttaatat tattttattg ggatagtgag  4920
aatagagttg gttaaggttt tgattaatag tatgttatta ttaggaaggt tataaaattt  4980
tatatttttt ttagggtgta ggatgaattt ggggtgggga agtttaatgt tttatttaag  5040
ggtatagagt taggagtaga atgtaggttt agtgttttag attttattat tttagatttt  5100
tttttatttg agttttaggt ttgagaaatt tggtttttta aattgtatgt tttaaaattt  5160
ttgttttttt tagggattat tttgttgttt ttggattttt ttagattatt aaattattgg  5220
gttataaata atgataattt taatatttgt tttttttta gttatttatt tatatttagt   5280
ttttttataa atgtttaggt ttggtttttt ggatataaat aggggtttta attagttaat  5340
tttaaaggtg tggggaaaat aaagttgttt gtgaattttg ttttgatagt agaggaaaag  5400
aagtagatat tttttatgg agtttttaa tttagtgtta tatgtagtgt taggttaata     5460
gggattaatt atttttgqq aggagatttt ttttggtttt tttttggtg attagtattt     5520
tattatgagg atttatttt agtttattgt ttttagaaag attaaaagtt gtagttttag   5580
ttggttgttt ttgttgttgt tgttaatgtt gttttgttgt ttttgtttgt taattataaa  5640
ataagaatat tgaataaaat aaagaattta tatttagtat tat                     5683
```

<210> 53
<211> 2347
<212> DNA
<213> Artificial Sequence

<220>
<223> chemically treated genomic DNA (Homo sapiens)

<400> 53

```
ttgaggttta agttagggag gttgagattg tagtgagttt taattgtgtt attgtatttt      60

agtttgggtt atagagtaag attttgttta aaaaataata agaaaggtgg aggaaatagg     120
tagagattag aggaaagtta tggttttttt ttgattattt tgagattttt ttaattttat     180
taatttttgg agagtttttt atttgtttta ttttatagat tgggaaattg agtttagggg     240
tatttagatt atttgaggtt ttagttggag atgaagagat ggatttaaat tatttttttgt     300
ggagtatagt tttgtttttt ttagagtgga gattgtgttt tagggttttt tttatttgta     360
ttaaggttgg ttttttttagg ttttattttt tttatttgta aaatgggttt ttgtgagtat     420
gaaggagggt ttggtttgta gtgagtgttg tttatttgtt ggttagtgtg attagttatt     480
gttgttgttt tttagtggtt tttgtttggt agttgttgga aaaatattga gggagggaga     540
tatagggttg gggaggtgaa aagttggata tagatgtttt tagtttttttg ggattaggaa     600
tagatggagg ttgggtgttt tgaagtggag tagggaggat tgtttggagg agggatattt     660
gagttagaat gtatagaatt atagggtatt tttggtagag gttatagtag aggtaaaagt     720
ttagaggagg tgttttgggg attattaaga tggatatagt gggtttgggt ttttgtgggg     780
tgatgggttg gggttttgag tgttaggtta aagggtttgg tttttttttga gggtggtagg     840
gagttatggt aggtgtgtga gttggagggg tattttagat aaaggtgaag tggtattttt     900
tttttttattg aagttataat ttgatatttt ttttgaagtt ttttatattt tattttttttt     960
ttgttatatt ttggtaagtt tattttttttt tttttgtttt taggtttgtt aagaggtttt    1020
gtgggttttt tatggtttta tttagttgtt ttagaagttt gtttgagata agtttttagg    1080
gttgttttat ttttaggtgt tttatagatt tgggggtggg tgaagtagga aggattgagt    1140
tatgagggtt ttagaaaata tgatgggagg aattatagta ttttttttatt tttttagttt    1200
ttgttttttt agggagagag atttttagtt tgaggaggag ggttgagttt tttttttatgt    1260
ggtaagagat tttaatgttg ttgaggatta tttaaagagg gtgttgaagg atgtatagga    1320
gttagttagg agataattta tttgttaatt taataaattt ataggtatta tttattaata    1380
agaataattt ttattattta attaggattt tttaatgttt ttgagggatt atttgaggat    1440
gtttaattat tatttttatt tgagataggg ttttattttg ttatttaggt tggagtgtag    1500
tgatataatt atagtttatt gtagttttaa tttttttagtt ttaagttatt tttttatttt    1560
agtttttgat atagttgaga tataggttaa tgttattatg atgtttggtt tttttttttt    1620
tttttttttt ttttattttt gagatagagt tttgtttagt tatttaggtt ggagtgtagt    1680
ggtgtgattt tagtttatta taatttttgt tttttgagtt taagtaattt ttttgtttta    1740
gtttttttgag tagttgggat tatagatatg tattattata tttagttaat ttttgtattt    1800
gtagtagaga tggggtttta ttatgttgtt taggttggtt ttaaatgttt gattttaagt    1860
gatttgttgg ttttggtttt ttaaagggtt gggattattg gtgtaagtta ttgttttttg    1920
attatttagt tttttttttt tttttttttag tttttggtag aaatgtggtt ttatgtttgt    1980
attttagtat tttgggaagg ttgaggtggg tagattattt gaggttagga atttgagatt    2040
agtttgatta atatggagaa attttgtttt tattaaaaat ataaaattag ttgggtatgg    2100
tggtttttgt ttgtaatttt agttatttgg gaggttgagg taagagaatt atttgaattt    2160
ggtaagtggg gtttgtggtg agttaagatt gtgttattgt attttagttt gggtaataag    2220
agtgaaattt tgttttaaaa aaataaaata aaataaaaat ataaaattag ttaggtatgg    2280
tggtatgtgt ttgtaatttt agttatttag gaggttgagg taggagaatt gtttgaattt    2340
ggtaggt                                                             2347
```

<210> 54
<211> 2347
<212> DNA
<213> Artificial Sequence

<220>
<223> chemically treated genomic DNA (Homo sapiens)

<400> 54

```
gtttattagg tttaagtgat ttttttgttt tagtttttg agtagttagg attataggtg 60
tgtgttatta tgtttggtta attttgtatt tttattttat tttatttttt tgagatagaa 120
ttttattttt gttgtttagg ttggagtgta atggtatgat tttggtttat tgtaaatttt 180
gtttattagg tttaagtgat ttttttgttt tagtttttta agtagttggg attatagata 240
ggagttatta tgtttggtta attttgtatt tttagtagag atggggtttt tttatgttgg 300
ttaggttagt tttgaatttt tgattttaag tgatttgttt attttggttt ttttaaagtg 360
ttgggatata ggtatgagat tatattttta ttaaaaattg aaaaaaaaaa aaaaaaagtt 420
ggatggttga ggggtggtgg tttatgttgg taattttagt tttttgggag gttgaggtta 480
gtagattatt taaggttagg tatttgagat tagtttggat aatatggtga aattttattt 540
ttattataaa tataaaaatt agttgggtgt ggtggtgtat gtttgtaatt ttagttattt 600
aggaggttga ggtaggagaa ttgtttaaat ttaggaggtg gaggttatag tgagttgaaa 660
ttgtattatt gtattttagt ttgggtgatt gagtaagatt ttgttttaaa agtaaaaaaa 720
aaaaaaaaaa aaagaaaagt taggtattat ggtggtattg gtttgtgttt tagttatatt 780

agaggttgag gtagaaggat agtttgaaat tgggaggttg aggttgtagt gagttatgat 840
tgtgttattg tattttagtt tgggtaatag agtgagattt tgttttaaat aaaaataata 900
attaaatatt tttaaatgat tttttagagg tattagaaag ttttgattga atgatgaaaa 960
ttgtttttgt tgatgagtga tgtttatgag tttgttaaat taataaatgg gttgtttttt 1020
ggttaatttt tatgtatttt ttaatatttt ttttagatga tttttagtga tgttagggtt 1080
ttttgttata tggagggaag tttggttttt ttttttgggt tgaaggtttt tttttttgag 1140
aaggtggaag ttgaggaggt ggggaagtgt tgtggttttt tttgttatgt tttttggggt 1200
ttttgtgatt tgattttttt tgttttattt gtttttgagt ttgtggggta tttgaaagtg 1260
gggtagtttt gggaatttat tttagatgag tttttggggt agttgggtgg ggttataagg 1320
ggtttatagg gtttttttggt aggtttagag atggagggaa ggaggtgggt ttgttaagat 1380
gtggtaaggg gaaggtggga tgtggggaat tttagaggga gtgttaggtt gtgattttaa 1440
taaggggaga aatattattt tatttttgtt tggggtattt ttttagttta tatatttgtt 1500
atggtttttt attgtttttg gagaaagtta ggtttttttag tttggtgttt gaggttttag 1560
tttattattt tatagaaatt tagatttatt gtgtttattt tggtggtttt taaagtattt 1620
tttttggatt tttgtttttg ttgtggtttt tgttggagat gttttgtaat tttatatatt 1680
ttagtttaag tgtttttttt ttaggtagtt tttttttgttt tattttaggg tatttggttt 1740
ttatttgttt ttgattttaa ggggttaggg gtgtttgtgt ttagtttttt atttttttag 1800
ttttgtattt ttttttttttg atgttttttt agtagttgtt aggtagaggt tattggggaa 1860
taataataat gattaattat attggttagt gggtggatag tgtttattgt aagttaagtt 1920
ttttttttata tttataaaag tttattttgt aggtgggaaa gatgaggttt gagagggtta 1980
attttggtgt gggtggggga agttttgagg tatggttttt attttggaag gggtaaaatt 2040
atattttata aggggtggtt tgaatttatt tttttatttt tagttggggt tttaggtggt 2100
ttgagtgttt ttggatttag ttttttaatt tgtgaaataa ggtaagtgag ggattttttta 2160
ggaattggtg gagttgggag ggttttagga tggttaggag ggggttatga ttttttttttg 2220
atttttattt atttttttta tttttttttgt tattttttga gtagggtttt gttttgtggt 2280
ttaggttgga gtgtagtggt gtaattagag tttattgtag ttttgatttt tttggtttaa 2340
gttttag 2347
```

<210> 55
<211> 6709
<212> DNA
<213> Artificial Sequence

<220>
<223> chemically treated genomic DNA (Homo sapiens)

<400> 55

```
atttttaaat tttttaattt atagagggtt ggagagttgg tattatgagt ttttagtttt      60
tatgggtgag aaaagaggtg tttgtgtgga gggtaggtgt gattttgggg gttagtagag     120
aggagaggtt gtgggaggat ttgttgtgaa gggtgagggg tggtgttggg tgggatgggt     180
gtttggggtt gtgtagggtg gtttttgggt gttgtttggg ttgtttgttg tttagtattg     240
gtgtaggaag atgggtttgt gtagtgtttg gtaatagtgg tttgttttat gtttggaggt     300
gattaagttt tgagttaaag gtgttgggtt tagttgggaa gtgtatggtg atttagaata     360
tggagtggag aaggttttag gggatatgag gttattgttt tagtttttgg gtttgtgttt     420
tgagtggtta gggtgtgtat ggatgggttt tgggatgtta gtggagaggt gataggagtt     480
tattatttta tggttaagtg tgtgaggttt ttggtgggtg ggggtttttt ttggggtagg     540
gtagggggag agttaggtat gtttttaatg ggtttttttag tagaattgtt aggaaagtgg    600
tttttaattt taggtatttt tgtggttttt aggtagttgg gtgattaaaa atttattttt     660
gtttgttgtg aaatgggata ggtggttgtt aggttagaga ttgtgtttat gaagttttgt     720
ttggtgggtt gggattgatg tggatttggg tttttttttgg aagtttggga atttgtgagt    780
tttttagttt tttgttttat tttgtttgtt tgaaaaaagg agttgtttga gaggaagagt     840
taaggtttaa aaggttttaa atgaagagta tgtggtttga tgtggagtgt aaagaagtga     900
gtagtgaatt gaatttggtg ggattgagat tagaggtgtt tgtatgtggt ggttgttttg     960
tgttttatta aagtaatttg ttatttatga taattatttt tgtaattatt ttggagtggt    1020
tgtgatttga tgtttgtggt gttttagggt atgtttttat tggatataga tattttaggg    1080
tgtttttttga tgttagtgtt ggtttagtgg gtgttagtgt tgtgaaggga gggtggtggg   1140
tgtgaatgtt tttttttttt gggtttgttg taggttttgg ttttttttatt ttgtttgatt   1200
ttggtgggtt tgttatttat tgaggttttt attggtgttt atgttgtttt gagaggtggg    1260
ataaatgttt ggtgtgtggt gtggagtttg tgaaagtgtt tgggggtttt tatttttttgt   1320
ttgggagttt ttttttttggg gtgggaagag gagttgtaga tttgtttgaa ttttgtttta   1380
tttgtttggt tgtttttttt tgtttggggt gggggtaggg gtggtggatg atttaggttt    1440
ggggttggga gttttgtttt taaaggtatt atttgtgtt tttttggaag tggtgtgttg     1500
```

ttggggtgtt tttgttgttg tagaggtttg gtttgtggtt ttataggggt gtgggtttag 1560
ggtttttagg gagtaagtga gtttaggttt tttagggggt ggtagagggg aatttttgat 1620
aatttttat ttttttttt aggtggttgt gaggttttg gggttttaga ggggttttt 1680
tttgttggtg ttggttgttt tgagtgtggt tatgtagttt gtgtttttat gtgggtaagg 1740
ggtggggatg ttggagggag gttgttttgg atgttggagt ttttggtttt tggttttgtt 1800
gagtagaaaa gttgaagtta aggaagggtt gaggtttttt tataagggtg tgtgtgtgtt 1860
tgggtgttgg tttagaagtt gagaatgtgt gtattgggga agggtttttt gtgttgagaa 1920
gatagaggat ttgttttagt tgttttttgt atagagggtg tggaggttgt gttttagttt 1980
gaatgtttat ttattggaaa gagggtagtg ttggggttta gggaagtttt ttgggaatga 2040
atggtttttg ttaagtggtt ttggattttt tgggtttttt gggtttatgg tatggtgttg 2100
tgtgagtttt tagtgtttat tggttttttt tgtttttttgt gtagatgttt ttaggtgggg 2160
aggtatattg gttttttttgg gtagttttgg ttagtgttgt tgtgggaaag gagagttagg 2220
gtttgggatg ggggatgagt atttttttgt ttattttggg ttttagtgtg taggaggtaa 2280
atttgttagt atagataaga tagtttgttt aagttgtatt ttaggttggg ttagagaata 2340
aaattgaggg ttagaaggtt tagaatgttg gatagtttag tggtatttgt tagggagttt 2400
taggtgtttg aaagaggtgt tgtatttttg gtgagtttag gatttatttt tttggatttg 2460
tgttttggag tgtttgtggg tttgggttta atttttgttt ttggtaggtg ttttttttt 2520
ttgttgttaa tattaggagt gttgttttttg tggtaattta ttttattttt agaagtttgt 2580
tttgggattg aagtgttagg gttttgtgtg tttttttatg tattgttttt tttagtgtaa 2640
agttttgtag atatgtttgg ttgaaaagtt ttagtggttt taatttttag attttgtttt 2700
tgtgtttggt tggaaaatta tttgggattt ggttgttgtt taaggttggg ggaaagtttt 2760
ttttggagga tgtttattat tattattttt ttttaggttt atttgggttt aaataaatgt 2820
taaagtttaa atgaatttga gaaggaatag tgaggtagtg gatgttgtta ttttgtggat 2880
gggagagtta tgtttattgt aggtgtggat attgtattga taggtattga ttattgtaaa 2940
attgtattta gtattaaata gaaatagttt tgttttttaa tttttataga atgtttaaat 3000
ttagtttagg gagttagttg gggtgtttta ataaatatgt ttttgagaat tagtgatgtt 3060
ttttttataa tgaaagttaa ttgttgtata ataattgggt taaagaattt tgttagttta 3120
aattttaaaa attagtattg tattttgttt ttaagtttgg gtggtaagta ttgaagggga 3180
gagggaagta gtgtttgtgt ggaaggaagt tttgtttttt gtttaaatat gtgggatttg 3240
ggtgtttatt tttttagttg agagtttttt gtaggttttt ttttaatttt ttgtattttt 3300
ttatttttat tttatttgtg gtgtttgggt tttatttttg tttggtgttt ttttttttagg 3360
gtgagtagag gtttttgagt gattggtttg ttagaggtag ttgtagagtt ggtgttttgt 3420
agggtagggt agggttgggt tgttgggttg gtttagtgtg tgtgggtttt ttttggtttt 3480
agttagttgt gggagatgtt attttgggtg atagtggtga gatgttgttg tttgtaattt 3540
ttgggtgggt aggattgttt gttgttttttg tggtttttgg ggtagatttt ttaggagttt 3600
tttttttttt ttttttgttt gggggttggg attgtgttgt gtttatggag ggaattgggt 3660
gtttttggtg tattggggtt ttagttgtat gatttttagtt tagattttaa gttttagtgg 3720
ggtgtaggtg ggatgggatg tgttttttagg agatgaggag attttggttg ttgagttgtt 3780
ttttaaatgt gtagtttttt aattttaggt gatatttgtt ttttggagga atgagtgaag 3840
gttgtttagg tgaaagaggg gtgggtttta gtatttggat tttttttttt agttttgtta 3900
attttaggtg tgtttatggt ggaggtgata gaaggaaggt gtgttggggg atttaggttt 3960
tgtaggtttt tggttgttaa tgaggtattg tttattgatt ttgtgttttg ttttttatag 4020
tgtggggttt ggtgttgtgt taggtttggg tgtggggtgg agttaggttg ggattggttg 4080
ggggttgttt tgtttggtgt ttgggttttt gtgttggttt ttggggagga gttatgataa 4140
tttttttttt attaaggtgt ttgggttttt tggttattgt taggatatat tgtttgggtg 4200
tggttttttt tgtttgtgga gtggttttga tatttgtggt ggtagtattt atgtttgtag 4260
agttgttgat gtgtgtttag tgatttggat agtaaggttt gtgtgtggtg ggggtggtgg 4320
tagatgtttg gttattgtga ttttgatttt ggatttattg tttgggggtt gggggggattt 4380
tggatttaat tggtgattgt tttgggggat gttggatgtt atgttgtgga aaataattga 4440
taatgttaag tatgaagagg attgtgaggt gagttggggt tttggggtgt agttttgttt 4500
tgttgaggat agtttgggag gtaggggtta ttggattgag gttggggatg agggtatagg 4560
agttttggtt ttttggtggg atgggattta tttttttgga tattttttag tttattttttg 4620
tggggttttt tttttgtata gggttttttt ttaaatagtt tttttttggg aatgaggttt 4680
ggaaagagaa tggtaaattt tatttatata gtttattggg tgtttttttta agtggtaaat 4740
agttttgttt tttgtttttt tggaaaagtg ttttgtttgt aatttttaga tgtggttttt 4800
atgtatgaag tttttgttta aaggggttag atgaggttat ttgttagtga tattttggtg 4860
gatatttttt taaggttggt ttgttatttg ttttgttatt tttttgggga agtatgaaaa 4920
taattgaagt ttggttttta aaagaagtgg gggttgggga gtggtggtgg gggtgtttga 4980
ggttagatgg ggttgtaaga gtttgtgagt gttttagttt gttggatagg ttgagttaga 5040
gttttggaga gtgggaggga agaaggaggt ggtgagtggg aagaggaaga agatgtaggt 5100
agtgttaggt gagtttaggg gtgttgggag tgtggagttt gggttatgga tttgtgggag 5160
tttattgtgt ttttgggttt tggagggttg tttttgtttg taggtttggg tgtttttgtt 5220
aggaggtgat agtgttatgt ttttttaggt ttttggtgtt ttgagatttt tgggtagatg 5280

gggatgtatt ttttagaaat tgagttgggt tgtttagggg tgagggaatg tgtgtgggta 5340
aggttgttta atttttaggg tttttatgt ttttttgtg ttttgatgtg tgagaatatt 5400
gtttttggta gtgtagggtg tttttattta ttattttttt tggttgggtt tggttagtag 5460
ggagggttgt tttgtgtgtg tgtttttttt ttatttttta gggtggtgta gggtggtggg 5520
ttttgttttt tgagtgttgt ttgtttggag gtttttgttt tgagggtgtg gaagggaggg 5580
ttttgggggt gggggaggtg tgtatttttt gtgttgtgta ttttatttgt gtttgttgtg 5640
ttgttatttt tagtagtttt tgtgttatgg gtgggtttta tgagatagtt ttgtattggg 5700
tgtttggttt ttttgttttg ggttttggtt tttttgtttt gggttttggt tatggatttt 5760
tttggtttaa gatttagggt ttggatttgg tgtttttaag tgttttgggt ttgggagtag 5820
tgtttagatt tttgttgttg ggttttgaga atttgttttt ttaggttttt tattagattt 5880
tttttttttt ttgtattttt tgtttataat gaaatttttg gggtgtattg tttttgggta 5940
tttttttgatt ttgggtaagt tttgttgggt ggggtgtggt tggtttttttg gggtttttttg 6000
tgtagtttgg tgatgttggt tagtttgtag tagtggggtt ttgtattaat ggggtttttt 6060
gttttttttt tttttttag gattgttatg atgggagtag taatgggaat ttgtgggttt 6120
tttatttttt ttttgttggg tagtattttt atagttttgt gttatttttt ttttatattg 6180
gagttgttga atattagttg ttatttttatt tttttttttt ttattagtag ttggtttatt 6240
tttagttggt tgatttttat ttgtatttgg gggaagtgta tgttgttgtt attaattttt 6300
tgtattagtt ggtgtttata ggtagttagt agtaggtttg gtttggttgt tagagttagg 6360
agggagtggg gttgtttttg tattatgggt gtttggttgg tttattgttt tatttttttg 6420
ggttggaggt gggtgtggtg agtgtttgta gggatgttta ttgttgtttt gatttgttgt 6480
tgttttatgt atatgttttg gatgttgtgg gtttggttga gaatttgggg ttttatgata 6540
tgttttatta gatggatgag gtgtaggtga gtggtgttgt ggtttttgat tggatttgtt 6600
tattttatgg tttttgttt ttatttgta tttttttagg tttttttgaa tttaagggaa 6660
ttttgttttt ttttttttag aatgttgatg attagtattt gttgttgta 6709

<210> 56
<211> 6709
<212> DNA
<213> Artificial Sequence

<220>
<223> chemically treated genomic DNA (Homo sapiens)

<400> 56

```
tgtagtaata ggtgttggtt gttgatattt tgggggagag aggataaaat ttttttaagt      60
ttgggggagt ttagaggagt gtggggtggg ggtagaaggt tgtagggtga ataggtttgg     120
ttaggagttg tagtgttgtt tatttgtatt ttgtttattt ggtgaggtat gttgtggagt     180
tttaggtttt tggttaggtt tgtggtattt agggtgtgtg tgtggggtag tagtaggttg     240
gagtggtggt aggtattttt gtgggtgttt attgtgtttg tttttagttt ggagaggtgg     300
ggtagtaggt tggttgggtg tttgtggtgt gagggtagtt ttgttttttt ttggttttgg     360
tggttgggtt aggtttgttg ttggttgttt gtgggtgttg gttggtgtag gggggttgatg     420
gtggtggtgt atgttttttt tagatgtgag taggggttgg ttgattggga gtaggttagt     480
tgttggtagg gagggggaaa gtagggtggt ggttgatatt tggtgatttt agtgtgggag     540
aggggtggtg tggggttgta gaggtgttgt ttggtggagg agaggtgggg gatttgtgga     600
tttttattgt tgtttttgtt gtggtgattt tggagggaaa aaaaaaaata ggagattttg     660
ttagtgtgaa attttgttat tgtgaattgg ttggtattgt tgggttatgt agagggtttt     720
gggggattaa ttgtattttg tttggtgggg tttgtttagg gttggaaggt atttgggggt     780
aatgtgtttt gaggattttg ttgtaagtaa agagtgtggg gagggaggag agtttggtga     840
aagatttggg ggaatgagtt tttaaagttt ggtggtgaag gtttaggtgt tgtttttaag     900
tttgaggtgt ttggaggtgt taagtttgaa ttttgggttt tgggttagaa aagtttgtgg     960
ttggagtttg gggtgaagga gttagagttt ggggtgaagg agttggatgt ttggtgtaga    1020
gttattttgt ggagtttgtt tatgatgtag ggattgttgg aggtggtagt gtggtaggtg    1080
tggatagagt gtgtggtgtg ggaaatgtgt attttttttg tttttgggat ttttttttt     1140
atgttttttgg gataaagatt tttgagtggg tggtgtttgg aaagtagggt ttgttatttt    1200
gtgttgtttt gggaagtgaa gaggagtgt gtgtataggg tggttttttt tgttggttgg     1260
gtttagttga ggggagtaat aagtgggggt gttttgtatt gttaagggta gtgtttttgt    1320
atgttggggt gtagaggggg tatgaagaat tttggaaatt gggtagtttt gtttgtgtat    1380
gtttttttgt ttttgggtgg tttgatttag tttttaaagg atgtgttttt atttgtttag    1440
gggtttttggg gtgttgggaa tttggaggaa tatggtgttg ttgtttttttg gtggaagtgt  1500
ttgggtttgt gggtaggggt agtttttttag ggtttggagg tgtagtgaat ttttgtgagt   1560
ttgtagtttg agttttgtgt ttttggtgtt tttgagtttg tttagtgttg tttgtgtttt    1620
ttttttttt ttgtttgttg ttttttttt tttttttatt ttttgaaatt ttagtttaat      1680
```

ttgtttgata ggttggagta tttgtgagtt tttatggttt tatttggttt tgggtatttt 1740
tattattatt ttttagtttt tatttttttt gaaagttaaa ttttggttgt ttttgtgttt 1800
ttttgaggag gtagtggggt aggtgataag ttggttttgg aggagtgttt gttaaggtgt 1860
tgttagtagg tagttttatt tgattttttt ggatagagat tttgtgtgta aaagttatgt 1920
ttgagggttg tagatggggt attttttga gaaaatggga agtagggttg tttgttgttt 1980
aggaaagtgt ttggtaagtt gtgtgggtgg gatttgttat ttttttttta ggttttgttt 2040
ttgaggggag gttatttagg aaaaggtttt atataggaaa ggggttttgt agaaatggat 2100
taagggtgt ttggagaagt ggatttgtt ttattgagag gttagggttt ttatgttttt 2160
gtttttgatt ttggtttagt ggtttttgtt ttttggattg tttttggtgg ggtggggttg 2220
tattttggag ttttagttta ttttgtagtt ttttttgtat ttgatattat tggttatttt 2280
ttataaatatg gtgtttggtg ttttttaaaa tagttgttag ttaaatttag gattttttta 2340
gtttttaagt ggtaaattta aaattggggt tatggtgatt aggtgtttgt tgttgttttt 2400
gttgtgtgtg ggttttgttg tttgggttat tggatatgta ttggtggttt tgtgagtgta 2460
gatgttgttg ttgtgagtgt taagattgtt ttgtggatgg ggaaagttgt gtttgaatag 2520
tgtgttttgg tgatagttgg gggatttgag tgttttaatg agaaggaaat tattataatt 2580
ttttttgggg ggttggtgtg gaggtttagg tgttgggtgg ggtggttttt agttagtttt 2640
agtttagttt tgttttgtat ttgggtttgg tgtagtgttg agttttatgt tgtgagggat 2700
gaagtgtgga gttagtgggt ggtgttttgt tgatagttga agatttgtga ggtttgggtt 2760
ttttgatgtg tttttttttt gttattttta ttgtaaatat atttgagatt ggtagagttg 2820
ggggaaggga tttaaatatt gaaatttatt tttttttgt ttaaatggtt tttatttgtt 2880
tttttaaaag atggatgtta tttggagtta agaaattgtg tatttgggga gtgatttaat 2940
aattggggtt tttttatttt ttaaaaatat attttatttt atttatattt tattgaggtt 3000
taaggtttga gttgggatta tgtagttgga gttttagtgt gttagagatg tttagttttt 3060
tttgtgggta tagtgtggtt ttggttttg ggtgggagga gagaggaagg gatttttggg 3120
gagtttgttt taagggttgt agaggtagta gatggtttta tttgtttagg agttgtggat 3180
agtggtgttt tgttgttgtt gtttgaagtg gtatttttttg tggttggttg aggttagggg 3240
gaatttgtgt gtattggatt agtttagtgg tttggttttg ttttgttttg tggaatgttg 3300
gttttgtagt tgtttttggt gggttggtta tttagaagtt tttatttgtt ttgggaaaga 3360
agtgttgggt aggaataaaa tttgagtgtt atgggtaagg tgagggtgag gaggtgtagg 3420
aagttgggag agagtttgta gagagttttt gattggagag gtagatattt aggttttata 3480
tgtttagata gaaaatgaaa ttttttttta tataaatgtt gttttttttt tttttttggt 3540
atttgttatt taaatttgaa agtaaagtgt aatattaatt tttaaaattt aaattaatga 3600
aattttttgg tttagttatt atgtagtaat tgattttat tgtaagggga atattgttaa 3660
tttttgaggg tgtatttgtt gggatgtttt aattggtttt ttgggttgga tttaaatatt 3720
ttgtggggat tagaggataa gattattttt atttgatatt gaatatgatt ttgtaataat 3780
taatgtttat tagtatggtg tttgtatttg tagtagatat gatttttta tttatggaat 3840
ggtggtattt attgttttgt tgtttttttt tgggtttgtt tgaattttag tatttattta 3900
gatttaggtg ggtttaaaag aaaataataa taataggtgt ttttttaaggg aaattttttt 3960
ttggttttag atagtagtta aattttaggt gatttttga ttggatgtgg gaataaaatt 4020
tggaagttga aattattgag atttttggt taagtatatt tgtagggttt tgtattaagg 4080
agaatagtgt ataaaaagat atataggatt ttgatatttt ggttttaaaa taggtttttg 4140
ggggtaaaat aagttattgt agaagtagtg tttttggtat tagtggtggg ggagggggat 4200
atttgttaga agtagaaatt agatttaggt ttgtaggtat tttagagtat aggtttagga 4260
agatgggttt tagatttgtt agaggtgtgg tgtttttttt gggtgtttgg gatttttga 4320
tgggtgttgt tgggttgttt gatattttgg gtttttagt ttttggtttt atttttgat 4380
ttggtttgag gtgtagtttg aataagttgt tttgtttgtg ttggtaagtt tattttttgt 4440
atgttggggt ttggaatggg taagaaggtg tttatttttt attttaggtt ttggtttttt 4500
ttttttatag taatattagt taaagttgtt tggaggaatt gatatgtttt tttgtttggg 4560
agtgtttatg taggaggtga agggagttga tgggtattga gggtttgtgt agtattgtgt 4620
tgtgggttta aaggatttag aggatttgga attgtttggt aaaggttatt tatttttaag 4680
gagtttttt ggatttttggt gttgtttttt ttttaatggg taagtgtttg aattggagtg 4740
taattttgt gttttttgta tggaaggtag ttagaataaa tttttttgttt ttttggtatg 4800
ggagattttt ttttaatatg tatattttttg gtttttgggt tggtatttag gtatgtgtgt 4860
gtttttgtga ggaagtttta attttttttt gatttaatt tttttgttta gtagagttgg 4920
gaattgaagg ttttagtatt taagatagtt tttttttaat gtttttgttt tttgtttatg 4980
tgaagatgtg gattgtgtgg ttgtgtttaa gatagttggt attggtagaa ggggattttt 5040
ttggaatttt ggaggttttg tagttgtttg gagaagagga tggaaggttg ttgggggttt 5100
tttttattg tttttttaggg agtttaaatt tatttatttt ttggaagttt tggatttatg 5160
tttttatgaa attgtaaatt aggtttttgt aatagtggga gtgttttaat agtgtgttat 5220
ttttaaagag gtgtgaggtg gtgtttttgg aaatagggtt tttggtttta ggtttgagtt 5280
atttgttatt tttgttttta ttttagatgg aagaaggtgg ttaggtgggt agagtaagat 5340
ttgggtagat ttgtgatttt ttttttgtt ttagggaggg gatttttgag tagaagatgg 5400
aggttttgga gtgtttttgt agattttgtg ttatgtgttg gatgtttgtt ttgtttttg 5460

```
ggataatgtg agtgttggta gggattttag taagtggtag atttgttagg attaagtaag 5520
gtggaaaggt tggggtttgt ggtaaattta ggaggagggg gtatttatgt ttgttatttt 5580
ttttttgtgg tattggtatt tattggattg atattggtgt taaagggtat tttgaagtat 5640
ttatgtttag taagggtatg ttttggggta ttatagatat taagttatag ttattttaaa 5700
ataattataa aaatgattat tgtaaataat agattgtttt aatagggtgt gaagtggttg 5760
ttgtatatag atgtttttgg ttttagtttt attagattta gtttattgtt tgtttttttg 5820
tgttttgtgt taggttgtat gtttttttgtt tagaattttt tgagttttaa tttttttttt 5880
taaataattt ttttttttaa ataggtaaag tgaggtgagg agttgagggg tttataggtt 5940
tttaagtttt tgaggagggt ttgggtttat gttagttttg gtttgttaaa tgagattttg 6000
tgggtatagt ttttaatttg ataattattt gttttatttt atagtaggta aaagtgaatt 6060
tttagttgtt taattatttg aaagttataa aggtgtttag ggttgaaggt tattttttta 6120
gtggttttat tagggagttt gttagagata tgtttgattt tttttttgtt ttgttttggg 6180
ggggattttt gtttgttgga ggttttatat gtttggttgt gggatggtag atttttgtta 6240
ttttttttatt aatattttgg agtttatttg tatgtatttt aattgtttgg agtgtaggtt 6300
tgaggattaa ggtggtagtt ttgtgttttt tgaggttttt tttgttttgt gttttgggtt 6360
gttatatatt ttttggttaa gtttggtgtt tttaatttag aatttggttg tttttaggtg 6420
tggagtaggt tattgttgtt agatgttgtg tggatttgtt tttttgtgtt agtattgggt 6480
gataggtagt ttgggtagta tttaggagtt atttgtatg gttttaggtg tttgttttat 6540
ttagtgttat tttttgtttt ttgtagtgag tttttttgta gtttttttttt tttgttagtt 6600
tttggggttg tatttgtttt ttatatagat attttttttt ttatttgtag gaattggaag 6660
tttatggtgt tagtttttta attttttgtg agttgaggag tttaagagt 6709
```

<210> 57
<211> 2296
<212> DNA
<213> Artificial Sequence

<220>
<223> chemically treated genomic DNA (Homo sapiens)

<400> 57

```
gttattgtat tttgttaata tttttaaaa gtaaaaaata aattaaaaa aaagtttta      60
gtggattgtt gagtttttt tgataggagg tattaaatgt gtttttttg aggataggg      120
aagagtttat tatttagtta tatttttgta tattttataa aatttatttt tattaaggat   180
gtttttttat aaaatgagga gtagtataaa gaatatttaa gaaattaggt ttgatttagg   240
gtttttgatg ttggggtagg tttttgtgt gttaatattt taggggattt tgttgtagtt    300
ttttgaatat tggttgtgag tttttaatt ttagttgagt tttagtattt tttttatatt    360
atttattttt tttgtttgtt tttggtgtgt ttagataatt ggaattttg ttgatatttt    420
aaatgggttg gttatttttt tagtgtggag gtatttagta taaggtgtga tagattagta   480
tttgttgatg agtaataaag ttattttggt aaattggatt tttttttga aatgttttgg    540
tttttatatt tttaatgatg ggttaagaaa tagtttttaa gaagaagggg tggagatagg   600
gggtaggatt ttgtttgaag tttttaagtt gattttgttt gtagttattt aatttattag   660
ggagggttgg atttgttttt ttgtggtgga tttttgtat ttttagaaag tgtaggggtg    720
gagaattaat ttagtttttt ttttttttat aagagttttg ttaggttagt ttgtatgagg   780
agggtatgtg tatattttta aaaggattgg tgtagttatt tttttaagtt tatagtttta   840
gtgtgagttt agggtttaga tttggggttt ttggtgtttt aggtgtgagt taagtttttt   900
gagtaagagg ttttatgata aattagttgg agtttggggg ttatgaaagt attggttatg   960
gtagaaatta ttagtttgtt attgtttatg ttgttttgtt taaaaatgat attgtgtaag   1020
ggattttgtt aatttttaat tttgggagat tatttattat ttggttgtgg tttgttgttg   1080
aataggaaag tattttgaga tgttaagaga ttaaaaggtt tgtaattgtt gtttagaaat   1140
gaaaatggtt ttaaattata attagagatg gaagtgtgtt ttaggggagg gtggggtgtt   1200
gtgtaattga gtttttttatg tgtaaggtgg atataaatgg tttatggatg tgggtgaagg  1260
tttttagagt aaagaatgtt tatttttatt agattaaaat atagtttggg aaaataatta   1320
tttgtttgag attttgggaa agtaaaattt tttggttttt ttgttaggaa gaatgattgt   1380
aaaataaggg ttgagaattt tttaaataag attttagaat tttatttaaa tagttgtagt   1440
ttatttttga ggagttttag atttatttgg gtttgatgga tgttgtaata ttaagtttga   1500
ggttttttta gtttggtgtt aagagtgggt tttaggggat aaatagagaa tagttgtttt   1560
tttaattgga gtagaaaata aaattttttt tttttttat ttttaatttt tttgttttgt    1620
tgtgaaattg ttattgtgtt tttgaggagt gattaaaatt ttagttatga ttaatttaaa   1680
gggtaggttt ttaggtaggg aagggaaagg ggtttaaggt aaaaaaaaaa aaaaaaagtt   1740
agttgaagtg agtttatgga ggatttgggg attataaaag ttatgggatt tgagttaatt   1800
aaaattaatg gaaaagtatt aatatgtaat ttaaaaaata ataataaaat aaagtagtaa   1860

ggatattttt ggtttgtttt ttgtttttat ttttgagttt gtttgggaat tttggagtag   1920
atttttttgaa gtatagtatg agtgtttaat ggttgtttta atttattttt ttgtttttta  1980
tatataaaga ttttgtaatg gagagtggga atgaatggtt tatttatttt tgaatatttt   2040
ttgtgagttg ggaatgtgtt tagtaaggtt gttggagtgg agggaaaaga aaaggtgttt   2100
gagtttttta gatatgggta gaaaaggaga gaatttgaga taggttgaga gaaagttgtt   2160
ttggttggta gtattgttag atttttagat atattgtata ttttagtttt tgaaaaggtt   2220
attttgagtt tttttagggt ttttggggtt tttattttat tttaaatatt tggaattgtt   2280
tgtgtttgtt ataata                                                  2296
```

<210> 58

<211> 2296

<212> DNA

<213> Artificial Sequence

<220>

<223> chemically treated genomic DNA (Homo sapiens)

<400> 58

tattatagta ggtatagata gttttaaata tttgggatga gatgagagtt ttaggggttt 60
tagagggatt taggatggtt tttttggggg ttggaatgta tggtgtgttt gagaatttgg 120
taatgttgtt aattggagta gttttttttt agtttgtttt aaattttttt tttttttgtt 180
tatgtttaga gaatttagat attttttttt ttttttttat tttagtagtt ttattgagtg 240
tatttttaat ttataggaaa tgtttagggg tgggtggatt atttattttt atttttttatt 300
gtagaatttt tgtgtgtgaa gggtaggaaa ataggttaaa atagttattg ggtatttatg 360
ttgtgtttta ggaagtttgt tttaagattt ttaagtaggt ttaagaatgg aagtagagag 420
taagttagaa atatttttat tgttttattt tattattatt ttttaaattg tatgttggtg 480
tttttttatt aattttaatt gatttaagtt ttatgatttt tgtgattttt aaatttttta 540
taaatttatt ttaattgatt tttttttttt tttttttgttt tgaatttttt tttttttttt 600
gtttgggaat ttgtttttta aattggttat ggttgggatt ttaattattt tttaaaggtg 660
tagtgataat tttataataa agtagggagg ttgggggtgg gaaggggagg gagttttgtt 720
ttttattttg attaagagag taattgtttt ttgtttgttt tttggggttt atttttggta 780
ttaggttggg gagattttaa atttggtgtt gtaatattta ttagatttaa gtgagtttgg 840
ggttttttaa aggtgaattg tagttgtttg gataaaattt taaaattttg tttgggagat 900
ttttagtttt tgttttataa ttgttttttt tggtaaagaa attaggaagt tttatttttt 960
taggatttta ggtaggtaat tgttttttta aattgtattt taatttgatg gaaatggata 1020
tttttttgttt tgagaatttt tgtttatatt tataaattgt ttgtatttgt tttgtgtatg 1080
ggaggtttga ttatatagtg ttttgttttt ttttgagatg tgttttttatt tttggttata 1140
atttagggtt atttttattt ttaaataata gttatgagtt ttttagtttt ttggtatttt 1200
ggagtgtttt tttgtttagt ggtggattat agttaggtgg tgggtgattt tttgaggttg 1260
ggaattggta aaatttttta tgtggtgtta tttttaagta aagtagtata aatggtgatg 1320
agttggtagt ttttgttata attaatgttt ttatggtttt taggttttaa ttgatttatt 1380
atgaggtttt ttgtttaggg gatttggttt atgtttgaaa tattagaggt tttgagtttg 1440
ggttttgagt ttatattggg attgtgagtt tagggaagtg gttgtattag tttttttgga 1500
ggtgtgtatg tgtttttttt atgtaggttg atttggtggg gtttttatgg ggaggagggg 1560
ggttgagtta atttttttatt tttgtatttt ttggagatgt ggagagttta ttatagggag 1620
gtagatttag tttttttttag tgaattaagt gattgtaaat agagttagtt tggaagtttt 1680
aggtagggtt ttattttttta tttttgtttt tttttttttgg gggttgtttt ttgatttatt 1740
attaaaaatg taaaagttaa aatgttttag gggaaaagtt taatttgtta gaataatttt 1800
gttatttatt aataaatatt gatttattat attttgtgtt aggtgttttt atgttggaga 1860
agtagttagt ttatttaaaa tgttagtaaa gattttagtt gtttaaatgt gttagggata 1920
ggtaaggagg ataagtggtg tgaggaggat gttgggattt agttgaagtt ggaaagttta 1980
tagttggtat ttagagaatt gtaatagggt tttttgggat attagtatgt ggggaatttg 2040
ttttagtatt agaggttttg ggttaggttt ggttttttag gtgtttttttg tgttattttt 2100
tattttgtgg agaggtattt ttggtagaag tgaattttgt aaaatatgta aaggtatggt 2160
tgaatagtga attttttttt tgtttttagg aaaagtatat ttggtatttt ttgttagagg 2220
ggatttagta atttattaaa gatttttttt ttaatttatt ttttattttt gaaaaatgtt 2280
ggtagggtgt agtgat 2296

<210> 59
<211> 4247
<212> DNA
<213> Artificial Sequence

<220>
<223> chemically treated genomic DNA (Homo sapiens)

<400> 59

ttttaaaaaa attaaaatat aaaataataa taattattaa aaattttatt tttgtttttt 60
taaataaaaa atttatttta aaaattttaa tttaaaaatt taaaaatgaa aaatgtgtat 120
tataattttt aaaaattttt gaatttaaaa atgattttttg tttttttgttt tttttgtgtg 180
atgaaattga atttattaat aaaaatttga aataaaatga ataaaaatga aatgaaatga 240
aattatgaaa aaataattga tgaattgtgt tgtgaattaa tattttaaaa aatgtgaaat 300
ttgaataatt gtgtgttaaa aattttgaaa tgaatgttgt gaaatttttt tgaattataa 360
ataaatgatt ttatgatgtg atttgaataa aaatttgatg attttataaa aaaatttaaa 420
atattgatgt tgaatttttt ttaattttttg taataattga tatgttatat aaaatgaaaa 480
aaaatttgaa ataaatttttg aaatgtgaat taaaaaaaat gaatttgatt tgtgatttta 540
atgtttttttg attgatgtga aaaaaataaa attaaatgtt tgtgtgaatt ttgaaaaaaa 600
aaaaaataaa aaattttaat gattaaaaat tttttgaatg tttattttga aatttttttt 660
gttgtttttt atttaaaata tattgaattt gatatgattt tattgtttta tttttttatta 720
tgatttttat tttgaattaa atttaattaa tttattataa taattattga taattgtttt 780
tttgttttgt aatattttgt aaattttaaa attgtgatgt tgttgaatat aaattttgaa 840
tttaaaatgt gtgatttttt tttttatgat tgttgttttt taaaaaaaat tatgtgaata 900
taaaaaattt atttaatatg ttttataaaa tttattattt ttttttgat tgtgaattat 960
gttgataatt tgaaaaattt gatttttttgt tttttatttt tttttttttt tttttttttt 1020
tttttttttt tttatttttt tgtttatttg ttgtaatttt ataaaaaaaa aattgttttt 1080
ttaattattt tttttttta ttttttatat atttttttat tttttttttga tttataaaaa 1140
aaatgaaata atgattaaaa aatgttaaaa aaaaaaaata aaaatgtaaa ttatgaattt 1200
ataaatgata tttgattttg tgtttgatga aaattatgaa ttaatttgaa tttgtgtttt 1260
gttttttgt gatatttaaa aattgtttga attaaaattg tgttattttt tgtttaatgt 1320
tgatgtaata aaatttatgt gatataaaat ttttgaaaat ttaaaaaatt ttaaaatgtt 1380
atgaatttaa tattttttta tttataaaat tttaaattaa taaaaaatat tttgtgaatt 1440
ttaaaaaaaa ataataaata tttttattt tatttgaatt tttttaataa aaatttatat 1500
gtatgataat gttattatgt ttataaataa tgttaaaata taaaaaaaat aaaaaattga 1560
aatttgaaaa ttttattttg taataaaatt taaaatttaa tttaaatttt aaaaatttgt 1620
ttaaaattta aaatttaata taataaaaat aaaaaaaatt tttgataatt tatttttata 1680
attaaaaaat gttaattaat atttttaaat ttttattttg taaataaaat aaaaatatat 1740
gaaattattt aaaaaatttt tattatttgt tattattaaa tataatttaa attttgtaaa 1800
aaaaaataat tttattttaa aattaaaatt tttaaaatat ttttaaaattt gataaaaaaa 1860
ataaaaaaaa taaaatttaa aaattaataa ttttatattt aatatttttt gtattttttt 1920
ttttttttat attttttaat aatttttata attttaatta tttttaaatt ttattttttt 1980
tgtttttttt taaattaaaa tattaaaatt aaattataat ttaaattata tttatatttt 2040
attaaaaaat aaaaatttta aatttattgt gtttttttttt attattaatt ttaatttaat 2100
atttaataaa tttttttttt taaattatta aaataaattt gtgtaaattt gatttaattg 2160
ttgtttttga attattgaat aaattgattg ttttgttgtt ttaaaaatta tttttatttt 2220
attttaattt aaatttaaaa aaatttaaat taataatttt aattgaaaaa atgaaatgat 2280
gtttattaat tattattaaa attattatta ttattattat aataattaat gtaatatttt 2340
taattgataa ttttttataa aaaaaaatat tatttttttt ttattatatt taaaataaat 2400
aaaatgaata ataaaaaaat aaaaaaaaaa aaaaaaaaaa aaatatatat ttttaataaa 2460
tttttaaatt taaaaattat ataattttat attaattatt attttttttt aaataataaa 2520
aatttataaa tttattatgt ttattttata tttaaaaaat taatattttt gaaatattta 2580
ttaaataatg aaatatttta aaaaaaaaat aaaataaata aatttttaat tttttttatta 2640
aatttaatta taatttataa aaaaaatttt tttttttttt ttattttttt ttattaaaaa 2700
tattaaatat attaaattta atatgtttat tttataaata aaaaaaaaaa aatataaaat 2760
taataattaa tttaataaaa aatataatta tataatatta aaatttttta ttttatttat 2820
tttttttaatt tatttttatt tatttttaat ttaatattta ataaaataaa aatttattaa 2880
ttttttttgta tttatttatt atttaatttt ataattattt tttaatttta attaaatttt 2940
gtaaaatatt ttaaatttat ttttaataat ttttaattat tgtttgatta tttttatttt 3000
tttattttta tatatttatt tttatttttgt taaattaatt aaattttaat taaaattaat 3060
tttttatttt tttaaataat aaataataat tttttaaata aaattttaat aaatattaat 3120
aaattttttt tttttttta aaattataaa aataatattt tttatttaaa aataataaat 3180
tttttaaatt tttttgatt taaattaaaa aaattaattt ataaaatata aaaattatat 3240
ttttttttga tttttttttt tttttgtta ttatttaatt ttttaattaa tttttttta 3300
tttttaaatt taaatatttt aataattaat ataaatttat ttttattaat aaaataataa 3360
aaatgatata ttaaaaaatt atatatttat ttaaaaaaaa aaaataaatt tatatttttt 3420

```
aaatataaat aaaaaattta aaattttttt atttttatta aaaaaaaaaa aatttttaat     3480
ttttaaaaat taatgttttt taaaaattaa attattttaa ttttaaattt taattttatt     3540
ataaataaat tttataattt taaataaatt attaaatttt tttaaatttt aaatattata     3600
tttattatat ttaaataatt aaaaaaattt aataatataa tattatataa aatatttaat     3660
ttatattaat taaaatatta atttaaattg ataaaatata taatttaaaa ataaataaat     3720
ttataaaaat attttttatt aaaaataata ataaattaat atttttaata ttaaaatttt     3780
aaaatttaaa aattaaattt aatttaaata aaaattataa aattaaaaaa aaattaaatt     3840
tataatataa aaaaaatttt aaataatttt tttatttaat attttattgt attttttttt     3900
tatttaataa aaaattaaat tattaaaaat ttaaatttat tttttaatgt aattatttaa     3960
tttattttga tttttttttt aataataaaa taaaaaaatt gtaaaaaaat ttttataaaa     4020
taaattatta ttattaaaat tttttttaata ttatttttta attatataaa atttaaaaaa     4080
taaattaaaa ataaattttt ttaaaaaaat ataaaaattt tttaaaaaaa aatttttaaa     4140
aattataaaa aaaaatttaa attataaaaa tatataattt taaaaaaaaa aatataatat     4200
tataaaaata ttattttttt ttaaaataat ttttaaattt aaatata                  4247
```

<210> 60
<211> 4247
<212> DNA
<213> Artificial Sequence

<220>
<223> chemically treated genomic DNA (Homo sapiens)

<400> 60

tgtatttaaa tttagaaatt attttgagga gaaataatat ttttatagta ttgtattttt 60
ttttttagga ttatgtgttt ttataattta ggtttttttt tgtagttttt agaagttttt 120
ttttaaaaag tttttgtgtt tttttgggga ggtttatttt tagtttgttt tttgagtttt 180
gtgtagttag aaaatggtgt tagaaaaatt ttgataatag tagtttattt tataaaagtt 240
tttttgtgat tttttttattt tattgttggg aagagggttg aggtaggtta ggtggttgtg 300
ttagaagatg agtttgggtt tttagtgatt tagtttttta ttaagtagag agggggtgtg 360
gtagggtgtt gggtaggagg attgtttgag gtttttttttg tgttatgagt ttgatttttt 420
tttaatttta tagtttttgt ttgagttaag tttaattttt agattttaaa attttagtat 480
tgaagatgtt agtttgttat tgtttttagt agaggatgtt tttataggtt tatttgtttt 540
taagttatat gttttattga tttaaattag tgttttggtt ggtgtgggtt aggtatttta 600
tataatattg tattgttgaa ttttttttagt tatttagata taatagatat aatgtttgag 660
gtttagagag gtttagtaat ttgtttgggg ttatagagtt tatttatggt agagttggga 720
tttagaatta gggtagtttg atttttgaga gatgttgatt tttgagagtt agagattttt 780
ttttttttaa tgggagtagg gagattttaa gtttttttatt tgtgtttaga ggatgtaaat 840
ttgttttttt tttttaagta gatgtgtagt tttttagtat attgtttttg ttattttgtt 900
aataagagta ggtttgtgtt gattgttaag gtatttaggt ttagggatgg gagggggttg 960
gttagagagt tgggtagtag tggggagagg gggagggttg ggaaggggta tggtttttat 1020
attttatgaa ttaatttttt tggtttgggt tggggagggt ttagaggatt tattattttt 1080
gaataagggg tgttattttt ataattttga ggaaggagga aagatttgtt gatatttatt 1140
gagattttat ttaaaaaatt gttatttgtt atttgagaga gtaggggggtt agttttggtt 1200
agggtttaat taatttggtg aagtaaaggt aggtatgtaa aggtaggaag gtgaaggtag 1260
ttgggtggtg gttgagagtt attgaagata aatttaagat gttttatggg gtttagttag 1320
gattagaagg tagttatggg gttaggtagt gggtggatgt ggggaagtta gtgggttttt 1380
gttttgttag atattaagtt ggaagtagat gaaagtgggt taagaggata gatggggtaa 1440
ggggttttag tattgtatgg ttgtattttt tattaagttg gttattggtt ttgtattttt 1500
tttttttttat ttataagatg ggtgtgttag atttaatgtg tttaatgttt ttagtagaaa 1560
aaagtgagaa gagaaaggag gtttttttta taaattatag ttaggtttag tgagaagatt 1620
agaggtttgt ttgttttgtt ttttttttaa ggtattttgt tgtttagtag gtgttttgga 1680
aatattagtt ttttgggtgt aggatgagtg taatggattt atagattttt gttgtttaga 1740
gaggggtagt gattagtata aggttatata gtttttaagt ttaagggttt gttggaggta 1800
tatatttttt tttttttttt ttttttttatt tttttgttgt ttgttttatt tatttttgggt 1860
gtgatgggaa ggaagtgata ttttttttta tgaaaagtta ttgattggga atattgtgtt 1920
aattattata ataatggtaa tagtagtttt aataataatt agtagatgtt gttttgtttt 1980
tttggttgag gttgttggtt taagtttttt taagtttgag ttggagtaaa ataaagatag 2040
tttttgaagt ggtgaaatgg ttggtttatt tggtagtttg ggagtggtgg ttaggttgag 2100
tttatgtgag tttgttttag tagtttgggg agggggggttt gttaagtgtt gagttggagt 2160
tagtagtgag aagaaatgtg atgggtttgg gatttttattt ttttaatagg gtataggtgt 2220
agtttaagtt atagtttaat tttagtattt tagtttgggg gagggtgaga gagatagggt 2280

ttgggggtag ttgaggttat aagagttatt agagggtata gaaagggaag gaggtgtggg 2340
ggatattgaa tgtgaggtta ttaattttta agttttattt tttttgtttt ttttattggg 2400
ttttgggatg ttttaaaagt tttagtttta ggatgggatt gttttttttt atggagtttg 2460
ggttatattt ggtggtgatg gatagtgaga gtttttaga tggttttgtg tgtttttatt 2520
ttatttatgg ggtagaggtt tagaggtgtt agttgatgtt ttttaattgt agaagtagat 2580
tattggaggt ttttttttatt tttgttatat taagttttag gttttaggtg ggtttttaag 2640
gtttggattg ggttttgagt tttgttgtgg ggtgaagttt ttgagttttg atttttttatt 2700
tttttgtgt tttagtgttg tttgtaggtg tagtgatgtt attgtgtgtg tgggtttta 2760
ttagaggagt ttgggtgggg taagaggtat ttgttgtttt tttttgggt ttgtggagta 2820
tttttattg gtttaagatt ttgtaaataa agggatattg gatttgtagt gttttgggat 2880
tttttgggtt tttgggggtt ttgtattgtg taagttttat tatgttggtg ttgggtggag 2940
ggtgatgtga ttttagtttg ggtgattttt agatattgtg gagggatgga gtgtgggttt 3000
gggttggttt gtagttttg ttgagtgtga agttgggtat tgtttgtaag tttgtagttt 3060
atgttttgt tttttttttt tgatgttttt tagttgttat tttgtttttt ttgtagattg 3120
agggaagata aggggatata taagggatag gagaggagag taattaggga agtgattttt 3180
tttttgtggg gttgtggtgg gtgggtggag agataggaga ggaaaagaga aaagggaaga 3240
gaaggaaggg gtaggggtg agaagttggg tttttggat tattggtgta gtttgtggtt 3300
gaaagaggaa tagtggattt tataaagtgt attgagtaag ttttttatat ttgtgtagtt 3360
tttttagga aatggtggtt gtagaggaaa gggttgtgtg ttttggattt ggagtttgtg 3420
tttggtggtg ttgtggtttt gaaatttgtg ggatgttatg gggtggaggg gtggttgttg 3480
atagttgtta taataaatta gttgagttta gtttgaggtg gaaattgtag taggaagtgg 3540
gatgatagag ttgtattggg tttggtatat tttaggtaag agatggtgaa aagagttttg 3600
agataaatgt ttgggaagtt tttggttgtt aaaatttttt gttttttttt tttttgaagt 3660
ttgtgtggat gtttagtttt attttttttg tgttggttgg ggagtgttga agttgtgggt 3720
tgggtttgtt tttttggtt tgtgttttga aatttatttt gggttttttt ttgttttata 3780
tagtgtattg attgttatga gggttggagg ggatttggtg ttggtatttt gaattttttt 3840
gtagggttgt tgagttttg tttgggttgt gttgtagagt tgtttattta tggtttggag 3900
aggttttgtg gtgtttgttt tgagattttt agtgtgtggt tgtttggatt ttgtgttttt 3960
tgagatattg gtttgtggtg tggtttgttg gttatttttt tgtagttttg ttttgttttg 4020
tttttgtttg ttttattttg ggtttttatt ggtaagtttg gttttgttgt gtggagaagg 4080
tgggggtgg ggattgtttt tgagtttggg gatttttggg aattgtagtg tgtgtttttt 4140
gtttttaggt ttttagattg gagttttgg aataggtttt ttatttagag gagtggaggt 4200
aggattttta gtaattattg ttgttttgtg ttttagtttt tttggag 4247

<210> 61
<211> 20
<212> DNA
<213> Artificial Sequence

<220>
<223> chemically treated genomic DNA (Homo sapiens)

<400> 61
ggagtggagg aaattgagat 20

<210> 62
<211> 22
<212> DNA
<213> Artificial Sequence

<220>
<223> chemically treated genomic DNA (Homo sapiens)

<400> 62
ccacacaaca aatactcaaa ac 22

<210> 63
<211> 33
<212> DNA
<213> Artificial Sequence

<220>
<223> chemically treated genomic DNA (Homo sapiens)

<400> 63
tgggtgtttg taatttttgt tttgtgttag gtt 33

<210> 64
<211> 22
<212> DNA
<213> Artificial Sequence

<220>
<223> chemically treated genomic DNA (Homo sapiens)

<400> 64
aaccgcaacg actaacaacg aa 22

<210> 65
<211> 21
<212> DNA
<213> Artificial Sequence

<220>
<223> chemically treated genomic DNA (Homo sapiens)

<400> 65
agggcgttta gttaattcgc g 21

<210> 66
<211> 30
<212> DNA
<213> Artificial Sequence

<220>
<223> chemically treated genomic DNA (Homo sapiens)

<400> 66
cgcgacgaca aaacgccctt taaaaacgaa 30

<210> 67
<211> 20
<212> DNA
<213> Artificial Sequence

<220>
<223> chemically treated genomic DNA (Homo sapiens)

<400> 67
cacgcacgat actaaacgcc 20

<210> 68
<211> 20
<212> DNA
<213> Artificial Sequence

<220>
<223> chemically treated genomic DNA (Homo sapiens)

<400> 68
tcgatcgtgt tggttgttcg 20

<210> 69
<211> 27
<212> DNA
<213> Artificial Sequence

<220>
<223> chemically treated genomic DNA (Homo sapiens)

<400> 69
cgaccgccta ccgcttcata ataacgt 27

<210> 70
<211> 21
<212> DNA
<213> Artificial Sequence

<220>
<223> chemically treated genomic DNA (Homo sapiens)

<400> 70
acccgataaa aacatcgcgt a 21

<210> 71
<211> 22
<212> DNA
<213> Artificial Sequence

<220>
<223> chemically treated genomic DNA (Homo sapiens)

<400> 71
gttcgtgcgt ttgtagttcg at 22

<210> 72
<211> 23
<212> DNA
<213> Artificial Sequence

<220>
<223> chemically treated genomic DNA (Homo sapiens)

<400> 72
aacgcgacca acgcttcaac gac 23

<210> 73
<211> 24
<212> DNA
<213> Artificial Sequence

<220>
<223> chemically treated genomic DNA (Homo sapiens)

<400> 73
tttttgttaa gcggtttcgg attt 24

<210> 74
<211> 22
<212> DNA
<213> Artificial Sequence

<220>
<223> chemically treated genomic DNA (Homo sapiens)

<400> 74
ccgcctaaaa acgtctacgc aa 22

<210> 75
<211> 29
<212> DNA
<213> Artificial Sequence

<220>
<223> chemically treated genomic DNA (Homo sapiens)

<400> 75
taaaaactcg cgcaacaccg taccgtaaa 29

<210> 76
<211> 30
<212> DNA
<213> Artificial Sequence

<220>
<223> chemically treated genomic DNA (Homo sapiens)

<400> 76
gttagttttt ttttgtcgtt tttttttcgt 30

<210> 77
<211> 22
<212> DNA
<213> Artificial Sequence

<220>
<223> chemically treated genomic DNA (Homo sapiens)

<400> 77
tcgcctaaac tcaactacac ga 22

<210> 78
<211> 21
<212> DNA
<213> Artificial Sequence

<220>
<223> chemically treated genomic DNA (Homo sapiens)

<400> 78
gcgttcggga gtttcgtgag g 21

<210> 79
<211> 4513
<212> DNA

<213> Homo Sapiens

<400> 79

```
agtgtatggg gaaagaccaa tccaactgtc catctgtggc tgggacagcc caggggtgt     60
gcccacggct gacccagggg tgtgcacacg gctgagctgg gagtcccgct ggtctccctg   120
aggactgagg gtgaacttcg ctctttgcct taaacctctt tatttcattg cagtaatagt   180
tttacgttgt acataatagt gtaaaccttt ttaaaaagga aagtataaaa acaaaagttg   240
taatttaaaa gtctgaataa ccatctgctg cttaggaaac tcaatgaaat gacatgcctt   300
tttagcagga agcaaagttg gtttctgttt tttgttttct ttgttgtttt agtttataaa   360
acatgtgcat tttacagttc cagtatcaaa tatttataat cttatgagaa atgaatgaat   420
gtttctattt acaactgtgc ttatcaaaat tgtgaacacc cccacccccg cattttttgtg   480
tgttgaaatt cttgaaggtt acattaaata aaacaaaatc tctttattat aaaatactga   540
aggtcccagg tggaatgatg agttttctaa gcatgtctga gtccagagga actgcctggg   600
catcctcccc agtactcagg tgctcatggc cttcgccccg cctgggagca ggtgccaggt   660
gcctttgtag cgagaggtga ttattatttt tttttaaata agacagtttg gatatgtcac   720
atgtgagcac agaataacca aaagaagtac acattttgtt accaggagga agtgttcaaa   780
actacggtgc tattccagtg actacatttt tttaaatttt gcgaagttca acaggtctcc   840
ctccggctgg gtgctcactg gccctgccac ccgtgctgga ccgaggcgct ttcccctggg   900
gacaggaact ttttactcca ctgagacaaa gagactctcc ttttcaaagt gtcatttttg   960
tttacctttt gttgtttccc ccccaaaaca aaagactttg tacaacttcc taagcaggat  1020
tgggggaggg atcagcttcg cctgtgctga ccgcgcctct ggattccgct catcaggaag  1080
tgtttggggt ctttgagcga gtgatctacg cgttgaattg ctgtttttcc aaactttcat  1140
ggcttaaaaa tttttttttct tctccctgta aaagaaactc agagactggt gatctgttac  1200
cgaagacgac tttttaggcg ttttctgttt ccccaggcag ctgggccgtt ggcgttccca  1260
ggcggaagcg gggcctctgg gctgggcctc tgccctgcca ggaagcggtc tgggggctgc  1320
ccctcgggat tttcagaacc ctggaaggag agagctgggg tctcaccgcg gagactcccg  1380
agttctcgtt gcctgggccc gagctgcgcc ccagggtcat cgctgatggc gcgagaccac  1440
gcattccccg cgaaccccga cttctgaaca gttcagaaag ttcgtagatt aaaaaccaaa  1500
caaaaaatac tggcacaact tctattgccg tattggcctc tctcttattt taataaaaat  1560
acggtaacat gaagcgatca tttttttaaa aatctgtttt caaagtataa cactgaaaag  1620
gggcaaggca cagctttgta aggtaatgag ttccccgtcc tcagaggtgt tcaagcagac  1680
gctaggctta cagtggcatg gagagtgttc tttctctttg gaactggatg agtcctcaga  1740
cccttgaaac gctgagtttt tttttttttt tttactccta tgtttattac attttaatcc  1800
aacagcatat aatctaagct gctccctcat ggagagccgc ggctgtgcca cctgcctgct  1860
ttcgccatgg agaccggagt caggcctgca gggaagtcgg tgcgcctttc ccaagtcctc  1920
tgcccgctgg ttcctctcgg gcctcccggg ctctgcgtgc cgcggatctg gacccggact  1980
gcagacaccg ccccacgact gggcccccgg ccagactgtt accccacagg aggagggttc  2040
cctaagtgtg cagaagagga aagctcattt ataaagtcct tgctccttcg agctgaacga  2100
cccccgcgcc cgaggcctag ctgtgtcccc gccgggccca ggctgagctt actccacact  2160
gctccatagc cgggcagaga ctccgcgagg gtctgggagt cctggcctcg gaggacccgc  2220
cgccctgcag gcctgcccaa gcaggcgccc ccgcgagagt gcgggacaga gagggcctgg  2280
cggggctcct tggcgctgag agccctcctt ctccgctgca cccagctctg ccaacggggc  2340
tggatgagtg gggctggag tcagaatccc agggtctaca cccgccgaca gcagcaatgc  2400
cgacccgccc tgcctttctc ctaaggagcg gagggaagga gccatgtgaa tgaccccagc  2460
ctgaccacca agagggcgcc agcatttctg ttttcctaga gtgaaaaggt caaatcgatg  2520
ggggctccta caccgctgga cacaggtgcg cccgggcgtc cagtgcgcgc aacacacgcc  2580
cagacactct atatgcaaat atgcctgccg ccgcggagcc ggccgggagg cagcgtttgt  2640
tcgctggggt tctgctagaa gtcattctgt ctcccgctgg tttaatatcc gccctctgcg  2700
gtctccgctc gcctcggcgg ctcccgaagc gcccccggga gggtcccggc tccgccgaaa  2760
actgcagcct gccaggctga gccgccgcgc gctgggaggt taatagcgac tgacgacaaa  2820
gggccaaggt gcaattccta aagcggggat tcgccgggtg aggcagaaat cagcctccgg  2880
ggagatgggt ccccccttcc cgacgcgccc ctgcacacac tgctgccccg gcgccggccc  2940
tccgggcggg gacccctctt ccgggcatcg cggccccatt cggcggagag ctaggccctc  3000
tccaggcctc caagtaggga gcggacgtgg gtggtgggga agggaagccg ccggcgggac  3060
ggagagtggg gtcccccgga ccctgggtcg ggcaggcctg gcgctggcag ctctgttgca  3120
cagatttgtg agcgatgctg gtggccgcat tgccgcgcgg cgcgaacccc aagtagacca  3180
ttcatgtttt tattaataat ctcaaacact tcattatatt tatgctgcct gcccagagaa  3240
aataaaaggc cattcttgaa atcgcctgaa atgtaataat attcattaaa gcgcctccta  3300
attatgtgat ttaatcaagc gtggtgcgcc cgcgctctcg gctctcgctg gaaaggctgg  3360
cgatgacggt tattattact gctgttgtta gtggcctcgt gatagcagcc ggccagctct  3420
tcagtccagg cccagtttcc tcagagagac tcggggagcc ccactttgag ggatggggtt  3480
```

```
ccccggaacg actccgaagt cccggagacc aggcgcggcc caggccctct gcccttctcc     3540
agccccagcc ccagccccag ccccagcccc gggcaaactc ttccaggccg agggcaggac     3600
tttctccaga cccttggggc cctctgtgtc ccctggggac gcccgacccg gctttctagg     3660
agggggaact ccgggtcctg agcccgggcc ccgccccgtt ggctgttccg ccccctccag     3720
ggctgagccc cgcgcaatgc ccgaagcaga aacaaagagt cgccgagatc tgcctcctgg     3780
gccgcctttt cacccccctca gcctggaagg cgtcccagag aggccccacc cccagcctcc     3840
acggagaccc aggagggaag aagagccggg acccggtagc cctgaggggc tgccgggcct     3900
gcggcgcttt ctccgccgca gagggggttgg gtgggagacg caggcgggaa ggaccctgtc     3960
ccagccgaga gtcctctgtg gggtcgagga atggtgagga tcactgtttg caccaaccgt     4020
ctggaagaaa ccgggcggcc agccgctcga ccccatctgt cgagatgtga aacttggccg     4080
cgcggtccgg gcgcgcgctg cctgcgcgga aggagccgcc tccctccttt cagcgaacgc     4140
attcggggtt ccccccctccc gccaagtcct ttgtctggaa ggggactggc aggctctgtc     4200
gtcgccgtgt gcccccctcc tttccccagc ctctaccccc tccaactaac tttcctttct     4260
tcccttctct tttcttccct ctctggatgg gtaatcacag gctccgctgc caagcgcgct     4320
gcgaggagcc gctgcccgtt cccgtcggtg cccctccccg cggcagcctg gccgagtccg     4380
cccagcctgc ctaggacgtc cctgacccgc gcgctcagtt tcattcagtg gccgtgggat     4440
gttgcatctg agtttttcct ttaggccaaa actttgtccc gaggtttttc tgagagggtt     4500
ttattctcca aca                                                        4513
```

<210> 80
<211> 2280
<212> DNA
<213> Homo Sapiens

<400> 80

tagtggcgag cactcgccgc tggccttcta gggcaggcag tattgccgct ccaggcgtgc 60
ggagtcgggg agaaaaaaac cgaacacgtg tgctacccag ggcccccaga taagcccctc 120
cgcgcgcaca cacaccctga accggagtcc agaaggccgg actcgcgtcc cggggtcgag 180
cccccttactc tccgctccca gttcggaaga tcccagcctg cccgaaggcg gcgtctgacc 240
cgctggtgga cctggtgggt gtctgttcct gatacctggg gacgaggcgg gagcgccttg 300
agcgctggga agcttcgcct cgcgagtccc cggcagtgac acgtgggagc gttgggcccg 360
gcacccccga cgcttccgag gtttcgggtg gtcgttttgc tctctcgggg gcttttattc 420
cttaagtggc tttaaacgac cgaggatggg cctcccgcgc cgccgcagct gtcacttcct 480
tcgggaaagg ctagcgacct tggacgcgca ggctctgaaa accaaggtcc agacccatac 540
cgagcgccca aagaccgcat ttgaattctc ctcgccggcc attgagggag agaaaggaac 600
gcttagttcc attcacattc acagaaagaa gcgccgaggg tgggggaaac gcagtcttgc 660
cgggtgagcc gggacaggtt cctcgcctgc cccccggccg ctgcttcctc ttagctgaat 720
ggggagcgac ccgccccggg cgcggccttc ggggctgaag actgaggtgc agcctcaccc 780
ccggcctggc agcggcttgg aagagagagg gaaaggagga acatctaccc ggctaagaga 840
cgccgccaga gtccctaaag ctggcggggg acggaggccg gggtcccgga tcttcagagc 900
gcccgcagcc gggcttctgg gctcctcggt gcggggaacc tgaccctgta gccttcgcca 960
gggcgcggcg gggctgcaag aagggaggaa ggagtgggac tagagccttc gagggcgcgg 1020
gcggcgtgca ctcagccttg tctccccgcg cgccctgccg cctgcttgcc cccttttgctg 1080
caccgggtgg ccctggtccc cgcgcccgcc cgcccccgac gccttcgcgt ggttccgcgc 1140
tgcccgcggc cagccccgtt tcgcttcgca aggagcgtgt tgttttctca atcgaaatgg 1200
gatcaagatc ccagggtttc ctcgtctcct tgttttgagg tgataggatc ttagccaata 1260
gatcgaaaac gtgaaaagta cccgtcgcct cttgtcccaa gtgcctctgt ggaaggatca 1320
tttacttgga aaagcagaat aaattctgtt gccgttttcc ttccggaggc gtgctcaccc 1380
cagagctcct agacccactg gcattcgcta tacttcctcc tgggctttac aggtaggatt 1440
tcaagaatga tatgggggca cctgggtgat ctgtgggttg caggtcctca agaggccgtt 1500
ttttcgtgca cggaagtgtc gtagctttat tttagcggaa gcaatatcaa accttatctt 1560
agaggtttgt tttgcaaaga atccagagtt tactatattt gacactattt taaaataaca 1620
cttgcttgtt ttaagaagtt gggagaaatc gataaaagaa aagttgaaat ccatgcttcc 1680
catttctact ctaggagata atcctgatca acagtttggt ttgctcccta cccccattcc 1740
ttaaaaacaa acaaacaaac aaacaaacca aaacccacta tacttttaga ttgtgtagga 1800
ttacgtagga ttttgtaaaa ataaaaatgg gattatactt cactcgtttc tgcaacggcc 1860
tagctcctac tagtaatcat tccctatcag tatagtagaa ttaaccttat tttagtatta 1920
tcattattat tttgagacag ggtctcactt tgtcacccag gctggagtac agtggcatga 1980
tcttggctca ttgcagcctc tgcctcctgg gctcaagctg tcctcccgtc tcaccacccg 2040
gccccttccc ctcaactagc tgggactaca gtcatgcgcc accaagcccg gctaattttt 2100
gtatattttt gtggagacag gtttcaccat gttgcccagg ctgatctcaa actactgaac 2160
tcaaggatc cacccacctt ggcctcccaa attgctggga tggcagacct gagccaccac 2220

accggcaaat ttattcttta taatggttgc gtggtacaga atttccattt tataggtgtg 2280

<210> 81
<211> 3640
<212> DNA
<213> Homo Sapiens

<400> 81

```
aatttctaga aattaatttg ttacaatttt atgataccgt aacatctctt ggaagagaca     60
tatgtggatg cttctatagc aagaccctgt gataagtaat ttttatgttt gtatcaaaat    120
aatcagattg tcttgtgatt ggtctagtca cctagcccaa tcttattatc tcaggaggaa    180
attaaacttc gttagtatta acatacttgt attaaaataa gaacctatac aaacctattg    240
atagaaggta ggaatttatg attgtgcact aaatccattc acataagaaa tatttttctt    300
tttactggac tctatgagac tgacattttt tatgtagaaa taatgtggct ttcttctccc    360
aaagatgctt atccatctta ttcaaataga gacacaatga gagtattatt taataaatat    420
ttgttaaatg ttttctccag cattataaga aatgcaccct ctaacaacca caacttcagt    480
aacaccttcc ttctgttgct accctaggtt gggggccatt caattttacc aagagtgtgg    540
aaattctgct gttttaatgg ggactgaaag gaaactagtc atttctcatt caaaccatca    600
aggctcctag attgatttaa aatattaagt cggtgtttaa atattccaat aaatggactt    660
tacaaagagc acaagcaatg tcacttgagg aacaaggcct caagctgagg agcaggatat    720
tggagagcct ccttcttaat tacccacctt tattagactt gaggattttc tgtcttacaa    780
gaaaatcaga aataaatttt attggtatgc tctgtaagaa cgatttcttc tgataacttg    840
gggtagcagc atttgtttga ttagctgaat ttgccatgaa tgtggaaata atgacatcaa    900
aacagctcgt gtaacaacct ccttaaagac ctcgtagatt agagatgact atagattttc    960
cccagcgcgg atcaaaggga ctgaattgaa cgttttagct taatgatcca acccctgtca   1020
cacccacagg gacgcgaatt ccgattttat aagcaggtgt gcacgtgcac tcagatactc   1080
acacaaagcc gcgtggaggg acgaaaagat caaccacccg atcgacgagg ataggtttga   1140
tcttttcgat tacctcagtg tgccagtgta tattcccggc tgggcctagc gccctaagaa   1200
acttcggaac tttagctgtt aatttttgtt ttcttactat gacttcccaa agacattcta   1260
tctgcctacc ggggcgaaga gaaatgggac caggcgtcag ggcggtggga ccctgtccag   1320
ggtcctgact ccgcgccagg gctccagacc agtcggcctc cgaaggccct ttcacccacc   1380
ccacacaaga ggaaacaaag acttctcagc tcaaggcctc agggctgcct cctgactccg   1440
gtcagtttgc aggaagagga aacaacaaaa caaaggaacc gccaatccgc cgggtattat   1500
atccctcagc tccaacctcc gacttcggac cgccagggtc accttctcta cgctgacccc   1560
gcttttctta aatgaaaaca cgccaacaaa agcatacttc ggatacaaaa tccaagtacg   1620
catctttttt ggggaggtta gagctgaggt gtacttcgga agatgagaat tttgttttca   1680
tgaattgggt aacacccagg cattgttagg taccccgaca gaccctctag atatccttct   1740
cttcctcctt cacactttct tcctatcaaa atagttattg ttttgaaatt cactagaaca   1800
acgacgttct aaaaacaaag gcgcagcaag catccctttc ttcgctgccg cgggctgaac   1860
cacggacgct cgcgggtcgc ccagccccga cggcccgcag gggcgcgcg ccgcagccgc   1920
agcacagccc ggctaccccc agaaagggag ccgaatggag ggaagcaggg agcgcggagg   1980
gctcgaggct tgcagataag gagaggcgca tcctgggatt tgggtcctct gctgctacaa   2040
cacaaccgtg ctattgttgg cactgtccga cccaagtgtc ggtggtaagc ggcgatgtcg   2100
gggctgggtc tctagcaacc gctgtgccct gggtcaggct ggtcgcccca gctaccggga   2160
tccctctccg gatgcttcca gggcgatagg tgctgcaccc attgacggga tagccgcata   2220
cctccgaaca ggtagtggag ttcgtctctg gcaggcatcc tagctgcgct gacaccaagg   2280
tcgccacaca atagccatca gcccccctta agccccagaa gtaggtttcc cctgccccag   2340
ccatagcggt tccagtcgtc gccacgagcc cacttcttat ccccaagcgc acctccctct   2400
cctcacccgg gtttatgccc gttacacaga gagaactaca caggggggaac tatggtccta   2460
caccctcgag gggacagaca ccggccgtga gacaggcacc acgcagagcc ttctggtgac   2520
tgtccgcagg agcgagacct tttttggttt tgcagtcggc taggtgtgtg tgtgtgaggg   2580
tccccagttg actaccggga tgcactgcca cttttcggcc tggcgggctc tgggactcct   2640
tggtctccgt aggaggccat cctaggcctt cggaggaggc gctcccagct ggcggcgccc   2700
ctcgccccgg gctcagaggc ggacacggtc ggtcgcgccc tgctggccct ttgttcgcgc   2760
cgcagcgggc tgggagcagc tgcgcgacac cagacccaca gcgccaagac gcgaagcgcg   2820
aggaaaccgc tgcgcttgac tccttctccc ccaactcttg gacccaggaa cgcttccagc   2880
tcctgcgtcc cacacgccca gtcctggctt ctctcccggc ccagaagtct tcaaggattg   2940
aagggctctg cctagggccc cgcactcctg cctgactccc atggcccaga aaagcagggg   3000
gacatttgaa atgtcacccc gggataaata ttaacaaaaa agcaaatgga cttgtgcagg   3060
ggccagttat caatcaacca ggccgcaagg ccactcaggg caaacacagc ccaggttggg   3120
ctgggcgaga ctttctcatg gccgcccccc gaccgaaccc ctcccccctt ggctcaggtc   3180
cttcttaggt cgttctgggg caaacaccgg acgggaaggg ggcgccgcca actcccccgc   3240

ggggtttgga ggtttcctcg cctccaagtc ccgcagggca gggtcggagt gcccaacacc   3300
caccccccgcc cgaacctcgg gcctgcgcgc cgccttcccc tgggtccgcg gtgctgcgct   3360
tgctgttggg tgtgtgtcgc tgtctctttc cgagccggca gctcctgctg tgtggccgaa   3420
gccctctgga atctttaatt ggaaactaat cttggtcttg atagacgccc acgtcagagg   3480
cgcgccaccc acccacaccc cccgcctcat cccggaggag acgcggcgag aaccctgtcg   3540
ctctgctctt taatggggca gcagcaagta gtacatggag ggaacatata ttgattttta   3600
aaaggtagtt aatttggtgg gaatttcctc ctgtcgccct                          3640
```

<210> 82
<211> 3240
<212> DNA
<213> Homo Sapiens

<400> 82

```
gaaactgagg cacaaggcag caatgattac tgagggtcct gcctccgctc ctctaggtga      60
ggagcctatt ccaggggctc cagtctgaaa gcctagaggc gaggggcgcg cactacaatt     120
cccagaggtc cccggactca agggcggtcc ccgccccccg ccttctccgc gcgacgaagc     180
cgggcttacc aatgggagcc cggagtgggg cgagcagggg cgggacgggg cgggactgcg     240
gggaggtggc cggcgggccg cgccgcgagc cagtatctca gagagcgcgg ggtccggaca     300
gccgcgcgct gagggtctcg ggcgggcgc cgcgggacct ctccgggcca tgggtaagcg      360
gctctgcggc gcggcccgga caggggctcg gcggccctgc agggaggttc aaggtgccgg     420
cgccgggtcc cctccagccc tcgtggcagt cggtgcgctg tgtggggcgg agaggggccc     480
ggggtgggtt tcggggcgcg ggccaagggg agcgagcccg gcccgcggct tcagcgctcc     540
ccggccggcg cggaaggggt gaggctgggc gtccgcgcga gcttcgagaa aaggaaagac     600
aaaaagtttt ggcggccagg ggcttcccgg gcgtttatct cgaaactctt ttcgccgtct     660
cttgcctgaa atatgccgag cccggtacgg ctctgtcgtc ccacttcctg ctgcggtttc     720
cacctcgagc tggctcagc taatttgttg tgacagctgt cggcagccgc ccctccgccc      780
cgtgacatcc cgcaggtttc agaaccgcgg cgccgccggg cacagactcc gggtccagag     840
cgcgcggccc tttcctctgc ggccgccgtt tcctgaaagg ggctgcgcgg gtgtgaagaa     900
cttgctcaat gcgctttgta ggatccactg ttcctctttc ggccgcgggc tgcgccgatg     960
gtccggggag cccggcttct cgccccctgc cccttccttc tcttcccttt tctcttttcc    1020
tctcctgtct ctccgcccac ccgccgcagc cccacaggag aggggtcgct tccctggttg    1080
ctctcctctc ctgtcccttg tgtgtcccct tgtcttccct cggtctgcag aagggacggg    1140
gtggcggctg gggagcgtca ggggaagagg gcaagagcgt gggctacgag cttgcaggcg    1200
atgcccggct tcgcgctcgg cgggagctgc gggccaaccc gggcccgcgc tccgtccctc    1260
cgcggtgtct aggggtcgcc cgggctgggg tcgcgtcacc ctccgcccag cgccggcgtg    1320
gtggggcttg cgcggtgcag gacccccgag ggcccagggg gtcccaaggc gctgcgggtc    1380
cagtgtccct ttgtttgcag agtcttgggc cagtaaggag tgctccgcgg gccccagggg    1440
aggacaacag gtgcctcttg ccccacccgg gctcctctgg tgggagccca cacgcacggt    1500
ggcgtcactg cgcctacagg caacgctaga gcacaggagg ggtgggaggt cggggctcga    1560
gggcttcacc ccgcagcaga gctcagagcc caatccaggc cttagggacc cgcctggggc    1620
ctagaacttg gtgtggcaga ggtagaaagg gcctccgata atctgcttct acagttgaga    1680
gacgtcaact ggcacctctg agcctctgcc ccgtaggtga aatgggaaca cacgaaacca    1740
tctgaggagc tctcactgtc cgtcaccacc aaatgtggcc cagactccgt ggggagggac    1800
agtcccatcc tggaactggg gcttttagga catcccagag cccggtaaga gggacaggga    1860
gaatggggct tggaagttgg tagcctcaca ttcagtgtcc cccgcacctc cttccctttc    1920
tgtaccctct ggtggctctt gtggcctcag ctgcccccag accctgtctc tctcgccctc    1980
ccccaagctg aggtactgag gttgggctgt ggcttgagct gcacctgtgc cctgttaggg    2040
agtgggggtc ccaggcccat cgcgtttctt ctcactgctg gctccagctc aacacttggc    2100
aggccccccct ccccaaactg ctggagcaag ctcgcgtaga ctcggcctgg ccgccgctcc    2160
cgggctgccg agtggaccgg ccgtttcgcc gcttcaaagg ctgtctttgt tttgctccag    2220
ctcaagctta gagaaactta agccaacaac ctcagccgag gggcggggc ggcgtctgct     2280
ggttgttatt gaggctgctg ttaccattat tgtggtaatt gacgcagtgt tcccagtcgg    2340
tgacttttca tgaaaggaag tgtcacttcc ttcccatcac acccagggtg agtggggcgg    2400
gcagcagaaa gatggggagg gagagaagga aaggggtgtg tgcctccagc agacccttaa    2460
acttgagagc tgtgtggcct tgtactagtc actgcccctc tctgggcaac aggggtctgt    2520
aagtccattg cgctcatcct gcacccagga ggctgatgtt tccggagcac ctgctgggca    2580
gcgaggtgcc ttggggaggg agcaggacag gcaggcctct gatcttctca ctgagcctgg    2640
ctgtggtttg tgggggaagc ctcctttctc ttctcacttt tttctgctga gaacattggg    2700
cacattgagt ctagcacgcc catcttgtag atggggaaag ggagatacag aaccagtgac    2760
caacttggta agaggtgcag ccatgcagtg ctggaacccc ttgccccatc tgtcctcttg    2820
gcccactttc atctgcttcc agcttgatgt ctggcagggc aggggcccac tgacttcccc    2880
```

gcatccaccc actgcctggc cccatagcta ccttctagtc ctggctaagc cccgtgagac 2940
atcttaagtt tgtcttcagt gactctcaac caccgcccga ctgccttcac cttcctgcct 3000
ttgcatacct gcctttgctt cgccaggttg gttgggccct ggccagagct ggccccctac 3060
tctctcaggt aacaggtagc agtttttttaa atggaatctc agtaggtatc aacaggtctt 3120
tcctccttcc tcaagattat ggagataaca ccccttattc aaagatggtg ggtcctctgg 3180
gccctccccg acccaggcca gggaagttga ttcatagagt gtgggggcca tgccccttcc 3240

<210> 83
<211> 2155
<212> DNA
<213> Homo Sapiens

<400> 83

tctcccttcc ttctcacccc tggagggtcc cagccaccag ccttgaagct gcagcgagag 60
ccacccactg ccttggcctt tgggacccct tgagcaggag aaaaaaacca gaaaaccaaa 120
agagagagag agacctggaa ggaagaagta atggaggagg gagacagggg gaacgagagg 180
ggagcccaca ccacagagag agaactcctt aaaggaagcg caggcgttta cgggacagga 240
gcgcggaaga aaagagtaga aaaggaaaag cagtttcaag caagagaaag taaggagagg 300
cggacaggga ggggaagagc caagagaagc caagctgagg gatctgcagc aactttgaga 360
aataaaagtg aactgtctgt gggcggctgc tgtgatctca gcaaaaagcg gagctcaagg 420
ctcagggact ccgccgccca cactgtgccc cgccttattt tggatgcctc caacttcagc 480
ttcaggcaca gtacacgagt aggggcagta aggcctggct agggccggtg ccgcacggga 540
gctgcgggtc cctgcaacac ccaggtgtgc aggcgctaca agaaggagcc ggggccgctg 600
cccccgcagc gcggacttcg ccccgtgctg cacacgcttt agaagagcgt gtccggaagt 660
gtcctccccg gccctcctcg cttcccaaga tttgtttgca acgtctacac ccgtcaccac 720
cgcataagca ccagggtgag gaggtctctt tgcaggaatt aatatcgatt tcagtctcct 780
gctccttcaa agtttctctt tgacgatggc gagacttata ttctaagtca gaagaaaata 840
aaaccttgcc tattctgggc ccatgattgc acaaaatcca aaatgctgcc ggcatgaaga 900
tgcagctgga agactctaga aacctagcat taggaggcag agactttata gtgcccagag 960
atgagttcct aaataaacta agaattacgc agtgggaaat taaggaggag ggtctgaggg 1020
gccgacctca gagggacaga tagagaaatc gagagcgaaa cactcaagga agtggaacaa 1080
atcgtcgtag ccaggcgtct ctgcctctcc ggaaacccga aatccgccca aacctcatcc 1140
caggcgctca cggtaccagt cgcagggccc cgcactatag gggctgccac cctctgcccc 1200
cggagtcccc acgccgagac tcatcatttg ctggctcttt cttccttttc ttcttttgtt 1260
tttttatcct tcaaatataa gacaaaaata cacctcattt atcatcatag atatatatat 1320
ttcactctca ccacataaat acaaaacatc caaatatcca aaacattcag aacaaagtta 1380
gtagcctgga ttcagtgaca cctacccgcg tttgaatcgt tcattatttt cttcactatt 1440
tgcctagaaa aaaaaaaaag atgaaaaaga gggggagagt ttccgacgta aataatttac 1500
aataaaatta ctattttaaa aaagtgtttt tcgcttgctt cagatggatg aatacattct 1560
agacactgcg gcacctcgca gtctgggtct gtgattttgg ttaaaatgtg ggtggccgtg 1620
gctgtaggga cgatcgccgc tgcgcagggt aaggaaagag ggtgtcgcgg ttcgtgttct 1680
gatttgggac cactgaaacc ctaagactgg ggaggcgagg ggagtgtctt tcctatcaat 1740
cacacaagac gctatctgga ctccgagact actgctagag gaggcccgac cacccagcgg 1800
cgtccctgcc tccccttccg caaagaactg ctcagaaatc cagacgtttc ctgcgtgcag 1860
gaagggtttt gtgaatccgg gtgtttggga gaggaggcaa tgagtgctga ctcgttttcc 1920
aaaccgagca attgtgcccg aagctacgcg cctgggaggc cgtagggtga agcgccggct 1980
gcgcaggcta ccgccggcag ccgctttgct ctttcctgga ggagtgggaa gctgtcggcc 2040
accgccccga acaggctcgg agaaaagatt ctgaatttcc tttgattagc ggcgttacct 2100
tttcctttgc ttttccccca ttaaattcct gtttaaagtt acaacaccca gaacc 2155

<210> 84
<211> 4513
<212> DNA
<213> Artificial Sequence

<220>
<223> chemically treated genomic DNA (Homo sapiens)

<400> 84

```
agtgtatggg gaaagattaa tttaattgtt tatttgtggt tgggatagtt taggggtgt       60
gtttacggtt gatttagggg tgtgtatacg gttgagttgg gagtttcgtt ggttttttg      120
```

aggattgagg gtgaatttcg tttttgttt taaattttt tattttattg tagtaatagt 180
tttacgttgt atataatagt gtaaatttt ttaaaaagga aagtataaaa ataaaagttg 240
taatttaaaa gtttgaataa ttatttgttg tttaggaaat ttaatgaaat gatatgtttt 300
tttagtagga agtaaagttg gtttttgttt tttgtttttt ttgttgtttt agtttataaa 360
atatgtgtat tttatagttt tagtattaaa tatttataat tttatgagaa atgaatgaat 420
gtttttattt ataattgtgt ttattaaaat tgtgaatatt tttattttcg tatttttgtg 480
tgttgaaatt tttgaaggtt atattaaata aaataaaatt tttttattat aaaatattga 540
aggttttagg tggaatgatg agtttttttaa gtatgtttga gtttagagga attgtttggg 600
tatttttttt agtatttagg tgtttatggt tttcgtttcg tttgggagta ggtgttaggt 660
gtttttgtag cgagaggtga ttattatttt tttttaaata agatagtttg gatatgttat 720
atgtgagtat agaataatta aaagaagtát atattttgtt attaggagga agtgtttaaa 780
attacggtgt tattttagtg attatatttt tttaaatttt gcgaagttta ataggttttt 840
tttcggttgg gtgtttattg gttttgttat tcgtgttgga tcgaggcgtt tttttttggg 900
gataggaatt ttttatttta ttgagataaa gagatttttt tttttaaagt gttattttttg 960
tttatttttt gttgtttttt ttttaaaata aaagattttg tataattttt taagtaggat 1020
tgggggaggg attagtttcg tttgtgttga tcgcgttttt ggatttcgtt tattaggaag 1080
tgtttggggt ttttgagcga gtgatttacg cgttgaattg ttgttttttt aaatttttat 1140
ggtttaaaaa tttttttttt tttttttgta aaagaaattt agagattggt gatttgttat 1200
cgaagacgat tttttaggcg tttttttgttt ttttaggtag ttgggtcgtt ggcgttttta 1260
ggcggaagcg gggtttttgg gttgggtttt tgttttgtta ggaagcggtt tgggggttgt 1320
ttttcgggat ttttagaatt ttggaaggag agagttgggg ttttatcgcg gagattttcg 1380
agttttcgtt gtttgggttc gagttgcgtt ttagggttat cgttgatggc gcgagattac 1440
gtatttttcg cgaatttcga tttttgaata gtttagaaag ttcgtagatt aaaaattaaa 1500
taaaaaatat tggtataatt tttattgtcg tattggtttt tttttttattt taataaaaat 1560
acggtaatat gaagcgatta ttttttttaaa aatttgtttt taaagtataa tattgaaaag 1620
gggtaaggta tagttttgta aggtaatgag tttttcgttt ttagaggtgt ttaagtagac 1680
gttaggttta tagtggtatg gagagtgttt tttttttttg gaattggatg agtttttaga 1740
tttttgaaac gttgagtttt tttttttttt tttattttta tgtttattat attttaattt 1800
aatagtatat aatttaagtt gttttttttat ggagagtcgc ggttgtgtta tttgtttgtt 1860
ttcgttatgg agatcggagt taggtttgta gggaagtcgg tgcgtttttt ttaagttttt 1920
tgttcgttgg tttttttcgg gtttttcggg ttttgcgtgt cgcggatttg gattcggatt 1980
gtagatatcg ttttacgatt gggtttttcgg ttagattgtt attttatagg aggagggttt 2040
tttaagtgtg tagaagagga aagtttattt ataaagtttt tgtttttttcg agttgaacga 2100
ttttcgcgtt cgaggtttag ttgtgttttc gtcgggttta ggttgagttt attttatatt 2160
gttttatagt cgggtagaga tttcgcgagg gtttgggagt tttggtttcg gaggattcgt 2220
cgttttgtag gtttgtttaa gtaggcgttt tcgcgagagt gcgggataga gagggtttgg 2280
cggggttttt tggcgttgag agtttttttt tttcgttgta tttagttttg ttaacggggt 2340
tggatgagtg ggggttggag ttagaatttt agggtttata ttcgtcgata gtagtaatgt 2400
cgattcgttt tgtttttttt ttaaggagcg gagggaagga gttatgtgaa tgattttagt 2460
ttgattatta agagggcgtt agtattttttg tttttttaga gtgaaaaggt taaatcgatg 2520
ggggttttta tatcgttgga tataggtgcg ttcgggcgtt tagtgcgcgt aatatacgtt 2580
tagatatttt atatgtaaat atgtttgtcg tcgcggagtc ggtcgggagg tagcgtttgt 2640
tcgttggggt tttgttagaa gttattttgt ttttcgttgg tttaatattc gttttttgcg 2700
gttttcgttc gtttcggcgg ttttcgaagc gttttcggga gggtttcggt ttcgtcgaaa 2760
attgtagttt gttaggttga gtcgtcgcgc gttgggaggt taatagcgat tgacgataaa 2820
gggttaaggt gtaattttta aagcggggat tcgtcgggtg aggtagaaat tagttttcgg 2880
ggagatgggt tttttttttt cgacgcgttt ttgtatatat tgttgtttcg gcgtcgggtt 2940
ttcgggcggg gatttttttt tcgggtatcg cggttttatt cggcggagag ttaggttttt 3000
tttaggtttt taagtaggga gcggacgtgg gtggtgggga agggaagtcg tcggcgggac 3060
ggagagtggg gtttttcgga ttttgggtcg ggtaggtttg gcgttggtag ttttgttgta 3120
tagatttgtg agcgatgttg gtggtcgtat tgtcgcgcgg cgcgaatttt aagtagatta 3180
tttatgtttt tattaataat tttaaatatt ttattatatt tatgttgttt gtttagagaa 3240
aataaaaggt tatttttgaa atcgtttgaa atgtaataat atttattaaa gcgtttttta 3300
attatgtgat ttaattaagc gtggtgcgtt cgcgttttcg gttttcgttg gaaaggttgg 3360
cgatgacggt tattattatt gttgttgtta gtggtttcgt gatagtagtc ggttagtttt 3420
ttagtttagg tttagttttt ttagagagat tcggggagtt ttattttgag ggatggggtt 3480
tttcggaacg atttcgaagt ttcggagatt aggcgcggtt taggtttttt gttttttttt 3540
agttttagtt ttagttttag ttttagtttc gggtaaattt ttttaggtcg agggtaggat 3600
tttttttaga tttttggggt tttttgtgtt ttttggggac gttcgattcg gtttttttagg 3660
agggggaatt tcgggttttg agttcgggtt tcgtttcgtt ggttgtttcg ttttttttag 3720
ggttgagttt cgcgtaatgt tcgaagtaga aataaagagt cgtcgagatt tgttttttgg 3780
gtcgtttttt tattttttta gtttggaagg cgttttagag aggttttatt tttagttttt 3840
acggagattt aggagggaag aagagtcggg attcggtagt tttgaggggt tgtcgggttt 3900

gcggcgtttt tttcgtcgta gaggggttgg gtgggagacg taggcgggaa ggattttgtt 3960
ttagtcgaga gtttttgtg gggtcgagga atggtgagga ttattgtttg tattaatcgt 4020
ttggaagaaa tcgggcggtt agtcgttcga ttttatttgt cgagatgtga aatttggtcg 4080
cgcggttcgg gcgcgcgttg tttgcgcgga aggagtcgtt tttttttttt tagcgaacgt 4140
attcggggtt ttttttttc gttaagtttt ttgtttggaa ggggattggt aggttttgtc 4200
gtcgtcgtgt gttttttttt ttttttagt ttttattttt tttaattaat tttttttttt 4260
tttttttttt tttttttttt ttttggatgg gtaattatag gtttcgttgt taagcgcgtt 4320
gcgaggagtc gttgttcgtt ttcgtcggtg ttttttttcg cggtagtttg gtcgagttcg 4380
tttagtttgt ttaggacgtt tttgattcgc gcgtttagtt ttatttagtg gtcgtgggat 4440
gttgtatttg agttttttttt ttaggttaaa attttgtttc gaggtttttt tgagagggtt 4500
ttattttttta ata 4513

<210> 85
<211> 4513
<212> DNA
<213> Artificial Sequence

<220>
<223> chemically treated genomic DNA (Homo sapiens)

<400> 85

```
tgttggagaa taaaattttt ttagaaaaat ttcgggataa agttttggtt taaaggaaaa     60
atttagatgt aatattttac ggttattgaa tgaaattgag cgcgcgggtt agggacgttt    120
taggtaggtt gggcggattc ggttaggttg tcgcggggag ggtatcgac gggaacgggt    180
agcggttttt cgtagcgcgt ttggtagcgg agtttgtgat tatttattta gagagggaag    240
aaaagagaag ggaagaaagg aaagttagtt ggagggggta gaggttgggg aaaggagggg    300
ggtatacggc gacgatagag tttgttagtt ttttttaga taaaggattt ggcgggaggg    360
gggaatttcg aatgcgttcg ttgaaaggag ggaggcggtt tttttcgcgt aggtagcgcg    420
cgttcggatc gcgcggttaa gttttatatt tcgatagatg gggtcgagcg gttggtcgtt    480
cggttttttt tagacggttg gtgtaaatag tgatttttat tatttttcga ttttatagag    540
gattttcggt tgggataggg tttttttcgt ttgcgttttt tatttaattt ttttgcggcg    600
gagaaagcgt cgtaggttcg gtagtttttt agggttatcg ggtttcggtt tttttttttt    660
tttgggtttt cgtggaggtt gggggtgggg tttttttggg acgtttttta ggttgagggg    720
gtgaaaaggc ggtttaggag gtagatttcg gcgatttttt gtttttgttt cgggtattgc    780
gcggggttta gttttggagg gggcggaata gttaacgggg cggggttcgg gtttaggatt    840
cggagttttt tttttagaa agtcgggtcg ggcgttttta ggggatatag agggttttaa    900
gggtttggag aaagttttgt tttcggtttg gaagagtttg ttcggggttg gggttggggt    960
tggggttggg gttggagaag ggtagagggt ttgggtcgcg tttggttttc gggatttcgg   1020
agtcgtttcg gggaatttta ttttttaaag tggggttttt cgagtttttt tgaggaaatt   1080
gggtttggat tgaagagttg gtcggttgtt attacgaggt tattaataat agtagtaata   1140
ataatcgtta tcgttagttt ttttagcgag agtcgagagc gcgggcgtat tacgtttgat   1200
taaattatat aattaggagg cgttttaatg aatattatta tattttaggc gattttaaga   1260
atggtttttt atttttttttg ggtaggtagt ataaatataa tgaagtgttt gagattatta   1320
ataaaaatat gaatggttta tttgggggttc gcgtcgcgcg gtaatgcggt tattagtatc   1380
gtttataaat ttgtgtaata gagttgttag cgttaggttt gttcgattta gggttcgggg   1440
gattttattt ttcgtttcgt cggcggtttt tttttttat tatttacgtt cgttttttat   1500
ttggaggttt ggagagggtt tagtttttcg tcgaatgggg tcgcgatgtt cggaagaggg   1560
gttttcgttc ggaggttcgg cgtcggggta gtagtgtgtg taggggcgcg tcgggaaggg   1620
gggatttatt ttttcggagg ttgattttttg ttttattcgg cgaattttcg ttttaggaat   1680
tgtattttgg ttttttgtcg ttagtcgtta ttaatttttt agcgcgcggc ggtttagttt   1740
ggtaggttgt agttttcggc ggagtcggga tttttcggg ggcgtttcgg gagtcgtcga   1800
ggcgagcgga gatcgtagag ggcggatatt aaattagcgg gagatagaat gatttttagt   1860
agaattttag cgaataaacg ttgtttttcg gtcggtttcg cggcggtagg tatatttgta   1920
tatagagtgt ttgggcgtgt gttgcgcgta ttggacgttc gggcgtattt gtgtttagcg   1980
gtgtaggagt ttttatcgat ttgatttttt tattttagga aaatagaaat gttggcgttt   2040
ttttggtggt taggttgggg ttatttatat ggtttttttt tttcgttttt taggagaaag   2100
gtagggcggg tcggtattgt tgttgtcggc gggtgtagat tttgggattt tgattttagt   2160
ttttatttat ttagtttcgt tggtagagtt gggtgtagcg gagaaggagg gtttttagcg   2220
ttaaggagtt tcgttaggtt ttttttgttt cgtattttcg cggggggcgtt tgtttgggta   2280
ggtttgtagg gcggcgggtt tttcgaggtt aggattttta gattttcgcg gagtttttgt   2340
tcggttatgg agtagtgtgg agtaagttta gtttgggttc ggcggggata tagttaggtt   2400
tcgggcgcgg gggtcgttta gttcgaagga gtaaggattt tataaatgag tttttttttt   2460
```

```
ttgtatattt agggaatttt tttttgtgg ggtaatagtt tggtcggggg tttagtcgtg 2520
gggcggtgtt tgtagttcgg gtttagattc gcggtacgta gagttcggga ggttcgagag 2580
gaattagcgg gtagaggatt tgggaaaggc gtatcgattt ttttgtaggt ttgatttcgg 2640
tttttatggc gaaagtaggt aggtggtata gtcgcggttt tttatgaggg agtagtttag 2700
attatatgtt gttggattaa aatgtaataa atataggagt aaaaaaaaaa aaaaaaattt 2760
agcgttttaa gggtttgagg atttatttag ttttaaagag aaagaatatt ttttatgtta 2820
ttgtaagttt agcgtttgtt tgaatatttt tgaggacggg gaatttatta ttttataaag 2880
ttgtgttttg ttttttttta gtgttatatt ttgaaaatag atttttaaaa aaatgatcgt 2940
tttatgttat cgtattttta ttaaaataag agagaggtta atacggtaat agaagttgtg 3000
ttagtatttt ttgtttggtt tttaatttac gaatttttttg aattgtttag aagtcggggt 3060
tcgcggggaa tgcgtggttt cgcgttatta gcgatgattt tggggcgtag ttcgggttta 3120
ggtaacgaga attcgggagt tttcgcggtg agattttagt tttttttttt tagggttttg 3180
aaaatttcga ggggtagttt ttagatcgtt ttttggtagg gtagaggttt agtttagagg 3240
tttcgttttc gtttgggaac gttaacggtt tagttgtttg gggaaataga aaacgtttaa 3300
aaagtcgttt tcggtaatag attattagtt tttgagtttt ttttataggg agaagaaaaa 3360
aaatttttaa gttatgaaag tttggaaaaa tagtaattta acgcgtagat tattcgttta 3420
aagattttaa atatttttttg atgagcggaa tttagaggcg cggttagtat aggcgaagtt 3480
gattttttttt ttaattttgt ttaggaagtt gtataaagtt ttttgttttg ggggggaaat 3540
aataaaaggt aaataaaaat gatattttga aaaggagagt ttttttgttt tagtggagta 3600
aaaagttttt gtttttaggg gaaagcgttt cggtttagta cgggtggtag ggttagtgag 3660
tatttagtcg gagggagatt tgttgaattt cgtaaaattt aaaaaaatgt agttattgga 3720
atagtatcgt agttttgaat attttttttt ggtaataaaa tgtgtatttt ttttggttat 3780
tttgtgttta tatgtgatat atttaaattg ttttatttaa aaaaaaataa taattatttt 3840
tcgttataaa ggtatttggt atttgttttt aggcggggcg aaggttatga gtatttgagt 3900
attggggagg atgtttaggt agttttttttg gatttagata tgtttagaaa atttattatt 3960
ttatttggga tttttagtat tttataataa agagattttg ttttatttaa tgtaattttt 4020
aagaatttta atatataaaa atgcggggt ggggtgttt ataattttga taagtatagt 4080
tgtaaataga aatatttatt tattttttat aagattataa atatttgata ttggaattgt 4140
aaaatgtata tgttttataa attaaaataa taaagaaaat aaaaaataga aattaatttt 4200
gtttttttgtt aaaaaggtat gttattttat tgagtttttt aagtagtaga tggttattta 4260
gatttttaaa ttataatttt tgtttttata tttttttttt taaaaaggtt tatattatta 4320
tgtataacgt aaaattatta ttgtaatgaa ataaagaggt ttaaggtaaa gagcgaagtt 4380
tatttttagt ttttagggag attagcggga tttttagttt agtcgtgtgt atatttttgg 4440
gttagtcgtg ggtatatttt ttgggttgtt ttagttatag atggatagtt ggattggttt 4500
tttttatat att 4513
```

<210> 86
<211> 2280
<212> DNA
<213> Artificial Sequence

<220>
<223> chemically treated genomic DNA (Homo sapiens)

<400> 86

tagtggcgag tattcgtcgt tggtttttta gggtaggtag tattgtcgtt ttaggcgtgc 60
ggagtcgggg agaaaaaaat cgaatacgtg tgttatttag ggtttttaga taagtttttt 120
cgcgcgtata tatattttga atcggagttt agaaggtcgg attcgcgttt cggggtcgag 180
ttttttattt ttcgttttta gttcggaaga ttttagtttg ttcgaaggcg gcgtttgatt 240
cgttggtgga tttggtgggt gtttgttttt gatatttggg gacgaggcgg gagcgttttg 300
agcgttggga agtttcgttt cgcgagtttt cggtagtgat acgtgggagc gttgggttcg 360
gtattttcga cgttttcgag gtttcgggtg gtcgttttgt tttttcgggg gtttttattt 420
tttaagtggt tttaaacgat cgaggatggg tttttcgcgt cgtcgtagtt gttatttttt 480
tcgggaaagg ttagcgattt tggacgcgta ggttttgaaa attaaggttt agatttatat 540
cgagcgttta aagatcgtat ttgaattttt ttcgtcggtt attgagggag agaaaggaac 600
gtttagtttt atttatattt atagaaagaa gcgtcgaggg tgggggaaac gtagttttgt 660
cgggtgagtc gggataggtt tttcgtttgt ttttcggtcg ttgttttttt ttagttgaat 720
ggggagcgat tcgtttcggg cgcggttttc ggggttgaag attgaggtgt agttttattt 780
tcggtttggt agcggtttgg aagagagagg gaaaggagga atatttattc ggttaagaga 840
cgtcgttaga gtttttaaag ttggcggggg acggaggtcg gggtttcgga tttttagagc 900
gttcgtagtc gggtttttgg gtttttcggt gcggggaatt tgattttgta gttttcgtta 960
gggcgcggcg gggttgtaag aagggaggaa ggagtgggat tagagttttc gagggcgcgg 1020

gcggcgtgta tttagttttg tttttttcgcg cgttttgtcg tttgtttgtt ttttttgttg 1080
tatcgggtgg ttttggtttt cgcgttcgtt cgttttcgac gttttcgcgt ggtttcgcgt 1140
tgttcgcggt tagtttcgtt tcgtttcgta aggagcgtgt tgttttttta atcgaaatgg 1200
gattaagatt ttagggtttt ttcgtttttt tgttttgagg tgataggatt ttagttaata 1260
gatcgaaaac gtgaaaagta ttcgtcgttt tttgttttaa gtgtttttgt ggaaggatta 1320
tttatttgga aaagtagaat aaattttgtt gtcgtttttt tttcggaggc gtgtttattt 1380
tagagttttt agatttattg gtattcgtta tatttttttt tgggttttat aggtaggatt 1440
ttaagaatga tatgggggta tttgggtgat ttgtgggttg taggttttta agaggtcgtt 1500
ttttcgtgta cggaagtgtc gtagttttat tttagcggaa gtaatattaa attttatttt 1560
agaggtttgt tttgtaaaga atttagagtt tattatattt gatattattt taaaataata 1620
tttgtttgtt ttaagaagtt gggagaaatc gataaaagaa aagttgaaat ttatgttttt 1680
tatttttatt ttaggagata attttgatta atagtttggt ttgtttttta tttttatttt 1740
ttaaaaataa ataaataaat aaataaatta aaatttatta tatttttaga ttgtgtagga 1800
ttacgtagga ttttgtaaaa ataaaaatgg gattatattt tattcgtttt tgtaacggtt 1860
tagtttttat tagtaattat tttttattag tatagtagaa ttaattttat tttagtatta 1920
ttattattat tttgagatag ggttttattt tgttatttag gttggagtat agtggtatga 1980
ttttggttta ttgtagtttt tgtttttttgg gtttaagttg ttttttcgtt ttattattcg 2040
gttttttttt tttaattagt tgggattata gttatgcgtt attaagttcg gttaattttt 2100
gtatattttt gtggagatag gttttattat gttgtttagg ttgattttaa attattgaat 2160
ttaagggatt tatttatttt ggttttttaa attgttggga tggtagattt gagttattat 2220
atcggtaaat ttattttttta taatggttgc gtggtataga atttttattt tataggtgtg 2280

<210> 87
<211> 2280
<212> DNA
<213> Artificial Sequence

<220>
<223> chemically treated genomic DNA (Homo sapiens)

<400> 87

```
tatatttata aaatggaaat tttgtattac gtaattatta taaagaataa atttgtcggt      60
gtggtggttt aggtttgtta ttttagtaat ttgggaggtt aaggtgggtg gatttttga      120
gtttagtagt ttgagattag tttgggtaat atggtgaaat ttgtttttat aaaaatatat      180
aaaaattagt cgggtttggt ggcgtatgat tgtagtttta gttagttgag gggaaggggt      240
cgggtggtga gacgggagga tagtttgagt ttaggaggta gaggttgtaa tgagttaaga      300
ttatgttatt gtattttagt ttgggtgata aagtgagatt ttgttttaaa ataataatga      360
taatattaaa ataaggttaa ttttattata ttgataggga atgattatta gtaggagtta      420
ggtcgttgta gaaacgagtg aagtataatt ttatttttat ttttataaaa ttttacgtaa      480
ttttatataa tttaaaagta tagtgggttt tggtttgttt gtttgtttgt ttgtttttaa      540
ggaatggggg tagggagtaa attaaattgt tgattaggat tatttttttag agtagaaatg      600
ggaagtatgg attttaattt ttttttatc gatttttttt aatttttaa aataagtaag      660
tgttatttta aaatagtgtt aaatatagta aattttggat tttttgtaaa ataaattttt      720
aagataaggt ttgatattgt tttcgttaaa ataaagttac gatattttcg tgtacgaaaa      780
aacggttttt tgaggatttg taatttatag attatttagg tgtttttata ttatttttga      840
aattttattt gtaaagttta ggaggaagta tagcgaatgt tagtgggttt aggagttttg      900
gggtgagtac gttttcggaa ggaaaacggt aatagaattt attttgtttt tttaagtaaa      960
tgattttttt atagaggtat ttgggataag aggcgacggg tatttttttac gttttcgatt     1020
tattggttaa gattttatta ttttaaaata aggagacgag gaaattttgg gattttgatt     1080
ttatttcgat tgagaaaata atacgttttt tgcgaagcga aacggggttg gtcgcgggta     1140
gcgcggaatt acgcgaaggc gtcgggggcg ggcgggcgcg gggattaggg ttattcggtg     1200
tagtaaaggg ggtaagtagg cggtagggcg cgcggggaga taaggttgag tgtacgtcgt     1260
tcgcgttttc gaaggtttta gttttatttt tttttttttt tttgtagttt cgtcgcgttt     1320
tggcgaaggt tataggggtta ggtttttcgt atcgaggagt ttagaagttc ggttgcgggc     1380
gttttgaaga ttcgggattt cggttttcgt ttttcgttag ttttagggat tttggcggcg     1440
tttttagtc gggtagatgt tttttttttt tttttttttt ttaagtcgtt gttaggtcgg     1500
gggtgaggtt gtattttagt ttttagtttc gaaggtcgcg ttcggggcgg gtcgtttttt     1560
atttagttaa gaggaagtag cggtcggggg gtaggcgagg aatttgtttc ggtttattcg     1620
gtaagattgc gtttttttta ttttcggcgt tttttttttgt gaatgtgaat ggaattaagc     1680
gttttttttt ttttttaat ggtcggcgag gagaatttaa atgcggtttt tgggcgttcg     1740
gtatgggttt ggattttggt ttttagagtt tgcgcgttta aggtcgttag tttttttcga     1800
aggaagtgat agttgcggcg gcgcgggagg tttattttcg gtcgtttaaa gttatttaag     1860

gaataaaagt tttcgagaga gtaaaacgat tattcgaaat ttcggaagcg tcgggggtgt     1920
cgggtttaac gtttttacgt gttattgtcg gggattcgcg aggcgaagtt ttttagcgtt     1980
taaggcgttt tcgtttcgtt tttaggtatt aggaatagat atttattagg tttattagcg     2040
ggttagacgt cgttttcggg taggttggga tttttcgaat tgggagcgga gagtaagggg     2100
ttcgatttcg ggacgcgagt tcggtttttt ggatttcggt ttagggtgtg tgtgcgcgcg     2160
gaggggttta tttgggggtt ttgggtagta tacgtgttcg gttttttttt tttcgatttc     2220
gtacgtttgg agcggtaata ttgtttgttt tagaaggtta gcggcgagtg ttcgttatta     2280
```

<210> 88
<211> 3640
<212> DNA
<213> Artificial Sequence

<220>
<223> chemically treated genomic DNA (Homo sapiens)

<400> 88

```
aattttaga aattaatttg ttataatttt atgatatcgt aatatttttt ggaagagata 60
tatgtggatg tttttatagt aagattttgt gataagtaat ttttatgttt gtattaaaat 120
aattagattg ttttgtgatt ggtttagtta tttagtttaa ttttattatt ttaggaggaa 180
attaaatttc gttagtatta atatatttgt attaaaataa gaatttatat aaatttattg 240
atagaaggta ggaatttatg attgtgtatt aaatttattt atataagaaa tatttttttt 300
tttattggat tttatgagat tgatattttt tatgtagaaa taatgtggtt tttttttttt 360
aaagatgttt atttatttta tttaaataga gatataatga gagtattatt taataaatat 420
ttgttaaatg ttttttttag tattataaga aatgtatttt ttaataatta taattttagt 480
aatatttttt ttttgttgtt attttaggtt ggggttatt taattttatt aagagtgtgg 540
aaattttgtt gttttaatgg ggattgaaag gaaattagtt atttttttatt taaattatta 600
aggtttttag attgatttaa aatattaagt cggtgtttaa atattttaat aaatggattt 660
tataaagagt ataagtaatg ttatttgagg aataaggttt taagttgagg agtaggatat 720
tggagagttt ttttttaat tatttatttt tattagattt gaggattttt tgttttataa 780
gaaaattaga aataaatttt attggtatgt tttgtaagaa cgattttttt tgataatttg 840
gggtagtagt atttgtttga ttagttgaat ttgttatgaa tgtggaaata atgatattaa 900
aatagttcgt gtaataattt ttttaaagat ttcgtagatt agagatgatt atagattttt 960
tttagcgcgg attaaaggga ttgaattgaa cgttttagtt taatgattta atttttgtta 1020
tatttatagg gacgcgaatt tcgattttat aagtaggtgt gtacgtgtat ttagatattt 1080
atataaagtc gcgtggaggg acgaaaagat taattattcg atcgacgagg ataggtttga 1140
tttttttcgat tattttagtg tgttagtgta tattttcggt tgggtttagc gttttaagaa 1200
atttcggaat tttagttgtt aatttttgtt tttttattat gatttttttaa agatatttta 1260
tttgtttatc ggggcgaaga gaaatgggat taggcgttag ggcggtggga ttttgtttag 1320
ggttttgatt tcgcgttagg gttttagatt agtcggtttt cgaaggtttt tttatttatt 1380
ttatataaga ggaaataaag atttttttagt ttaaggtttt agggttgttt tttgatttcg 1440
gttagtttgt aggaagagga aataataaaa taaaggaatc gttaattcgt cgggtattat 1500
atttttttagt tttaattttc gatttcggat cgttagggtt attttttttta cgttgatttc 1560
gtttttttta aatgaaaata cgttaataaa agtatatttc ggatataaaa tttaagtacg 1620
tatttttttt ggggaggtta gagttgaggt gtatttcgga agatgagaat tttgtttttta 1680
tgaattgggt aatatttagg tattgttagg tatttcgata gatttttttag atattttttt 1740
tttttttttt tatattttttt ttttattaaa atagttattg ttttgaaatt tattagaata 1800
acgacgtttt aaaaataaag gcgtagtaag tatttttttt ttcgttgtcg cgggttgaat 1860
tacggacgtt cgcgggtcgt ttagtttcga cggttcgtag ggggcgcgcg tcgtagtcgt 1920
agtatagttc ggttattttt agaaagggag tcgaatggag ggaagtaggg agcgcggagg 1980
gttcgaggtt tgtagataag gagaggcgta ttttgggatt tgggtttttt gttgttataa 2040
tataatcgtg ttattgttgg tattgttcga tttaagtgtc ggtggtaagc ggcgatgtcg 2100
gggttgggtt tttagtaatc gttgtgtttt gggttaggtt ggtcgtttta gttatcggga 2160
tttttttttcg gatgttttta gggcgatagg tgttgtattt attgacggga tagtcgtata 2220
ttttcgaata ggtagtggag ttcgtttttg gtaggtattt tagttgcgtt gatattaagg 2280
tcgttatata atagttatta gttttttttta agttttagaa gtaggttttt tttgttttag 2340
ttatagcggt tttagtcgtc gttacgagtt tatttttttat ttttaagcgt attttttttt 2400
ttttattcgg gtttatgttc gttatataga gagaattata taggggggaat tatggtttta 2460
tattttcgag gggatagata tcggtcgtga gataggtatt acgtagagtt ttttggtgat 2520
tgttcgtagg agcgagattt tttttggttt tgtagtcggt taggtgtgtg tgtgtgaggg 2580
tttttagttg attatcggga tgtattgtta tttttcggtt tggcgggttt tgggattttt 2640
tggttttcgt aggaggttat tttaggtttt cggaggaggc gtttttagtt ggcggcgttt 2700
```

```
ttcgtttcgg gtttagaggc ggatacggtc ggtcgcgttt tgttggtttt ttgttcgcgt    2760
cgtagcgggt tgggagtagt tgcgcgatat tagatttata gcgttaagac gcgaagcgcg    2820
aggaaatcgt tgcgtttgat tttttttttt ttaatttttg gatttaggaa cgtttttagt    2880
ttttgcgttt tatacgttta gttttggttt ttttttcggt ttagaagttt ttaaggattg    2940
aagggttttg tttagggttt cgtattttttg tttgattttt atggtttaga aaagtagggg    3000
gatatttgaa atgttatttc gggataaata ttaataaaaa agtaaatgga tttgtgtagg    3060
ggttagttat taattaatta ggtcgtaagg ttatttaggg taaatatagt ttaggttggg    3120
ttgggcgaga ttttttttatg gtcgtttttc gatcgaattt tttttttttt ggtttaggtt    3180
tttttttaggt cgttttgggg taaatatcgg acgggaaggg ggcgtcgtta atttttttcgc    3240
ggggtttgga ggttttttcg tttttaagtt tcgtagggta gggtcggagt gtttaatatt    3300
tattttcgtt cgaatttcgg gtttgcgcgt cgtttttttt tgggttcgcg gtgttgcgtt    3360
tgttgttggg tgtgtgtcgt tgtttttttt cgagtcggta gtttttgttg tgtggtcgaa    3420
gtttttttgga atttttaatt ggaaattaat tttggttttg atagacgttt acgttagagg    3480
cgcgttattt atttatattt ttcgttttat ttcggaggag acgcggcgag aattttgtcg    3540
ttttgttttt taatggggta gtagtaagta gtatatggag ggaatatata ttgattttta    3600
aaaggtagtt aatttggtgg gaattttttt ttgtcgtttt                          3640
```

<210> 89

<211> 3640

<212> DNA

<213> Artificial Sequence

<220>

<223> chemically treated genomic DNA (Homo sapiens)

<400> 89

agggcgatag gaggaaattt ttattaaatt aattattttt taaaaattaa tatatgtttt 60
ttttatgtat tatttgttgt tgttttatta aagagtagag cgataggggtt ttcgtcgcgt 120
ttttttcggg atgaggcggg gggtgtgggt gggtggcgcg tttttgacgt gggcgtttat 180
taagattaag attagttttt aattaaagat tttagagggt ttcggttata tagtaggagt 240
tgtcggttcg gaaagagata gcgatatata tttaatagta agcgtagtat cgcggattta 300
ggggaaggcg gcgcgtaggt tcgaggttcg ggcgggggtg ggtgttgggt atttcgattt 360
tgttttgcgg gatttggagg cgaggaaatt tttaaatttc gcgggggagt tggcggcgtt 420
ttttttttcgt tcggtgtttg ttttagaacg atttaagaag gatttgagtt aagggggggag 480
gggttcggtc gggggggcggt tatgagaaag tttcgtttag tttaatttgg gttgtgtttg 540
ttttgagtgg ttttgcggtt tggttgattg ataattggtt tttgtataag tttatttgtt 600
tttttgttaa tatttatttc ggggtgatat tttaaatgtt tttttgtttt ttttgggttat 660
gggagttagg taggagtgcg gggttttagg tagagttttt taatttttga agatttttgg 720
gtcgggagag aagttaggat tgggcgtgtg ggacgtagga gttggaagcg tttttgggtt 780
taagagttgg gggagaagga gttaagcgta gcggtttttt cgcgtttcgc gttttggcgt 840
tgtgggtttg gtgtcgcgta gttgttttta gttcgttgcg gcgcgaataa agggttagta 900
gggcgcgatc gatcgtgttc gtttttgagt tcggggcgag gggcgtcgtt agttgggagc 960
gtttttttcg aaggtttagg atggtttttt acggagatta aggagtttta gagttcgtta 1020
ggtcgaaaag tggtagtgta tttcggtagt taattgggga tttttatata tatatattta 1080
gtcgattgta aaattaaaaa aggtttcgtt tttgcggata gttattagaa ggttttgcgt 1140
ggtgtttgtt ttacggtcgg tgtttgtttt ttcgagggtg taggattata gtttttttttg 1200
tgtagttttt tttgtgtaac gggtataaat tcgggtgagg agagggaggt gcgtttgggg 1260
ataagaagtg ggttcgtggc gacgattgga atcgttatgg ttggggtagg ggaaatttat 1320
ttttggggtt taagggggggt tgatggttat tgtgtggcga ttttggtgtt agcgtagtta 1380
ggatgtttgt tagagacgaa ttttattatt tgttcggagg tatgcggtta tttcgttaat 1440
gggtgtagta tttatcgttt tggaagtatt cggagaggga tttcggtagt tggggcgatt 1500
agtttgattt agggtatagc ggttgttaga gatttagttt cgatatcgtc gtttattatc 1560
gatatttggg tcggatagtg ttaataatag tacggttgtg ttgtagtagt agaggattta 1620
aattttagga tgcgtttttt tttatttgta agtttcgagt ttttcgcgtt ttttgttttt 1680
ttttattcgg tttttttttt ggggggtagtc gggttgtgtt gcggttgcgg cgcgcgtttt 1740
ttgcgggtcg tcggggttgg gcgattcgcg agcgttcgtg gtttagttcg cggtagcgaa 1800
gaaagggatg tttgttgcgt ttttgttttt agaacgtcgt tgttttagtg aattttaaaa 1860
taataattat tttgatagga agaaagtgtg aaggaggaag agaaggatat ttagagggtt 1920
tgtcggggta tttaataatg tttgggtgtt atttaattta tgaaaataaa atttttattt 1980
ttcgaagtat attttagttt taattttttt aaaaaagatg cgtatttgga ttttgtattc 2040
gaagtatgtt tttgttggcg tgtttttatt taagaaaagc ggggttagcg tagagaaggt 2100
gattttggcg gttcgaagtc ggaggttgga gttgagggat ataatattcg gcggattggc 2160

ggtttttttg ttttgttgtt tttttttttt gtaaattgat cggagttagg aggtagtttt 2220
gaggttttga gttgagaagt ttttgttttt ttttgtgtgg ggtgggtgaa agggttttcg 2280
gaggtcgatt ggtttggagt tttggcgcgg agttaggatt ttggataggg ttttatcgtt 2340
ttgacgtttg gttttatttt ttttcgtttc ggtaggtaga tagaatgttt ttgggaagtt 2400
atagtaagaa aataaaaatt aatagttaaa gtttcgaagt tttttagggc gttaggttta 2460
gtcgggaata tatattggta tattgaggta atcgaaaaga ttaaatttat tttcgtcgat 2520
cgggtggttg atttttttcgt tttttttacgc ggttttgtgt gagtatttga gtgtacgtgt 2580
atatttgttt ataaaatcgg aattcgcgtt tttgtgggtg tgatagggggt tggattatta 2640
agttaaaacg tttaatttag ttttttttgat tcgcgttggg gaaaatttat agttattttt 2700
aatttacgag gtttttaagg aggttgttat acgagttgtt ttgatgttat tatttttata 2760
tttatggtaa atttagttaa ttaaataaat gttgttattt taagttatta gaagaaatcg 2820
tttttataga gtatattaat aaaatttatt tttgattttt ttgtaagata gaaaattttt 2880
aagtttaata aaggtgggta attaagaagg aggtttttta atattttgtt ttttagtttg 2940
aggttttgtt ttttaagtga tattgtttgt gtttttttgta aagtttattt attggaatat 3000
ttaaatatcg atttaatatt ttaaattaat ttaggagttt tgatggtttg aatgagaaat 3060
gattagtttt tttttagttt ttattaaaat agtagaattt ttatattttt ggtaaaattg 3120
aatggttttt aatttagggt agtaatagaa ggaaggtgtt attgaagttg tggttgttag 3180
agggtgtatt ttttataatg ttggagaaaa tatttaataa atatttatta aataatattt 3240
ttattgtgtt tttatttgaa taagatggat aagtattttt gggagaagaa agttatatta 3300
tttttatata aaaaatgtta gttttataga gtttagtaaa aagaaaaata ttttttatgt 3360
gaatggattt agtgtataat tataaatttt tattttttat taataggttt gtataggttt 3420
ttattttaat ataagtatgt taatattaac gaagtttaat ttttttttga gataataaga 3480
ttgggttagg tgattagatt aattataaga taatttgatt attttgatat aaatataaaa 3540
attatttatt atagggtttt gttatagaag tatttatata tgtttttttt aagagatgtt 3600
acggtattat aaaattgtaa taaattaatt tttagaaatt 3640

<210> 90
<211> 3240
<212> DNA
<213> Artificial Sequence

<220>
<223> chemically treated genomic DNA (Homo sapiens)

<400> 90

```
gaaattgagg tataaggtag taatgattat tgagggtttt gttttcgttt ttttaggtga      60
ggagtttatt ttagggggtt tagtttgaaa gtttagaggc gagggggcgcg tattataatt    120
tttagaggtt ttcggattta agggcggttt tcgtttttcg ttttttttcgc gcgacgaagt    180
cgggtttatt aatgggagtt cggagtgggg cgagtagggg cgggacgggg cgggattgcg    240
gggaggtggt cggcgggtcg cgtcgcgagt tagtatttta gagagcgcgg ggttcggata    300
gtcgcgcgtt gagggtttcg gggcgggcgt cgcgggattt tttcgggtta tgggtaagcg    360
gttttgcggc gcggttcgga taggggttcg gcggttttgt agggaggttt aaggtgtcgg    420
cgtcgggttt tttttagttt tcgtggtagt cggtgcgttg tgtggggcgg agaggggttc    480
ggggtgggtt tcggggcgcg ggttaagggg agcgagttcg gttcgcggtt ttagcgtttt    540
tcggtcggcg cggaaggggt gaggttgggc gttcgcgcga gtttcgagaa aaggaaagat    600
aaaaagtttt ggcggttagg ggtttttcgg gcgtttattt cgaaattttt ttcgtcgttt    660
tttgtttgaa atatgtcgag ttcggtacgg ttttgtcgtt ttattttttg ttgcggtttt    720
tatttcgagt tgggtttagt taatttgttg tgatagttgt cggtagtcgt tttttcgttt    780
cgtgatattt cgtaggtttt agaatcgcgg cgtcgtcggg tatagatttc gggtttagag    840
cgcgcggttt ttttttttgc ggtcgtcgtt ttttgaaagg ggttgcgcgg gtgtgaagaa    900
tttgtttaat gcgttttgta ggatttattg tttttttttc ggtcgcgggt tgcgtcgatg    960
gttcggggag ttcggttttt cgtttttttgt tttttttttt tttttttttt tttttttttt   1020
tttttttgttt tttcgtttat tcgtcgtagt tttataggag aggggtcgtt tttttggttg   1080
tttttttttt ttgtttttttg tgtgtttttt tgtttttttt cggtttgtag aagggacggg   1140
gtggcggttg ggagcgttag gggaagaggg gtaagagcgt gggttacgag tttgtaggcg   1200
atgttcggtt tcgcgttcgg cgggagttgc gggttaattc gggttcgcgt ttcgtttttt   1260
cgcggtgttt aggggtcgtt cgggttgggg tcgcgttatt tttcgtttag cgtcggcgtg   1320
gtggggtttg cgcggtgtag gattttcgag ggtttagggg gttttaaggc gttgcgggtt   1380
tagtgttttt ttgtttgtag agttttgggt tagtaaggag tgtttcgcgg gttttagggg   1440
aggataatag gtgttttttg ttttattcgg gttttttttgg tgggagttta tacgtacggt   1500
ggcgttattg cgtttatagg taacgttaga gtataggagg ggtgggaggt cggggttcga   1560
gggttttatt tcgtagtaga gtttagagtt taatttaggt tttagggatt cgtttggggt   1620
```

ttagaatttg gtgtggtaga ggtagaaagg gttttcgata atttgttttt atagttgaga 1680
gacgttaatt ggtatttttg agtttttgtt tcgtaggtga aatgggaata tacgaaatta 1740
tttgaggagt ttttattgtt cgttattatt aaatgtggtt tagatttcgt ggggagggat 1800
agttttattt tggaattggg gtttttagga tattttagag ttcggtaaga gggataggga 1860
gaatggggtt tggaagttgg tagttttata tttagtgttt ttcgtatttt tttttttttt 1920
tgtatttttt ggtggttttt gtggttttag ttgtttttag attttgtttt tttcgttttt 1980
ttttaagttg aggtattgag gttgggttgt ggtttgagtt gtatttgtgt tttgttaggg 2040
agtggggtt ttaggtttat cgcgtttttt tttattgttg gttttagttt aatatttggt 2100
aggttttttt ttttaaattg ttggagtaag ttcgcgtaga ttcggtttgg tcgtcgtttt 2160
cgggttgtcg agtggatcgg tcgtttcgtc gttttaaagg ttgtttttgt tttgttttag 2220
tttaagttta gagaaattta agttaataat tttagtcgag ggggcggggc ggcgtttgtt 2280
ggttgttatt gaggttgttg ttattattat tgtggtaatt gacgtagtgt ttttagtcgg 2340
tgatttttta tgaaaggaag tgttattttt tttttattat atttagggtg agtggggcgg 2400
gtagtagaaa gatggggagg gagagaagga aaggggtgtg tgtttttagt agatttttaa 2460
atttgagagt tgtgtggttt tgtattagtt attgtttttt tttgggtaat aggggtttgt 2520
aagtttattg cgtttatttt gtatttagga ggttgatgtt ttcggagtat ttgttgggta 2580
gcgaggtgtt ttggggaggg agtaggatag gtaggttttt gatttttta ttgagtttgg 2640
ttgtggtttg tgggggaagt tttttttttt tttttatttt tttttgttga gaatattggg 2700
tatattgagt ttagtacgtt tattttgtag atggggaaag ggagatatag aattagtgat 2760
taatttggta agaggtgtag ttatgtagtg ttggaatttt ttgttttatt tgttttttg 2820
gtttattttt atttgttttt agtttgatgt ttggtagggt aggggtttat tgatttttc 2880
gtatttattt attgtttggt tttatagtta ttttttagtt ttggttaagt ttcgtgagat 2940
attttaagtt tgtttttagt gatttttaat tatcgttcga ttgtttttat tttttttgttt 3000
ttgtatattt gtttttgttt cgttaggttg gttgggtttt ggttagagtt ggttttttat 3060
ttttttaggt aataggtagt agtttttttaa atggaatttt agtaggtatt aataggtttt 3120
tttttttttt ttaagattat ggagataata ttttttattt aaagatggtg ggtttttggg 3180
gttttttcg atttaggtta gggaagttga tttatagagt gtgggggtta tgtttttttt 3240

<210> 91

<211> 3240

<212> DNA

<213> Artificial Sequence

<220>

<223> chemically treated genomic DNA (Homo sapiens)

<400> 91

```
ggaaggggta tggtttttat attttatgaa ttaatttttt tggtttgggt cggggagggt      60
ttagaggatt tattattttt gaataagggg tgttattttt ataattttga ggaaggagga     120
aagatttgtt gatatttatt gagattttat ttaaaaaatt gttatttgtt atttgagaga     180
gtaggggggtt agttttggtt agggtttaat taatttggcg aagtaaaggt aggtatgtaa    240
aggtaggaag gtgaaggtag tcgggcggtg gttgagagtt attgaagata aatttaagat     300
gttttacggg gtttagttag gattagaagg tagttatggg gttaggtagt gggtggatgc     360
ggggaagtta gtgggttttt gttttgttag atattaagtt ggaagtagat gaaagtgggt     420
taagaggata gatggggtaa ggggttttag tattgtatgg ttgtattttt tattaagttg     480
gttattggtt ttgtattttt ttttttttat ttataagatg ggcgtgttag atttaatgtg     540
tttaatgttt ttagtagaaa aaagtgagaa gagaaaggag gttttttttta taaattatag    600
ttaggtttag tgagaagatt agaggtttgt ttgttttgtt ttttttttaa ggtatttcgt     660
tgtttagtag gtgtttcgga aatattagtt ttttgggtgt aggatgagcg taatggattt     720
atagattttt gttgtttaga gaggggtagt gattagtata aggttatata gtttttaagt     780
ttaagggttt gttggaggta tatatttttt tttttttttt tttttttatt tttttgttgt     840
tcgttttatt tattttgggt gtgatgggaa ggaagtgata tttttttttta tgaaaagtta    900
tcgattggga atattgcgtt aattattata ataatggtaa tagtagtttt aataataatt     960
agtagacgtc gtttcgtttt ttcggttgag gttgttggtt taagtttttt taagtttgag    1020
ttggagtaaa ataaagatag tttttgaagc ggcgaaacgg tcggtttatt cggtagttcg    1080
ggagcggcgg ttaggtcgag tttacgcgag tttgttttag tagtttgggg aggggggttt    1140
gttaagtgtt gagttggagt tagtagtgag aagaaacgcg atgggtttgg gatttttatt    1200
ttttaatagg gtataggtgt agtttaagtt atagtttaat tttagtattt tagtttgggg    1260
gagggcgaga gagatagggt ttgggggtag ttgaggttat aagagttatt agagggtata    1320
gaaagggaag gaggtgcggg ggatattgaa tgtgaggtta ttaattttta agttttattt    1380
tttttgtttt ttttatcggg ttttgggatg ttttaaaagt tttagtttta ggatgggatt    1440
gttttttttt acggagtttg ggttatattt ggtggtgacg gatagtgaga gtttttttaga   1500

tggtttcgtg tgtttttatt ttatttacgg ggtagaggtt tagaggtgtt agttgacgtt    1560
ttttaattgt agaagtagat tatcggaggt ttttttttatt tttgttatat taagttttag   1620
gttttaggcg ggtttttaag gtttggattg ggttttgagt tttgttgcgg ggtgaagttt    1680
tcgagtttcg atttttttatt tttttttgtgt tttagcgttg tttgtaggcg tagtgacgtt  1740
atcgtgcgtg tgggttttta ttagaggagt tcgggtgggg taagaggtat ttgttgtttt    1800
tttttggggt tcgcggagta tttttttattg gtttaagatt ttgtaaataa agggatattg   1860
gattcgtagc gttttgggat tttttgggtt ttcgggggtt ttgtatcgcg taagttttat    1920
tacgtcggcg ttgggcggag ggtgacgcga ttttagttcg ggcgattttt agatatcgcg    1980
gagggacgga gcgcgggttc gggttggttc gtagttttcg tcgagcgcga agtcgggtat    2040
cgtttgtaag ttcgtagttt acgtttttgt tttttttttt tgacgttttt tagtcgttat    2100
ttcgtttttt ttgtagatcg agggaagata aggggatata taagggatag gagaggagag    2160
taattaggga agcgattttt tttttgtggg gttgcggcgg gtgggcggag agataggaga    2220
ggaaaagaga aaagggaaga gaaggaaggg gtaggggggcg agaagtcggg tttttcggat   2280
tatcggcgta gttcgcggtc gaaagaggaa tagtggattt tataaagcgt attgagtaag    2340
ttttttatat tcgcgtagtt tttttttagga aacggcggtc gtagaggaaa gggtcgcgcg   2400
ttttggattc ggagtttgtg ttcggcggcg tcgcggtttt gaaatttgcg ggatgttacg    2460
gggcggaggg gcggttgtcg atagttgtta taataaatta gttgagttta gttcgaggtg    2520
gaaatcgtag taggaagtgg gacgatagag tcgtatcggg ttcggtatat tttaggtaag    2580
agacggcgaa aagagtttcg agataaacgt tcgggaagtt tttggtcgtt aaaatttttt    2640
gttttttttt ttttcgaagt tcgcgcggac gtttagtttt atttttttcg cgtcggtcgg    2700
ggagcgttga agtcgcgggt cgggttcgtt tttttttggtt cgcgtttcga aatttatttc   2760
gggttttttt tcgttttata tagcgtatcg attgttacga gggttggagg ggattcggcg    2820
tcggtatttt gaattttttt gtagggtcgt cgagtttttg ttcgggtcgc gtcgtagagt    2880
cgtttattta tggttcggag aggtttcgcg gcgttcgttt cgagattttt agcgcgcggt    2940
tgttcggatt tcgcgttttt tgagatattg gttcgcggcg cggttcgtcg gttatttttt    3000
cgtagtttcg tttcgtttcg tttttgttcg ttttatttcg ggtttttatt ggtaagttcg    3060
gtttcgtcgc gcggagaagg cggggggcgg ggatcgtttt tgagttcggg gatttttggg    3120
aattgtagtg cgcgtttttc gtttttaggt ttttagattg gagtttttgg aataggtttt    3180
ttatttagag gagcggaggt aggattttta gtaattattg ttgttttgtg ttttagtttt    3240
```

<210> 92
<211> 2155
<212> DNA
<213> Artificial Sequence

<220>
<223> chemically treated genomic DNA (Homo sapiens)

<400> 92

```
tttttttttt tttttatttt tggagggttt tagttattag ttttgaagtt gtagcgagag      60
ttatttattg ttttggtttt tgggattttt tgagtaggag aaaaaaatta gaaaattaaa     120
agagagagag agatttggaa ggaagaagta atggaggagg gagataggggg gaacgagagg    180
ggagtttata ttatagagag agaatttttt aaaggaagcg taggcgttta cgggatagga     240
gcgcggaaga aaagagtaga aaaggaaaag tagttttaag taagagaaag taaggagagg     300
cggataggga ggggaagagt taagagaagt taagttgagg gatttgtagt aattttgaga     360
aataaaagtg aattgtttgt gggcggttgt tgtgatttta gtaaaaagcg gagtttaagg     420
tttagggatt tcgtcgttta tattgtgttt cgttttattt tggatgtttt taattttagt     480
tttaggtata gtatacgagt aggggtagta aggtttggtt agggtcggtg tcgtacggga     540
gttgcgggtt tttgtaatat ttaggtgtgt aggcgttata agaaggagtc ggggtcgttg     600
ttttcgtagc gcggatttcg tttcgtgttg tatacgtttt agaagagcgt gttcggaagt     660
gtttttttcg gtttttttcg tttttaaga tttgtttgta acgtttatat tcgttattat      720
cgtataagta ttagggtgag gaggtttttt tgtaggaatt aatatcgatt ttagtttttt     780
gtttttttaa agtttttttt tgacgatggc gagatttata ttttaagtta gaagaaaata     840
aaatttgtt tattttgggt ttatgattgt ataaaattta aaatgttgtc ggtatgaaga      900
tgtagttgga agattttaga aatttagtat taggaggtag agattttata gtgtttagag     960
atgagttttt aaataaatta agaattacgt agtgggaaat taaggaggag ggtttgaggg    1020
gtcgatttta gagggataga tagagaaatc gagagcgaaa tatttaagga agtggaataa    1080
atcgtcgtag ttaggcgttt ttgtttttc ggaaattcga aattcgttta aattttattt     1140
taggcgttta cggtattagt cgtagggttt cgtattatag gggttgttat tttttgtttt    1200
cggagttttt acgtcgagat ttattatttg ttggtttttt tttttttttt tttttttgtt    1260
tttttatttt ttaaatataa gataaaaata tattttattt attattatag atatatatat    1320
tttattttta ttatataaat ataaaatatt taaatattta aaatatttag aataaagtta    1380

gtagtttgga tttagtgata tttattcgcg tttgaatcgt ttattatttt ttttattatt    1440
tgtttagaaa aaaaaaaaag atgaaaaaga gggggagagt tttcgacgta aataatttat    1500
aataaaatta ttattttaaa aaagtgtttt tcgtttgttt tagatggatg aatatatttt    1560
agatattgcg gtatttcgta gtttgggttt gtgattttgg ttaaaatgtg ggtggtcgtg    1620
gttgtaggga cgatcgtcgt tgcgtagggt aaggaaagag ggtgtcgcgg ttcgtgtttt    1680
gatttgggat tattgaaatt ttaagattgg ggaggcgagg ggagtgtttt ttttattaat    1740
tatataagac gttatttgga tttcgagatt attgttagag gaggttcgat tatttagcgg    1800
cgtttttgtt tttttttcg taaagaattg tttagaaatt tagacgtttt ttgcgtgtag     1860
gaagggtttt gtgaattcgg gtgtttggga gaggaggtaa tgagtgttga ttcgtttttt    1920
aaatcgagta attgtgttcg aagttacgcg tttgggaggt cgtagggtga agcgtcggtt    1980
gcgtaggtta tcgtcggtag tcgttttgtt ttttttgga ggagtgggaa gttgtcggtt     2040
atcgtttcga ataggttcgg agaaaagatt ttgaattttt tttgattagc ggcgttattt    2100
ttttttttgt ttttttttta ttaaattttt gtttaaagtt ataatattta gaatt         2155
```

<210> 93
<211> 2155
<212> DNA
<213> Artificial Sequence

<220>
<223> chemically treated genomic DNA (Homo sapiens)

<400> 93

ggttttgggt gttgtaattt taaataggaa tttaatgggg gaaaagtaaa ggaaaaggta 60
acgtcgttaa ttaaaggaaa tttagaattt ttttttcgag tttgttcggg gcggtggtcg 120
atagtttttt atttttttag gaaagagtaa agcggttgtc ggcggtagtt tgcgtagtcg 180
gcgttttatt ttacggtttt ttaggcgcgt agtttcgggt ataattgttc ggtttggaaa 240
acgagttagt atttattgtt ttttttttta aatattcgga tttataaaat tttttttgta 300
cgtaggaaac gtttggattt ttgagtagtt ttttgcggaa ggggaggtag ggacgtcgtt 360
gggtggtcgg gtttttttta gtagtagttt cggagtttag atagcgtttt gtgtgattga 420
taggaaagat atttttttcg tttttttagt tttagggttt tagtggtttt aaattagaat 480
acgaatcgcg atattttttt tttttatttt gcgtagcggc gatcgttttt atagttacgg 540
ttatttatat tttaattaaa attatagatt tagattgcga ggtgtcgtag tgtttagaat 600
gtatttattt atttgaagta agcgaaaaat atttttttaa aatagtaatt ttattgtaaa 660
ttatttacgt cggaaatttt tttttttttt tttatttttt tttttttttt aggtaaatag 720
tgaagaaaat aatgaacgat ttaaacgcgg gtaggtgtta ttgaatttag gttattaatt 780
ttgttttgaa tgttttggat atttggatgt tttgtattta tgtggtgaga gtgaaatata 840
tatatttatg atgataaatg aggtgtattt ttgttttata tttgaaggat aaaaaaataa 900
aagaagaaaa ggaagaaaga gttagtaaat gatgagtttc ggcgtgggga tttcgggggt 960
agagggtggt agtttttata gtgcggggtt ttgcgattgg tatcgtgagc gtttgggatg 1020
aggtttgggc ggatttcggg ttttcggaga ggtagagacg tttggttacg acgatttgtt 1080
ttattttttt gagtgtttcg ttttcgattt ttttatttgt ttttttgagg tcggtttttt 1140
agattttttt ttttaatttt ttattgcgta atttttagtt tatttaggaa tttatttttg 1200
ggtattataa agtttttgtt ttttaatgtt aggtttttag agttttttag ttgtattttt 1260
atgtcggtag tattttggat tttgtgtaat tatgggttta gaataggtaa ggttttattt 1320
ttttttgatt tagaatataa gtttcgttat cgttaaagag aaattttgaa ggagtaggag 1380
attgaaatcg atattaattt ttgtaaagag atttttttat tttggtgttt atgcggtggt 1440
gacgggtgta gacgttgtaa ataaattttg ggaagcgagg agggtcgggg aggatatttt 1500
cggatacgtt tttttaaagc gtgtgtagta cggggcgaag ttcgcgttgc gggggtagcg 1560
gtttcggttt ttttttgtag cgtttgtata tttgggtgtt gtagggattc gtagttttcg 1620
tgcggtatcg gttttagtta ggttttattg tttttattcg tgtattgtgt ttgaagttga 1680
agttggaggt atttaaaata aggcggggta tagtgtgggc ggcggagttt ttgagttttg 1740
agtttcgttt tttgttgaga ttatagtagt cgtttataga tagtttattt ttatttttta 1800
aagttgttgt agatttttta gtttggtttt ttttggtttt tttttttttt gttcgttttt 1860
ttttattttt ttttgtttga aattgttttt ttttttttat tttttttttt cgcgtttttg 1920
tttcgtaaac gtttgcgttt tttttaagga gttttttttt tgtggtgtgg gttttttttt 1980
cgtttttttt gttttttttt tttattattt ttttttttta ggtttttttt ttttttttgg 2040
ttttttggtt tttttttttt gtttaagggg ttttaaaggt taaggtagtg ggtggttttc 2100
gttgtagttt taaggttggt ggttgggatt ttttaggggt gagaaggaag ggaga 2155

<210> 94
<211> 4513
<212> DNA
<213> Artificial Sequence

<220>
<223> chemically treated genomic DNA (Homo sapiens)

<400> 94

agtgtatggg gaaagattaa tttaattgtt tatttgtggt tgggatagtt taggggggtgt 60
gtttatggtt gatttagggg tgtgtatatg gttgagttgg gagttttgtt ggttttttttg 120
aggattgagg gtgaattttg tttttttgttt taaatttttt tattttattg tagtaatagt 180
tttatgttgt atataatagt gtaaattttt ttaaaaagga aagtataaaa ataaaagttg 240
taatttaaaa gtttgaataa ttatttgttg tttaggaaat ttaatgaaat gatatgtttt 300
tttagtagga agtaaagttg gtttttgttt tttgtttttt ttgttgtttt agtttataaa 360
atatgtgtat tttatagttt tagtattaaa tatttataat tttatgagaa atgaatgaat 420
gtttttattt ataattgtgt ttattaaaat tgtgaatatt tttattttttg tatttttgtg 480
tgttgaaatt tttgaaggtt atattaaata aaataaaatt tttttattat aaaatattga 540
aggttttagg tggaatgatg agtttttttaa gtatgtttga gtttagagga attgtttggg 600
tattttttttt agtatttagg tgtttatggt ttttgttttg tttgggagta ggtgttaggt 660
gtttttgtag tgagaggtga ttattatttt tttttaaata agatagtttg gatatgttat 720
atgtgagtat agaataatta aaagaagtat atattttgtt attaggagga agtgtttaaa 780
attatggtgt tattttagtg attatatttt tttaaatttt gtgaagttta ataggttttt 840
ttttggttgg gtgtttattg gttttgttat ttgtgttgga ttgaggtgtt ttttttttggg 900
gataggaatt ttttatttta ttgagataaa gagatttttt tttttaaagt gttatttttg 960
tttattttttt gttgtttttt ttttaaaata aaagatttttg tataattttt taagtaggat 1020
tgggggaggg attagttttg tttgtgttga ttgtgttttt ggattttgtt tattaggaag 1080
tgtttggggt ttttgagtga gtgatttatg tgttgaattg ttgttttttt aaatttttat 1140
ggtttaaaaa ttttttttttt ttttttttgta aaagaaattt agagattggt gatttgttat 1200
tgaagatgat tttttaggtg tttttttgttt ttttaggtag ttgggttgtt ggtgttttta 1260
ggtggaagtg gggtttttgg gttgggtttt tgttttgtta ggaagtggtt tgggggttgt 1320
tttttgggat ttttagaatt ttggaaggag agagttgggg ttttattgtg gagatttttg 1380
agttttttgtt gtttgggttt gagttgtgtt ttagggttat tgttgatggt gtgagattat 1440
gtattttttg tgaattttga tttttgaata gtttagaaag tttgtagatt aaaaattaaa 1500
taaaaaatat tggtataatt tttattgttg tattggtttt tttttttattt taataaaaat 1560
atggtaatat gaagtgatta ttttttttaaa aatttgtttt taaagtataa tattgaaaag 1620
gggtaaggta tagttttgta aggtaatgag tttttttgttt ttagaggtgt ttaagtagat 1680
gttaggttta tagtggtatg gagagtgttt ttttttttttg gaattggatg agtttttaga 1740
tttttgaaat gttgagtttt ttttttttttt tttattttta tgtttattat attttaattt 1800
aatagtatat aatttaagtt gttttttttat ggagagttgt ggttgtgtta tttgtttgtt 1860
tttgttatgg agattggagt taggtttgta gggaagttgg tgtgtttttt ttaagttttt 1920
tgtttgttgg ttttttttttgg gtttttttggg ttttgtgtgt tgtggatttg gatttggatt 1980
gtagatattg ttttatgatt gggtttttgg ttagattgtt attttatagg aggagggttt 2040
tttaagtgtg tagaagagga aagtttattt ataaagtttt tgttttttttg agttgaatga 2100
tttttgtgtt tgaggtttag ttgtgttttt gttgggttta ggttgagttt attttatatt 2160
gttttatagt tgggtagaga ttttgtgagg gtttgggagt tttggttttg gaggatttgt 2220
tgttttgtag gtttgtttaa gtaggtgttt ttgtgagagt gtgggataga gagggtttgg 2280
tggggttttt tggtgttgag agtttttttt ttttgttgta tttagttttg ttaatggggt 2340
tggatgagtg ggggttggag ttagaatttt agggtttata tttgttgata gtagtaatgt 2400
tgatttgttt tgtttttttt ttaaggagtg gagggaagga gttatgtgaa tgattttagt 2460
ttgattatta agagggtgtt agtattttttg tttttttaga gtgaaaaggt taaattgatg 2520
ggggttttta tattgttgga tataggtgtg tttgggtgtt tagtgtgtgt aatatatgtt 2580
tagatatttt atatgtaaat atgtttgttg ttgtggagtt ggttgggagg tagtgtttgt 2640
ttgttggggt tttgttagaa gttattttgt tttttgttgg tttaatattt gtttttttgtg 2700
gtttttgttt gttttggtgg tttttgaagt gtttttggga gggttttggt tttgttgaaa 2760
attgtagttt gttaggttga gttgttgtgt gttgggaggt taatagtgat tgatgataaa 2820
gggttaaggt gtaattttta aagtggggat ttgttgggtg aggtagaaat tagtttttgg 2880
ggagatgggt tttttttttt tgatgtgttt ttgtatatat tgttgttttg gtgttgggtt 2940
tttgggtggg gatttttttt ttgggtattg tggttttatt tggtggagag ttaggttttt 3000
tttaggtttt taagtaggga gtggatgtgg gtggtgggga agggaagttg ttggtgggat 3060
ggagagtggg gtttttttgga ttttgggttg ggtaggtttg gtgttggtag ttttgttgta 3120
tagatttgtg agtgatgttg gtggttgtat tgttgtgtgg tgtgaatttt aagtagatta 3180
tttatgtttt tattaataat tttaaatatt ttattatatt tatgttgttt gtttagagaa 3240
aataaaaggt tatttttgaa attgtttgaa atgtaataat atttattaaa gtgtttttta 3300

```
attatgtgat ttaattaagt gtggtgtgtt tgtgtttttg gtttttgttg gaaaggttgg     3360
tgatgatggt tattattatt gttgttgtta gtggttttgt gatagtagtt ggttagtttt     3420
ttagtttagg tttagttttt ttagagagat ttggggagtt ttattttgag ggatggggtt     3480
ttttggaatg attttgaagt tttggagatt aggtgtggtt taggtttttt gttttttttt     3540
agttttagtt ttagttttag ttttagtttt gggtaaattt ttttaggttg agggtaggat     3600
tttttttaga tttttggggt tttttgtgtt ttttggggat gtttgatttg gtttttttagg     3660
agggggaatt ttgggttttg agtttgggtt ttgttttgtt ggttgttttg ttttttttag     3720
ggttgagttt tgtgtaatgt ttgaagtaga aataaagagt tgttgagatt tgtttttttgg     3780
gttgtttttt tatttttttta gtttggaagg tgttttagag aggttttatt tttagttttt     3840
atggagattt aggagggaag aagagttggg atttggtagt tttgaggggt tgttgggttt     3900
gtggtgtttt ttttgttgta gaggggttgg gtgggagatg taggtgggaa ggattttgtt     3960
ttagttgaga gtttttttgtg gggttgagga atggtgagga ttattgtttg tattaattgt     4020
ttggaagaaa ttgggtggtt agttgtttga ttttatttgt tgagatgtga aatttggttg     4080
tgtggtttgg gtgtgtgttg tttgtgtgga aggagttgtt tttttttttt tagtgaatgt     4140
atttggggtt tttttttttt gttaagtttt ttgtttggaa ggggattggt aggttttgtt     4200
gttgttgtgt gttttttttt tttttttagt ttttattttt tttaattaat tttttttttt     4260
tttttttttt tttttttttt ttttggatgg gtaattatag gttttgttgt taagtgtgtt     4320
gtgaggagtt gttgtttgtt tttgttggtg tttttttttg tggtagtttg gttgagtttg     4380
tttagtttgt ttaggatgtt tttgatttgt gtgtttagtt ttatttagtg gttgtgggat     4440
gttgtatttg agtttttttt ttaggttaaa attttgtttt gaggtttttt tgagagggtt     4500
ttattttttta ata                                                       4513
```

<210> 95
<211> 4513
<212> DNA
<213> Artificial Sequence

<220>
<223> chemically treated genomic DNA (Homo sapiens)

<400> 95

```
tgttggagaa taaaattttt ttagaaaaat tttgggataa agttttggtt taaaggaaaa      60
atttagatgt aatattttat ggttattgaa tgaaattgag tgtgtgggtt agggatgttt     120
taggtaggtt gggtggattt ggttaggttg ttgtggggag gggtattgat gggaatgggt     180
agtggttttt tgtagtgtgt ttggtagtgg agtttgtgat tatttattta gagagggaag     240
aaaagagaag ggaagaaagg aaagttagtt ggagggggta gaggttgggg aaaggagggg     300
ggtatatggt gatgatagag tttgttagtt ttttttaga taaaggattt ggtgggaggg      360
gggaattttg aatgtgtttg ttgaaaggag ggaggtggtt tttttgtgt aggtagtgtg      420
tgtttggatt gtgtggttaa gttttatatt ttgatagatg gggttgagtg gttggttgtt     480
tggttttttt tagatggttg gtgtaaatag tgatttttat tatttttga ttttatagag      540
gatttttggt tgggataggg ttttttttgt ttgtgttttt tatttaattt ttttgtggtg     600
gagaaagtgt tgtaggtttg gtagtttttt agggttattg ggttttggtt tttttttttt     660
tttgggtttt tgtggaggtt gggggtgggg ttttttggg atgtttttta ggttgagggg      720
gtgaaaaggt ggtttaggag gtagattttg gtgatttttt gtttttgttt tgggtattgt     780
gtggggttta gttttggagg gggtggaata gttaatgggg tggggtttgg gtttaggatt     840
tggagttttt tttttagaa agttgggttg ggtgttttta ggggatatag agggttttaa      900
gggtttggag aaagttttgt ttttggtttg gaagagtttg tttggggttg gggttggggt     960
tggggttggg gttggagaag ggtagagggt ttgggttgtg tttggttttt gggattttgg    1020
agttgttttg gggaatttta ttttttaaag tggggttttt tgagtttttt tgaggaaatt    1080
gggtttggat tgaagagttg gttggttgtt attatgaggt tattaataat agtagtaata    1140
ataattgtta ttgttagttt ttttagtgag agttgagagt gtgggtgtat tatgtttgat    1200
taaattatat aattaggagg tgttttaatg aatattatta tattttaggt gattttaaga    1260
atggtttttt atttttttg ggtaggtagt ataaatataa tgaagtgttt gagattatta     1320
ataaaaatat gaatggttta tttgggttt gtgttgtgtg gtaatgtggt tattagtatt     1380
gtttataaat ttgtgtaata gagttgttag tgttaggttt gtttgattta gggtttgggg    1440
gattttattt tttgttttgt tggtggtttt ttttttttat tatttatgtt tgtttttttat   1500
ttggaggttt ggagagggtt tagtttttg ttgaatgggg ttgtgatgtt tggaagaggg     1560
gtttttgttt ggaggtttgg tgttggggta gtagtgtgtg taggggtgtg ttgggaaggg    1620
gggatttatt tttttggagg ttgattttg ttttatttgg tgaattttg ttttaggaat      1680
tgtattttgg tttttgttg ttagttgtta ttaattttt agtgtgtggt ggtttagttt      1740
ggtaggttgt agtttttggt ggagttggga tttttttggg ggtgttttgg gagttgttga    1800
ggtgagtgga gattgtagag ggtggatatt aaattagtgg gagatagaat gatttttagt    1860
```

```
agaattttag tgaataaatg ttgtttttg gttggttttg tggtggtagg tatatttgta 1920
tatagagtgt ttgggtgtgt gttgtgtgta ttggatgttt gggtgtattt gtgtttagtg 1980
gtgtaggagt ttttattgat ttgatttttt tattttagga aaatagaaat gttggtgttt 2040
ttttggtggt taggttgggg ttatttatat ggttttttttt ttttgttttt taggagaaag 2100
gtagggtggg ttggtattgt tgttgttggt gggtgtagat tttgggattt tgattttagt 2160
ttttatttat ttagttttgt tggtagagtt gggtgtagtg gagaaggagg gtttttagtg 2220
ttaaggagtt ttgttaggtt tttttgttt tgtatttttg tggggtgtt tgtttgggta 2280
ggtttgtagg gtggtgggtt ttttgaggtt aggattttta gatttttgtg gagtttttgt 2340
ttggttatgg agtagtgtgg agtaagttta gtttgggttt ggtggggata tagttaggtt 2400
ttgggtgtgg gggttgttta gtttgaagga gtaaggattt tataaatgag ttttttttttt 2460
ttgtatattt agggaatttt tttttgtgg ggtaatagtt tggttggggg tttagttgtg 2520
gggtggtgtt tgtagtttgg gtttagattt gtggtatgta gagtttggga ggtttgagag 2580
gaattagtgg gtagaggatt tgggaaaggt gtattgattt ttttgtaggt ttgattttgg 2640
ttttatggt gaaagtaggt aggtggtata gttgtggttt tttatgaggg agtagtttag 2700
attatatgtt gttggattaa aatgtaataa atataggagt aaaaaaaaaa aaaaaaattt 2760
agtgttttaa gggtttgagg atttatttag ttttaaagag aaagaatatt ttttatgtta 2820
ttgtaagttt agtgtttgtt tgaatatttt tgaggatggg gaatttatta ttttataaag 2880
ttgtgttttg ttttttttta gtgttatatt ttgaaaatag atttttaaaa aaatgattgt 2940
tttatgttat tgtattttta ttaaaataag agagaggtta atatggtaat agaagttgtg 3000
ttagtatttt ttgtttggtt tttaatttat gaatttttg aattgtttag aagttggggt 3060
ttgtggggaa tgtgtggttt tgtgttatta gtgatgattt tggggtgtag tttgggttta 3120
ggtaatgaga atttgggagt ttttgtggtg agattttagt ttttttttt tagggttttg 3180
aaaattttga ggggtagttt ttagattgtt ttttggtagg gtagaggttt agtttagagg 3240
ttttgttttt gtttgggaat gttaatggtt tagttgtttg gggaaataga aaatgtttaa 3300
aaagttgttt ttggtaatag attattagtt tttgagtttt ttttataggg agaagaaaaa 3360
aaatttttaa gttatgaaag tttggaaaaa tagtaattta atgtgtagat tatttgttta 3420
aagattttaa atattttttg atgagtggaa tttagaggtg tggttagtat aggtgaagtt 3480
gattttttt ttaattttgt ttaggaagtt gtataaagtt ttttgttttg ggggggaaat 3540
aataaaaggt aaataaaaat gatattttga aaaggagagt ttttttgttt tagtggagta 3600
aaaagttttt gtttttaggg gaaagtgttt tggtttagta tgggtggtag ggttagtgag 3660
tatttagttg gagggagatt tgttgaattt tgtaaaattt aaaaaaatgt agttattgga 3720
atagtattgt agttttgaat atttttttt ggtaataaaa tgtgtatttt ttttggttat 3780
tttgtgttta tatgtgatat atttaaattg ttttatttaa aaaaaaataa taattatttt 3840
ttgttataaa ggtatttggt atttgttttt aggtggggtg aaggttatga gtatttgagt 3900
attggggagg atgtttaggt agttttttg gatttagata tgtttagaaa atttattatt 3960
ttatttggga tttttagtat tttataataa agagattttg ttttatttaa tgtaattttt 4020
aagaatttta atatataaaa atgtgggggt gggggtgttt ataattttga taagtatagt 4080
tgtaaataga aatatttatt tatttttat aagattataa atatttgata ttggaattgt 4140
aaaatgtata tgttttataa attaaaataa taaagaaaat aaaaaaatag aattaatttt 4200
gtttttgtt aaaaaggtat gttattttat tgagtttttt aagtagtaga tggttattta 4260
gatttttaaa ttataatttt tgtttttata tttttttttt taaaaaggtt tatattatta 4320
tgtataatgt aaaattatta ttgtaatgaa ataaagaggt ttaaggtaaa gagtgaagtt 4380
tatttttagt ttttagggag attagtggga ttttagttt agttgtgtgt atatttttgg 4440
gttagttgtg ggtatatttt ttgggttgtt ttagttatag atggatagtt ggattggttt 4500
tttttatat att 4513
```

<210> 96
<211> 2280
<212> DNA
<213> Artificial Sequence

<220>
<223> chemically treated genomic DNA (Homo sapiens)

<400> 96

tagtggtgag tatttgttgt tggtttttta gggtaggtag tattgttgtt ttaggtgtgt 60
ggagttgggg agaaaaaaat tgaatatgtg tgttatttag ggtttttaga taagtttttt 120
tgtgtgtata tatattttga attggagttt agaaggttgg atttgtgttt tggggttgag 180
tttttttattt tttgttttta gtttggaaga ttttagtttg tttgaaggtg gtgtttgatt 240
tgttggtgga tttggtgggt gtttgttttt gatatttggg gatgaggtgg gagtgttttg 300
agtgttggga agttttgttt tgtgagtttt tggtagtgat atgtgggagt gttgggtttg 360
gtatttttga tgtttttgag gttttgggtg gttgttttgt ttttttgggg gtttttattt 420

tttaagtggt tttaaatgat tgaggatggg ttttttgtgt tgttgtagtt gttatttttt 480
ttgggaaagg ttagtgattt tggatgtgta ggttttgaaa attaaggttt agatttatat 540
tgagtgttta aagattgtat ttgaattttt tttgttggtt attgagggag agaaaggaat 600
gtttagtttt atttatattt atagaaagaa gtgttgaggg tgggggaaat gtagttttgt 660
tgggtgagtt gggataggtt ttttgtttgt tttttggttg ttgttttttt ttagttgaat 720
ggggagtgat ttgttttggg tgtggttttt ggggttgaag attgaggtgt agttttattt 780
ttggtttggt agtggtttgg aagagagagg gaaaggagga atatttattt ggttaagaga 840
tgttgttaga gtttttaaag ttggtggggg atggaggttg gggttttgga tttttagagt 900
gtttgtagtt gggtttttgg gtttttttggt gtggggaatt tgattttgta gtttttgtta 960
gggtgtggtg gggttgtaag aagggaggaa ggagtgggat tagagttttt gagggtgtgg 1020
gtggtgtgta tttagttttg ttttttttgtg tgttttgttg tttgtttgtt tttttttgttg 1080
tattgggtgg ttttggtttt tgtgtttgtt tgtttttgat gtttttgtgt ggttttgtgt 1140
tgtttgtggt tagttttgtt ttgttttgta aggagtgtgt tgttttttta attgaaatgg 1200
gattaagatt ttagggtttt tttgtttttt tgttttgagg tgataggatt ttagttaata 1260
gattgaaaat gtgaaaagta tttgttgttt tttgttttaa gtgtttttgt ggaaggatta 1320
tttatttgga aaagtagaat aaattttgtt gttgtttttt ttttggaggt gtgtttattt 1380
tagagttttt agatttattg gtatttgtta tatttttttt tgggttttat aggtaggatt 1440
ttaagaatga tatgggggta tttgggtgat ttgtgggttg taggttttta agaggttgtt 1500
tttttgtgta tggaagtgtt gtagttttat tttagtggaa gtaatattaa attttatttt 1560
agaggtttgt tttgtaaaga atttagagtt tattatattt gatattattt taaaataata 1620
tttgtttgtt ttaagaagtt gggagaaatt gataaaagaa aagttgaaat ttatgttttt 1680
tatttttatt ttaggagata attttgatta atagtttggt ttgtttttta tttttatttt 1740
ttaaaaataa ataaataaat aaataaatta aaatttatta tatttttaga ttgtgtagga 1800
ttatgtagga ttttgtaaaa ataaaaatgg gattatattt tatttgtttt tgtaatggtt 1860
tagtttttat tagtaattat tttttattag tatagtagaa ttaattttat tttagtatta 1920
ttattattat tttgagatag ggttttattt tgttatttag gttggagtat agtggtatga 1980
ttttggttta ttgtagtttt tgttttttgg gtttaagttg tttttttgtt ttattatttg 2040
gtttttttttt tttaattagt tgggattata gttatgtgtt attaagtttg gttaattttt 2100
gtatattttt gtggagatag gttttattat gttgtttagg ttgattttaa attattgaat 2160
ttaagggatt tatttatttt ggttttttaa attgttggga tggtagattt gagttattat 2220
attggtaaat ttattttttta taatggttgt gtggtataga atttttattt tataggtgtg 2280

<210> 97
<211> 2280
<212> DNA
<213> Artificial Sequence

<220>
<223> chemically treated genomic DNA (Homo sapiens)

<400> 97

```
tatatttata aaatggaaat tttgtattat gtaattatta taaagaataa atttgttggt 60
gtggtggttt aggtttgtta ttttagtaat ttgggaggtt aaggtgggtg gatttttga 120
gtttagtagt ttgagattag tttgggtaat atggtgaaat ttgtttttat aaaaatatat 180
aaaaattagt tgggtttggt ggtgtatgat tgtagtttta gttagttgag gggaaggggt 240
tgggtggtga gatgggagga tagtttgagt ttaggaggta gaggttgtaa tgagttaaga 300
ttatgttatt gtattttagt ttgggtgata aagtgagatt ttgttttaaa ataataatga 360
taatattaaa ataaggttaa ttttattata ttgataggga atgattatta gtaggagtta 420
ggttgttgta gaaatgagtg aagtataatt ttatttttat ttttataaaa ttttatgtaa 480
ttttatataa tttaaaagta tagtgggttt tggtttgttt gtttgtttgt ttgtttttaa 540
ggaatggggg tagggagtaa attaaattgt tgattaggat tattttttag agtagaaatg 600
ggaagtatgg attttaattt ttttttattt gatttttttt aatttttttaa aataagtaag 660
tgttatttta aaatagtgtt aaatatagta aattttggat tttttgtaaa ataaattttt 720
aagataaggt ttgatattgt ttttgttaaa ataaagttat gatattttttg tgtatgaaaa 780
aatggttttt tgaggatttg taatttatag attatttagg tgtttttata ttatttttga 840
aattttattt gtaaagttta ggaggaagta tagtgaatgt tagtgggttt aggagttttg 900
gggtgagtat gtttttggaa ggaaaatggt aatagaattt attttgtttt tttaagtaaa 960
tgatttttttt atagaggtat ttgggataag aggtgatggg tattttttat gtttttgatt 1020
tattggttaa gattttatta ttttaaaata aggagatgag gaaattttgg gattttgatt 1080
ttattttgat tgagaaaata atatgttttt tgtgaagtga aatggggttg gttgtgggta 1140
gtgtggaatt atgtgaaggt gttgggggtg ggtgggtgtg gggattaggg ttatttggtg 1200
tagtaaaggg ggtaagtagg tggtagggtg tgtggggaga taaggttgag tgtatgttgt 1260

ttgtgttttt gaaggtttta gtttattttt tttttttttt tttgtagttt tgttgtgttt 1320
tggtgaaggt tatagggtta ggttttttgt attgaggagt ttagaagttt ggttgtgggt 1380
gttttgaaga tttgggattt tggtttttgt tttttgttag ttttagggat tttggtggtg 1440
tttttagtt gggtagatgt tttttttttt tttttttttt ttaagttgtt gttaggttgg 1500
gggtgaggtt gtattttagt ttttagtttt gaaggttgtg tttggggtgg gttgtttttt 1560
atttagttaa gaggaagtag tggttggggg gtaggtgagg aatttgtttt ggtttatttg 1620
gtaagattgt gtttttttta tttttggtgt tttttttttgt gaatgtgaat ggaattaagt 1680
gttttttttt ttttttttaat ggttggtgag gagaatttaa atgtggtttt tgggtgtttg 1740
gtatgggttt ggattttggt ttttagagtt tgtgtgttta aggttgttag ttttttttga 1800
aggaagtgat agttgtggtg gtgtgggagg tttatttttg gttgtttaaa gttatttaag 1860
gaataaaagt ttttgagaga gtaaaatgat tatttgaaat tttggaagtg ttgggggtgt 1920
tgggtttaat gtttttatgt gttattgttg gggatttgtg aggtgaagtt ttttagtgtt 1980
taaggtgttt ttgttttgtt tttaggtatt aggaatagat atttattagg tttattagtg 2040
ggttagatgt tgtttttggg taggttggga ttttttgaat tgggagtgga gagtaagggg 2100
tttgattttg ggatgtgagt ttggtttttt ggattttggt ttagggtgtg tgtgtgtgtg 2160
gaggggttta tttgggggtt ttgggtagta tatgtgtttg gtttttttttt ttttgatttt 2220
gtatgtttgg agtggtaata ttgtttgttt tagaaggtta gtggtgagtg tttgttatta 2280
```

<210> 98

<211> 3640

<212> DNA

<213> Artificial Sequence

<220>

<223> chemically treated genomic DNA (Homo sapiens)

<400> 98

```
aatttttaga aattaatttg ttataatttt atgatattgt aatatttttt ggaagagata     60
tatgtggatg tttttatagt aagattttgt gataagtaat ttttatgttt gtattaaaat    120
aattagattg ttttgtgatt ggtttagtta tttagtttaa ttttattatt ttaggaggaa    180
attaaatttt gttagtatta atatatttgt attaaaataa gaatttatat aaatttattg    240
atagaaggta ggaatttatg attgtgtatt aaatttattt atataagaaa tatttttttt    300
tttattggat tttatgagat tgatattttt tatgtagaaa taatgtggtt tttttttttt    360
aaagatgttt atttatttta tttaaataga gatataatga gagtattatt taataaatat    420
ttgttaaatg tttttttttag tattataaga aatgtatttt ttaataatta taattttagt    480
aatattttttt ttttgttgtt attttaggtt gggggttatt taattttatt aagagtgtgg    540
aaattttgtt gttttaatgg ggattgaaag gaaattagtt atttttttatt taaattatta    600
aggtttttag attgatttaa aatattaagt tggtgtttaa atattttaat aaatggattt    660
tataaagagt ataagtaatg ttatttgagg aataaggttt taagttgagg agtaggatat    720
tggagagttt tttttttaat tatttatttt tattagattt gaggattttt tgttttataa    780
gaaaattaga aataaatttt attggtatgt tttgtaagaa tgattttttt tgataatttg    840
gggtagtagt atttgtttga ttagttgaat ttgttatgaa tgtggaaata atgatattaa    900
aatagtttgt gtaataattt ttttaaagat tttgtagatt agagatgatt atagattttt    960
tttagtgtgg attaaaggga ttgaattgaa tgttttagtt taatgattta atttttgtta   1020
tatttatagg gatgtgaatt ttgattttat aagtaggtgt gtatgtgtat ttagatattt   1080
atataaagtt gtgtggaggg atgaaaagat taattatttg attgatgagg ataggtttga   1140
ttttttttgat tattttagtg tgttagtgta tatttttggt tgggtttagt gttttaagaa   1200
attttggaat tttagttgtt aatttttgtt tttttattat gatttttttaa agatatttta   1260
tttgtttatt ggggtgaaga gaaatgggat taggtgttag ggtggtggga ttttgtttag   1320
ggttttgatt ttgtgttagg gttttagatt agttggtttt tgaaggtttt tttatttatt   1380
ttatataaga ggaaataaag atttttttagt ttaaggtttt agggttgttt tttgattttg   1440
gttagtttgt aggaagagga aataataaaa taaaggaatt gttaatttgt tgggtattat   1500
atttttttagt tttaattttt gattttggat tgttagggtt attttttttta tgttgatttt   1560
gtttttttta aatgaaaata tgttaataaa agtatatttt ggatataaaa tttaagtatg   1620
tatttttttt ggggaggtta gagttgaggt gtattttgga agatgagaat tttgttttta   1680
tgaattgggt aatatttagg tattgttagg tattttgata gatttttttag atattttttt   1740
tttttttttt tatatttttt ttttattaaa atagttattg ttttgaaatt tattagaata   1800
atgatgtttt aaaaataaag gtgtagtaag tatttttttt tttgttgttg tgggttgaat   1860
tatggatgtt tgtgggttgt ttagttttga tggtttgtag gggtgtgtg ttgtagttgt   1920
agtatagttt ggttattttt agaaagggag ttgaatggag ggaagtaggg agtgtggagg   1980
gtttgaggtt tgtagataag gagaggtgta ttttgggatt tgggtttttt gttgttataa   2040
tataattgtg ttattgttgg tattgtttga tttaagtgtt ggtggtaagt ggtgatgttg   2100
```

gggttgggtt tttagtaatt gttgtgtttt gggttaggtt ggttgtttta gttattggga 2160
tttttttttg gatgttttta gggtgatagg tgttgtattt attgatggga tagttgtata 2220
tttttgaata ggtagtggag tttgtttttg gtaggtattt tagttgtgtt gatattaagg 2280
ttgttatata atagttatta gtttttttta agttttagaa gtaggttttt tttgttttag 2340
ttatagtggt tttagttgtt gttatgagtt tatttttat ttttaagtgt attttttttt 2400
ttttatttgg gtttatgttt gttatataga gagaattata taggggggaat tatggtttta 2460
tatttttgag gggatagata ttggttgtga gataggtatt atgtagagtt ttttggtgat 2520
tgtttgtagg agtgagattt tttttggttt tgtagttggt taggtgtgtg tgtgtgaggg 2580
tttttagttg attattggga tgtattgtta tttttttggtt tggtgggttt tgggattttt 2640
tggtttttgt aggaggttat tttaggtttt tggaggaggt gtttttagtt ggtggtgttt 2700
tttgttttgg gtttagaggt ggatatggtt ggttgtgttt tgttggtttt ttgtttgtgt 2760
tgtagtgggt tgggagtagt tgtgtgatat tagatttata gtgttaagat gtgaagtgtg 2820
aggaaattgt tgtgtttgat tttttttttt ttaattttttg gatttaggaa tgtttttagt 2880
ttttgtgtttt tatatgttta gttttggttt ttttttggt ttagaagttt ttaaggattg 2940
aagggttttg tttagggttt tgtatttttg tttgattttt atggtttaga aaagtagggg 3000
gatatttgaa atgttatttt gggataaata ttaataaaaa agtaaatgga tttgtgtagg 3060
ggttagttat taattaatta ggttgtaagg ttatttaggg taaatatagt ttaggttggg 3120
ttgggtgaga tttttttatg gttgtttttt gattgaattt tttttttttt ggtttaggtt 3180
ttttttaggt tgttttgggg taaatattgg atgggaaggg ggtgttgtta atttttttgt 3240
ggggtttgga ggttttttttg tttttaagtt ttgtagggta gggttggagt gtttaatatt 3300
tatttttgtt tgaattttgg gtttgtgtgt tgtttttttt tgggtttgtg gtgttgtgtt 3360
tgttgttggg tgtgtgttgt tgtttttttt tgagttggta gtttttgttg tgtggttgaa 3420
gtttttttgga atttttaatt ggaaattaat tttggttttg atagatgttt atgttagagg 3480
tgtgttattt atttatattt tttgttttat tttggaggag atgtggtgag aattttgttg 3540
ttttgttttt taatggggta gtagtaagta gtatatggag ggaatatata ttgattttta 3600
aaaggtagtt aatttggtgg gaattttttt ttgttgtttt 3640

<210> 99

<211> 3640

<212> DNA

<213> Artificial Sequence

<220>

<223> chemically treated genomic DNA (Homo sapiens)

<400> 99

```
agggtgatag gaggaaattt ttattaaatt aattattttt taaaaattaa tatatgtttt     60
ttttatgtat tatttgttgt tgttttatta aagagtagag tgatagggtt tttgttgtgt    120
ttttttttggg atgaggtggg gggtgtgggt gggtggtgtg tttttgatgt gggtgtttat    180
taagattaag attagttttt aattaaagat tttagagggt tttggttata tagtaggagt    240
tgttggtttg gaaagagata gtgatatata tttaatagta agtgtagtat tgtggattta    300
ggggaaggtg gtgtgtaggt ttgaggtttg ggtgggggtg ggtgttgggt attttgattt    360
tgttttgtgg gatttggagg tgaggaaatt tttaaatttt gtgggggagt tggtggtgtt    420
tttttttttgt ttggtgtttg ttttagaatg atttaagaag gatttgagtt aagggggggag    480
gggtttggtt gggggtggt tatgagaaag ttttgtttag tttaatttgg gttgtgtttg    540
ttttgagtgg ttttgtggtt tggttgattg ataattggtt tttgtataag tttatttgtt    600
ttttgttaa tatttatttt ggggtgatat tttaaatgtt tttttgtttt tttgggttat    660
gggagttagg taggagtgtg gggttttagg tagagttttt taattttga agattttttgg    720
gttgggagag aagttaggat tgggtgtgtg ggatgtagga gttggaagtg tttttgggtt    780
taagagttgg gggagaagga gttaagtgta gtggtttttt tgtgttttgt gttttggtgt    840
tgtgggtttg gtgttgtgta gttgttttta gtttgttgtg gtgtgaataa agggttagta    900
gggtgtgatt gattgtgttt gtttttgagt ttggggtgag gggtgttgtt agttgggagt    960
gtttttttttg aaggtttagg atggtttttt atggagatta aggagtttta gagtttgtta   1020
ggttgaaaag tggtagtgta ttttggtagt taattgggga tttttatata tatatattta   1080
gttgattgta aaattaaaaa aggttttgtt tttgtggata gttattagaa ggttttgtgt   1140
ggtgtttgtt ttatggttgg tgtttgtttt tttgagggtg taggattata gtttttttttg   1200
tgtagttttt tttgtgtaat gggtataaat ttgggtgagg agagggaggt gtgtttgggg   1260
ataagaagtg ggtttgtggt gatgattgga attgttatgg ttggggtagg ggaaatttat   1320
ttttggggtt taagggggggt tgatggttat tgtgtggtga ttttggtgtt agtgtagtta   1380
ggatgtttgt tagagatgaa ttttattatt tgtttggagg tatgtggtta ttttgttaat   1440
gggtgtagta tttattgttt tggaagtatt tggagaggga ttttggtagt tggggtgatt   1500
agtttgattt agggtatagt ggttgttaga gatttagttt tgatattgtt gtttattatt   1560
```

```
gatatttggg ttggatagtg ttaataatag tatggttgtg ttgtagtagt agaggattta 1620
aattttagga tgtgtttttt tttatttgta agttttgagt tttttgtgtt ttttgttttt 1680
ttttatttgg tttttttttt gggggtagtt gggttgtgtt gtggttgtgg tgtgtgtttt 1740
ttgtgggttg ttggggttgg gtgatttgtg agtgtttgtg gtttagtttg tggtagtgaa 1800
gaaagggatg tttgttgtgt ttttgttttt agaatgttgt tgttttagtg aattttaaaa 1860
taataattat tttgatagga agaaagtgtg aaggaggaag agaaggatat ttagagggtt 1920
tgttggggta tttaataatg tttgggtgtt atttaattta tgaaaataaa atttttattt 1980
tttgaagtat attttagttt taattttttt aaaaaagatg tgtatttgga ttttgtattt 2040
gaagtatgtt tttgttggtg tgttttttatt taagaaaagt ggggttagtg tagagaaggt 2100
gattttggtg gtttgaagtt ggaggttgga gttgagggat ataatatttg gtggattggt 2160
ggtttttttg ttttgttgtt tttttttttt gtaaattgat tggagttagg aggtagtttt 2220
gaggttttga gttgagaagt ttttgttttt ttttgtgtgg ggtgggtgaa agggtttttg 2280
gaggttgatt ggtttggagt tttggtgtgg agttaggatt ttggataggg ttttattgtt 2340
ttgatgtttg gttttatttt tttttgtttt ggtaggtaga tagaatgttt ttgggaagtt 2400
atagtaagaa aataaaaatt aatagttaaa gttttgaagt tttttagggt gttaggttta 2460
gttgggaata tatattggta tattgaggta attgaaaaga ttaaatttat ttttgttgat 2520
tgggtggttg atttttttgt tttttatgt ggttttgtgt gagtatttga gtgtatgtgt 2580
atatttgttt ataaaattgg aatttgtgtt tttgtgggtg tgataggggt tggattatta 2640
agttaaaatg tttaatttag ttttttgat ttgtgttggg gaaaatttat agttattttt 2700
aatttatgag gtttttaagg aggttgttat atgagttgtt ttgatgttat tatttttata 2760
tttatggtaa atttagttaa ttaaataaat gttgttattt taagttatta gaagaaattg 2820
tttttataga gtatattaat aaaatttatt tttgattttt ttgtaagata gaaaattttt 2880
aagtttaata aaggtgggta attaagaagg aggtttttta atatttgtt ttttagtttg 2940
aggttttgtt ttttaagtga tattgtttgt gtttttttgta aagtttattt attggaatat 3000
ttaaatattg atttaatatt ttaaattaat ttaggagttt tgatggtttg aatgagaaat 3060
gattagtttt tttttagttt ttattaaaat agtagaattt ttatattttt ggtaaaattg 3120
aatggttttt aatttagggt agtaatagaa ggaaggtgtt attgaagttg tggttgttag 3180
agggtgtatt ttttataatg ttggagaaaa tatttaataa atatttatta aataatattt 3240
ttattgtgtt tttatttgaa taagatggat aagtattttt gggagaagaa agttatatta 3300
tttttatata aaaaatgtta gttttataga gtttagtaaa aagaaaaata ttttttatgt 3360
gaatggattt agtgtataat tataaatttt tattttttat taataggttt gtataggttt 3420
ttattttaat ataagtatgt taatattaat gaagtttaat tttttttga gataataaga 3480
ttgggttagg tgattagatt aattataaga taatttgatt attttgatat aaatataaaa 3540
attatttatt atagggtttt gttatagaag tatttatata tgtttttttt aagagatgtt 3600
atggtattat aaaattgtaa taaattaatt tttagaaatt 3640
```

<210> 100
<211> 3240
<212> DNA
<213> Artificial Sequence

<220>
<223> chemically treated genomic DNA (Homo sapiens)

<400> 100

```
gaaattgagg tataaggtag taatgattat tgagggtttt gtttttgttt ttttaggtga     60
ggagtttatt ttagggtttt tagtttgaaa gtttagaggt gaggggtgtg tattataatt    120
tttagaggtt tttggattta agggtggttt ttgttttttg ttttttttgt gtgatgaagt    180
tgggtttatt aatgggagtt tggagtgggg tgagtagggg tgggatgggg tgggattgtg    240
gggaggtggt tggtgggttg tgttgtgagt tagtatttta gagagtgtgg ggtttggata    300
gttgtgtgtt gagggttttg gggtgggtgt tgtgggattt ttttgggtta tgggtaagtg    360
gttttgtggt gtggtttgga taggggtttg gtggttttgt agggaggttt aaggtgttgg    420
tgttgggttt tttttagttt ttgtggtagt tggtgtgttg tgtggggtgg agaggggttt    480
ggggtgggtt ttggggtgtg ggttaagggg agtgagtttg gtttgtggtt ttagtgtttt    540
ttggttggtg tggaaggggt gaggttgggt gtttgtgtga gttttgagaa aaggaaagat    600
aaaaagtttt ggtggttagg ggtttttggg gtgtttattt tgaaattttt tttgttgttt    660
tttgtttgaa atatgttgag tttggtatgg ttttgttgtt ttattttttg ttgtggtttt    720
tattttgagt tgggtttagt taatttgttg tgatagttgt tggtagttgt tttttttgttt    780
tgtgatattt tgtaggtttt agaattgtgg tgttgttggg tatagatttt gggtttagag    840
tgtgtggttt tttttttgt ggttgttgtt ttttgaaagg ggttgtgtgg gtgtgaagaa    900
tttgtttaat gtgttttgta ggatttattg tttttttttt ggttgtgggt tgtgttgatg    960
gtttggggag tttggttttt tgtttttttgt tttttttttt tttttttttt tttttttttt   1020

tttttttgttt ttttgtttat ttgttgtagt tttataggag aggggttgtt tttttggttg   1080
tttttttttt ttgtttttttg tgtgtttttt tgtttttttt tggtttgtag aagggatggg   1140
gtggtggttg gggagtgtta ggggaagagg gtaagagtgt gggttatgag tttgtaggtg   1200
atgtttggtt ttgtgtttgg tgggagttgt gggttaattt gggtttgtgt tttgtttttt   1260
tgtggtgttt aggggttgtt tgggttgggg ttgtgttatt ttttgtttag tgttggtgtg   1320
gtggggtttg tgtggtgtag gatttttgag ggtttagggg gttttaaggt gttgtgggtt   1380
tagtgttttt ttgtttgtag agttttgggt tagtaaggag tgttttgtgg gttttagggg   1440
aggataatag gtgtttttttg ttttatttgg gtttttttgg tgggagttta tatgtatggt   1500
ggtgttattg tgtttatagg taatgttaga gtataggagg ggtgggaggt tggggtttga   1560
gggtttttatt ttgtagtaga gtttagagtt taatttaggt tttagggatt tgtttggggt   1620
ttagaatttg gtgtggtaga ggtagaaagg gtttttgata atttgttttt atagttgaga   1680
gatgttaatt ggtattttttg agtttttgtt ttgtaggtga aatgggaata tatgaaatta   1740
tttgaggagt ttttattgtt tgttattatt aaatgtggtt tagattttgt ggggagggat   1800
agttttattt tggaattggg gtttttagga tattttagag tttggtaaga gggatagggga   1860
gaatggggtt tggaagttgg tagttttata tttagtgttt tttgtatttt tttttttttt   1920
tgtatttttt ggtggttttt gtggttttag ttgtttttag attttgtttt ttttgttttt   1980
ttttaagttg aggtattgag gttgggttgt ggtttgagtt gtatttgtgt tttgttaggg   2040
agtgggggtt ttaggtttat tgtgtttttt ttttattgttg gttttagttt aatatttggt   2100
aggtttttttt ttttaaattg ttggagtaag tttgtgtaga tttggtttgg ttgttgtttt   2160
tgggttgttg agtggattgg ttgttttgtt gttttaaagg ttgtttttgt tttgttttag   2220
tttaagttta gagaaattta agttaataat tttagttgag gggtggggt ggtgtttgtt   2280
ggttgttatt gaggttgttg ttattattat tgtggtaatt gatgtagtgt ttttagttgg   2340
tgatttttta tgaaaggaag tgttattttt tttttattat atttagggtg agtggggtgg   2400
gtagtagaaa gatggggagg gagagaagga aaggggtgtg tgtttttagt agattttttaa   2460
atttgagagt tgtgtggttt tgtattagtt attgtttttt tttgggtaat aggggtttgt   2520
aagtttattg tgtttatttt gtatttagga ggttgatgtt tttggagtat ttgttgggta   2580
gtgaggtgtt ttggggaggg agtaggatag gtaggttttt gattttttta ttgagtttgg   2640
ttgtggtttg tgggggaagt tttttttttt tttttatttt tttttgttga gaatattggg   2700
tatattgagt ttagtatgtt tattttgtag atggggaaag ggagatatag aattagtgat   2760
taatttggta agaggtgtag ttatgtagtg ttggaatttt ttgttttatt tgtttttttg   2820
gtttattttt atttgttttt agtttgatgt ttggtagggt aggggtttat tgattttttt   2880
gtatttattt attgtttggt tttatagtta tttttttagtt ttggttaagt tttgtgagat   2940
attttaagtt tgtttttagt gatttttaat tattgtttga ttgtttttat ttttttgttt   3000
ttgtatattt gtttttgttt tgttaggttg gttgggtttt ggttagagtt ggttttttat   3060
tttttttaggt aataggtagt agtttttttaa atggaatttt agtaggtatt aataggtttt   3120
tttttttttt ttaagattat ggagataata ttttttattt aaagatggtg ggttttttgg   3180
gttttttttg atttaggtta gggaagttga tttatagagt gtgggggtta tgtttttttt   3240
```

<210> 101
<211> 3240
<212> DNA
<213> Artificial Sequence

<220>
<223> chemically treated genomic DNA (Homo sapiens)

<400> 101

```
ggaaggggta tggtttttat attttatgaa ttaattttt tggtttgggt tggggagggt 60
ttagaggatt tattattttt gaataagggg tgttattttt ataattttga ggaaggagga 120
aagatttgtt gatatttatt gagattttat ttaaaaaatt gttatttgtt atttgagaga 180
gtaggggggtt agttttggtt agggtttaat taatttggtg aagtaaaggt aggtatgtaa 240
aggtaggaag gtgaaggtag ttgggtggtg gttgagagtt attgaagata aatttaagat 300
gttttatggg gtttagttag gattagaagg tagttatggg gttaggtagt gggtggatgt 360
ggggaagtta gtgggttttt gttttgttag atattaagtt ggaagtagat gaaagtgggt 420
taagaggata gatggggtaa ggggttttag tattgtatgg ttgtattttt tattaagttg 480
gttattggtt ttgtattttt tttttttat ttataagatg ggtgtgttag atttaatgtg 540
tttaatgttt ttagtagaaa aaagtgagaa gagaaaggag gtttttttta taaattatag 600
ttaggtttag tgagaagatt agaggtttgt ttgttttgtt tttttttaa ggtattttgt 660
tgtttagtag gtgttttgga aatattagtt ttttgggtgt aggatgagtg taatggattt 720
atagattttt gttgtttaga gaggggtagt gattagtata aggttatata gtttttaagt 780
ttaagggttt gttggaggta tatattttt ttttttttt ttttttttatt tttttgttgt 840
ttgttttatt tattttgggt gtgatgggaa ggaagtgata ttttttttta tgaaaagtta 900

ttgattggga atattgtgtt aattattata ataatggtaa tagtagtttt aataataatt 960
agtagatgtt gttttgtttt tttggttgag gttgttggtt taagtttttt taagtttgag 1020
ttggagtaaa ataaagatag tttttgaagt ggtgaaatgg ttggtttatt tggtagtttg 1080
ggagtggtgg ttaggttgag tttatgtgag tttgttttag tagtttgggg aggggggttt 1140
gttaagtgtt gagttggagt tagtagtgag aagaaatgtg atgggtttgg gatttttatt 1200
ttttaatagg gtataggtgt agtttaagtt atagtttaat tttagtattt tagtttgggg 1260
gagggtgaga gagatagggt ttgggggtag ttgaggttat aagagttatt agagggtata 1320
gaaagggaag gaggtgtggg ggatattgaa tgtgaggtta ttaattttta agttttattt 1380
tttttgtttt ttttattggg ttttgggatg ttttaaaagt tttagtttta ggatggattt 1440
gttttttttt atggagtttg ggttatattt ggtggtgatg gatagtgaga gtttttttaga 1500
tggttttgtg tgtttttatt ttatttatgg ggtagaggtt tagaggtgtt agttgatgtt 1560
ttttaattgt agaagtagat tattggaggt ttttttttatt tttgttatat taagttttag 1620
gttttaggtg ggtttttaag gtttggattg ggttttgagt tttgttgtgg ggtgaagttt 1680
ttgagttttg atttttttatt tttttttgtgt tttagtgttg tttgtaggtg tagtgatgtt 1740
attgtgtgtg tgggttttta ttagaggagt ttgggtgggg taagaggtat ttgttgtttt 1800
tttttgggggt ttgtggagta ttttttattg gtttaagatt ttgtaaataa agggatattg 1860
gatttgtagt gttttgggat tttttgggtt tttgggggtt ttgtattgtg taagttttat 1920
tatgttggtg ttgggtggag ggtgatgtga ttttagtttg ggtgattttt agatattgtg 1980
gagggatgga gtgtgggttt gggttggttt gtagtttttg ttgagtgtga agttgggtat 2040
tgtttgtaag tttgtagttt atgtttttgt ttttttttt tgatgttttt tagttgttat 2100
tttgtttttt ttgtagattg agggaagata aggggatata taagggatag gagaggagag 2160
taattaggga agtgattttt tttttgtggg gttgtggtgg gtgggtggag agataggaga 2220
ggaaaagaga aaagggaaga gaaggaaggg gtaggggggtg agaagttggg ttttttggat 2280
tattggtgta gtttgtggtt gaaagaggaa tagtggattt tataaagtgt attgagtaag 2340
ttttttatat ttgtgtagtt tttttttagga aatggtggtt gtagaggaaa gggttgtgtg 2400
ttttggattt ggagtttgtg tttggtggtg ttgtggtttt gaaatttgtg ggatgttatg 2460
gggtggaggg gtggttgttg atagttgtta taataaatta gttgagttta gtttgaggtg 2520
gaaattgtag taggaagtgg gatgatagag ttgtattggg tttggtatat tttaggtaag 2580
agatggtgaa aagagttttg agataaatgt ttgggaagtt tttggttgtt aaaatttttt 2640
gttttttttt tttttgaagt ttgtgtggat gtttagtttt atttttttttg tgttggttgg 2700
ggagtgttga agttgtgggt tgggtttgtt ttttttggtt tgtgttttga aatttatttt 2760
gggttttttt ttgttttata tagtgtattg attgttatga gggttggagg ggatttggtg 2820
ttggtatttt gaattttttt gtagggttgt tgagtttttg tttgggttgt gttgtagagt 2880
tgtttattta tggtttggag aggttttgtg gtgtttgttt tgagattttt agtgtgtggt 2940
tgtttggatt ttgtgttttt tgagatattg gtttgtggtg tggtttgttg gttatttttt 3000
tgtagttttg ttttgttttg tttttgtttg ttttattttg ggtttttatt ggtaagtttg 3060
gttttgttgt gtggagaagg tggggggtgg ggattgtttt tgagtttggg gatttttggg 3120
aattgtagtg tgtgtttttt gtttttaggt ttttagattg gagtttttgg aataggtttt 3180
ttatttagag gagtggaggt aggattttta gtaattattg ttgttttgtg ttttagtttt 3240
```

<210> 102
<211> 2155
<212> DNA
<213> Artificial Sequence

<220>
<223> chemically treated genomic DNA (Homo sapiens)

<400> 102

```
ttttttttt tttttatttt tggagggttt tagttattag ttttgaagtt gtagtgagag      60
ttatttattg ttttggtttt tgggattttt tgagtaggag aaaaaaatta gaaaattaaa    120
agagagagag agatttggaa ggaagaagta atggaggagg gagatagggg gaatgagagg    180
ggagtttata ttatagagag agaatttttt aaaggaagtg taggtgttta tgggatagga    240
gtgtggaaga aaagagtaga aaaggaaaag tagttttaag taagagaaag taaggagagg    300
tggataggga ggggaagagt taagagaagt taagttgagg gatttgtagt aattttgaga    360
aataaaagtg aattgtttgt gggtggttgt tgtgatttta gtaaaaagtg gagtttaagg    420
tttagggatt ttgttgttta tattgtgttt tgttttattt tggatgtttt taattttagt    480
tttaggtata gtatatgagt aggggtagta aggtttggtt agggttggtg ttgtatggga    540
gttgtgggtt tttgtaatat ttaggtgtgt aggtgttata agaaggagtt ggggttgttg    600
tttttgtagt gtggattttg ttttgtgttg tatatgtttt agaagagtgt gtttggaagt    660
gtttttttg gtttttttg tttttaaga tttgtttgta atgtttatat ttgttattat    720
tgtataagta ttagggtgag gaggtttttt tgtaggaatt aatattgatt ttagtttttt    780

gtttttttaa agttttttt tgatgatggt gagatttata ttttaagtta gaagaaaata    840
aaatttgtt tattttgggt ttatgattgt ataaaattta aaatgttgtt ggtatgaaga    900
tgtagttgga agattttaga aatttagtat taggaggtag agattttata gtgtttagag    960
atgagttttt aaataaatta agaattatgt agtgggaaat taaggaggag ggtttgaggg   1020
gttgatttta gagggataga tagagaaatt gagagtgaaa tatttaagga agtggaataa   1080
attgttgtag ttaggtgttt ttgttttttt ggaaatttga aatttgttta aattttattt   1140
taggtgttta tggtattagt tgtagggttt tgtattatag gggttgttat tttttgtttt   1200
tggagttttt atgttgagat ttattatttg ttggtttttt ttttttttt ttttttgtt   1260
tttttatttt ttaaatataa gataaaaata tattttattt attattatag atatatatat   1320
tttattttta ttatataaat ataaaatatt taaatattta aaatatttag aataaagtta   1380
gtagtttgga tttagtgata tttatttgtg tttgaattgt ttattatttt ttttattatt   1440
tgtttagaaa aaaaaaaaag atgaaaaaga gggggagagt ttttgatgta aataatttat   1500
aataaaatta ttattttaaa aaagtgtttt ttgtttgttt tagatggatg aatatatttt   1560
agatattgtg gtattttgta gtttgggttt gtgattttgg ttaaaatgtg ggtggttgtg   1620
gttgtaggga tgattgttgt tgtgtagggt aaggaaagag ggtgttgtgg tttgtgtttt   1680
gatttgggat tattgaaatt ttaagattgg ggaggtgagg ggagtgtttt ttttattaat   1740
tatataagat gttatttgga ttttgagatt attgttagag gaggtttgat tatttagtgg   1800
tgtttttgtt ttttttttg taaagaattg tttagaaatt tagatgtttt ttgtgtgtag   1860
gaagggtttt gtgaatttgg gtgtttggga gaggaggtaa tgagtgttga tttgtttttt   1920
aaattgagta attgtgtttg aagttatgtg tttgggaggt tgtagggtga agtgttggtt   1980
gtgtaggtta ttgttggtag ttgttttgtt tttttttgga ggagtgggaa gttgttggtt   2040
attgttttga ataggtttgg agaaaagatt ttgaattttt tttgattagt ggtgttattt   2100
tttttttgt ttttttttta ttaaattttt gtttaaagtt ataatattta gaatt         2155
```

<210> 103
<211> 2155
<212> DNA
<213> Artificial Sequence

<220>
<223> chemically treated genomic DNA (Homo sapiens)

<400> 103

```
ggttttgggt gttgtaattt taaataggaa tttaatgggg gaaaagtaaa ggaaaaggta     60
atgttgttaa ttaaaggaaa tttagaattt tttttttgag tttgtttggg gtggtggttg    120
atagtttttt attttttag gaaagagtaa agtggttgtt ggtggtagtt tgtgtagttg    180
gtgttttatt ttatggtttt ttaggtgtgt agttttgggt ataattgttt ggtttggaaa    240
atgagttagt atttattgtt tttttttta aatatttgga tttataaaat tttttttgta    300
tgtaggaaat gtttggattt ttgagtagtt ttttgtggaa ggggaggtag ggatgttgtt    360
gggtggttgg gttttttta gtagtagttt tggagtttag atagtgtttt gtgtgattga    420
taggaaagat atttttttg ttttttagt tttagggttt tagtggtttt aaattagaat    480
atgaattgtg atatttttt tttttatttt gtgtagtggt gattgttttt atagttatgg    540
ttatttatat tttaattaaa attatagatt tagattgtga ggtgttgtag tgtttagaat    600
gtatttattt atttgaagta agtgaaaaat atttttttaa aatagtaatt ttattgtaaa    660
ttatttatgt tggaaatttt tttttttttt tttatttttt tttttttttt aggtaaatag    720
tgaagaaaat aatgaatgat ttaaatgtgg gtaggtgtta ttgaatttag gttattaatt    780
ttgttttgaa tgttttggat atttggatgt tttgtattta tgtggtgaga gtgaaatata    840
tatatttatg atgataaatg aggtgtattt ttgttttata tttgaaggat aaaaaaataa    900
aagaagaaaa ggaagaaaga gttagtaaat gatgagtttt ggtgtgggga ttttggggt    960
agagggtggt agtttttata gtgtggggtt ttgtgattgg tattgtgagt gtttgggatg   1020
aggtttgggt ggattttggg tttttggaga ggtagagatg tttggttatg atgatttgtt   1080
ttattttttt gagtgttttg tttttgattt ttttatttgt ttttttgagg ttggtttttt   1140
agattttttt ttttaatttt ttattgtgta atttttagtt tatttaggaa tttatttttg   1200
ggtattataa agttttgtt ttttaatgtt aggtttttag agttttttag ttgtattttt   1260
atgttggtag tattttggat tttgtgtaat tatgggttta gaataggtaa ggttttattt   1320
tttttgatt tagaatataa gttttgttat tgttaaagag aaattttgaa ggagtaggag   1380
attgaaattg atattaattt ttgtaaagag attttttat tttggtgttt atgtggtggt   1440
gatgggtgta gatgttgtaa ataaatttg ggaagtgagg agggttgggg aggatatttt   1500
tggatatgtt tttttaaagt gtgtgtagta tggggtgaag tttgtgttgt ggggtagtg   1560
gttttggttt tttttgtag tgtttgtata tttgggtgtt gtagggattt gtagttttg   1620
tgtggtattg gttttagtta ggttttattg tttttatttg tgtattgtgt ttgaagttga   1680
agttggaggt atttaaaata aggtggggta tagtgtgggt ggtggagttt ttgagttttg   1740


agttttgttt tttgttgaga ttatagtagt tgtttataga tagtttattt ttattttta   1800
aagttgttgt agatttttta gtttggtttt ttttggtttt ttttttttt gtttgttttt   1860
ttttattttt ttttgtttga aattgtttt ttttttttat ttttttttt tgtgttttg   1920
ttttgtaaat gtttgtgttt tttttaagga gttttttttt tgtggtgtgg gttttttttt   1980
tgttttttt gtttttttt tttattattt ttttttttta ggttttttt ttttttttgg   2040
tttttggtt ttttttttt gtttaagggg ttttaaaggt taaggtagtg ggtggttttt   2100
gttgtagttt taaggttggt ggttgggatt ttttaggggt gagaaggaag ggaga        2155
```

<210> 104
<211> 28
<212> DNA
<213> Artificial Sequence

<220>
<223> chemically treated genomic DNA (Homo sapiens)

<400> 104
tatcgcggag attttcgagt tttcgttg 28

<210> 105
<211> 19
<212> DNA
<213> Artificial Sequence

<220>
<223> chemically treated genomic DNA (Homo sapiens)

<400> 105
ggggttgttt ttcgggatt 19

<210> 106
<211> 24
<212> DNA
<213> Artificial Sequence

<220>
<223> chemically treated genomic DNA (Homo sapiens)

<400> 106
gataacccta aaacgcaact cgaa 24

<210> 107
<211> 20
<212> DNA
<213> Artificial Sequence

<220>
<223> chemically treated genomic DNA (Homo sapiens)

<400> 107
cgtttcgtaa ggagcgtgtt 20

<210> 108
<211> 24
<212> DNA
<213> Artificial Sequence

<220>
<223> chemically treated genomic DNA (Homo sapiens)

<400> 108
cgcgttgttc gcggttagtt tcgt 24

<210> 109
<211> 16
<212> DNA
<213> Artificial Sequence

<220>
<223> chemically treated genomic DNA (Homo sapiens)

<400> 109
cgacgttttc gcgtgg 16

<210> 110
<211> 29
<212> DNA
<213> Artificial Sequence

<220>
<223> chemically treated genomic DNA (Homo sapiens)

<400> 110
gttttgaaat ttattagaat aacgacgtt 29

<210> 111
<211> 31
<212> DNA

<213> Artificial Sequence

<220>
<223> chemically treated genomic DNA (Homo sapiens)

<400> 111
ctttctaaaa ataaccgaac tatactacga c 31

<210> 112
<211> 21
<212> DNA
<213> Artificial Sequence

<220>
<223> chemically treated genomic DNA (Homo sapiens)

<400> 112
tacggacgtt cgcgggtcgt t 21

<210> 113
<211> 24
<212> DNA
<213> Artificial Sequence

<220>
<223> chemically treated genomic DNA (Homo sapiens)

<400> 113
agtaagttcg cgtagattcg gttt 24

<210> 114
<211> 21
<212> DNA
<213> Artificial Sequence

<220>
<223> chemically treated genomic DNA (Homo sapiens)

<400> 114
taaaacgacg aaacgaccga t 21

<210> 115
<211> 24
<212> DNA
<213> Artificial Sequence

<220>
<223> chemically treated genomic DNA (Homo sapiens)

<400> 115
tcgtcgtttt cgggttgtcg agtg 24

<210> 116
<211> 24
<212> DNA
<213> Artificial Sequence

<220>

<223> chemically treated genomic DNA (Homo sapiens)

<400> 116
aaggaagtgg aataaatcgt cgta 24

<210> 117
<211> 33
<212> DNA
<213> Artificial Sequence

<220>
<223> chemically treated genomic DNA (Homo sapiens)

<400> 117
aggcgttttt gtttttcgg aaattcgaaa ttc 33

<210> 118
<211> 25
<212> DNA
<213> Artificial Sequence

<220>
<223> chemically treated genomic DNA (Homo sapiens)

<400> 118
ctacgactaa taccgtaaac gccta 25

## Description

FIELD OF THE INVENTION

The present invention relates to genomic DNA sequences that exhibit altered expression patterns in disease states relative to normal. Particular embodiments provide methods, nucleic acids, nucleic add arrays and kits useful for detecting, or for diagnosing cell proliferative disorders.

BACKGROUND

*CpG island methylation.* Apart from mutations aberrant methylation of CpG islands has been shown to lead to the transcriptional silencing of certain genes that have been previously linked to the pathogenesis of various cell proliferative disorders, including cancer. CpG islands are short sequences which are rich in CpG dinucleotides and can usually be found in the 5' region of approximately 50% of all human genes. Methylation of the cytosines in these islands leads to the loss of gene expression and has been reported in the inactivation of the X chromosome and genomic imprinting.

*Development of medical tests.* Two key evaluative measures of any medical screening or diagnostic test are its sensitivity and specificity, which measure how well the test performs to accurately detect all affected individuals without exception, and without falsely including individuals who do not have the target disease (predicitive value). Historically, many diagnostic tests have been criticized due to poor sensitivity and specificity.

A true positive (TP) result is where the test is positive and the condition is present. A false positive (FP) result is where the test is positive but the condition is not present. A true negative (TN) result is where the test is negative and the condition is not present. A false negative (FN) result is where the test is negative but the condition is not present. In this context: Sensitivity = TP/(TP+FN); Specificity = TN/(FP+TN); and Predictive value = TPI(TP+FP).

Sensitivity is a measure of a test's ability to correctly detect the target disease in an individual being tested. A test having poor sensitivity produces a high rate of false negatives, i.e., individuals who have the disease but are falsely identified as being free of that particular disease. The potential danger of a false negative is that the diseased individual will remain undiagnosed and untreated for some period of time, during which the disease may progress to a later stage wherein treatments, if any, may be less effective. An example of a test that has low sensitivity is a protein-based blood test for HIV. This type of test exhibits poor sensitivity because it fails to detect the presence of the virus until the disease is well established and the virus has invaded the bloodstream in substantial numbers. In contrast, an example of a test that has high sensitivity is viral-load detection using the polymerase chain reaction (PCR). High sensitivity is achieved because this type of test can detect very small quantities of the virus. High sensitivity is particularly important when the consequences of missing a diagnosis are high.

Specificity, on the other hand, is a measure of a test's ability to identify accurately patients who are free of the disease state. A test having poor specificity produces a high rate of false positives, i.e., individuals who are falsely identified as having the disease. A drawback of false positives is that they force patients to undergo unnecessary medical procedures treatments with their attendant risks, emotional and financial stresses, and which could have adverse effects on the patient's health. A feature of diseases which makes it difficult to develop diagnostic tests with high specificity is that disease mechanisms, particularly in cell proliferative disorders, often involve a plurality of genes and proteins. Additionally, certain proteins may be elevated for reasons unrelated to a disease state. Specificity is important when the cost or risk associated with further diagnostic procedures or further medical intervention are very high.

WO 2006/060653 discloses that the expression levels of a group of genes comprising SHOX2 can be used to predict the progression of lung cancer in a patient already diagnosed with lung cancer. Methylation of the SHOX2 gene is not mentioned.

WO 2006/008128 describes a large number of methylation and expression markers for breast cancer. However, it does not mention SHOX2.

SUMMARY OF THE INVENTION

The present invention provides a method for detecting lung, breast and bladder carcinomas, in a subject comprising determining the methylation levels of the SHOX2 in a biological sample isolated from said subject wherein hyper-methylation is indicative of the presence of said disorder. Various aspects of the present invention provide an efficient and unique genetic marker, whereby expression analysis of said marker enables the detection of cell proliferative disorders, preferably those according to Table 2 with a particularly high sensitivity, specificity and/or predictive value. Preferred is a lung cancer selected from the group consisting of Lung adenocarcinoma; Large cell lung cancer; squamous cell lung carcinoma; Small cell lung carcinoma.

In one embodiment the invention provides a method for detecting lung, breast or bladder cancer, in a subject comprising determining the methylation level of the gene SHOX2 in a biological sample isolated from said subject wherein CpG methylation is indicative of the presence of said disorder.

In a further preferred embodiment said expression is determined by detecting the presence or absence of CpG methylation within said gene, wherein under-expression indicates the presence of cell proliferative disorders, preferably those according to (most preferably lung carcinoma). Said method comprises the following steps: i) contacting genomic DNA isolated from a biological sample (preferably selected from the group consisting of cells or cell lines, histological slides, biopsies, paraffin-embedded tissue, body fluids, ejaculate, urine, blood plasma, blood serum, whole blood, isolated blood cells, sputum and biological matter derived from bronchoscopy (including but not limited to bronchial lavage, bronchial alveolar lavage, bronchial brushing, bronchial abrasion) obtained from the subject with at least one reagent, or series of reagents that distinguishes between methylated and non-methylated CpG dinucleotides within at least one target region of the genomic DNA, wherein the nucleotide sequence of said target region comprises at least one CpG dinucleotide sequence of SHOX2 and ii) detecting cell proliferative disorders, preferably those according to (most preferably lung carcinoma), at least in part. Preferably the target region comprises, or hybridizes under stringent conditions to a sequence of at least 16 contiguous nucleotides of SEQ ID NO: 1 to SEQ ID NO: 12; SEQ ID NO: 79 to SEQ ID NO: 83.

Preferably, the sensitivity of said detection is from about 75% to about 96%, or from about 80% to about 90%, or from about 80% to about 85%. Preferably, the specificity is from about 75% to about 96%, or from about 80% to about 90%, or from about 80% to about 85%.

However, in the most preferred embodiment of the invention the detection of cell proliferative disorders, preferably those according to (most preferably lung carcinoma), is enabled by means of analysis of the *methylation status* of at least the SHOX2 gene and/or promoter or regulatory elements thereof.

The invention provides a method for the analysis of biological samples for features associated with the development of cell proliferative disorders, preferably those according to (most preferably lung carcinoma), the method characterized in that the nucleic acid, or a fragment thereof of SEQ ID NO: 5 is contacted with a reagent or series of reagents capable of distinguishing between methylated and non methylated CpG dinucleotides within the genomic sequence.

The present invention provides a method for ascertaining epigenetic parameters of genomic DNA associated with the development of cell proliferative disorders, preferably those according to (most preferably lung carcinoma). The method has utility for the improved detection and diagnosis of said disease.

Preferably, the source of the test sample is selected from the group consisting of cells or cell lines, histological slides, biopsies, paraffin-embedded tissue, body fluids, ejaculate, urine, blood plasma, blood serum, whole blood, isolated blood cells, sputum and biological matter derived from bronchoscopy (including but not limited to bronchial lavage, bronchial alveolar lavage, bronchial brushing, bronchial abrasion, and combinations thereof. More preferably the samnpel type is selected form the group consisting of blood plasma, sputum and biological matter derived from bronchoscopy (including but not limited to bronchial lavage, bronchial alveolar lavage, bronchial brushing, bronchial abrasion) and all possible combinations thereof.

Specifically, the present invention provides a method for detecting cell proliferative disorders, preferably those according to (most preferably lung carcinoma) suitable for use in a diagnostic tool, comprising: obtaining a biological sample comprising genomic nucleic acid(s); contacting the nucleic acid(s), or a fragment thereof, with a reagent or a plurality of reagents sufficient for distinguishing between methylated and non methylated CpG dinucleotide sequences within a target sequence of the subject nucleic acid, wherein the target sequence comprises, or hybridises under stringent conditions to, a sequence comprising at least 16 contiguous nucleotides of SEQ ID NO: 5 said contiguous nucleotides comprising at least one CpG dinucleotide sequence; and determining, based at least in part on said distinguishing, the methylation state of at least one target CpG dinucleotide sequence, or an average, or a value reflecting an average methylation state of a plurality of target CpG dinucleotide sequences.

Preferably, distinguishing between methylated and non methylated CpG dinucleotide sequences within the target sequence comprises methylation state-dependent conversion or non-conversion of at least one such CpG dinucleotide sequence to the corresponding converted or non-converted dinucleotide sequence within a sequence selected from the group consisting of SEQ ID NO: 21, 22, 45 and 46, and contiguous regions thereof corresponding to the target sequence.

Additional embodiments provide a method for the detection of cell proliferative disorders, preferably those according to (most preferably lung carcinoma) comprising: obtaining a biological sample having subject genomic DNA; extracting the genomic DNA; treating the genomic DNA, or a fragment thereof, with one or more reagents to convert 5-position unmethylated cytosine bases to uracil or to another base that is detectably dissimilar to cytosine in terms of hybridization properties; contacting the treated genomic DNA, or the treated fragment thereof, with an amplification enzyme and at least two primers comprising, in each case a contiguous sequence at least 9 nucleotides in length that is complementary to, or hybridizes under moderately stringent or stringent conditions to a sequence selected from the group consisting SEQ ID NO: 21, 22, 45 and 46, and complements thereof, wherein the treated DNA or the fragment thereof is either amplified to produce an amplificate, or is not amplified; and determining, based on a presence or absence of, or on a property of said amplificate, the methylation state or an average, or a value reflecting an average of the methylation level of at least one , but more preferably a plurality of CpG dinucleotides of SEQ ID NO: 5.

Preferably, determining comprises use of at least one method selected from the group consisting of: i) hybridizing at least one nucleic acid molecule comprising a contiguous sequence at least 9 nucleotides in length that is complementary to, or hybridizes under moderately stringent or stringent conditions to a sequence selected from the group consisting of SEQ ID NO: 21, 22, 45 and 46, and complements thereof; ii) hybridizing at least one nucleic acid molecule, bound to a solid phase, comprising a contiguous sequence at least 9 nucleotides in length that is complementary to, or hybridizes under moderately stringent or stringent conditions to a sequence selected from the group consisting of SEQ ID NO: 21, 22, 45 and 46, and complements thereof; iii) hybridizing at least one nucleic acid molecule comprising a contiguous sequence at least 9 nucleotides in length that is complementary to, or hybridizes under moderately stringent or stringent conditions to a sequence selected from the group consisting of SEQ ID NO: 21, 22, 45 and 46, and complements thereof, and extending at least one such hybridized nucleic acid molecule by at least one nucleotide base; and iv) sequencing of the amplificate.

Further embodiments provide a method for the analysis (i.e. detection or diagnosis) of cell proliferative disorders, preferably those according to (most preferably lung carcinoma), comprising: obtaining a biological sample having subject genomic DNA; extracting the genomic DNA; contacting the genomic DNA, or a fragment thereof, of SEQ ID NO: 5 or a sequence that hybridizes under stringent conditions thereto, with one or more methylation-sensitive restriction enzymes, wherein the genomic DNA is either digested thereby to produce digestion fragments, or is not digested thereby; and determining, based on a presence or absence of, or on property of at least one such fragment, the methylation state of at least one CpG dinucleotide sequence of SEQ ID NO: 5 or an average, or a value reflecting an average methylation state of a plurality of CpG dinucleotide sequences thereof. Preferably, the digested or undigested genomic DNA is amplified prior to said determining.

Additional embodiments provide novel genomic and chemically modified nucleic acid sequences, as well as oligonucleotides and/or PNA-oligomers for analysis of cytosine methylation patterns within SEQ ID NO: 5.

## DETAILED DESCRIPTION OF THE INVENTION

Definitions:

The term "Observed/Expected Ratio" ("O/E Ratio") refers to the frequency of CpG dinucleotides within a particular DNA sequence, and corresponds to the [number of CpG sites / (number of C bases x number of G bases)] / band length for each fragment.

The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 KB, or to about 2kb in length.

The term "methylation state" or "methylation status" refers to the presence or absence of 5-methylcytosine ("5-mCyt") at one or a plurality of CpG dinucleotides within a DNA sequence. Methylation states at one or more particular CpG methylation sites (each having two CpG dinucleotide sequences) within a DNA sequence include "unmethylated," "fully-methylated" and "hemi-methylated."

The term "hemi-methylation" or "hemimethylation" refers to the methylation state of a double stranded DNA wherein only one strand thereof is methylated.

The term 'AUC' as used herein is an abbreviation for the area under a curve. In particular it refers to the area under a Receiver Operating Characteristic (ROC) curve. The ROC curve is a plot of the true positive rate against the false positive rate for the different possible cut points of a diagnostic test. It shows the trade-off between sensitivity and specificity depending on the selected cut point (any increase in sensitivity will be accompanied by a decrease in specificity). The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis, Academic Press, New York, 1975).

The term "microarray" refers broadly to both "DNA microarrays," and 'DNA chip(s),' as recognized in the art, encompasses all art-recognized solid supports, and encompasses all methods for affixing nucleic acid molecules thereto or synthesis of nucleic acids thereon.

"Genetic parameters" are mutations and polymorphisms of genes and sequences further required for their regulation. To be designated as mutations are, in particular, insertions, deletions, point mutations, inversions and polymorphisms and, particularly preferred, SNPs (single nucleotide polymorphisms).

"Epigenetic parameters" are, in particular, cytosine methylation. Further epigenetic parameters include, for example, the acetylation of histones which, however, cannot be directly analysed using the described method but which, in turn, correlate with the DNA methylation.

The term "bisulfite reagent" refers to a reagent comprising bisulfite, disulfite, hydrogen sulfite or combinations thereof, useful as disclosed herein to distinguish between methylated and unmethylated CpG dinucleotide sequences.

The term "Methylation assay" refers to any assay for determining the methylation state of one or more CpG dinucleotide sequences within a sequence of DNA.

The term "MS.AP-PCR" (Methylation-Sensitive Arbitrarily-Primed Polymerase Chain Reaction) refers to the art-recognized technology that allows for a global scan of the genome using CG-rich primers to focus on the regions most likely to contain CpG dinucleotides, and described by Gonzalgo et al., cell proliferative disorders, preferably those according to Cancer Research 57:594-599, 1997.

The term "MethyLight™" refers to the art-recognized fluorescence-based real-time PCR technique described by Eads et al., cell proliferative disorders, preferably those according to Cancer Res. 59:2302-2306, 1999.

The term "HeavyMethyl™" assay, in the embodiment thereof implemented herein, refers to an assay, wherein methylation specific *blocking* probes (also referred to herein as *blockers*) covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers.

The term "Ms-SNuPE" (Methylation-sensitive Single Nucleotide Primer Extension) refers to the art-recognized assay described by Gonzalgo & Jones, Nucleic Acids Res. 25:2529-2531, 1997.

The term "MSP" (Methylation-specific PCR) refers to the art-recognized methylation assay described by Herman et al. Proc. Natl. Acad. Sci. USA 93:9821-9826, 1996, and by US Patent No. 5,786,146.

The term "COBRA" (Combined Bisulfite Restriction Analysis) refers to the art-recognized methylation assay described by Xiong & Laird, Nucleic Acids Res. 25:2532-2534, 1997.

The term "MCA" (Methylated CpG Island Amplification) refers to the methylation assay described by Toyota et al., cell proliferative disorders, preferably those according to Cancer Res. 59:2307-12, 1999, and In WO 00/26401A1.

The term "hybridisation" is to be understood as a bond of an oligonucleotide to a complementary sequence along the lines of the Watson-Crick base pairings in the sample DNA, forming a duplex structure.

"Stringent hybridisation conditions," as defined herein, involve hybridising at 68°C in 5x SSC/5x Denhardt's solution/1.0% SDS, and washing in 0.2x SSC/0.1% SDS at room temperature, or involve the art-recognized equivalent thereof (e.g., conditions in which a hybridisation is carried out at 60°C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

The terms "Methylation-specific restriction enzymes" or "methylation-sensitive restriction enzymes" shall be taken to mean an enzyme that selectively digests a nucleic acid dependant on the methylation state of its recognition site. In the case of such restriction enzymes which specifically cut if the recognition site is not methylated or hemimethylated, the cut will not take place, or with a significantly reduced efficiency, if the recognition site is methylated. In the case of such restriction enzymes which specifically cut if the recognition site is methylated, the cut will not take place, or with a significantly reduced efficiency if the recognition site is not methylated. Preferred are methylation-specific restriction enzymes, the recognition sequence of which contains a CG dinucleotide (for instance cgcg or cccggg). Further preferred for some embodiments are restriction enzymes that do not cut if the cytosine in this dinucleotide is methylated at the carbon atom C5. "Non-methylation-specific restriction enzymes" or "non-methylation-sensitive restriction enzymes" are restriction enzymes that cut a nucleic acid sequence irrespective of the methylation state with nearly identical efficiency. They are also called "methylation-unspecific restriction enzymes."

In reference to composite array sequences, the phrase "contiguous nucleotides" refers to a contiguous sequence region of any individual contiguous sequence of the composite array, but does not include a region of the composite array sequence that includes a "node," as defined herein above.

The term "at least one gene or genomic sequence selected from the group consisting of PTGER4;RUNX1;EVX2;EVX-1; SHOX2;SEQ ID NO: 6;CN027;LRAT;IL-12RB1;TFAP2C;BCL2; EN2; PRDM14; SEQ ID NO: 81; ARID5A;VAX1" shall be taken to include all transcript variants thereof and all promoter and regulatory elements thereof. Furthermore as a plurality of SNPs are known within said gene the term shall be taken to include all sequence variants thereof.

<u>Overview</u>:

The present invention provides a method for detecting lung, breast or bladder cancer in a subject comprising determining the methylation level of the gene SHOX2 in a biological sample isolated from said subject wherein hypermethylation is indicative of the presence of said disorder. Said markers may be used for the diagnosis of said cancer.

In addition to the embodiments above wherein the methylation analysis of SHOX2 is analysed, the invention presents further panels of genes comprising at least one gene or genomic sequence selected from the group consisting of PTGER4;RUNX1;EVX2;EVX-1; SHOX2;SEQ ID NO: 6;CN027;LRAT;IL-12RB1;TFAP2C;BCL2; EN2; PRDM14; SEQ ID NO: 81; ARID5A;VAX1 with novel utility for the detection of cell proliferative disorders, preferably those according to Table 2 (most preferably lung carcinoma).

*Bisulfite modification of DNA is an art-recognized tool used to assess CpG methylation status.* 5-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing, because 5-methylcytosine has the same base pairing behavior as cytosine. Moreover, the epigenetic information carried by 5-methylcytosine is completely lost during, *e.g.*, PCR amplification.

The most frequently used method for analyzing DNA for the presence of 5-methylcytosine is based upon the specific reaction of bisulfite with cytosine whereby, upon subsequent alkaline hydrolysis, cytosine is converted to uracil which corresponds to thymine in its base pairing behavior. Significantly, however, 5-methylcytosine remains unmodified under these conditions. Consequently, the original DNA is *converted* in such a manner that methylcytosine, which originally could not be distinguished from cytosine by its hybridization behavior, can now be detected as the only remaining cytosine using standard, art-recognized molecular biological techniques, for example, by amplification and hybridization, or by sequencing. All of these techniques are based on differential base pairing properties, which can now be fully exploited.

The prior art, in terms of sensitivity, is defined by a method comprising enclosing the DNA to be analysed in an agarose matrix, thereby preventing the diffusion and renaturation of the DNA (bisulfite only reacts with single-stranded DNA), and replacing all precipitation and purification steps with fast dialysis (Olek A, et al., A modified and improved method for bisulfite based cytosine methylation analysis, Nucleic Acids Res. 24:5064-6, 1996). It is thus possible to analyse individual cells for methylation status, illustrating the utility and sensitivity of the method. An overview of art-recognized methods for detecting 5-methylcytosine is provided by Rein, T., et al., Nucleic Acids Res., 26:2255, 1998.

The bisulfite technique, barring few exceptions (*e.g.*, Zeschnigk M, et al., Eur J Hum Genet. 5:94-98, 1997), is currently only used in research. In all instances, short, specific fragments of a known gene are amplified subsequent to a bisulfite treatment, and either completely sequenced (Olek & Walter, Nat Genet. 1997 17:275-6, 1997), subjected to one or more primer extension reactions (Gonzalgo & Jones, Nucleic Acids Res., 25:2529-31, 1997; WO 95/00669; U.S. Patent No. 6,251,594) to analyse individual cytosine positions, or treated by enzymatic digestion (Xiong & Laird, Nucleic Acids Res., 25:2532-4, 1997). Detection by hybridisation has also been described in the art (Olek et al., WO 99/28498). Additionally, use of the bisulfite technique for methylation detection with respect to individual genes has been described (Grigg & Clark, Bioessays, 16:431-6, 1994; Zeschnigk M, et al., Hum Mol Genet., 6:387-95, 1997; Feil R, et al., Nucleic Acids Res., 22:695-, 1994; Martin V, et al., Gene, 157:261-4, 1995; WO 9746705 and WO 9515373).

The present invention provides for the use of the bisulfite technique, in combination with one or more methylation assays, for determination of the methylation status of CpG dinucleotide sequences within SEQ ID NO: 5. Genomic CpG dinucleotides can be methylated or unmethylated (alternatively known as up- and down- methylated respectively). However the methods of the present invention are suitable for the analysis of biological samples of a heterogeneous nature e.g. a low concentration of tumor cells within a background of blood. Accordingly, when analyzing the methylation status of a CpG position within such a sample the person skilled in the art may use a quantitative assay for determining the level (e.g. percent, fraction, ratio, proportion or degree) of methylation at a particular CpG position as opposed to a methylation state. Accordingly the term methylation status or methylation state should also be taken to mean a value reflecting the degree of methylation at a CpG position. Unless specifically stated the terms "hypermethylated" or "upmethylated" shall be taken to mean a methylation level above that of a specified cut-off point, wherein said cut-off may be a value representing the average or median methylation level for a given population, or is preferably an optimized cut-off level. The "cut-off" is also referred herein as a "threshold". In the context of the present invention the terms "methylated", "hypermethylated" or "upmethylated" shall be taken to include a methylation level above the cut-off be zero (0) % (or equivalents thereof) methylation for all CpG positions within and associated with (e.g. in promoter or regulatory regions) the gene or genomic sequence of SHOX2.

According to the present invention, determination of the methylation status of CpG dinucleotide sequences within SEQ ID NO: 5 has utility in the diagnosis and detection of cell proliferative disorders, preferably those according to Table 2 (most preferably lung carcinoma).

*Methylation Assay Procedures.* Various methylation assay procedures are known in the art, and can be used in conjunction with the present invention. These assays allow for determination of the methylation state of one or a plurality of CpG dinucleotides (*e.g.*, CpG islands) within a DNA sequence. Such assays involve, among other techniques, DNA sequencing of bisulfite-treated DNA, PCR (for sequence-specific amplification), Southern blot analysis, and use of methylation-sensitive restriction enzymes.

For example, genomic sequencing has been simplified for analysis of DNA methylation patterns and 5-methylcytosine distribution by using bisulfite treatment (Frommer et al., Proc. Natl. Acad. Sci. USA 89:1827-1831, 1992).

Additionally, restriction enzyme digestion of PCR products amplified from bisulfite-converted DNA is used, *e.g.*, the method described by Sadri & Hornsby (Nucl. Acids Res. 24:5058-5059, 1996), or COBRA (Combined Bisulfite Restriction Analysis) (Xiong & Laird, Nucleic Acids Res. 25:2532-2534, 1997).

COBRA. COBRA™ analysis is a quantitative methylation assay useful for determining DNA methylation levels at specific gene loci in small amounts of genomic DNA (Xiong & Laird, Nucleic Acids Res. 25:2532-2534, 1997). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992). PCR amplification of the bisulfite converted DNA is then performed using primers specific for the CpG islands of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples.

Typical reagents (*e.g.*, as might be found in a typical COBRA™-based kit) for COBRA™ analysis may include, but are not limited to: PCR primers for specific gene (or bisulfite treated DNA sequence or CpG island); restriction enzyme and appropriate buffer; gene-hybridization oligonucleotide; control hybridization oligonucleotide; kinase labeling kit for oligonucleotide probe; and labeled nucleotides. Additionally, bisulfite conversion reagents may include: DNA denaturation buffer; sulfonation buffer; DNA recovery reagents or kits (*e.g.*, precipitation, ultrafiltration, affinity column); desulfonation buffer; and DNA recovery components.

Preferably, assays such as "MethyLight™" (a fluorescence-based real-time PCR technique) (Eads et al., cell proliferative disorders, preferably those according to Cancer Res. 59:2302-2306, 1999), Ms-SNuPE™ (Methylation-sensitive Single Nucleotide Primer Extension) reactions (Gonzalgo & Jones, Nucleic Acids Res. 25:2529-2531, 1997), methylation-specific PCR ("MSP"; Herman et al., Proc. Natl. Acad. Sci. USA 93:9821-9826, 1996; US Patent No. 5,786,146), and methylated CpG island amplification ("MCA"; Toyota et al., cell proliferative disorders, preferably those according to Cancer Res. 59:2307-12, 1999) are used alone or in combination with other of these methods.

The "HeavyMethyl™" assay, technique is a quantitative method for assessing methylation differences based on methylation specific amplification of bisulfite treated DNA. Methylation specific *blocking* probes (also referred to herein as *blockers*) covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers. The HeavyMethyl™ assay may also be used in combination with methylation specific amplification primers.

Typical reagents (*e.g.*, as might be found in a typical MethyLight□-based kit) for HeavyMethyl™ analysis may include, but are not limited to: PCR primers for specific genes (or bisulfite treated DNA sequence or CpG island); blocking oligonucleotides; optimized PCR buffers and deoxynucleotides; and Taq polymerase.

*MSP.* MSP (methylation-specific PCR) allows for assessing the methylation status of virtually any group of CpG sites within a CpG island, independent of the use of methylation-sensitive restriction enzymes (Herman et al. Proc. Natl. Acad. Sci. USA 93:9821-9826, 1996; US Patent No. 5,786,146). Briefly, DNA is modified by sodium bisulfite converting all unmethylated, but not methylated cytosines to uracil, and subsequently amplified with primers specific for methylated versus unmethylated DNA. MSP requires only small quantities of DNA, is sensitive to 0.1% methylated alleles of a given CpG island locus, and can be performed on DNA extracted from paraffin-embedded samples. Typical reagents (*e.g.*, as might be found in a typical MSP-based kit) for MSP analysis may include, but are not limited to: methylated and unmethylated PCR primers for specific gene (or bisulfite treated DNA sequence or CpG island), optimized PCR buffers and deoxynucleotides, and specific probes.

*MefhyLight*™. The MethyLight™ assay is a high-throughput quantitative methylation assay that utilizes fluorescence-based real-time PCR (TaqMan□) technology that requires no further manipulations after the PCR step (Eads et al., Cancer Res. 59:2302-2306, 1999). Briefly, the MethyLight™ process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed in a "biased" (with PCR primers that overlap known CpG dinucleotides) reaction. Sequence discrimination can occur both at the level of the amplification process and at the level of the fluorescence detection process.

The MethyLight™ assay may be used as a quantitative test for methylation patterns in the genomic DNA sample, wherein sequence discrimination occurs at the level of probe hybridization. In this quantitative version, the PCR reaction provides for a methylation specific amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the HeavyMethyl™ and MSP techniques), or with oligonucleotides covering potential methylation sites.

The MethyLight™ process can by used with any suitable probes e.g. "TaqMan®" , Lightcycler® etc.... For example, double-stranded genomic DNA is treated with sodium bisulfite and subjected to one of two sets of PCR reactions using TaqMan® probes; *e.g.*, with MSP primers and/ or HeavyMethyl blocker oligonucleotides and TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (*e.g.*, as might be found in a typical MethyLight□-based kit) for MethyLight™ analysis may include, but are not limited to: PCR primers for specific gene (or bisulfite treated DNA sequence or CpG island); TaqMan® or Lightcycler® probes; optimized PCR buffers and deoxynucleotides; and Taq polymerase.

The QM™ (quantitative methylation) assay is an alternative quantitative test for methylation patterns in genomic DNA samples, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the HeavyMethyl™ and MSP techniques), or with oligonucleotides covering potential methylation sites.

The QM™ process can by used with any suitable probes e.g. "TaqMan®", Lightcycler® etc... in the amplification process. For example, double-stranded genomic DNA is treated with sodium bisulfite and subjected to unbiased primers and the TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (*e.g.*, as might be found in a typical QM™ -based kit) for QM™ analysis may include, but are not limited to: PCR primers for specific gene (or bisulfite treated DNA sequence or CpG island); TaqMan® or Lightcycler® probes; optimized PCR buffers and deoxynucleotides; and Taq polymerase.

*Ms-SNuPE.* The Ms-SNuPE™ technique is a quantitative method for assessing methylation differences at specific CpG sites based on bisulfite treatment of DNA, followed by single-nucleotide primer extension (Gonzalgo & Jones, Nucleic Acids Res. 25:2529-2531, 1997). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest. Small amounts of DNA can be analyzed (*e.g.*, microdissected pathology sections), and it avoids utilisation of restriction enzymes for determining the methylation status at CpG sites.

Typical reagents (*e.g.*, as might be found in a typical Ms-SNuPE™-based kit) for Ms-SNuPE™ analysis may include, but are not limited to: PCR primers for specific gene (or bisulfite treated DNA sequence or CpG island); optimized PCR buffers and deoxynucleotides; gel extraction kit; positive control primers; Ms-SNuPE™ primers for specific gene; reaction buffer (for the Ms-SNuPE reaction); and labelled nucleotides. Additionally, bisulfite conversion reagents may include: DNA denaturation buffer; sulfonation buffer; DNA recovery regents or kit (*e.g.*, precipitation, ultrafiltration, affinity column); desulfonation buffer; and DNA recovery components.

The Genomic Sequence(s) According to SEQ ID NO: 1 TO SEQ ID NO: 12; SEQ ID NO: 79 TO SEQ ID NO: 83, and Non-naturally Occurring Treated Variants Thereof According to SEQ ID NO: 13 TO SEQ ID NO: 60; SEQ ID NO: 84 TO SEQ ID NO: 103, were Determined to have Novel Utility for the Detection of cell proliferative disorders, preferably those according to Table 2 (most preferably lung carcinoma).

In one embodiment the method of the invention comprises the following steps: i) determining the methylation level of the gene SHOX2 and ii) determining the presence or absence of lung, breast or bladder cancer. Preferred is a lung cancer selected from the group consisting of Lung adenocarcinoma; Large cell lung cancer; squamous cell lung carcinoma; Small cell lung carcinoma.

The method of the invention may be enabled by means of any analysis of the expression of an RNA transcribed therefrom or polypeptide or protein translated from said RNA, preferably by means of mRNA expression analysis or polypeptide expression analysis. However, in the most preferred embodiment of the invention the detection of cell proliferative disorders, preferably those according to Table 2 (most preferably lung carcinoma), is enabled by means of analysis of the *methylation status* of SHOX2 and/or promoter or regulatory elements thereof.

Also disclosed are diagnostic assays and methods, both quantitative and qualitative for detecting the expression of at least one gene or genomic sequence selected from the group consisting of PTGER4;RUNX1;EVX2;EVX-1; SHOX2;SEQ ID NO: 6;CN027;LRAT;IL-12RB1;TFAP2C;BCL2; EN2; PRDM14; SEQ ID NO: 81; ARID5A;VAX1 in a subject and determining therefrom upon the presence or absence of cell proliferative disorders, preferably those according to Table 2 (most preferably lung carcinoma) in said subject: Particularly preferred is a lung cancer selected from the group consisting of lung adenocarcinoma; large cell lung cancer; squamous cell lung carcinoma; small cell lung carcinoma.

Aberrant expression of mRNA transcribed from at least one gene or genomic sequence selected from the group consisting of PTGER4;RUNX1;EVX2;EVX-1; SHOX2;SEQ ID NO: 6;CN027;LRAT;IL-12RB1;TFAP2C;BCL2; EN2; PRDM14; SEQ ID NO: 81; ARID5A;VAX1 is associated with the presence of cell proliferative disorders, preferably those according to Table 2 (most preferably lung carcinoma) in a subject. Particularly preferred is a lung cancer selected from the group consisting of lung adenocarcinoma; large cell lung cancer; squamous cell lung carcinoma; small cell lung carcinoma. According to the present invention, hyper-methylation and /or under-expression is associated with the presence of cell proliferative disorders, in particular those according to Table 2 (most preferably lung carcinoma).

To detect the presence of mRNA encoding a gene or genomic sequence, a sample is obtained from a patient. The sample may be any suitable sample comprising cellular matter of the tumor. Suitable sample types include cells or cell lines, histological slides, biopsies, paraffin-embedded tissue, body fluids, ejaculate, urine, blood plasma, blood serum, whole blood, isolated blood cells, sputum and biological matter derived from bronchoscopy (including but not limited to bronchial lavage, bronchial alveolar lavage, bronchial brushing, bronchial abrasion and all possible combinations thereof. More preferably the samnpel type is selected form the group consisting of blood plasma, sputum and biological matter derived from bronchoscopy (including but not limited to bronchial lavage, bronchial alveolar lavage, bronchial brushing, bronchial abrasion) and all possible combinations thereof. The sample may be treated to extract the RNA contained therein. The resulting nucleic acid from the sample is then analysed. Many techniques are known in the state of the art for determining absolute and relative levels of gene expression, commonly used techniques suitable for use in the present invention include in situ hybridisation (e.g. FISH), Northern analysis, RNase protection assays (RPA), microarrays and PCR-based techniques, such as quantitative PCR and differential display PCR or any other nucleic acid detection method.

Particularly preferred is the use of the reverse transcription/polymerisation chain reaction technique (RT-PCR). The method of RT-PCR is well known in the art (for example, see Watson and Fleming, supra).

The RT-PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end oligonucleotide dT primer and/or random hexamer primers. The cDNA thus produced is then amplified by means of PCR. (Belyavsky et al, Nucl Acid Res 17:2919-2932, 1989; Krug and Berger, Methods in Enzymology, Academic Press, N.Y., Vol.152, pp. 316-325, 1987). Further preferred is the "Real-time" variant of RT- PCR, wherein the PCR product is detected by means of hybridisation probes (e.g. TaqMan, Lightcycler, Molecular Beacons & Scorpion) or SYBR green. The detected signal from the probes or SYBR green is then quantitated either by reference to a standard curve or by comparing the Ct values to that of a calibration standard. Analysis of housekeeping genes is often used to normalize the results.

In Northern blot analysis total or poly(A)+ mRNA is run on a denaturing agarose gel and detected by hybridisation to a labelled probe in the dried gel itself or on a membrane. The resulting signal is proportional to the amount of target RNA in the RNA population.

Comparing the signals from two or more cell populations or tissues reveals relative differences in gene expression levels. Absolute quantitation can be performed by comparing the signal to a standard curve generated using known amounts of an in vitro transcript corresponding to the target RNA. Analysis of housekeeping genes, genes whose expression levels are expected to remain relatively constant regardless of conditions, is often used to normalize the results, eliminating any apparent differences caused by unequal transfer of RNA to the membrane or unequal loading of RNA on the gel.

The first step in Northern analysis is isolating pure, intact RNA from the cells or tissue of interest. Because Northern blots distinguish RNAs by size, sample integrity influences the degree to which a signal is localized in a single band. Partially degraded RNA samples will result in the signal being smeared or distributed over several bands with an overall loss in sensitivity and possibly an erroneous interpretation of the data. In Northern blot analysis, DNA, RNA and oligonucleotide probes can be used and these probes are preferably labelled (e.g. radioactive labels, mass labels or fluorescent labels). The size of the target RNA, not the probe, will determine the size of the detected band, so methods such as random-primed labelling, which generates probes of variable lengths, are suitable for probe synthesis. The specific activity of the probe will determine the level of sensitivity, so it is preferred that probes with high specific activities, are used..

In an RNase protection assay, the RNA target and an RNA probe of a defined length are hybridised in solution. Following hybridisation, the RNA is digested with RNases specific for single-stranded nucleic acids to remove any unhybridized, single-stranded target RNA and probe. The RNases are inactivated, and the RNA is separated e.g. by denaturing polyacrylamide gel electrophoresis. The amount of intact RNA probe is proportional to the amount of target RNA in the RNA population. RPA can be used for relative and absolute quantitation of gene expression and also for mapping RNA structure, such as intron/exon boundaries and transcription start sites. The RNase protection assay is preferable to Northern blot analysis as it generally has a lower limit of detection.

The antisense RNA probes used in RPA are generated by in vitro transcription of a DNA template with a defined endpoint and are typically in the range of 50-600 nucleotides. The use of RNA probes that include additional sequences not homologous to the target RNA allows the protected fragment to be distinguished from the full-length probe. RNA probes are typically used instead of DNA probes due to the ease of generating single-stranded RNA probes and the reproducibility and reliability of RNA: RNA duplex digestion with RNases (Ausubel *et al*. 2003), particularly preferred are probes with high specific activities.

Particularly preferred is the use of microarrays. The microarray analysis process can be divided into two main parts. First is the immobilization of known gene sequences onto glass slides or other solid support followed by hybridisation of the fluorescently labelled cDNA (comprising the sequences to be interrogated) to the known genes immobilized on the glass slide (or other solid phase). After hybridisation, arrays are scanned using a fluorescent microarray scanner. Analysing the relative fluorescent intensity of different genes provides a measure of the differences in gene expression. DNA arrays can be generated by immobilizing presynthesized oligonucleotides onto prepared glass slides or other solid surfaces. In this case, representative gene sequences are manufactured and prepared using standard oligonucleotide synthesis and purification methods. These synthesized gene sequences are complementary to the RNA transcript(s) of at least one gene or genomic sequence selected from the group consisting of PTGER4;RUNX1;EVX2;EVX-1; SHOX2;SEQ ID NO: 6;CN027;LRAT;IL-12RB1;TFAP2C;BCL2; EN2; PRDM14; SEQ ID NO: 81; ARID5A;VAX1 and tend to be shorter sequences in the range of 25-70 nucleotides. Alternatively, immobilized oligos can be chemically synthesized in situ on the surface of the slide. In situ oligonucleotide synthesis involves the consecutive addition of the appropriate nucleotides to the spots on the microarray; spots not receiving a nucleotide are protected during each stage of the process using physical or virtual masks. Preferably said synthesized nucleic acids are locked nucleic acids.

In expression profiling microarray experiments, the RNA templates used are representative of the transcription profile of the cells or tissues under study. RNA is first isolated from the cell populations or tissues to be compared. Each RNA sample is then used as a template to generate fluorescently labelled cDNA via a reverse transcription reaction. Fluorescent labelling of the cDNA can be accomplished by either direct labelling or indirect labelling methods. During direct labelling, fluorescently modified nucleotides (e.g., Cy®3- or Cy®5-dCTP) are incorporated directly into the cDNA during the reverse transcription. Alternatively, indirect labelling can be achieved by incorporating aminoallyl-modified nucleotides during cDNA synthesis and then conjugating an N-hydroxysuccinimide (NHS)-ester dye to the aminoallyl-modified cDNA after the reverse transcription reaction is complete. Alternatively, the probe may be unlabelled, but may be detectable by specific binding with a ligand which is labelled, either directly or indirectly. Suitable labels and methods for labelling ligands (and probes) are known in the art, and include, for example, radioactive labels which may be incorporated by known methods (e.g., nick translation or kinasing). Other suitable labels include but are not limited to biotin, fluorescent groups, chemiluminescent groups (e.g., dioxetanes, particularly triggered dioxetanes), enzymes, antibodies, and the like.

To perform differential gene expression analysis, cDNA generated from different RNA samples are labelled with Cy®3. The resulting labelled cDNA is purified to remove unincorporated nucleotides, free dye and residual RNA. Following purification, the labelled cDNA samples are hybridised to the microarray. The stringency of hybridisation is determined by a number of factors during hybridisation and during the washing procedure, including temperature, ionic strength, length of time and concentration of formamide. These factors are outlined in, for example, Sambrook et al. (Molecular Cloning: A Laboratory Manual, 2nd ed., 1989). The microarray is scanned post-hybridisation using a fluorescent microarray scanner. The fluorescent intensity of each spot indicates the level of expression of the analysed gene; bright spots correspond to strongly expressed genes, while dim spots indicate weak expression.

Once the images are obtained, the raw data must be analysed. First, the background fluorescence must be subtracted from the fluorescence of each spot. The data is then normalized to a control sequence, such as exogenously added nucleic acids (preferably RNA or DNA), or a housekeeping gene panel to account for any nonspecific hybridisation, array imperfections or variability in the array set-up, cDNA labelling, hybridisation or washing. Data normalization allows the results of multiple arrays to be compared.

Also disclosed is a kit for use in diagnosis of cell proliferative disorders, preferably those according to Table 2 (most preferably lung carcinoma and further preferred is a lung cancer selected from the group consisting of lung adenocarcinoma; large cell lung cancer; squamous cell lung carcinoma; small cell lung carcinoma.) in a subject according to the methods of the present invention, said kit comprising: a means for measuring the level of transcription of at least one gene or genomic sequence selected from the group consisting of PTGER4;RUNX1;EVX2;EVX-1; SHOX2;SEQ ID NO: 6;CN027;LRAT;IL-12RB1;TFAP2C;BCL2; EN2; PRDM14; SEQ ID NO: 81; ARID5A;VAX1. The means for measuring the level of transcription may comprise oligonucleotides or polynucleotides able to hybridise under stringent or moderately stringent conditions to the transcription products of at least one gene or genomic sequence selected from the group consisting of PTGER4;RUNX1;EVX2;EVX-1; SHOX2;SEQ ID NO: 6;CN027;LRAT;IL-12RB1;TFAP2C;BCL2; EN2; PRDM14; SEQ ID NO: 81; ARID5A;VAX1. In a most preferred embodiment the level of transcription is determined by techniques selected from the group of Northern Blot analysis, reverse transcriptase PCR, real-time PCR, RNAse protection, and microarray. In another embodiment of the invention the kit further comprises means for obtaining a biological sample of the patient. Preferred is a kit, which further comprises a container which is most preferably suitable for containing the means for measuring the level of transcription and the biological sample of the patient, and most preferably further comprises instructions for use and interpretation of the kit results.

In a preferred embodiment the kit comprises (a) a plurality of oligonucleotides or polynucleotides able to hybridise under stringent or moderately stringent conditions to the transcription products of at least one gene or genomic sequence selected from the group consisting of PTGER4;RUNX1;EVX2;EVX-1; SHOX2;SEQ ID NO: 6;CN027;LRAT;IL-12RB1;TFAP2C;BCL2; EN2; PRDM14; SEQ ID NO: 81; ARID5A;VAX1; (b) a container, preferably suitable for containing the oligonucleotides or polynucleotides and a biological sample of the patient comprising the transcription products wherein the oligonucleotides or polynucleotides can hybridise under stringent or moderately stringent conditions to the transcription products, (c) means to detect the hybridisation of (b); and optionally, (d) instructions for use and interpretation of the kit results

The kit may also contain other components such as hybridisation buffer (where the oligonucleotides are to be used as a probe) packaged in a separate container. Alternatively, where the oligonucleotides are to be used to amplify a target region, the kit may contain, packaged in separate containers, a polymerase and a reaction buffer optimised for primer extension mediated by the polymerase, such as PCR. Preferably said polymerase is a reverse transcriptase. It is further preferred that said kit further contains an Rnase reagent.

The present invention further discloses for methods for the detection of the presence of the polypeptide encoded by said gene sequences in a sample obtained from a patient.

Aberrant levels of polypeptide expression of the polypeptides encoded at least one gene or genomic sequence selected from the group consisting of PTGER4;RUNX1;EVX2;EVX-1; SHOX2;SEQ ID NO: 6;CN027;LRAT;IL-12RB1;TFAP2C;BCL2; EN2; PRDM14; SEQ ID NO: 81; ARID5A;VAX1 are associated with the presence of cell proliferative disorders, preferably those according to Table 2 (most preferably lung carcinoma). Particularly preferred is a lung cancer selected from the group consisting of lung adenocarcinoma; large cell lung cancer; squamous cell lung carcinoma; small cell lung carcinoma.

According to the present invention under-expression of said polypeptides is associated with the presence of cell proliferative disorders, preferably those according to Table 2 (most preferably lung carcinoma). Particularly preferred is a lung cancer selected from the group consisting of lung adenocarcinoma; large cell lung cancer; squamous cell lung carcinoma; small cell lung carcinoma.

Any method known in the art for detecting polypeptides can be used. Such methods include, but are not limited to masss-spectrometry, immunodiffusion, immunoelectrophoresis, immunochemical methods, binder-ligand assays, immunohistochemical techniques, agglutination and complement assays (e.g., see Basic and Clinical Immunology, Sites and Terr, eds., Appleton & Lange, Norwalk, Conn. pp 217-262, 1991 which is incorporated by reference). Preferred are binder-ligand immunoassay methods including reacting antibodies with an epitope or epitopes and competitively displacing a labelled polypeptide or derivative thereof.

Certain embodiments of the above mentioned kit comprise the use of antibodies specific to the polypeptide(s) encoded by at least one gene or genomic sequence selected from the group consisting of PTGER4;RUNX1;EVX2;EVX-1; SHOX2;SEQ ID NO: 6;CN027;LRAT;IL-12RB1;TFAP2C;BCL2; EN2; PRDM14; SEQ ID NO: 81; ARID5A;VAX1.

Such antibodies are useful for cell proliferative disorders, preferably of those diseases according to Table 2, and most preferably in the diagnosis of lung carcinoma. Particularly preferred is a lung cancer selected from the group consisting of lung adenocarcinoma; large cell lung cancer; squamous cell lung carcinoma; small cell lung carcinoma. In certain embodiments production of monoclonal or polyclonal antibodies can be induced by the use of an epitope encoded by a polypeptide of at least one gene or genomic sequence selected from the group consisting of PTGER4;RUNX1;EVX2;EVX-1; SHOX2;SEQ ID NO: 6;CN027;LRAT;IL-12RB1;TFAP2C;BCL2; EN2; PRDM14; SEQ ID NO: 81; ARID5A;VAX1 as an antigene. Such antibodies may in turn be used to detect expressed polypeptides as markers for cell proliferative disorders, preferably those according to Table 2 and most preferably the diagnosis of lung carcinoma. Particularly preferred is a lung cancer selected from the group consisting of lung adenocarcinoma; large cell lung cancer; squamous cell lung carcinoma; small cell lung carcinoma. The levels of such polypeptides present may be quantified by conventional methods. Antibody-polypeptide binding may be detected and quantified by a variety of means known in the art, such as labelling with fluorescent or radioactive ligands. The invention further discloses kits for performing the above-mentioned procedures, wherein such kits contain antibodies specific for the investigated polypeptides.

Numerous competitive and non-competitive polypeptide binding immunoassays are well known in the art. Antibodies employed in such assays may be unlabelled, for example as used in agglutination tests, or labelled for use a wide variety of assay methods. Labels that can be used include radionuclides, enzymes, fluorescers, chemiluminescers, enzyme substrates or co-factors, enzyme inhibitors, particles, dyes and the like. Preferred assays include but are not limited to radioimmunoassay (RIA), enzyme immunoassays, e.g., enzyme-linked immunosorbent assay (ELISA), fluorescent immunoassays and the like. Polyclonal or monoclonal antibodies or epitopes thereof can be made for use in immunoassays by any of a number of methods known in the art.

In an alternative embodiment of the method the proteins may be detected by means of western blot analysis. Said analysis is standard in the art, briefly proteins are separated by means of electrophoresis e.g. SDS-PAGE. The separated proteins are then transferred to a suitable membrane (or paper) e.g. nitrocellulose, retaining the spacial separation achieved by electrophoresis. The membrane is then incubated with a blocking agent to bind remaining sticky places on the membrane, commonly used agents include generic protein (e.g. milk protein). An antibody specific to the protein of interest is then added, said antibody being detectably labelled for example by dyes or enzymatic means (e.g. alkaline phosphatase or horseradish peroxidase). The location of the antibody on the membrane is then detected.

In an alternative embodiment of the method the proteins may be detected by means of immunohistochemistry (the use of antibodies to probe specific antigens in a sample). Said analysis is standard in the art, wherein detection of antigens in tissues is known as immunohistochemistry, while detection in cultured cells is generally termed immunocytochemistry. Briefly the primary antibody to be detected by binding to its specific antigen. The antibody-antigen complex is then bound by a secondary enzyme conjugated antibody. In the presence of the necessary substrate and chromogen the bound enzyme is detected according to coloured deposits at the antibody-antigen binding sites. There is a wide range of suitable sample types, antigen-antibody affinity, antibody types, and detection enhancement methods. Thus optimal conditions for immunohistochemical or immunocytochemical detection must be determined by the person skilled in the art for each individual case.

One approach for preparing antibodies to a polypeptide is the selection and preparation of an amino acid sequence of all or part of the polypeptide, chemically synthesising the amino acid sequence and injecting it into an appropriate animal, usually a rabbit or a mouse (Milstein and Kohler Nature 256:495-497, 1975; Gulfre and Milstein, Methods in Enzymology: Immunochemical Techniques 73:1-46, Langone and Banatis eds., Academic Press, 1981 which are incorporated by reference in its entirety). Methods for preparation of the polypeptides or epitopes thereof include, but are not limited to chemical synthesis, recombinant DNA techniques or isolation from biological samples.

In the final step of the method the diagnosis of the patient is determined, whereby under-expression (of mRNA or polypeptides) is indicative of the presence of cell proliferative disorders, preferably those according to Table 2 (most preferably lung carcinoma). Particularly preferred is a lung cancer selected from the group consisting of lung adenocarcinoma; large cell lung cancer; squamous cell lung carcinoma; small cell lung carcinoma. The term under-expression shall be taken to mean expression at a detected level less than a pre-determined cut off which may be selected from the group consisting of the mean, median or an optimised threshold value. The term over-expression shall be taken to mean expression at a detected level greater than a pre-determined cut off which may be selected from the group consisting of the mean, median or an optimised threshold value.

Another aspect of the invention discloses a kit for use in diagnosis of cell proliferative disorders, preferably those according to Table 2 (most preferably lung carcinoma) in a subject according to the methods of the present invention, comprising: a means for detecting at least one gene or genomic sequence selected from the group consisting of PTGER4;RUNX1;EVX2;EVX-1; SHOX2;SEQ ID NO: 6;CN027;LRAT;IL-12RB1;TFAP2C;BCL2; EN2; PRDM14; SEQ ID NO: 81; ARID5A;VAX1 polypeptides. The means for detecting the polypeptides comprise preferably antibodies, antibody derivatives, or antibody fragments. The polypeptides are most preferably detected by means of Western Blotting utilizing a labelled antibody. In another embodiment of the invention the kit further comprising means for obtaining a biological sample of the patient. Preferred is a kit, which further comprises a container suitable for containing the means for detecting the polypeptides in the biological sample of the patient, and most preferably further comprises instructions for use and interpretation of the kit results. In a preferred embodiment the kit comprises: (a) a means for detecting at least one gene or genomic sequence selected from the group consisting of PTGER4;RUNX1;EVX2;EVX-1; SHOX2;SEQ ID NO: 6;CN027;LRAT;IL-12RB1;TFAP2C;BCL2; EN2; PRDM14; SEQ ID NO: 81; ARID5A;VAX1 polypeptides; (b) a container suitable for containing the said means and the biological sample of the patient comprising the polypeptides wherein the means can form complexes with the polypeptides; (c) a means to detect the complexes of (b); and optionally (d) instructions for use and interpretation of the kit results.

The kit may also contain other components such as buffers or solutions suitable for blocking, washing or coating , packaged in a separate container.

Particular embodiments of the present invention provide a novel application of the analysis of methylation levels and/or patterns within SHOX2 that enables a precise detection, characterisation and/or treatment of cell proliferative disorders, preferably those according to Table 2 (most preferably lung carcinoma). Particularly preferred is a lung cancer selected from the group consisting of lung adenocarcinoma; large cell lung cancer; squamous cell lung carcinoma; small cell lung carcinoma. Early detection of cell proliferative disorders, I prticular lung carcinoma, is directly linked with disease prognosis, and the disclosed method thereby enables the physician and patient to make better and more informed treatment decisions.

In the most preferred embodiment of the method, the presence or absence of cell proliferative disorders, preferably those according to Table 2 (most preferably lung carcinoma, in particular a lung cancer selected from the group consisting of lung adenocarcinoma; large cell lung cancer; squamous cell lung carcinoma; small cell lung carcinoma.) is determined by analysis of the methylation status of one or more CpG dinucleotides of SHOX2.

In one embodiment the invention of said method comprises the following steps: i) contacting genomic DNA (preferably isolated from body fluids) obtained from the subject with at least one reagent, or series of reagents that distinguishes between methylated and non-methylated CpG dinucleotides within SHOX2 (including promoter and regulatory regions thereof) and ii) detecting cell proliferative disorders, preferably those according to Table 2 (most preferably lung carcinoma). Particularly preferred is a lung cancer selected from the group consisting of lung adenocarcinoma; large cell lung cancer; squamous cell lung carcinoma; small cell lung carcinoma.

It is preferred that said one or more CpG dinucleotides of SHOX2 are comprised within a respective genomic target sequence thereof as provided in SEQ ID NO:5 and complements thereof. The present invention further provides a method for ascertaining genetic and/or epigenetic parameters of SHOX2 and/or the genomic sequence according to SEQ ID NO:5 within a subject by analysing cytosine methylation. Said method comprising contacting a nucleic acid comprising SEQ ID NO:5 in a biological sample obtained from said subject with at least one reagent or a series of reagents, wherein said reagent or series of reagents, distinguishes between methylated and non-methylated CpG dinucleotides within the target nucleic acid.

In a preferred embodiment, said method comprises the following steps: In the *first step,* a sample of the tissue to be analysed is obtained. The source may be any suitable source, such as cells or cell lines, histological slides, biopsies, paraffin-embedded tissue, body fluids, ejaculate, urine, blood plasma, blood serum, whole blood, isolated blood cells, sputum and biological matter derived from bronchoscopy (including but not limited to bronchial lavage, bronchial alveolar lavage, bronchial brushing, bronchial abrasion and all possible combinations thereof. More preferably the samnpel type is selected form the group consisting of blood plasma, sputum and biological matter derived from bronchoscopy (including but not limited to bronchial lavage, bronchial alveolar lavage, bronchial brushing, bronchial abrasion) and all possible combinations thereof.

The genomic DNA is then isolated from the sample. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA.

Wherein the sample DNA is not enclosed in a membrane (e.g. circulating DNA from a blood sample) methods standard in the art for the isolation and/or purification of DNA may be employed. Such methods include the use of a protein degenerating reagent e.g. chaotropic salt e.g. guanidine hydrochloride or urea; or a detergent e.g. sodium dodecyl sulphate (SDS), cyanogen bromide. Alternative methods include but are not limited to ethanol precipitation or propanol precipitation, vacuum concentration amongst others by means of a centrifuge. The person skilled in the art may also make use of devices such as filter devices e.g.ultrafiltration , silica surfaces or membranes, magnetic particles, polystyrol particles, polystyrol surfaces, positively charged surfaces, and positively charged membranse, charged membranes, charged surfaces, charged switch membranes, charged switched surfaces.

Once the nucleic acids have been extracted, the genomic double stranded DNA is used in the analysis.

In the *second step* of the method, the genomic DNA sample is treated in such a manner that cytosine bases which are unmethylated at the 5'-position are converted to uracil, thymine, or another base which is dissimilar to cytosine in terms of hybridisation behaviour. This will be understood as 'pre-treatment' or 'treatment' herein.

This is preferably achieved by means of treatment with a bisulfite reagent. The term "bisulfite reagent" refers to a reagent comprising bisulfite, disulfite, hydrogen sulfite or combinations thereof, useful as disclosed herein to distinguish between methylated and unmethylated CpG dinucleotide sequences. Methods of said treatment are known in the art (e.g. PCT/EP2004/011715). It is preferred that the bisulfite treatment is conducted in the presence of denaturing solvents such as but not limited to n-alkylenglycol, particularly diethylene glycol dimethyl ether (DME), or in the presence of dioxane or dioxane derivatives. In a preferred embodiment the denaturing solvents are used in concentrations between 1% and 35% (v/v). It is also preferred that the bisulfite reaction is carried out in the presence of scavengers such as but not limited to chromane derivatives, e.g., 6-hydroxy-2, 5,7,8, -tetramethylchromane 2-carboxylic acid or trihydroxybenzoe acid and derivates thereof, e.g. Gallic acid (see: PCT/EP2004/011715). The bisulfite conversion is preferably carried out at a reaction temperature between 30°C and 70°C, whereby the temperature is increased to over 85°C for short periods of times during the reaction (see: PCT/EP2004/011715). The bisulfite treated DNA is preferably purified priori to the quantification. This may be conducted by any means known in the art, such as but not limited to ultrafiltration, preferably carried out by means of Microcon^(TM) columns (manufactured by Millipore^(TM)). The purification is carried out according to a modified manufacturer's protocol (see: PCT/EP2004/011715).

In the *third step* of the method, fragments of the treated DNA are amplified, using sets of primer oligonucleotides according to the present invention, and an amplification enzyme. The amplification of several DNA segments can be carried out simultaneously in one and the same reaction vessel. Typically, the amplification is carried out using a polymerase chain reaction (PCR). Preferably said amplificates are 100 to 2,000 base pairs in length. The set of primer oligonucleotides includes at least two oligonucleotides whose sequences are each reverse complementary, identical, or hybridise under stringent or highly stringent conditions to an at least 16-base-pair long segment of the base sequences of one of SEQ ID NO: 21, 22, 45 and 46 and sequences complementary thereto. In an alternate embodiment of the method, the methylation status of pre-selected CpG positions within SHOX2 and preferably within the nucleic acid sequence according to SEQ ID NO:5, may be detected by use of methylation-specific primer oligonucleotides. This technique (MSP) has been described in United States Patent No. 6,265,171 to Herman. The use of methylation status specific primers for the amplification of bisulfite treated DNA allows the differentiation between methylated and unmethylated nucleic acids. MSP primers pairs contain at least one primer which hybridises to a bisulfite treated CpG dinucleotide. Therefore, the sequence of said primers comprises at least one CpG dinucleotide. MSP primers specific for non-methylated DNA contain a "T' at the position of the C position in the CpG. Preferably, therefore, the base sequence of said primers is required to comprise a sequence having a length of at least 9 nucleotides which hybridises to a treated nucleic acid sequence according to one of SEQ ID NO: 21, 22, 45 and 46 and sequences complementary thereto, wherein the base sequence of said oligomers comprises at least one CpG dinucleotide .A further preferred embodiment of the method comprises the use of *blocker* oligonucleotides (the HeavyMethyl™ assay). The use of such blocker oligonucleotides has been described by Yu et al., BioTechniques 23:714-720, 1997. Blocking probe oligonucleotides are hybridised to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpA' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivitized at the 3' position with other than a "free" hydroxyl group. For example, 3'-O-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-terminii thereof that render the blocker molecule nuclease-resistant. Particular applications may not require such 5' modifications of the blocker. For example, if the blocker- and primer-binding sites overlap, thereby precluding binding of the primer (*e.g.*, with excess blocker), degradation of the blocker oligonucleotide will be substantially precluded. This is because the polymerase will not extend the primer toward, and through (in the 5'-3' direction) the blocker-a process that normally results in degradation of the hybridized blocker oligonucleotide.

A particularly preferred blocker/PCR embodiment, for purposes of the present invention and as implemented herein, comprises the use of peptide nucleic acid (PNA) oligomers as blocking oligonucleotides. Such PNA blocker oligomers are ideally suited, because they are neither decomposed nor extended by the polymerase.

Preferably, therefore, the base sequence of said *blocking oligonucleotides* is required to comprise a sequence having a length of at least 9 nucleotides which hybridises to a treated nucleic acid sequence according to one of SEQ ID NO: 21, 22, 45 and 46 and sequences complementary thereto, wherein the base sequence of said oligonucleotides comprises at least one CpG, TpG or CpA dinucleotide.

The fragments obtained by means of the amplification can carry a directly or indirectly detectable label. Preferred are labels in the form of fluorescence labels, radionuclides, or detachable molecule fragments having a typical mass which can be detected in a mass spectrometer. Where said labels are mass labels, it is preferred that the labelled amplificates have a single positive or negative net charge, allowing for better delectability in the mass spectrometer. The detection may be carried out and visualized by means of, e.g., matrix assisted laser desorption/ionization mass spectrometry (MALDI) or using electron spray mass spectrometry (ESI).

Matrix Assisted Laser Desorption/Ionization Mass Spectrometry (MALDI-TOF) is a very efficient development for the analysis of biomolecules (Karas & Hillenkamp, Anal Chem., 60:2299-301, 1988). An analyte is embedded in a light-absorbing matrix. The matrix is evaporated by a short laser pulse thus transporting the analyte molecule into the vapor phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut & Beck, Current Innovations and Future Trends, 1:147-57, 1995). The sensitivity with respect to nucleic acid analysis is approximately 100-times less than for peptides, and decreases disproportionally with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallisation. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut & Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

In the *fourth step* of the method, the amplificates obtained during the third step of the method are analysed in order to ascertain the methylation status of the CpG dinucleotides prior to the treatment.

In embodiments where the amplificates were obtained by means of MSP amplification, the presence or absence of an amplificate is in itself indicative of the methylation state of the CpG positions covered by the primer, according to the base sequences of said primer.

Amplificates obtained by means of both standard and methylation specific PCR may be further analysed by means of based-based methods such as, but not limited to, array technology and probe based technologies as well as by means of techniques such as sequencing and template directed extension.

In one embodiment of the method, the amplificates synthesised in *step three* are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9 nucleotides which is reverse complementary or identical to a segment of the base sequences specified in the present Sequence Listing; and the segment comprises at least one CpG , TpG or CpA dinucleotide. The hybridizing portion of the hybridizing nucleic acids is typically at least 9, 15, 20, 25, 30 or 35 nucleotides in length. However, longer molecules have inventive utility, and are thus within the scope of the present invention. In a preferred embodiment, said dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within a sequence according to SEQ ID NO: 5, and the equivalent positions within SEQ ID NO: 21, 22, 45 or 46. Said oligonucleotides may also be present in the form of peptide nucleic acids. The non-hybridised amplificates are then removed. The hybridised amplificates are then detected. In this context, it is preferred that labels attached to the amplificates are identifiable at each position of the solid phase at which an oligonucleotide sequence is located.

In yet a further embodiment of the method, the genomic methylation status of the CpG positions may be ascertained by means of oligonucleotide probes (as detailed above) that are hybridised to the bisulfite treated DNA concurrently with the PCR amplification primers (wherein said primers may either be methylation specific or standard).

A particularly preferred embodiment of this method is the use of fluorescence-based Real Time Quantitative PCR (Heid et al., Genome Res. 6:986-994, 1996; *also* see United States Patent No. 6,331,393) employing a dual-labelled fluorescent oligonucleotide probe (TaqMan™ PCR, using an ABI Prism 7700 Sequence Detection System, Perkin Elmer Applied Biosystems, Foster City, California). The TaqMan™ PCR reaction employs the use of a non-extendible interrogating oligonucleotide, called a TaqMan™ probe, which, in preferred embodiments, is designed to hybridise to a CpG-rich sequence located between the forward and reverse amplification primers. The TaqMan™ probe further comprises a fluorescent "reporter moiety" and a "quencher moiety" covalently bound to linker moieties (e.g., phosphoramidites) attached to the nucleotides of the TaqMan™ oligonucleotide. For analysis of methylation within nucleic acids subsequent to bisulfite treatment, it is required that the probe be methylation specific, as described in United States Patent No. 6,331,393, also known as the MethyLightMT™ assay. Variations on the TaqMan™ detection methodology that are also suitable for use with the described invention include the use of dual-probe technology (Lightcycler™) or fluorescent amplification primers (Sunrise™ technology). Both these techniques may be adapted in a manner suitable for use with bisulfite treated DNA, and moreover for methylation analysis within CpG dinucleotides.

In a further preferred embodiment of the method, the *fourth step* of the method comprises the use of template-directed oligonucleotide extension, such as MS-SNuPE as described by Gonzalgo & Jones, Nucleic Acids Res. 25:2529-2531, 1997.

In yet a further embodiment of the method, the *fourth step* of the method comprises sequencing and subsequent sequence analysis of the amplificate generated in the *third step* of the method (Sanger F., et al., Proc Natl Acad Sci USA 74:5463-5467, 1977).

Best mode

In the most preferred embodiment of the method the genomic nucleic acids are isolated and treated according to the first three steps of the method outlined above, namely:

a) obtaining, from a subject, a biological sample having subject genomic DNA;
b) extracting or otherwise isolating the genomic DNA;
c) treating the genomic DNA of b), or a fragment thereof, with one or more reagents to convert cytosine bases that are unmethylated in the 5-position thereof to uracil or to another base that is detectably dissimilar to cytosine in terms of hybridization properties; and wherein
d) amplifying subsequent to treatment in c) is carried out in a methylation specific manner, namely by use of methylation specific primers or *blocking oligonucleotides,* and further wherein
e) detecting of the amplificates is carried out by means of a real-time detection probe, as described above.

Preferably, where the subsequent amplification of d) is carried out by means of methylation specific primers, as described above, said methylation specific primers comprise a sequence having a length of at least 9 nucleotides which hybridises to a treated nucleic acid sequence according to one of SEQ ID NO: 21, 22, 45 and 46 and sequences complementary thereto, wherein the base sequence of said oligomers comprise at least one CpG dinucleotide.

Step e) of the method, namely the detection of the specific amplificates indicative of the methylation status of one or more CpG positions according to SEQ ID NO: 5 is carried out by means of *real-time* detection methods as described above.

· Additional embodiments of the invention provide a method for the analysis of the methylation status of SHOX2 (preferably SEQ ID NO: 5 and complements thereof) without the need for bisulfite conversion. Methods are known in the art wherein a methylation sensitive restriction enzyme reagent, or a series of restriction enzyme reagents comprising methylation sensitive restriction enzyme reagents that distinguishes between methylated and non-methylated CpG dinucleotides within a target region are utilized in determining methylation, for example but not limited to DMH.

In the *first step* of such additional embodiments, the genomic DNA sample is isolated from tissue or cellular sources. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants, e.g., by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. All clinical sample types comprising neoplastic or potentially neoplastic matter are suitable for us e in the present method, preferred are cells or cell lines, histological slides, biopsies, paraffin-embedded tissue, body fluids, ejaculate, urine, blood plasma, blood serum, whole blood, isolated blood cells, sputum and biological matter derived from bronchoscopy (including but not limited to bronchial lavage, bronchial alveolar lavage, bronchial brushing, bronchial abrasion and combinations thereof. More preferably the samnpel type is selected form the group consisting of blood plasma, sputum and biological matter derived from bronchoscopy (including but not limited to bronchial lavage, bronchial alveolar lavage, bronchial brushing, bronchial abrasion) and all possible combinations thereof.

Once the nucleic acids have been extracted, the genomic double-stranded DNA is used in the analysis.

In a preferred embodiment, the DNA may be cleaved prior to treatment with methylation sensitive restriction enzymes. Such methods are known in the art and may include both physical and enzymatic means. Particularly preferred is the use of one or a plurality of restriction enzymes which are not methylation sensitive, and whose recognition sites are AT rich and do not comprise CG dinucleotides. The use of such enzymes enables the conservation of CpG islands and CpG rich regions in the fragmented DNA. The non-methylation-specific restriction enzymes are preferably selected from the group consisting of MseI, BfaI, Csp6I, Tru1I, Tvu1I, Tru9I, Tvu9I, MaeI and XspI. Particularly preferred is the use of two or three such enzymes. Particularly preferred is the use of a combination of MseI, BfaI and Csp6I.

The fragmented DNA may then be ligated to adaptor oligonucleotides in order to facilitate subsequent enzymatic amplification. The ligation of oligonucleotides to blunt and sticky ended DNA fragments is known in the art, and is carried out by means of dephosphorylation of the ends (e.g. using calf or shrimp alkaline phosphatase) and subsequent ligation using ligase enzymes (e.g. T4 DNA ligase) in the presence of dATPs. The adaptor oligonucleotides are typically at least 18 base pairs in length.

In the *third step,* the DNA (or fragments thereof) is then digested with one or more methylation sensitive restriction enzymes. The digestion is carried out such that hydrolysis of the DNA at the restriction site is informative of the methylation status of a specific CpG dinucleotide of the SHOX2 gene.

Preferably, the methylation-specific restriction enzyme is selected from the group consisting of *Bsi E1, Hga I HinPI, Hpy99I, Ava I, Bce AI, Bsa HI, BisI, BstUI, BshI236I, AccII, BstFNI, McrBC; GlaI, MvnI, HpaII (HapII), HhaI, AciI, SmaI, HinP1I', HpyCH4IV, EagI* and mixtures of two or more of the above enzymes. Preferred is a mixture containing the restriction enzymes BstUI, HpaII, HpyCH4IV and HinP1I.

In the *fourth step,* which is optional but a preferred embodiment, the restriction fragments are amplified. This is preferably carried out using a polymerase chain reaction, and said amplificates may carry suitable detectable labels as discussed above, namely fluorophore labels, radionuclides and mass labels. Particularly preferred is amplification by means of an amplification enzyme and at least two primers comprising, in each case a contiguous sequence at least 16 nucleotides in length that is complementary to, or hybridizes under moderately stringent or stringent conditions to a sequence as defined by SEQ ID NO: 5, and complements thereof. Preferably said contiguous sequence is at least 16, 20 or 25 nucleotides in length. In an alternative embodiment said primers may be complementary to any adaptors linked to the fragments.

In the *fifth step* the amplificates are detected. The detection may be by any means standard in the art, for example, but not limited to, gel electrophoresis analysis, hybridisation analysis, incorporation of detectable tags within the PCR products, DNA array analysis, MALDI or ESI analysis. Preferably said detection is carried out by hybridisation to at least one nucleic acid or peptide nucleic acid comprising in each case a contiguous sequence at least 16 nucleotides in length that is complementary to, or hybridizes under moderately stringent or stringent conditions to a sequence as defined by SEQ ID NO: 5, and complements thereof. Preferably said contiguous sequence is at least 16, 20 or 25 nucleotides in length.

Subsequent to the determination of the methylation state or level of the genomic nucleic acids the presence, absence of cell proliferative disorders, preferably those according to Table 2 (most preferably lung carcinoma), is deduced based upon the methylation state or level of at least one CpG dinucleotide sequence of SEQ ID NO: 5, or an average, or a value reflecting an average methylation state of a plurality of CpG dinucleotide sequences of SEQ ID NO: 5 wherein methylation is associated with the presence of cell proliferative disorders, preferably those according to Table 2 (most preferably lung carcinoma). Wherein said methylation is determined by quantitative means the cut-off point for determining said the presence of methylation is preferably zero (i.e. wherein a sample displays any degree of methylation it is determined as having a methylated status at the analysed CpG position). Nonetheless, it is foreseen that the person skilled in the art may wish to adjust said cut-off value in order to provide an assay of a particularly preferred sensitivity or specificity. Accordingly said cut-off value may be increased (thus increasing the specificity), said cut off value may be within a range selected form the group consisting of 0%-5%, 5%-10%, 10%-15%, 15%-20%, 20%-30% and 30%-50%. Particularly preferred are the cut-offs 10%, 15%, 25%, and 30%.

Upon determination of the methylation of SHOX2 the presence or absence of cell proliferative disorders, preferably those according to Table 2 (most preferably lung carcinoma) is determined, wherein hyper-methylation indicates the presence of cell proliferative disorders, preferably those according to Table 2 (most preferably lung carcinoma) and hypo-methylation indicates the absence of cell proliferative disorders, preferably those according to Table 2 (most preferably lung carcinoma) within the subject. Particularly preferred is a lung cancer selected from the group consisting of lung adenocarcinoma; large cell lung cancer; squamous cell lung carcinoma; small cell lung carcinoma.

## FURTHER IMPROVEMENTS

The disclosed invention discloses treated nucleic acids, derived from genomic SEQ ID NO: 1 to SEQ ID NO: 12; SEQ ID NO: 79 to SEQ ID NO: 83, wherein the treatment is suitable to convert at least one unmethylated cytosine base of the genomic DNA sequence to uracil or another base that is detectably dissimilar to cytosine in terms of hybridization. The genomic sequences in question may comprise one, or more consecutive methylated CpG positions. Said treatment preferably comprises use of a reagent selected from the group consisting of bisulfite, hydrogen sulfite, disulfite, and combinations thereof. In a preferred embodiment of the invention, the invention discloses a non-naturally occurring modified nucleic acid comprising a sequence of at least 16 contiguous nucleotide bases in length of a sequence selected from the group consisting of SEQ ID NO: 13 TO SEQ ID NO: 60; SEQ ID NO: 84 TO SEQ ID NO: 103. Said nucleic acid is at least 50, 100, 150, 200, 250 or 500 base pairs in length of a segment of the nucleic acid sequence disclosed in SEQ ID NO: 13 to SEQ ID NO: 60; SEQ ID NO: 84 to SEQ ID NO: 103. Also disclosed is a nucleic acid molecule that is not identical or complementary to all or a portion of the sequences SEQ ID NO: 13 to SEQ ID NO: 60; SEQ ID NO: 84 to SEQ ID NO: 103 but not SEQ ID NO: 1 to SEQ ID NO: 12; SEQ ID NO: 79 to SEQ ID NO: 83 or other naturally occurring DNA.

It is preferred that said sequence comprises at least one CpG, TpA or CpA dinucleotide and sequences complementary thereto. The sequences of SEQ ID NO: 13 TO SEQ ID NO: 60; SEQ ID NO: 84 TO SEQ ID NO: 103 provide non-naturally occurring modified versions of the nucleic acid according to SEQ ID NO: 1 TO SEQ ID NO: 12; SEQ ID NO: 79 TO SEQ ID NO: 83, wherein the modification of each genomic sequence results in the synthesis of a nucleic acid having a sequence that is unique and distinct from said genomic sequence as follows. For each sense strand genomic DNA, e.g., SEQ ID NO: 1 to SEQ ID NO: 12; SEQ ID NO: 79 to SEQ ID NO: 83, four converted versions are disclosed. A first version wherein "C" is converted to "T," but "CpG" remains "CpG" (*i.e.*, corresponds to case where, for the genomic sequence, all "C" residues of CpG dinucleotide sequences are methylated and are thus not converted); a second version discloses the complement of the disclosed genomic DNA sequence (i.e. antisense strand), wherein "C" is converted to "T," but "CpG" remains "CpG" (*i.e.*, corresponds to case where, for all "C" residues of CpG dinucleotide sequences are methylated and are thus not converted). The 'upmethylated' converted sequences of SEQ ID NO: 1 to SEQ ID NO: 12; SEQ ID NO: 79 to SEQ ID NO: 83 correspond to SEQ ID NO: 13 & SEQ ID NO: 36; SEQ ID NO: 84 to SEQ ID NO: 93. A third chemically converted version of each genomic sequences is disclosed, wherein "C" is converted to "T" for all "C" residues, including those of "CpG" dinucleotide sequences (*i.e.*, corresponds to case where, for the genomic sequences, all "C" residues of CpG dinucleotide sequences are unmethylated); a final chemically converted version of each sequence, discloses the complement of the disclosed genomic DNA sequence (i.e. *anti*sense strand), wherein "C" is converted to "T" for all "C" residues, including those of "CpG" dinucleotide sequences (*i.e.*, corresponds to case where, for the complement (*anti*sense strand) of each genomic sequence, all "C" residues of CpG dinucleotide sequences are unmethylated). The 'downmethylated' converted sequences of SEQ ID NO: 1 to SEQ ID NO: 12; SEQ ID NO: 79 to SEQ ID NO: 83 corresponds to SEQ ID NO: 37 to SEQ ID NO: 60; ; SEQ ID NO: 84 to SEQ ID NO: 93.

Significantly, heretofore, the nucleic acid sequences and molecules according SEQ ID NO: 13 to SEQ ID NO: 60; SEQ ID NO: 84 to SEQ ID NO: 103 were not implicated in or connected with the detection or diagnosis of cell proliferative disorders, preferably those according to Table 2 (most preferably lung carcinoma). Particularly preferred is a lung cancer selected from the group consisting of lung adenocarcinoma; large cell lung cancer; squamous cell lung carcinoma; small cell lung carcinoma.

In an alternative preferred embodiment, the invention further provides oligonucleotides or oligomers suitable for use in the methods of the invention for detecting the cytosine methylation state within genomic or treated (chemically modified) DNA, according to SEQ ID NO: 5, 21, 22, 45 or 46. Said oligonucleotide or oligomer nucleic acids provide novel diagnostic means. Said oligonucleotide or oligomer comprising a nucleic acid sequence having a length of at least nine (9) nucleotides which is identical to, hybridizes, under moderately stringent or stringent conditions (as defined herein above), to a treated nucleic acid sequence according to SEQ ID NO: 21, 22, 45 and 46 and/or sequences complementary thereto, or to a genomic sequence according to SEQ ID NO: 5 and/or sequences complementary thereto.

Thus, the present invention includes nucleic acid molecules (e.g., oligonucleotides and peptide nucleic acid (PNA) molecules (PNA-oligomers)) that hybridize under moderately stringent and/or stringent hybridization conditions to all or a portion of the sequences SEQ ID NO: 5, 21, 22, 45 or 46 or to the complements thereof. Particularly preferred is a nucleic acid molecule that hybridizes under moderately stringent and/or stringent hybridization conditions to all or a portion of the sequences SEQ ID NO: 21, 22, 45 or 46; but not SEQ ID NO: 5 or other human genomic DNA.

The identical or hybridizing portion of the hybridizing nucleic acids is typically at least 9, 16, 20, 25, 30 or 35 nucleotides in length. However, longer molecules have inventive utility, and are thus within the scope of the present invention.

Preferably, the hybridizing portion of the inventive hybridizing nucleic acids is at least 95%, or at least 98%, or 100% identical to the sequence, or to a portion thereof of SEQ ID NO: 5, 21, 22, 45 or 46, or to the complements thereof.

Hybridizing nucleic acids of the type described herein can be used, for example, as a primer (*e.g.*, a PCR primer), or a diagnostic probe or primer. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions.

For target sequences that are related and substantially identical to the corresponding sequence of SEQ ID NO: 5 (such as allelic variants and SNPs), rather than identical, it is useful to first establish the lowest temperature at which only homologous hybridization occurs with a particular concentration of salt (*e.g.*, SSC or SSPE). Then, assuming that 1% mismatching results in a 1 °C decrease in the Tm, the temperature of the final wash in the hybridization reaction is reduced accordingly (for example, if sequences having > 95% identity with the probe are sought, the final wash temperature is decreased by 5°C). In practice, the change in Tm can be between 0.5°C and 1.5°C per 1% mismatch.

Examples of oligonucleotides of length X (in nucleotides), as indicated by polynucleotide positions with reference to, *e.g.*, SEQ ID NO: 1, include those corresponding to sets (sense and antisense sets) of consecutively overlapping oligonucleotides of length X, where the oligonucleotides within each consecutively overlapping set (corresponding to a given X value) are defined as the finite set of Z oligonucleotides from nucleotide positions:

$$n \text{ to } (n + (X-1));$$

where n=1, 2, 3,...(Y-(X-1));
where Y equals the length (nucleotides or base pairs) of SEQ ID NO: 1 (3905);
where X equals the common length (in nucleotides) of each oligonucleotide in the set (*e.g.*, X=20 for a set of consecutively overlapping 20-mers); and where the number (Z) of consecutively overlapping oligomers of length X for a given SEQ ID NO 1 of length Y is equal to Y- (X-1). For example Z= 3905 -19= 3886 for either sense or antisense sets of SEQ ID NO: 1, where X=20.

Preferably, the set is limited to those oligomers that comprise at least one CpG, TpG or CpA dinucleotide.

Examples of inventive 20-mer oligonucleotides include the following set of 3905 oligomers (and the antisense set complementary thereto), indicated by polynucleotide positions with reference to SEQ ID NO: 1:

1-20, 2-21, 3-22, 4-23, 5-24, ............. and 3886- 3905

Preferably, the set is limited to those oligomers that comprise at least one CpG, TpG or CpA dinucleotide.

Likewise, examples of 25-mer oligonucleotides include the following set of 3881 oligomers (and the antisense set complementary thereto), indicated by polynucleotide positions with reference to SEQ ID NO: 1:

1-25, 2-26, 3-27, 4-28, 5-29, ...... and 3881- 3905.

Preferably, the set is limited to those oligomers that comprise at least one CpG, TpG or CpA dinucleotide.

The present invention encompasses, for *each* of SEQ ID NO: 5, 21, 22, 45 or 46. (sense and antisense), multiple consecutively overlapping sets of oligonucleotides or modified oligonucleotides of length X, where, *e.g.,* X= 9, 10, 17, 20, 22, 23, 25, 27, 30 or 35 nucleotides.

The oligonucleotides or oligomers according to the present invention constitute effective tools useful to ascertain genetic and epigenetic parameters of the genomic sequence corresponding to SEQ ID NO: 5. Preferred sets of such oligonucleotides or modified oligonucleotides of length X are those consecutively overlapping sets of oligomers corresponding to SEQ ID NO: 5, 21, 22, 45 or 46 (and to the complements thereof). Preferably, said oligomers comprise at least one CpG, TpG or CpA dinucleotide.

Particularly preferred oligonucleotides or oligomers according to the present invention are those in which the cytosine of the CpG dinucleotide (or of the corresponding converted TpG or CpA dinculeotide) sequences is within the middle third of the oligonucleotide; that is, where the oligonucleotide is, for example, 13 bases in length, the CpG, TpG or CpA dinucleotide is positioned within the fifth to ninth nucleotide from the 5'-end.

The oligonucleotides of the invention can also be modified by chemically linking the oligonucleotide to one or more moieties or conjugates to enhance the activity, stability or detection of the oligonucleotide. Such moieties or conjugates include chromophores, fluorophors, lipids such as cholesterol, cholic acid, thioether, aliphatic chains, phospholipids, polyamines, polyethylene glycol (PEG), palmityl moieties, and others as disclosed in, for example, United States Patent Numbers 5,514,758, 5,565,552, 5,567,810, 5,574,142, 5,585,481, 5,587,371, 5,597,696 and 5,958,773. The probes may also exist in the form of a PNA (peptide nucleic acid) which has particularly preferred pairing properties. Thus, the oligonucleotide may include other appended groups such as peptides, and may include hybridization-triggered cleavage agents (Krol et al., BioTechniques 6:958-976, 1988) or intercalating agents (Zon, Pharm. Res. 5:539-549, 1988). To this end, the oligonucleotide may be conjugated to another molecule, e.g., a chromophore, fluorophor, peptide, hybridization-triggered cross-linking agent, transport agent, hybridization-triggered cleavage agent, etc.

The oligonucleotide may also comprise at least one art-recognized modified sugar and/or base moiety, or may comprise a modified backbone or non-natural internucleoside linkage.

The oligonucleotides or oligomers according to particular embodiments of the present invention are typically used in 'sets,' which contain at least one oligomer for analysis of each of the CpG dinucleotides of SEQ ID NO: 5 and sequences complementary thereto, or to the corresponding CpG, TpG or CpA dinucleotide within a sequence of the treated nucleic acids according to SEQ ID NO: 21, 22, 45 or 46 and sequences complementary thereto. However, it is anticipated that for economic or other factors it may be preferable to analyse a limited selection of the CpG dinucleotides within said sequences, and the content of the set of oligonucleotides is altered accordingly.

Therefore, in particular embodiments, the present invention provides a set of at least two (2) (oligonucleotides and/or PNA-oligomers) useful for detecting the cytosine methylation state in treated genomic DNA (SEQ ID NO: 21, 22, 45 or 46), or in genomic DNA (SEQ ID NO: 5 and sequences complementary thereto). These probes enable diagnosis and detection of cell proliferative disorders, preferably those according to Table 2 (most preferably lung carcinoma). Particularly preferred is a lung cancer selected from the group consisting of lung adenocarcinoma; large cell lung cancer; squamous cell lung carcinoma; small cell lung carcinoma.The set of oligomers may also be used for detecting single nucleotide polymorphisms (SNPs) in treated genomic DNA (SEQ ID NO: 21, 22, 45 or 46), or in genomic DNA (SEQ ID NO: 5 and sequences complementary thereto).

In preferred embodiments, at least one, and more preferably all members of a set of oligonucleotides is bound to a solid phase.

In further embodiments, the present invention provides a set of at least two (2) oligonucleotides that are used as 'primer' oligonucleotides for amplifying DNA sequences of one of SEQ ID NO:5, 21, 22, 45 or 46 and sequences complementary thereto, or segments thereof.

It is anticipated that the oligonucleotides may constitute all or part of an "array" or "DNA chip" (*i.e.*, an arrangement of different oligonucleotides and/or PNA-oligomers bound to a solid phase). Such an array of different oligonucleotide- and/or PNA-oligomer sequences can be characterized, for example, in that it is arranged on the solid phase in the form of a rectangular or hexagonal lattice. The solid-phase surface may be composed of silicon, glass, polystyrene, aluminium, steel, iron, copper, nickel, silver, or gold. Nitrocellulose as well as plastics such as nylon, which can exist in the form of pellets or also as resin matrices, may also be used. An overview of the Prior Art in oligomer array manufacturing can be gathered from a special edition of Nature Genetics (Nature Genetics Supplement, Volume 21, January 1999, and from the literature cited therein). Fluorescently labelled probes are often used for the scanning of immobilized DNA arrays. The simple attachment of Cy3 and Cy5 dyes to the 5'-OH of the specific probe are particularly suitable for fluorescence labels. The detection of the fluorescence of the hybridised probes may be carried out, for example, via a confocal microscope. Cy3 and Cy5 dyes, besides many others, are commercially available.

It is also anticipated that the oligonucleotides, or particular sequences thereof, may constitute all or part of an "virtual array" wherein the oligonucleotides, or particular sequences thereof, are used, for example, as 'specifiers' as part of, or in combination with a diverse population of unique labeled probes to analyze a complex mixture of analytes. Such a method, for example is described in US 2003/0013091 (United States serial number 09/898,743, published 16 January 2003). In such methods, enough labels are generated so that each nucleic acid in the complex mixture (*i.e.,* each analyte) can be uniquely bound by a unique label and thus detected (each label is directly counted, resulting in a digital read-out of each molecular species in the mixture).

It is particularly preferred that the oligomers according to the invention are utilised for detecting, or for diagnosing cell proliferative disorders, preferably those according to Table 2 (most preferably lung carcinoma) . Particularly preferred is a lung cancer selected from the group consisting of lung adenocarcinoma; large cell lung cancer; squamous cell lung carcinoma; small cell lung carcinoma.

<u>Kits</u>

Moreover, an additional aspect of the present invention is the use of a kit for detecting lung, breast or bladder cancer the kit comprising a bisulfite-containing reagent; one or a plurality of oligonucleotides consisting whose sequences in each case are identical, are complementary, or hybridise under stringent or highly stringent conditions to a 9 or more preferably 18 base long segment of a sequence selected from SEQ ID NO: 21, 22, 45 and 46; and optionally instructions for carrying out and evaluating the described method of methylation analysis. In one embodiment the base sequence of said oligonucleotides comprises at least one CpG, CpA or TpG dinucleotide.

In a further embodiment, said kit may further comprise standard reagents for performing a CpG position-specific methylation analysis, wherein said analysis comprises one or more of the following techniques: MS-SNuPE, MSP, MethyLight™, HeavyMethyl, COBRA, and nucleic acid sequencing. However, a kit along the lines of the present invention can also contain only part of the aforementioned components.

In a preferred embodiment the kit may comprise additional bisulfite conversion reagents selected from the group consisting: DNA denaturation buffer; sulfonation buffer; DNA recovery reagents or kits (*e.g.*, precipitation, ultrafiltration, affinity column); desulfonation buffer; and DNA recovery components.

In a further alternative embodiment, the kit may contain, packaged in separate containers, a polymerase and a reaction buffer optimised for primer extension mediated by the polymerase, such as PCR. In another embodiment of the invention the kit further comprising means for obtaining a biological sample of the patient. Preferred is a kit, which further comprises a container suitable for containing the means for determining methylation of the SHOX2 in the biological sample of the patient, and most preferably further comprises instructions for use and interpretation of the kit results. In a preferred embodiment the kit comprises: (a) a bisulfite reagent; (b) a container suitable for containing the said bisulfite reagent and the biological sample of the patient; (c) at least one set of primer oligonucleotides containing two oligonucleotides whose sequences in each case are identical, are complementary, or hybridise under stringent or highly stringent conditions to a 9 or more preferably 18 base long segment of a sequence selected from SEQ ID NO: 21, 22, 45 and 46; and optionally (d) instructions for use and interpretation of the kit results. In an alternative preferred embodiment the kit comprises: (a) a bisulfite reagent; (b) a container suitable for containing the said bisulfite reagent and the biological sample of the patient; (c) at least one oligonucleotides and/or PNA-oligomer having a length of at least 9 or 16 nucleotides which is identical to or hybridises to a pre-treated nucleic acid sequence according to one of SEQ ID NO: 21, 22, 45 and 46 and sequences complementary thereto; and optionally (d) instructions for use and interpretation of the kit results.

In an alternative embodiment the kit comprises: (a) a bisulfite reagent; (b) a container suitable for containing the said bisulfite reagent and the biological sample of the patient; (c) at least one set of primer oligonucleotides containing two oligonucleotides whose sequences in each case are identical, are complementary, or hybridise under stringent or highly stringent conditions to a 9 or more preferably 18 base long segment of a sequence selected from SEQ ID NO: 21, 22, 45 and 46; (d) at least one oligonucleotides and/or PNA-oligomer having a length of at least 9 or 16 nucleotides which is identical to or hybridises to a pre-treated nucleic acid sequence according to one of SEQ ID NO: 21, 22, 45 and 46 and sequences complementary thereto; and optionally (e) instructions for use and interpretation of the kit results.

The kit may also contain other components such as buffers or solutions suitable for blocking, washing or coating, packaged in a separate container.

Another aspect of the invention relates to a kit for use in determining the presence of and/or diagnosing lung, breast or bladder cancer. Particularly preferred is a lung cancer selected from the group consisting of lung adenocarcinoma; large cell lung cancer; squamous cell lung carcinoma; small cell lung carcinoma.

Said kit prefereably comprises: a means for measuring the level of transcription of at least one gene or genomic sequence selected from the group consisting of SHOX2 and a means for determining methylation of the gene SHOX2.

Typical reagents (*e.g.*, as might be found in a typical COBRA™-based kit) for COBRA™ analysis may include, but are not limited to: PCR primers for the gene SHOX2; restriction enzyme and appropriate buffer; gene-hybridization oligo; control hybridization oligo; kinase labeling kit for oligo probe; and labeled nucleotides. Typical reagents (*e.g.*, as might be found in a typical MethyLight™ -based kit) for MethyLight™ analysis may include, but are not limited to: PCR primers for the bisulfite converted sequence of the gene SHOX2; bisulfite specific probes (e.g. TaqMan™ or Lightcycler ™); optimized PCR buffers and deoxynucleotides; and Taq polymerase.

Typical reagents (*e.g.*, as might be found in a typical Ms-SNuPE™-based kit) for Ms-SNuPE™ analysis may include, but are not limited to: PCR primers for specific gene (or bisulfite treated DNA sequence or CpG island); optimized PCR buffers and deoxynucleotides; gel extraction kit; positive control primers; Ms-SNuPE™ primers for the bisulfite converted sequence of the gene of SHOX2; reaction buffer (for the Ms-SNuPE reaction); and labelled nucleotides.

Typical reagents (*e.g.*, as might be found in a typical MSP-based kit) for MSP analysis may include, but are not limited to: methylated and unmethylated PCR primers for the bisulfite converted sequence of at least one gene or genomic sequence selected from the group consisting of PTGER4;RUNX1;EVX2;EVX-1; SHOX2;SEQ ID NO: 6;CN027;LRAT;IL-12RB1;TFAP2C;BCL2; EN2; PRDM14; SEQ ID NO: 81; ARID5A;VAX1, optimized PCR buffers and deoxynucleotides, and specific probes.

Moreover, an additional aspect of the present application discloses an alternative kit comprising a means for determining at least one gene or genomic sequence selected from the group consisting of PTGER4;RUNX1;EVX2;EVX-1; SHOX2;SEQ ID NO: 6;CN027;LRAT;IL-12RB1;TFAP2C;BCL2; EN2; PRDM14; SEQ ID NO: 81; ARID5A;VAX1 methylation, wherein said means comprise preferably at least one methylation specific restriction enzyme; one or a plurality of primer oligonucleotides (preferably one or a plurality of primer pairs) suitable for the amplification of a sequence comprising at least one CpG dinucleotide of a sequence selected from SEQ ID NO: 1 to SEQ ID NO: 12; SEQ ID NO: 79 to SEQ ID NO: 83; and optionally instructions for carrying out and evaluating the described method of methylation analysis. In one embodiment the base sequence of said oligonucleotides are identical, are complementary, or hybridise under stringent or highly stringent conditions to an at least 18 base long segment of a sequence selected from SEQ ID NO: 1 to SEQ ID NO: 12; SEQ ID NO: 79 to SEQ ID NO: 83. Said kit may comprise one or a plurality of oligonucleotide probes for the analysis of the digest fragments, preferably said oligonucleotides are identical, are complementary, or hybridise under stringent or highly stringent conditions to an at least 16 base long segment of a sequence selected from SEQ ID NO: 1 to SEQ ID NO: 12; SEQ ID NO: 79 to SEQ ID NO: 83.

The kit may comprise additional reagents selected from the group consisting: buffer (e.g. restriction enzyme, PCR, storage or washing buffers); DNA recovery reagents or kits (*e.g.*, precipitation, ultrafiltration, affinity column) and DNA recovery components. The kit may contain, packaged in separate containers, a polymerase and a reaction buffer optimised for primer extension mediated by the polymerase, such as PCR. In another embodiment of the invention the kit further comprising means for obtaining a biological sample of the patient. In a preferred embodiment the kit comprises: (a) a methylation sensitive restriction enzyme reagent; (b) a container suitable for containing the said reagent and the biological sample of the patient; (c) at least one set of oligonucleotides one or a plurality of nucleic acids or peptide nucleic acids which are identical, are complementary, or hybridise under stringent or highly stringent conditions to an at least 9 base long segment of a sequence selected from SEQ ID NO: 1 to SEQ ID NO: 12; SEQ. ID NO: 79 to SEQ ID NO: 83; and optionally (d) instructions for use and interpretation of the kit results.

The kit may comprise: (a) a methylation sensitive restriction enzyme reagent; (b) a container suitable for containing the said reagent and the biological sample of the patient; (c) at least one set of primer oligonucleotides suitable for the amplification of a sequence comprising at least one CpG dinucleotide of a sequence selected from SEQ ID NO: 1 to SEQ ID NO: 12; SEQ ID NO: 79 to SEQ ID NO: 83; and optionally (d) instructions for use and interpretation of the kit results.

The kit may comprise: (a) a methylation sensitive restriction enzyme reagent; (b) a container suitable for containing the said reagent and the biological sample of the patient; (c) at least one set of primer oligonucleotides suitable for the amplification of a sequence comprising at least one CpG dinucleotide of a sequence selected from SEQ ID NO: 1 to SEQ ID NO: 12; SEQ ID NO: 79 to SEQ ID NO: 83; (d) at least one set of oligonucleotides one or a plurality of nucleic acids or peptide nucleic acids which are identical , are complementary, or hybridise under stringent or highly stringent conditions to an at least 9 base long segment of a sequence selected from SEQ ID NO: 1 to SEQ ID NO: 12; SEQ ID NO: 79 to SEQ ID NO: 83 and optionally (e) instructions for use and interpretation of the kit results.

The kit may also contain other components such as buffers or solutions suitable for blocking, washing or coating, packaged in a separate container.

The application further discloses a kit for use in providing a diagnosis of the presence or absence of cell proliferative disorders, preferably those according to Table 2 (most preferably lung carcinoma), in a subject by means of methylationsensitive restriction enzyme analysis. Said kit comprises a container and a DNA microarray component. Said DNA microarray component being a surface upon which a plurality of oligonucleotides are immobilized at designated positions and wherein the oligonucleotide comprises at least one CpG methylation site. At least one of said oligonucleotides is specific for at least one gene or genomic sequence selected from the group consisting of PTGER4;RUNX1;EVX2;EVX-1; SHOX2;SEQ ID NO: 6;CN027;LRAT;IL-12RB1;TFAP2C;BCL2; EN2; PRDM14; SEQ ID NO: 81; ARIDSA;VAX1 and comprises a sequence of at least 15 base pairs in length but no more than 200 bp of a sequence according to one of SEQ ID NO: 1 to SEQ ID NO: 12; SEQ ID NO: 79 to SEQ ID NO: 83. Preferably said sequence is at least 15 base pairs in length but no more than 80 bp of a sequence according to one of SEQ ID NO: 1 to SEQ ID NO: 12; SEQ ID NO: 79 to SEQ ID NO: 83. It is further preferred that said sequence is at least 20 base pairs in length but no more than 30 bp of a sequence according to one of SEQ ID NO: 1 to SEQ ID NO: 12; SEQ ID NO: 79 to SEQ ID NO: 83.

Said test kit preferably further comprises a restriction enzyme component comprising one or a plurality of methylation-sensitive restriction enzymes.

Said test kit is further characterized in that it comprises at least one methylation-specific restriction enzyme, and wherein the oligonucleotides comprise a restriction site of said at least one methylation specific restriction enzymes.

The kit may further comprise one or several of the following components, which are known in the art for DNA enrichment: a protein component, said protein binding selectively to methylated DNA; a triplex-forming nucleic acid component, one or a plurality of linkers, optionally in a suitable solution; substances or solutions for performing a ligation e.g. ligases, buffers; substances or solutions for performing a column chromatography; substances or solutions for performing an immunology based enrichment (e.g. immunoprecipitation); substances or solutions for performing a nucleic acid amplification e.g. PCR; a dye or several dyes, if applicable with a coupling reagent, if applicable in a solution; substances or solutions for performing a hybridization; and/or substances or solutions for performing a washing step.

The application further discloses a composition of matter useful for detecting, or for diagnosing cell proliferative disorders, preferably those according to Table 2 (most preferably lung carcinoma). Particularly preferred is a lung cancer selected from the group consisting of lung adenocarcinoma; large cell lung cancer; squamous cell lung carcinoma; small cell lung carcinoma.

Said composition preferably comprises at least one nucleic acid 18 base pairs In length of a segment of the nucleic acid sequence disclosed in SEQ ID NO: 13 to SEQ ID NO: 60; SEQ ID NO: 84 to SEQ ID NO: 103, and one or more substances taken from the group comprising:

1-5 mM Magnesium Chloride, 100-500 μM dNTP, 0.5-5 units of taq polymerase, bovine serum albumen, an oligomer in particular an oligonucleotide or peptide nucleic acid (PNA)-oligomer, said oligomer comprising in each case at least one base sequence having a length of at least 9 nucleotides which is complementary to, or hybridizes under moderately stringent or stringent conditions to a pretreated genomic DNA according to one of the SEQ ID NO: 13 to SEQ ID NO: 60; SEQ ID NO: 84 to SEQ ID NO: 103 and sequences complementary thereto. It is preferred that said composition of matter comprises a buffer solution appropriate for the stabilization of said nucleic acid in an aqueous solution and enabling polymerase based reactions within said solution.. Suitable buffers are known in the art and commercially available.

Said at least one nucleic acid is at least 50, 100, 150, 200, 250 or 500 base pairs in length of a segment of the nucleic acid sequence disclosed in SEQ ID NO: 13 to SEQ ID NO: 60; SEQ ID NO: 84 to SEQ ID NO: 103.

Table 1

| Gene | Genomic SEQ ID NO: | Pretreated methylated sequence (sense) SEQ ID NO: | Pretreated methylated strand (antisense) SEQ ID NO: | Pretreated unmethylated sequence (sense) SEQ ID NO: | Pretreated unmethylated sequence (antisense) SEQ ID NO: |
|---|---|---|---|---|---|
| RUNX1 | 1 | 13 | 14 | 37 | 38 |
| EVX2 | 2 | 15 | 16 | 39 | 40 |
| EVX-1 | 3 | 17 | 18 | 41 | 42 |
| PTGER4 | 4 | 19 | 20 | 43 | 44 |
| SHOX2 | 5 | 21 | 22 | 45 | 46 |
| No annotated gene, sequence located between TIM14 & SOX-2 genes | 6 | 23 | 24 | 47 | 48 |
| CN027 | 7 | 25 | 26 | 49 | 50 |
| LRAT | 8 | 27 | 28 | 51 | 52 |
| IL-12RB1 | 9 | 29 | 30 | 53 | 54 |
| TFAP2C | 10 | 31 | 32 | 55 | 56 |
| BCL2 | 11 | 33 | 34 | 57 | 58 |
| ARID5A | 12 | 35 | 36 | 59 | 60 |
| Homeobox protein engrailed-2 (Hu-En-2); EN2; HME2 | 79 | 84 | 85 | 94 | 95 |
| PR domain zinc finger protein 14 (PR domain-containing protein 14); PRDM 14 | 80 | 86 | 87 | 96 | 97 |
| Chromosomal location (NCBI36) chromosome: 15 bp45139161 to 45139783 | 81 | 88 | 89 | 98 | 99 |
| AT rich interactive domain 5A; ARID5A | 82 | 90 | 91 | 100 | 101 |
| Ventral anterior homeobox 1; VAX1 | 83 | 92 | 93 | 102 | 103 |

Table 2

| Gene | Preferred disorder |
|---|---|
| EVX2 | Cancers, preferably lung |
| RUNX1 | Cancers, preferably lung, prostate and/or breast |
| PTGER4 | Cancers, preferably lung, prostate and/or breast |

(continued)

| Gene | Preferred disorder |
|---|---|
| SHOX2 | Cancers, preferably lung, breast and/or bladder |
| none; upstream: TIM14/ downstream: SOY-2 | Cancers, preferably prostate |
| CN027 | Cancers, preferably lung and/or prostate |
| LRAT | Cancers, preferably colon |
| IL-12RB1 | Cancers, preferably prostate and/or breast |
| EVX-1 | Cancers |
| TFAP2C | Cancers |
| BCL2 | Cancers, preferably lung |
| Homeobox protein engrailed-2 (Hu-En-2); EN2; HME2 | Cancers, preferably lung |
| PR domain zinc finger protein 14 (PR domain-containing protein 14); PRDM 14 | Cancers, preferably lung |
| Chromosomal location (NCBI36) chromosome: 15 bp45139161 to 45139783 | Cancers, preferably lung |
| AT rich interactive domain 5A; ARID5A | Cancers, preferably lung |
| Ventral anterior homeobox 1; VAX1 | Cancers, preferably lung |

Table 3A

| Gene/ Genomic region | primer 1 SEQ ID NO: | primer 2 SEQ ID NO: | Probe SEQ ID NO: |
|---|---|---|---|
| SEQ ID NO: 6 | 64 | 65 | 66 |
| CN027 | 67 | 68 | 69 |
| LRAT | 70 | 71 | 72 |
| TFAP2C | 73 | 74 | 75 |
| EVX-1 | 76 | 77 | 78 |
| EVX2 | 79 | 80 | 81 |
| SHOX2 | 82 | 83 | 84 |
| ARID5A | 85 | 86 | 87 |
| RUNX1 | 88 | 89 | 90 |
| PTGER4 | 91 | 92 | 93 |
| BCL2 | 94 | 95 | 96 |
| IL-12RB1 | 97 | 98 | 99 |

Table 3B

| Target sequence (Genomic) | Oligo SEQ ID NO: | Sequence |
|---|---|---|
| SEQ ID NO: 79 Forward Primer | 104 | tatcgcggagattttcgagttttcgttg |
| SEQ ID NO: 79 Probe | 105 | ggggttgtttttcgggatt |
| SEQ ID NO: 79 Reverse Primer | 106 | gataaccctaaaacgcaactcgaa |
| SEX ID NO: 80 Reverse Primer | 107 | cgtttcgtaaggagcgtgtt |

(continued)

| Target sequence (Genomic) | Oligo SEQ ID NO: | Sequence |
|---|---|---|
| SEQ ID NO: 80 Forward Primer | 108 | cgcgttgttcgcggttagtttcgt |
| SEQ ID NO: 80 Probe | 109 | cgacgttttcgcgtgg |
| SEQ ID NO: 81 Forward Primer | 110 | gttttgaaatttattagaataacgacgtt |
| SEQ ID NO: 81 Reverse Primer | 111 | ctttctaaaaataaccgaactatactacgac |
| SEQ ID NO: 81 Probe | 112 | tacggacgttcgcgggtcgtt |
| SEQ ID NO: 82 Probe | 113 | agtaagttcgcgtagattcggttt |
| SEQ ID NO: 82 Reverse Primer | 114 | taaaacgacgaaacgaccgat |
| SEQ ID NO: 82 Forward Primer | 115 | tcgtcgttttcgggttgtcgagtg |
| SEQ ID NO: 83 Probe | 116 | aaggaagtggaaataatcgtcgta |
| SEQ ID NO: 83 Forward Primer | 117 | aggcgttttgtttttcggaaattcgaaattc |
| SEQ ID NO: 83 Reverse Primer | 118 | ctacgactaataccgtaaacgccta |

Table 4A

| Tissue type | All lung cancers | Lung adenocarcinoma | Large cell lung cancer | Squamouscell lung carcinoma | Small cell lung carcinoma | Prostate carcinoma |
|---|---|---|---|---|---|---|
| PMR threshold (%) | >20 | >20 | >20 | >20 | >20 | >20 |
| N (total) | 61 | 21 | 12 | 21 | 7 | 10 |
| **Gene** | | | | | | |
| EVX2 | 76.8 | 52.4 | 75.0 | 90,5 | 100.0 | 60.0 |
| RUNX1 | 76,7 | 61,9 | 58,3 | 85,7 | 100,0 | 100,0 |
| PTGER4 | 66,1 | 66,7 | 66,7 | 61,9 | 71,4 | 100,0 |
| SHOX2 | 63,0 | 38,1 | 50.0 | 90.5 | 71,4 | 20,0 |
| SEQ ID NO: 6 | 68,2 | 38,1 | 33,3 | 95,2 | 100,0 | 100,0 |
| CNO27 | 55,9 | 38,1 | 41,7 | 71.4 | 71,4 | 100,0 |
| LRAT | 41,7 | 42,9 | 33,3 | 42,9 | 42,9 | 30,0 |
| IL-12RB1 | 24,9 | 14,3 | 16,7 | 28,6 | 42,9 | 100,0 |
| EVX-1 | 47,4 | 38,1 | 33,3 | 57,1 | 57,1 | 100,0 |
| TFAP2C | 68,1 | 38,1 | 58,3 | 85,7 | 100,0 | 100,0 |
| BCL2 | 14,1 | 23,8 | 8,3 | 14,3 | 0,0 | 50,0 |

Table 4B

| Tissue type | Colorectal cancer | Breast carcinoma | Bladder carcinoma | Lung diseasesd (Not cancer) | Lung healthy | Blood |
|---|---|---|---|---|---|---|
| PMR threshold (%) | >20 | >20 | >20 | >5 | >5 | >0.2 |

(continued)

| Tissue type | Colorectal cancer | Breast carcinoma | Bladder carcinoma | Lung diseasesd (Not cancer) | Lung healthy | Blood |
|---|---|---|---|---|---|---|
| N (total) | 10 | 10 | 10 | 7 | 12 | 20 |
| Gene | | | | | | |
| EVX2 | 40,0 | 80,0 | 80,0 | 0,0 | 0,0 | 55,0 |
| RUNX1 | 20,0 | 80,0 | 60,0 | 0,0 | 0,0 | 0,0 |
| PTGER4 | 0,0 | 80,0 | 30,0 | 0,0 | 0,0 | 0,0 |
| SHOX2 | 60,0 | 80,0 | 70,0 | 0,0 | 0,0 | 10,0 |
| SEQ ID NO: 6 | 60,0 | 50,0 | 50,0 | 28,6 | 33,3 | 25,0 |
| CN027 | 90,0 | 70,0 | 70,0 | 0,0 | 8,3 | 25,0 |
| LRAT | 90,0 | 60,0 | 60,0 | 0,0 | 0,0 | 0,0 |
| IL-12RB1 | 0,0 | 80,0 | 30,0 | 85,7 | 100,0 | 0,0 |
| EVX-1 | 80,0 | 80,0 | 90,0 | 0,0 | 0,0 | 0,0 |
| TFAP2C | 100,0 | 100,0 | 60,0 | 14,3 | 0,0 | 25,0 |
| BCL2 | 0,0 | 10,0 | 60,0 | 0,0 | 0,0 | 0,0 |

Table 4C

| Tissue type | All lung cancers | Lung adenocarcinoma | Large cell lung cancer | Squamouscell lung carcinoma | Small cell lung carcinoma | Prostate carcinoma |
|---|---|---|---|---|---|---|
| PMR threshold (%) | >20 | >20 | >20 | >20 | >20 | >20 |
| N (total) | 61 | 21 | 12 | 21 | 7 | 10 |
| SEQ ID NO: | | | | | | |
| SEQ ID NO: 79 | 38 | 29 | 33 | 57 | 29 | 55 |
| SEQ ID NO: 80 | 42 | 29 | 16 | 66 | 43 | 11 |
| SEQ ID NO: 81 | 24 | 24 | 8 | 28 | 29 | 11 |
| SEQ ID NO: 82 | 58 | 57 | 50 | 62 | 57 | 66 |
| SEQ ID NO: 83 | 45 | 24 | 33 | 66 | 57 | 25 |

Table 4D

| Tissue type | Healthy Prostate | Colorectal cancer | Healthy Colon | Breast carcinoma | Healthy breast | Bladder carcinoma | Healthy Bladder | Lung diseasesd (Not cancer) | Lung healthy | Blood |
|---|---|---|---|---|---|---|---|---|---|---|
| PMR threshold %) | >5 | >20 | >5 | >20 | >5 | >20 | >5 | >5 | >5 | >0.2 |
| N (total) | 11 | 10 | 9 | 10 | 12 | 10 | 10 | 7 | 12 | 20 |
| SEQ ID NO: | | | | | | | | | | |
| SEQ ID NO: 79 | 0 | 40 | 0 | 50 | 0 | 40 | 0 | 0 | 0 | 0 |
| SEQ ID NO: 80 | 0 | 50 | 0 | 40 | 0 | 30 | 0 | 0 | 0 | 0 |
| SEQ ID NO: 81 | 0 | 40 | 0 | 20 | 0 | 10 | 0 | 0 | 0 | 0 |
| SEQ ID NO: 82 | 12 | 10 | 22 | 60 | 0 | 90 | 70 | 0 | 0 | 0 |
| SEQ ID NO: 83 | 12 | 60 | 0 | 50 | 0 | 60 | 10 | 0 | 0 | 0 |

Table 5

| | PTGER4 | | RUNX1 | |
|---|---|---|---|---|
| NSCLC | Sens | Spec | Sens | Spec |
| Bronchial Lavage | 52% | 91% | 39% | 91% |
| Blood Plasma | 69% | 91% | --- | --- |

EXAMPLES

Example 1

Analysis of methylation of the genes/sequences according to Table 1 in multiple cancer and control tissue samples was carried out by means of a Real-Time MSP assay using the components according to Table 3.

Samples

| Tissue Type | No. of samples |
|---|---|
| Lung adenocarcinoma | 21 |
| Large cell lung cancer | 12 |
| Squamouscell lung carcinoma | 21 |
| Small cell lung carcinoma | 7 |
| Prostate carcinoma | 10 |
| Colorectal cancer | 10 |
| Breast carcinoma | 10 |
| Bladder carcinoma | 10 |
| Lung diseasesd (Not cancer) | 7 |
| Lung healthy | 12 |
| Blood/white blood cells | 20 |

DNA extraction and bisulfite treatment

The DNA was isolated from the all samples according to a modified protocol based on that disclosed in the Qiagen Genomic DNA Handbook (August 2001) (pg 28-31, 44-47). The DNA in the eluate was quantified and the eulate was treated according to the following bisulfite reaction.

The eluate was mixed with 354 μl of bisulfite solution (5.89 mol/l) and 146 μl of dioxane containing a radical scavenger(6-hydroxy-2,5,7,8-tetramethylchromane 2-carboxylic acid, 98.6 mg in 2.5 ml of dioxane). The reaction mixture was denatured for 3 min at 99°C and subsequently incubated at the following temperature program for a total of 7 h min 50°C; one thermospike (99.9°C) for 3 min; 1.5 h 50°C; one thermospike (99°C) for 3 min; 3 h 50°C. The reaction mixture was subsequently purified by ultrafiltration using a Millipore Microcon ™ column. The purification was conducted essentially according to the manufacturer's instructions. For this purpose, the reaction mixture was mixed with 300 μl of water, loaded onto the ultrafiltration membrane, centrifuged for 15 min and subsequently washed with 1 x TE buffer. The DNA remains on the membrane in this treatment. Then desulfonation is performed. For this purpose, 0.2 mol/l NaOH was added and incubated for 10 min. A centrifugation (10 min) was then conducted, followed by a washing step with 1 x TE buffer. After this, the DNA was eluted. For this purpose, the membrane was mixed for 10 minutes with 75 μl of warm 1 x TE buffer (50°C). The membrane was turned over according to the manufacturer's instructions. Subsequently a repeated centrifugation was conducted, with which the DNA was removed from the membrane. 10 μl of the eluate was utilized for the Lightcycler Real Time PCR assay.

Reference assay

The GSTP1 reference assay design makes it suitable for quantitating bisulfite converted DNA from different sources, including fresh/frozen samples, remote samples such as plasma or serum, and DNA obtained from archival specimen such as paraffin embedded material. The assay measures the total DNA independly from its methylation status, as long as no CpG is covered by the PCR oligonucleotides.

The following components were used in the reaction to amplify the control amplificate:

- 10 μl of template DNA
- 2 μl of FastStart LightCycler Mix for hybridization probes (Roche Diagnostics)
- 3.5 mmol/l MgCl2 (Roche Diagnostics)
- 0.60 μmol/l forward primer (SEQ ID NO: 61, TIB-MolBiol)
- 0.60 μmol/l reverse primer (SEQ ID NO: 62, TIB-MolBiol)
- 0.2 μmol/l probe (SEQ ID NO: 63, TIB-MolBiol)

The following oligonucleotides were used in the reaction to amplify the control amplificate:

Primer1: GGAGTGGAGGAAATTGAGAT (SEQ ID NO: 61)
Primer2: CCACACAACAAATACTCAAAAC (SEQ ID NO: 62)
Probe: FAM-TGGGTGTTTGTAATTTTTGTTTTGTGTTAGGTT-TAMRA (SEQ ID NO: 63)

The assay was performed in the LightCycler 480 according to the following temperature-time-profile:

- Activation 10 min at 95 °C
- 50 cycles:

  10 sec at 95 °C
  30 sec at 56 °C
  10 sec at 72 °C

The detection was carried out during the annealing phase at 56 °C in channel for 530 nm with a target specific fluorescence probe (Seq ID-63).

Methylation Specific Real Time PCR

The methylation specific real time PCR (MSP) reactions were performed on the bisulfite converted sample DNA. All MSP mastermixes were the Roche FastStart TaqMan Probe Master containing 300 nM ROX reference dye on the ABI7900 instrument. Each reaction contained 600 nM of the forward primer, 600 nM reverse primer, and 200 nM of the detection probe (see table with Seq-IDs of the markers). The reactions were performed in a final volume of 20 microL using the ABI7900 instrument with following temperature and time profile :

- Activation 10 min at 95 °C
- 50 cycles:

  15 sec at 95 °C
  60 sec at 60 °C

Data interpretation

Calculation of DNA concentration.

The CP (crossing point values) as calculated by the ABI7900 instrument software using a specific threshold was used for each assay to determine DNA concentration. The DNA concentration was calculated by reference of the CP value of each well to a calibration standard. The calibration standard was prepared from methylated bisulfite converted human DNA containing 10, 5, 2.5, 1, 0.4, 0.1, and 0.05 ng per reaction. The calibration curves was used for both the methylation specific marker assays and the C3 reference assay.

Percentage methylation

For each sample the detected percentage methylation was calculated as the measured concentration of DNA quantified using the methylation assays over the concentration of DNA in the sample as quantified by the C3 assay. The methylation ratios were calculated according to the PMR value method (Eads et al., Cancer Res. 2001 Apr 15; 61 (8):

3410-8, PMID 11309301) against the total DNA quantified by the C3 reference PCR.

Detection of methylation was determined at multiple different threshold levels, see Tables 4A to 4D).

Results are provided in Table 4A to 4D indicating the %of samples of each tissue class with methylation above the PMR (% methylation) threshold.

Exampe 2

Analysis of methylation of the genes PTGER4 and RUNX1 was confirmed in cancer and control body fluid samples (plasma, bronchial savage).

Samples

| Analysis Group | plasma | lavage |
|---|---|---|
| Lung adenocarcinoma | 50 | 50 |
| Benign lung disease | 50 | 50 |

DNA was extracted from plasma and bronchial lavage using MagnaPure (Roche Diagnostics)

For bronchial lavage (BL) samples the following preprocessing was performed: 1 ml of BL sample was centrifuged for 10 minutes at 8,000 x g to pellet sample. After removing 900 ul of supernatant the cells were resuspended in the remaining 100 ul of liquid. Then 130 ul of Bacteria Lysis Buffer and 20 ul of Proteinase K were added to the sampe, which was vortexed for 10 seconds. After a quick spin down to collect the sample at the bottom of the tube it was incubated for 10 minutes at 60 C and for 15 minutes at 90 C. Then the sample was cooled down for 60 seconds and collected at the bottom of the tube by briefly centrifuging it.

Bisulfite treatment and reference assays were carried out, substantially as above. Methylation analysis was carried out by means of a Real-Time PCR HM assay.

Results are provided in Table 5.

**Claims**

1. A method for detecting lung, breast or bladder cancer in a subject comprising determining the methylation level of the gene SHOX2 in a biological sample isolated from said subject wherein hyper-methylation is indicative for the presence of said cancer.

2. A method for detecting lung, breast or bladder cancer according to claim 1, comprising contacting genomic DNA isolated from a biological sample obtained from said subject with at least one reagent, or series of reagents that distinguishes between methylated and non-methylated CpG dinucleotides within at least one target region of the genomic DNA, wherein the target region comprises, or hybridizes under stringent conditions to a sequence of at least 16 contiguous nucleotides of SEQ ID NO: 5, and wherein said contiguous nucleotides comprise at least one CpG dinucleotide sequence.

3. A method for detecting lung, breast or bladder cancer according to claim 1 or 2, comprising:

a) extracting or otherwise isolating genomic DNA from a biological sample obtained from said subject,
b) treating the genomic DNA of a), or a fragment thereof, with one or more reagents to convert cytosine bases that are unmethylated in the 5-position thereof to uracil or to another base that is detectably dissimilar to cytosine in terms of hybridization properties;
c) contacting the treated genomic DNA, or the treated fragment thereof, with an amplification enzyme and at least one primer comprising, a contiguous sequence of at least 9 nucleotides that is complementary to, or hybridizes under moderately stringent or stringent conditions to a sequence selected from the group consisting of SEQ ID NOs: 21, 22, 45 and 46, and complements thereof, wherein the treated genomic DNA or the fragment thereof is either amplified to produce at least one amplificate, or is not amplified; and
d) determining, based on a presence or absence of, or on a property of said amplificate, the methylation state or level of at least one CpG dinucleotide of SEQ ID NO: 5, or an average, or a value reflecting an average methylation state or level of a plurality of CpG dinucleotides of SEQ ID NO: 5.

4. The method of any of claims 1 to 3, wherein the biological sample obtained from said subject is selected from the group comprising cells or cell lines, histological slides, biopsies, paraffin-embedded tissue, body fluids, ejaculate, urine, blood plasma, blood serum, whole blood, isolated blood cells, sputum and biological matter derived from bronchoscopy (including but not limited to bronchial lavage, bronchial alveolar lavage, bronchial brushing, bronchial abrasion and combinations thereof.

**5.** A method for detecting lung, breast or bladder cancer according to claim 1, comprising:

a) extracting or otherwise isolating genomic DNA from a biological sample obtained from the subject
b) digesting the genomic DNA of a), or a fragment thereof, with one or more methylation sensitive restriction enzymes;
c) contacting the DNA restriction enzyme digest of b), with an amplification enzyme and at least two primers suitable for the amplification of a sequence comprising at least one CpG dinucleotide of SEQ ID NO: 5,
d) determining, based on a presence or absence of an amplificate the methylation state or level of at least one CpG dinucleotide of SEQ ID NO: 5.

**6.** Use of a nucleic acid comprising at least 16 contiguous nucleotides of a treated genomic DNA sequence selected from the group consisting of SEQ ID NOs: 21, 22, 45, 46, and sequences complementary thereto for detecting lung, breast or bladder cancer according to a method according to any of claims 1 to 5.

**7.** Use according to claim 6, wherein said nucleic acid comprises at least 50 contiguous nucleotides.

**8.** Use of a kit for performing the method according to any of claims 1 to 4, the kit comprising (a) a bisulfite reagent; (b) a container suitable for containing said bisulfite reagent and the biological sample of the patient; (c) at least one set of oligonucleotides containing two oligonucleotides whose sequences in each case are identical, are complementary, or hybridize under stringent or highly stringent conditions to a 9 or more preferably 18 base long segment of a sequence selected from SEQ ID NOs: 21, 22, 45 and 46.

## Patentansprüche

**1.** Verfahren zum Nachweis von Lungen-, Brust- oder Blasenkrebs in einem Probanden, umfassend:

Ermitteln des Methylierungsgrades des SHOX2-Gens in einer biologischen Probe, die aus dem Probanden isoliert wurde, wobei Hypermethylierung indikativ für das Vorhandensein des Krebses ist.

**2.** Verfahren zum Nachweisen von Lungen-, Brust- oder Blasenkrebs gemäß Anspruch 1, umfassend:

In-Kontakt-Bringen von genomischer DNA, die aus einer biologischen Probe isoliert wurde, welche von dem Probanden erhalten wurde, mit mindestens einem Reagens oder einer Reihe von Reagentien, das / die zwischen methylierten und nicht-methylierten CpG-Dinukleotiden innerhalb mindestens einer Zielregion der genomischen DNA unterscheidet, wobei die Zielregion eine Sequenz von mindestens 16 zusammenhängenden Nukleotiden nach SEQ ID NO: 5 umfasst oder unter stringenten Bedingungen daran hybridisiert, und wobei die zusammenhängenden Nukleotide mindestens eine CpG-Dinukleotidsequenz umfassen.

**3.** Verfahren zum Nachweis von Lungen-, Brust- oder Blasenkrebs gemäß Anspruch 1 oder 2, umfassend:

a) Extrahieren oder auf andere Weise Isolieren von genomischer DNA aus einer biologischen Probe, die von dem Probanden erhalten wurde,
b) Behandeln der genomischen DNA aus a) oder eines Fragments davon mit einem oder mehreren Reagentien, um Cytosinbasen, die in ihrer 5-Position unmethyliert sind, zu Uracil oder zu einer anderen Base umzuwandeln, die im Hinblick auf ihre Hybridisierungseigenschaften nachweisbar unterschiedlich zu Cytosin ist;
c) In-Kontakt-Bringen der behandelten genomischen DNA oder des behandelten Fragments derselben mit einem Amplifikationsenzym und mindestens einem Primer, der eine zusammenhängende Sequenz von mindestens 9 Nukleotiden umfasst, die zu einer Sequenz, welche aus der Gruppe ausgewählt ist, die aus SEQ ID NOs: 21, 22, 45 und 46 und Komplementärsequenzen davon besteht, komplementär ist oder unter moderat stringenten oder stringenten Bedingungen daran hybridisiert, wobei die behandelte genomische DNA oder das Fragment derselben entweder amplifiziert wird, um mindestens ein Amplifikat zu erzeugen, oder nicht amplifiziert wird; und
d) Bestimmen des Methylierungszustands oder des Methylierungsgrades mindestens eines CpG-Dinukleotids von SEQ ID NO: 5 oder eines Durchschnittswertes oder eines Wertes, der einen durchschnittlichen Methylierungszustand oder einen durchschnittlichen Methylierungsgrad einer Vielzahl von CpG-Dinukleotiden von SEQ ID NO: 5 reflektiert, auf der Grundlage einer Anwesenheit oder Abwesenheit oder einer Eigenschaft des Amplifikats.

**4.** Verfahren nach einem der Ansprüche 1 bis 3, wobei die biologische Probe, die von dem Probanden erhalten wurde, aus der Gruppe ausgewählt ist, welche Zellen oder Zelllinien, histologische Schnitte, Biopsien, in Paraffin eingebettetes Gewebe, Körperflüssigkeiten, Ejakulat, Urin, Blutplasma, Blutserum, Vollblut, isolierte Blutzellen, Sputum und biologisches Material, das aus Bronchioskopie abgeleitet ist (einschließlich aber nicht beschränkt auf bronchiale Lavage, bronchiale alveolare Lavage, bronchiales Bürsten, bronchiale Abrasion) und Kombinationen davon umfasst.

**5.** Verfahren zum Nachweis von Lungen-, Brust- oder Blasenkrebs gemäß Anspruch 1 umfassend:

a) Extrahieren oder auf andere Weise Isolieren von genomischer DNA aus einer biologischen Probe, die von dem Probanden erhalten wurde,
b) Verdau der genomischen DNA aus a) oder eines Fragments davon mit einem oder mehreren methylierungssensitiven Restriktionsenzymen;
c) In-Kontakt-Bringen des DNA-Restriktionsenzymverdaus aus b) mit einem Amplifikationsenzym und mindestens 2 Primern, die für die Amplifikation einer Sequenz geeignet sind, die mindestens ein CpG-Dinukleotid von SEQ ID NO: 5 umfasst,
d) Bestimmen des Methylierungszustands oder Methylierungsgrades mindestens eines CpG-Dinukleotids von SEQ ID NO: 5, auf der Grundlage einer Anwesenheit oder Abwesenheit eines Amplifikats.

**6.** Verwendung einer Nukleinsäure, die mindestens 16 zusammenhängende Nukleotide einer behandelten genomischen DNA-Sequenz umfasst, welche aus der Gruppe ausgewählt ist, die aus SEQ ID NOs: 21, 22, 45, 46 und dazu komplementären Sequenzen besteht, zum Nachweis von Lungen-, Brust- oder Blasenkrebs gemäß einem Verfahren gemäß einem der Ansprüche 1 bis 5.

**7.** Verwendung gemäß Anspruch 6, wobei die Nukleinsäure mindestens 50 zusammenhängende Nukleotide umfasst.

**8.** Verwendung eines Kits zum Durchführen des Verfahrens gemäß einem der Ansprüche 1 bis 4, wobei das Kit umfasst: (a) ein Bisulfit-Reagens; (b) einen Behälter, der dafür geeignet ist, dieses Bisulfit-Reagens und die biologische Probe des Patienten aufzunehmen; (c) mindestens einen Satz von Oligonukleotiden, der 2 Oligonukleotide enthält, deren Sequenzen in jedem Fall identisch sind, komplementär sind oder unter stringenten oder hoch stringenten Bedingungen an ein 9 oder mehr Basen langes, bevorzugterweise 18 Basen langes Segment einer Sequenz hybridisieren, die aus SEQ ID NOs: 21, 22, 45 und 46 ausgewählt ist.

## Revendications

**1.** Procédé pour la détection d'un cancer du poumon, du sein ou de la vessie chez un sujet, comprenant la détermination du niveau de méthylation du gène SHOX2 dans un échantillon biologique isolé à partir dudit sujet, une hyperméthylation étant indicative de la présence dudit cancer.

**2.** Procédé pour la détection d'un cancer du poumon, du sein ou de la vessie selon la revendication 1, comprenant la mise en contact d'ADN génomique isolé à partir d'un échantillon biologique obtenu à partir dudit sujet avec au moins un réactif ou une série de réactifs, qui distingue entre dinucléotides CpG méthyles et non-méthylés dans au moins une région cible de l'ADN génomique, tandis que la région cible comprend, ou hybride dans des conditions stringentes à une séquence d'au moins 16 nucléotides contigus de SEQ ID NO: 5, et tandis que lesdits nucléotides contigus comprennent au moins une séquence dinucléotidique CpG.

**3.** Procédé pour la détection d'un cancer du poumon, du sein ou de la vessie selon la revendication 1 ou 2, comprenant:

a) l'extraction ou l'isolement d'une autre manière d'ADN génomique à partir d'un échantillon biologique obtenu à partir dudit sujet,
b) le traitement de l'ADN génomique de a), ou d'un fragment de celui-ci, avec un ou plusieurs réactifs pour convertir les bases cytosine qui sont non-méthylées dans la position 5 de celui-ci en uracile ou en une autre base qui est différente de la cytosine de manière détectable en termes de propriétés d'hybridation;

c) la mise en contact de l'ADN génomique traité, ou du fragment traité de celui-ci, avec une enzyme d'amplification et au moins une amorce comprenant une séquence contiguë d'au moins 9 nucléotides, qui est complémentaire de, ou hybride dans des conditions modérément stringentes ou stringentes à une séquence choisie dans le groupe consistant en SEQ ID Nos: 21, 22, 45 et 46, et leurs compléments, tandis que l'ADN génomique traité ou le fragment de celui-ci, soit est amplifié pour produire au moins un produit d'amplification, soit n'est pas amplifié; et

d) la détermination, sur la base d'une présence ou d'une absence de, ou sur la base d'une propriété dudit produit d'amplification, de l'état ou du niveau de méthylation d'au moins un dinucléotide CpG de SEQ ID NO: 5, ou d'une moyenne, ou d'une valeur reflétant un état ou un niveau de méthylation moyen d'une pluralité de dinucléotides CpG de SEQ ID NO: 5.

**4.** Procédé selon l'une quelconque des revendications 1 à 3, dans lequel l'échantillon biologique obtenu à partir dudit sujet est choisi dans le groupe comprenant des cellules ou des lignées cellulaires, des lames histologiques, des biopsies, des tissus enrobés de paraffine, des fluides corporels, des éjaculats, de l'urine, du plasma sanguin, du sérum sanguin, du sang total, des cellules sanguines isolées, du crachat et de la matière biologique dérivée d'une bronchoscopie, notamment mais sans que cela soit limitatif un lavage des bronches, un lavage broncho-alvéolaire, un brossage bronchique, une abrasion des bronches et leurs combinaisons.

**5.** Procédé pour la détection d'un cancer du poumon, du sein ou de la vessie selon la revendication 1, comprenant:

a) l'extraction ou l'isolement d'une autre manière d'ADN génomique à partir d'un échantillon biologique obtenu à partir d'un sujet,

b) la digestion de l'ADN génomique de a), ou d'un fragment de celui-ci, avec une ou plusieurs enzymes de restriction sensibles à la méthylation,

c) la mise en contact du produit de digestion par l'enzyme de restriction d'ADN de b), avec une enzyme d'amplification et au moins deux amorces appropriées pour l'amplification d'une séquence comprenant au moins un dinucléotide CpG selon SEQ ID NO: 5,

d) la détermination, sur la base d'une présence ou d'une absence d'un produit d'amplification, de l'état ou du niveau de méthylation d'un moins un dinucléotide CpG selon SEQ ID NO: 5.

**6.** Utilisation d'un acide nucléique comprenant au moins 16 nucléotides contigus d'une séquence d'ADN génomique traitée, choisie dans le groupe consistant en SEQ ID NOs: 21, 22, 45, 46 et des séquences complémentaires de celles-ci, pour la détection d'un cancer du poumon, du sein ou de la vessie par un procédé selon l'une quelconque des revendications 1 à 5.

**7.** Utilisation selon la revendication 6, dans laquelle ledit acide nucléique comprend au moins 50 nucléotides contigus.

**8.** Utilisation d'un kit pour la mise en oeuvre d'un procédé selon l'une quelconque des revendications 1 à 4, le kit comprenant (a) un réactif au bisulfite; (b) un récipient convenant pour contenir ledit réactif au bisulfite et l'échantillon biologique du patient; (c) au moins un ensemble d'oligonucléotides contenant deux oligonucléotides dont les séquences dans chaque cas sont identiques, sont complémentaires, ou hybrident dans des conditions stringentes ou fortement stringentes à un segment ayant une longueur de 9 ou plus, de préférence 18 bases d'une séquence choisie parmi SEQ ID NOs: 21, 22, 45 et 46.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006060653 A
- WO 2006008128 A
- US 5786146 A
- WO 0026401 A1
- WO 9500669 A
- US 6251594 B
- WO 9928498 A, Olek
- WO 9746705 A
- WO 9515373 A
- EP 2004011715 W
- US 6265171 B, Herman
- JP 6331393 B
- US 6331393 B
- US 5514758 A
- US 5565552 A
- US 5567810 A
- US 5574142 A
- US 5585481 A
- US 5587371 A
- US 5597696 A
- US 5958773 A
- US 20030013091 A
- US 09898743 B


### Non-patent literature cited in the description


- **J.P. EGAN.** Signal Detection Theory and ROC Analysis. Academic Press, 1975
- **GONZALGO et al.** *cell proliferative disorders, preferably those according to Cancer Research,* 1997, vol. 57, 594-599
- **EADS et al.** *cell proliferative disorders, preferably those according to Cancer Res.,* 1999, vol. 59, 2302-2306
- **GONZALGO ; JONES.** *Nucleic Acids Res.,* 1997, vol. 25, 2529-2531
- **HERMAN et al.** *Proc. Natl. Acad. Sci. USA,* 1996, vol. 93, 9821-9826
- **XIONG ; LAIRD.** *Nucleic Acids Res.,* 1997, vol. 25, 2532-2534
- **TOYOTA et al.** *cell proliferative disorders, preferably those according to Cancer Res.,* 1999, vol. 59, 2307-12
- **SAMBROOK et al.** Molecular Cloning, A Laboratory Manual. Cold Spring Harbor Press, 1989
- Current Protocols in Molecular Biology. John Wiley & Sons, 1995
- **OLEK A et al.** A modified and improved method for bisulfite based cytosine methylation analysis. *Nucleic Acids Res.,* 1996, vol. 24, 5064-6
- **REIN, T. et al.** *Nucleic Acids Res.,* 1998, vol. 26, 2255
- **ZESCHNIGK M et al.** *Eur J Hum Genet.,* 1997, vol. 5, 94-98
- **OLEK ; WALTER.** *Nat Genet.,* 1997, vol. 17, 275-6
- **GONZALGO ; JONES.** *Nucleic Acids Res.,* 1997, vol. 25, 2529-31
- **XIONG ; LAIRD.** *Nucleic Acids Res.,* 1997, vol. 25, 2532-4
- **GRIGG ; CLARK.** *Bioessays,* 1994, vol. 16, 431-6
- **ZESCHNIGK M et al.** *Hum Mol Genet.,* 1997, vol. 6, 387-95
- **FEIL R et al.** *Nucleic Acids Res.,* 1994, vol. 22, 695
- **MARTIN V et al.** *Gene,* 1995, vol. 157, 261-4
- **FROMMER et al.** *Proc. Natl. Acad. Sci. USA,* 1992, vol. 89, 1827-1831
- **SADRI ; HORNSBY.** *Nucl. Acids Res.,* 1996, vol. 24, 5058-5059
- **EADS et al.** *Cancer Res.,* 1999, vol. 59, 2302-2306
- **BELYAVSKY et al.** *Nucl Acid Res,* 1989, vol. 17, 2919-2932
- **KRUG ; BERGER.** Methods in Enzymology. Academic Press, 1987, vol. 152, 316-325
- **SAMBROOK et al.** Molecular Cloning: A Laboratory Manual. 1989
- Basic and Clinical Immunology. Appleton & Lange, 1991, 217-262
- **MILSTEIN ; KOHLER.** *Nature,* 1975, vol. 256, 495-497
- **GULFRE ; MILSTEIN.** Methods in Enzymology: Immunochemical Techniques. Academic Press, 1981, vol. 73, 1-46
- **YU et al.** *BioTechniques,* 1997, vol. 23, 714-720
- **KARAS ; HILLENKAMP.** *Anal Chem.,* 1988, vol. 60, 2299-301

- **GUT ; BECK.** *Current Innovations and Future Trends,* 1995, vol. 1, 147-57
- **GUT ; BECK.** *Nucleic Acids Res.,* 1995, vol. 23, 1367-73
- **HEID et al.** *Genome Res.,* 1996, vol. 6, 986-994
- **SANGER F. et al.** *Proc Natl Acad Sci USA,* 1977, vol. 74, 5463-5467
- **KROL et al.** *BioTechniques,* 1998, vol. 6, 958-976
- **ZON.** *Pharm. Res.,* 1988, vol. 5, 539-549
- *Nature Genetics,* January 1999, vol. 21
- Qiagen Genomic DNA Handbook. August 2001, 28-3144-47
- **EADS et al.** *Cancer Res.,* 15 April 2001, vol. 61 (8), 3410-8